# EUROPEAN PATENT APPLICATION

(11) **EP 2 540 310 A1**
(43) Date of publication of application: **02.01.2013**
(21) Application number: 12173833.0
(22) Date of filing: 17.05.2007
(51) Int. Cl.: A61K 38/18, A61P 19/02, A61P 19/04

(54) **Methods of treating cartilage defects using a soluble morphogenic protein complex**

(30) Priority: 17.05.2006 US 801551 P
(62) Divisional of application: 07795187.9
(71) Applicant: Stryker Corporation, Kalamazoo, MI 49002 (US)
(72) Inventor: Engelman, Donald, Guilford, CT 06437 (US)
(74) Representative: Vossius & Partner

(57) **Abstract**

The present invention provides methods of repairing and regenerating cartilage tissue using a soluble morphogenic protein complex comprising (a) a morphogenic protein; and (b) a morphogenic protein pro region isolated from a morphogenic protein, or a conservative substitution variant or a fragment of said pro region, wherein said pro region or variant or fragment is noncovalently linked to the morphogenic protein, and wherein said complex is more soluble in an aqueous solvent than said morphogenic protein alone.

## Description

### FIELD OF THE INVENTION

The present invention relates to orthopaedic tissue repair. More particularly, it relates to methods of repairing or regenerating cartilage.

### BACKGROUND OF THE INVENTION

Cartilage repair and regeneration is one of the major obstacles in current orthopaedics. The importance is enormous because cartilage injury and degenerative disorders such as osteoarthritis, intervertebral disc degeneration and meniscal tears are a major cause of disability among the adult population in the United States.

Cartilage is connective tissue composed of chondrocytes embedded in an extracellular matrix of collagen fibers, proteoglycans, and other non-collagenous proteins. There are two forms of cartilage - articular and non-articular. Articular cartilage is a thin layer of connective tissue, which covers the ends of bones in joints. Non-articular cartilage includes fibrocartilage and elastic cartilage and includes intervertebral discs, meniscus, trachea, larynx, nose, ear and ribs.

The function of cartilage is to cushion load bearing, resist wear, and allow for almost frictionless movement of joints. Defects in cartilage tissue, often caused by trauma, abnormal wear or disease, can lead to pain and stiffness, and if left untreated, may progress and ultimately require replacement of the entire joint. For example, articular cartilage defects often lead to early degradation of the articular surface and may eventually result in osteochondral defects, osteoarthritis or both.

Osteoarthritis is considered a process of attempted, but gradually failing, repair of damaged cartilage extracellular matrix, as the balance between synthesis and breakdown of matrix components is disturbed and shifted toward catabolism.

The ability of cartilage tissue to regenerate on its own is severely limited due to its avascular nature. Repair of osteochondral defects, which involves both the cartilage tissue and the underlying bone, occurs to a limited extent promoted by the presence of both stem cells and growth and differentiation factors brought into the defect by the blood and/or marrow. In animal studies, these defects undergo some repair with formation of a new layer of bone and cartilage, but the macrornolecular organization and the biochemical characteristics of the cartilage matrix are imperfect. Type I collagen, rather than Type II collagen, and proteoglycans that are not cartilage specific, such as dermatan sulfate containing proteoglycans, make up the repair tissue and result in fibrillations and degenerative changes over time. And, repair of cartilage defects that do not penetrate into the subchondral bone does not occur, even to a limited extent.

Moreover, surgical treatment of cartilage defects is complex and has been demonstrated to have only limited success. For example, articular cartilage defects are treated with an arthroscopic approach where loose bodies are debrided and transition areas are smoothed. However, this method alone frequently does not provide long lasting relief of the symptoms. Knee replacements often require resecting significant amounts of bone and often require multiple surgeries.

The meniscus is a small horseshoe shaped tissue located between the bone ends inside the knee joint, which acts as a shock absorber. There are two menisci in each knee on either side of the knee. They are usually strong in young people and with age become more brittle and tear more easily. Tears are extremely common with anterior cruciate ligament (ACL) injuries. Meniscal fibrocartilage, like articular hyaline cartilage, has a limited capacity to heal, particularly in the middle and inner avascular regions. The current treatment for small tears is to leave them alone if they do not cause much trouble. Surgical options for treating meniscal tears depend on a number of factors including the nature and extent of the injury and most importantly, its location. Tears in the vascularized region, which is integrated with the highly vascularized synovium have been successfully repaired by suturing. Partial or total meniscectomy is the normal surgical treatment for symptomatic tears within the avascular two thirds of the meniscus. Tears in the latter meniscus regions are the most common types seen clinically. Irrespective of whether open, arthroscopic, total or partial meniscectomy are employed, osteoarthritis is a frequent sequela in these patients within a few years post surgery. Therefore, the common form of repair is to only partially remove the torn bits and to repair the cartilage by stapling it. Unfortunately, the healing process following this procedure is slow. Moreover, if the repair is not successful, then the entire torn meniscus must subsequently be removed.

The major cause of persistent and often debilitating back pain is intervertebral disc (IVD) degeneration. As discs degenerate, they cause the adjoining vertebrae to become compressed, often resulting in severe pain.

The IVD as a syndesmosis provides articulation between adjoining vertebral bodies and acts as a weight bearing cushion which dissipates axially applied spinal loads. These biomechanical functions are made possible by the unique structure of the IVD which is composed of an outer collagen-rich annulus fibrosus surrounding a central hydrated proteoglycan rich gelatinous nucleus pulposus. Superior and inferior cartilaginous endplates, thin layers of hyaline-like cartilage covers the interfaces of the vertebral bodies within the disc.

Lumbar disc degeneration represents a substantial social and economic burden to the community which is manifest principally as low back pain (LBP). It is estimated that as much as 80% of the population experience at least one significant episode of LBP during life, and approximately 2.5% of the working population will take some sick leave during the year as a result of LBP. The direct costs of LBP in modem Western countries has been estimated at $9 billion, most of which is spent on consulting general practitioners, physical therapists and other conservative practitioners (Williams DA et al., (1998) Health care and indemnity costs across the natural history of disability in occupational low back pain, Spine 23:2329-36). Total indirect expenditure, including surgical management may be ten times higher (Maetzel and Li (2002) The economic burden of low back pain: a review of studies published between 1996 and 2001, Best Prac Res Clin Rheumatol 16:23-30; Walker et al., (2003) The economic burden, Proceedings of the Spine Society of Australia Annual Scientific Meeting, Canberra, Australia).

Disc degeneration is a natural phenomenon that occurs, in most instances, from the time of skeletal maturity (Vemon-Roberts (1992) Age-related and degenerative pathology of intervertebral discs and apophyseal joints, In: The lumbar spine and back pain. Fourth edition, Jayson MIV, Ed. Churchill Livingstone, Edinburgh, Chapter 2, 17-41). It is consistent with advancing age but in many cases is also associated with pain, particularly in the lumbar spine, and restricted mobility. Symptoms of LBP often resolve spontaneously over time as patients modify their lifestyles to accommodate restricted mobility. In many cases however, it remains a significant factor that requires surgical intervention. The traditional "gold standard" surgical treatment for chronic LBP has been spinal fusion to immobilize the one or more painful level. Fusion is expensive because it requires prolonged hospitalization and specialist surgical expertise, and although most of these patients will experience short-term pain relief there is evidence now that fusion does not provide the best outcome. Long-term studies suggest that spinal fusion actually promotes degeneration at levels adjacent to the fusion site (Lee (1988) Accelerated degeneration of the segment adjacent to a lumbar fusion, Spine 13:375-7). In the same way that artificial prostheses were developed 50 years ago to restore function to arthritic and fractured hips and knees, prostheses are now being developed with the aim of restoring full mechanical function to discs that have become painful and arthritic due to chronic degeneration (Szpaalski et al (2002) V Spine arthroplasty: a historical review, Eur Spine J 11:S65-S84). It is however too early to know if any of the myriad models undergoing trials will provide long-term benefit.

A class of proteins known as "osteogenic proteins" or "morphogenic proteins" or "morphogens," are competent to act as true bone and cartilage tissue morphogens, able, on their own, to induce the proliferation and differentiation of progenitor cells into functional bone, cartilage, tendon, and/or ligamentous tissue. These proteins, include members of the family of bone morphogenetic proteins (BMPs) which were initially identified by their ability to induce ectopic, endochondral bone morphogenesis. The osteogenic proteins generally are classified in the art as a subgroup of the TGF-β superfamily of growth factors (Hogan (1996) Genes & Development 10:1580-1594). Members of the morphogen family of proteins include the mammalian osteogenic protein-1 (OP-1, also known as BMP-7, and the *Drosophilia* homolog 60A), osteogenic protein-2 (OP-2, also known as BMP-8a), osteogenic protein-3 (OP-3, also known as BMP-8b), BMP-2 (also known as BMP-2A or CBMP-2A, and the *Drosophila* homolog DPP), BMP-3, BMP-4 (also known as BMP-2B or CBMP-2B), BMP-5, BMP-6 and its murine homolog Vgr-1, BMP-9, BMP-10, BMP-11, BMP-12, GDF3 (also known as Vgr2), GDF8, GDF9, GDF10, GDF11, GDF12, BMP-13, BMP-14, BMP-15, BMP-16, BMP-17, BMP-18, GDF-5 (also known as CDMP-1 or MP52), GDF-6 (also known as CDMP-2), GDF-7 (also known as CDMP-3), the *Xenopus* homolog Vg1, NODAL, UNIVIN, SCREW, ADMP, and NEURAL. Members of this family encode secreted polypeptide chains sharing common structural features, including processing from a precursor "pro-form" to yield a mature polypeptide chain competent to dimerize, and containing a carboxy terminal active domain of approximately 97-106 amino acids. All members share a conserved pattern of cysteines in this domain and the active form of these proteins can be either a disulfide-bonded homodimer of a single family member, or a heterodimer of two different members (see, e.g., Massague (1990) Annu. Rev. Cell Biol. 6:597; Sampath, et al. (1990) J. Biol. Chem. 265:13198). See also, U.S. 5,011,691; U.S. 5,266,683, Ozkaynak et al. (1990) EMBO J. 9: 2085-2093, Wharton et al. (1991) PNAS 88:9214-9218, Ozkaynak (1992) J. Biol. Chem. 267:25220-25227 and U.S. 5,266,683; Celeste et al. (1991) PNAS 87:9843-9847; Lyons et al. (1989) PNAS 86:4554-4558; Wozney et al. (1988) Science 242:1528-1534; WO93/00432; Padgett et al. (1987) Nature 325:81-84; Weeks (1987) Cell 51:861-867.

In their mature dimeric forms, the morphogenic proteins typically are fairly insoluble under physiological conditions. It has been discovered that morphogenic proteins secreted into cultured medium from mammalian cell expression systems contain as a significant fraction of the secreted protein a soluble form of the protein. This soluble form, also referred to as "soluble morphogenic protein complex" comprises the mature dimeric species, including truncated forms thereof, noncovalently associated with at least one, and preferably two pro domains. See e.g., U.S. 6,395,883.

The currently preferred methods of repairing cartilage defects include debridement, microfracture, autologous cell transplantation, mosaicplasty and joint replacement. However, none of these methods, result in actual repair and replacement of cartilage tissue. These methods result in imperfect repair tissue with scar-like characteristics.

Therefore, there remains a need for compositions and methods for repairing and regenerating cartilage defects which overcome the problems associated with the currently available methods and compositions.

### SUMMARY OF THE INVENTION

The present invention provides methods of repairing and regenerating cartilage tissue using a soluble morphogenic protein complex. In some embodiments, the present invention provides a method of repairing a cartilage defect in a patient comprising the step of administering into the cartilage or into the area surrounding the cartilage a composition comprising a therapeutically effective amount of an isolated soluble morphogenic protein complex comprising: (a) a morphogenic protein; and (b) a morphogenic protein pro region isolated from a morphogenic protein, or a conservative substitution variant or a fragment of said pro region, wherein said pro region or variant or fragment is noncovalently linked to the morphogenic protein, and wherein said complex is more soluble in an aqueous solvent than said morphogenic protein alone.

In some embodiments, the invention provides a method of regenerating or producing cartilage in a patient comprising the step of administering into the cartilage or the area surrounding the cartilage a composition comprising a therapeutically effective amount of an isolated soluble morphogenic protein complex comprising: (a) a morphogenic protein; and (b) a morphogenic protein pro region isolated from a morphogenic protein, or a conservative substitution variant or a fragment of said pro region, wherein said pro region or variant or fragment is noncovalently linked to the morphogenic protein, and wherein said complex is more soluble in an aqueous solvent than said morphogenic protein alone.

In other embodiments, the invention provides a method of promoting cartilage growth or accelerating cartilage formation in a patient comprising the step of administering into the cartilage or into the area surrounding the cartilage a composition comprising a therapeutically effective amount of an isolated soluble morphogenic protein complex comprising: (a) a morphogenic protein; and (b) a morphogenic protein pro region isolated from a morphogenic protein, or a conservative substitution variant or a fragment of said pro region, wherein said pro region or variant or fragment is nancovalently linked to the morphogenic protein, and wherein said complex is more soluble in an aqueous solvent than said morphogenic protein alone.

In yet other embodiments, the invention provides a method of preventing cartilage degradation or treating cartilage injury or degenerative disease or disorder in a patient comprising the step of administering into the cartilage or into the area surrounding the cartilage a composition comprising a therapeutically effective amount of an isolated soluble morphogenic protein complex comprising: (a) a morphogenic protein; and (b) a morphogenic protein pro region isolated from a morphogenic protein, or a conservative substitution variant or a fragment of said pro region, wherein said pro region or variant or fragment is noncovalently linked to the morphogenic protein, and wherein said complex is more soluble in an aqueous solvent than said morphogenic protein alone.

The present invention also provides a method of treating cartilage tissue injury in a patient comprising the step of administering to the patient a composition comprising a therapeutically effective amount of an isolated soluble morphogenic protein complex comprising: (a) a morphogenic protein; and (b) a morphogenic protein pro region isolated from a morphogenic protein, or a conservative substitution variant or a fragment of said pro region, wherein said pro region or variant or fragment is noncovalently linked to the morphogenic protein, and wherein said complex is more soluble in an aqueous solvent than said morphogenic protein alone. In some embodiments the tissue injury includes but is not limited to meniscus tears, chondral voids or defects, osteochondral voids or defects or ACL injury.

The present invention also provides a method of treating cartilage tissue degenerative disease or disorder in a patient comprising the step of administering to the patient a composition comprising a therapeutically effective amount of an isolated soluble morphogenic protein complex comprising: (a) a morphogenic protein; and (b) a morphogenic protein pro region isolated from a morphogenic protein, or a conservative substitution variant or a fragment of said pro region, wherein said pro region or variant or fragment is noncovalently linked to the morphogenic protein, and wherein said complex is more soluble in an aqueous solvent than said morphogenic protein alone. In some embodiments, the cartilage tissue degenerative disease or disorder includes but is not limited to osteoarthritis and disc degeneration. In some embodiments, the composition of this invention is administered into the asteoarthritic or disc degeneration defect site or into the area surrounding the osteoarthritic or disc degeneration defect site.

In some embodiments, the cartilage is articular cartilage. In some embodiments, the articular cartilage is within an articular joint. In other embodiments, the cartilage is non-articular cartilage. In some embodiments, the non-articular cartilage is a meniscus or an intervertebral disc.

In some embodiments, the composition is administered into the cartilage. In some embodiments, the composition is administered into a meniscus or an intervertebral disc. In some embodiments, the composition is administered into the areas surrounding the cartilage. In some embodiments, the area surrounding the cartilage is synovial fluid.

In some embodiments, the morphogenic protein in the composition used in the methods of this invention includes but is not limited to OP-1, OP-2, OP-3, BMP-2, BMP-3, BMP-4, BMP-5, BMP-6, BMP-8, BMP-9, BMP-10, BMP-11, BMP-12, BMP-13, BMP-15, BMP-16, BMP-17, BMP-18, DPP, Vg1, Vgr, 60A protein, GDF-1, GDF-2, GDF-3, GDF-5, GDF-6, GDF-7, GDF-8, GDF-9, GDF-10, GDF-11, GDF-12, CDMP-1, CDMP-2, CDMP-3, NODAL, UNIVIN, SCREW, ADMP, NEURAL, and conservative substitution variants and fragments thereof. In a preferred embodiment, the morphogenic protein is selected from OP-1, BMP-5, BMP-6, GDF-5, GDF-6, GDF-7, CDMP-1, CDMP-2, CDMP-3, BMP-12, and BMP-13. In a more preferred embodiment, the morphogenic protein is OP-1. In other embodiments, the morphogenic protein comprises an amino acid sequence having at least 70% homology with the C-terminal 102-106 amino acids, including the conserved seven cysteine domain, of human OP-1, said morphogenic protein being capable of inducing repair of the cartilage defect. In some embodiments, the morphogenic protein in the composition used in the methods of this invention is a dimer. In some embodiments, the morphogenic protein in the composition of this invention is a non-covalent dimer, including, for example, monomers in which the cysteine residue responsible for interchain disulfide bond formation has been replaced with another amino acid residue.

In some embodiments, the morphogenic protein pro region comprises a pro region amino acid sequence of OP-1, OP-2, OP-3, BMP-2, BMP-3, BMP-4, BMP-5, BMP-6, BMP-8, BMP-9, BMP-10, BMP-11, BMP-12, BMP-13, BMP-15, BMP-16, BMP-17, BMP-18, DPP, Vg1, Vgr, 60A protein, GDF-1, GDF-2, GDF-3, GDF-5, GDF-6, GDF-7, GDF-8, GDF-9, GDF-10, GDF-11, GDF-12, CDMP-1, CDMP-2, CDMP-3, NODAL, UNIVIN, SCREW, ADMP, NEURAL, or conservative substitution variants or fragments thereof. In other embodiments, the morphogenic protein pro region comprises a pro region amino acid sequence of OP-1, BMP-5, BMP-6, GDF-5, GDF-6, GDF-7, CDMP-1, CDMP-2, CDMP-3, BMP-12, BMP-13, or conservative substitution variants or fragments thereof. In yet another preferred embodiment the morphogenic protein pro region comprises a pro region amino acid sequence of OP-1 or a conservative substitution variant or fragment thereof.

### BRIEF DESCRIPTION OF THE DRAWINGS

FIGURE 1 is a schematic representation of a morphogen polypeptide chain as expressed from a nucleic acid encoding the sequence, wherein the crosshatched region represents the signal sequence; the stippled region represents the pro domain; the hatched region represents the N-terminus ("N-terminal extension") of the mature protein sequence; and the open region represents the C-terminal region of the mature protein sequence defining the conserved seven cysteine domain, the conserved cysteines being indicated by vertical hatched lines.

FIGURE 2 lists the sequences of the N-terminal extensions of the mature forms of various morphogens OP-2, OP-1, Vgr-1, BMP-5, 60A, DPP, BMP-2, BMP-4, Vg-1, BMP-3, respectively.

FIGURE 3 is a gel filtration column elution profile of a soluble morphogen (OP-1) produced and purified from a mammalian cell culture by IMAC, S-Sepharose and S-200HR chromatography in TBS (Tris-buffered saline), wherein Vₒ is the void volume, ADH is alcohol dehydrogenase (MW 150 kDa), BSA is bovine serum albumin (MW 67 kDa), CA is carbonic anhydrase (MW 29 kDa) and CytC is cytochrome C (MW 12.5 kDa).

FIGURE 4 is a schematic of a joint showing the site of the bilateral impact injuries.

FIGURE 5 depicts the zonal dissection scheme to separate the disc into annulus fibrosus (AF) quadrants and the nucleus pulposus (NP) and the location and extent of the anterolateral annular lesion in quadrant 1 in horizontal and vertical sections through lumbar ovine intervertebral discs.

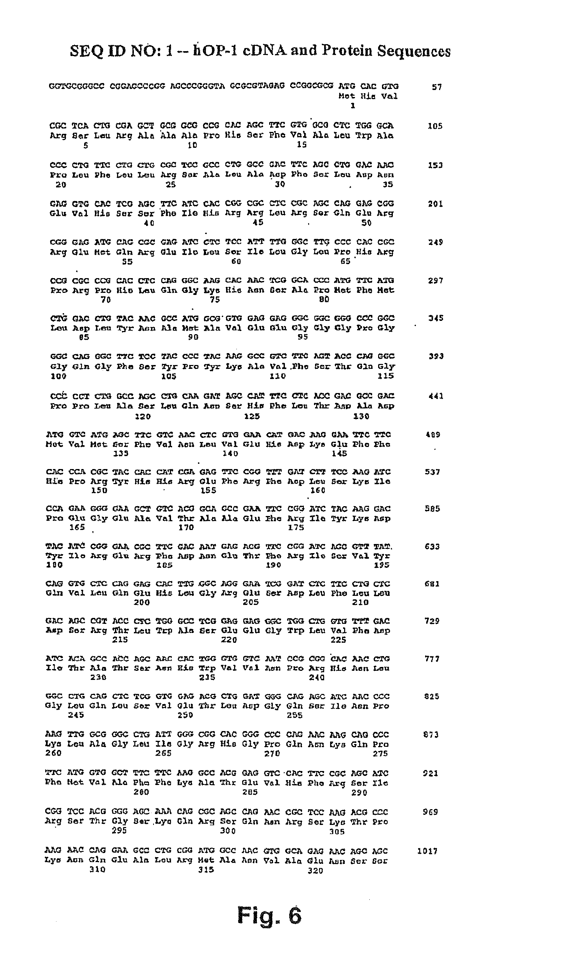

FIGURE 6 depicts human OP-1 cDNA and protein sequences (SEQ ID NO:1).

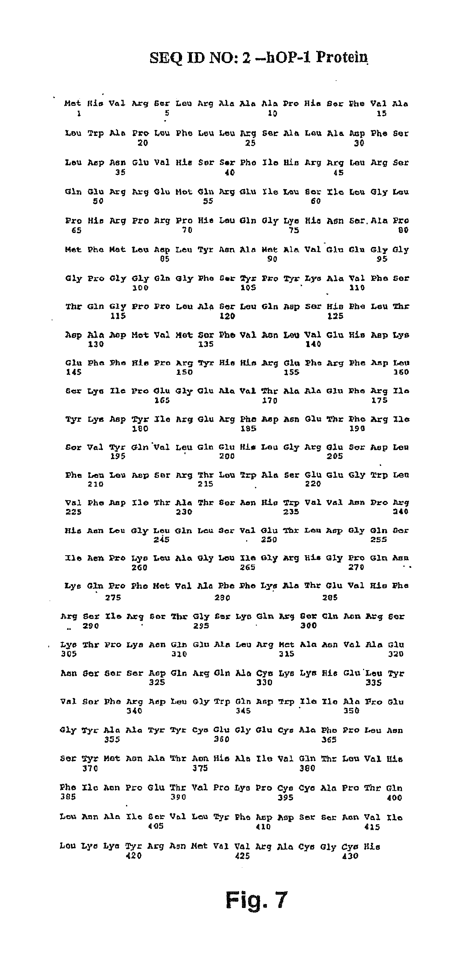

FIGURE 7 depicts a human OP-1 protein sequence (SEQ ID NO:2).

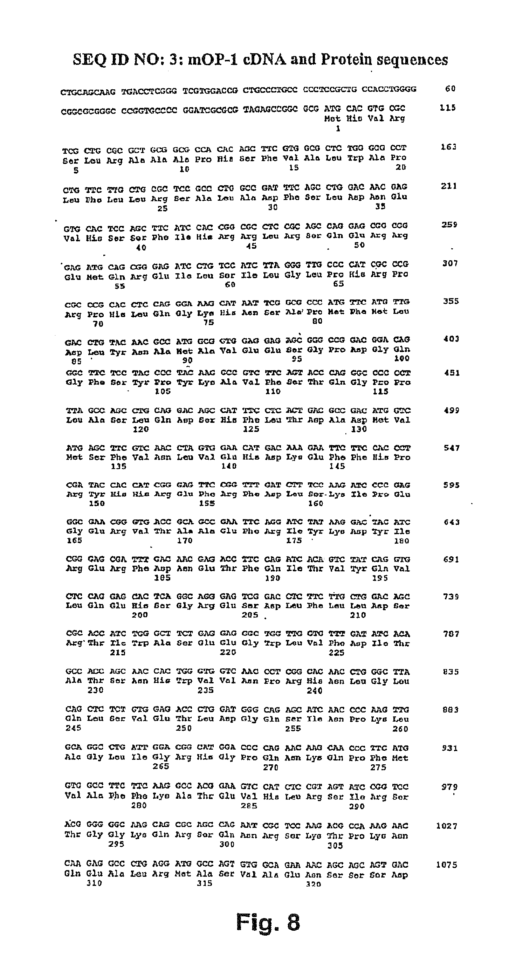

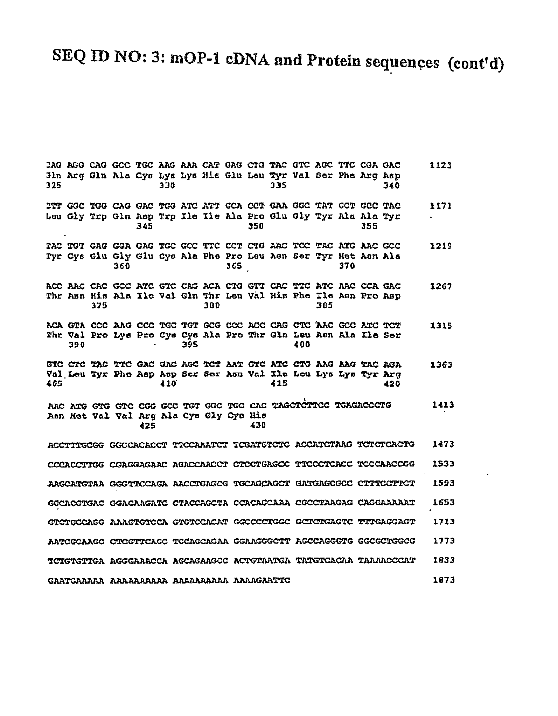

FIGURE 8 depicts murine OP-1 cDNA and protein sequences (SEQ ID NO:3).

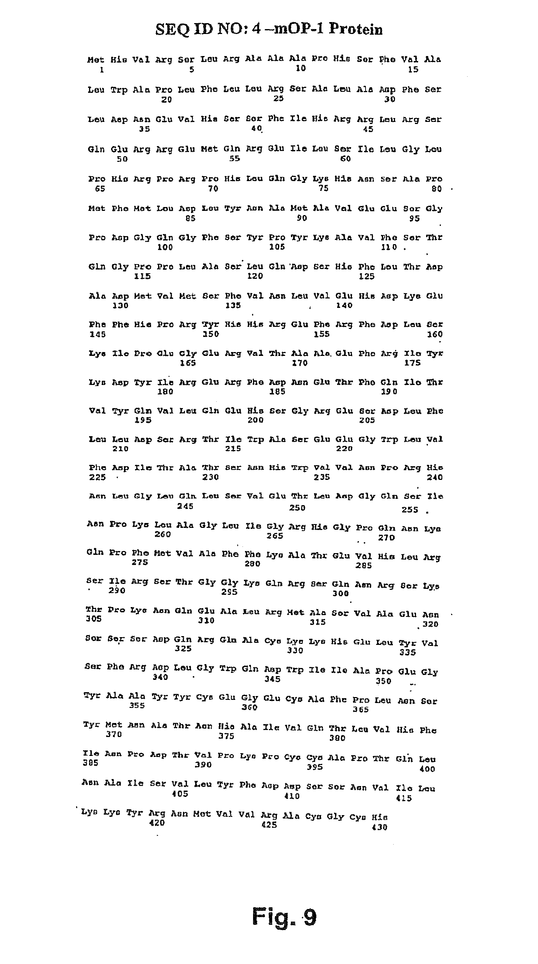

FIGURE 9 depicts a murine protein sequence (SEQ ID NO:4).

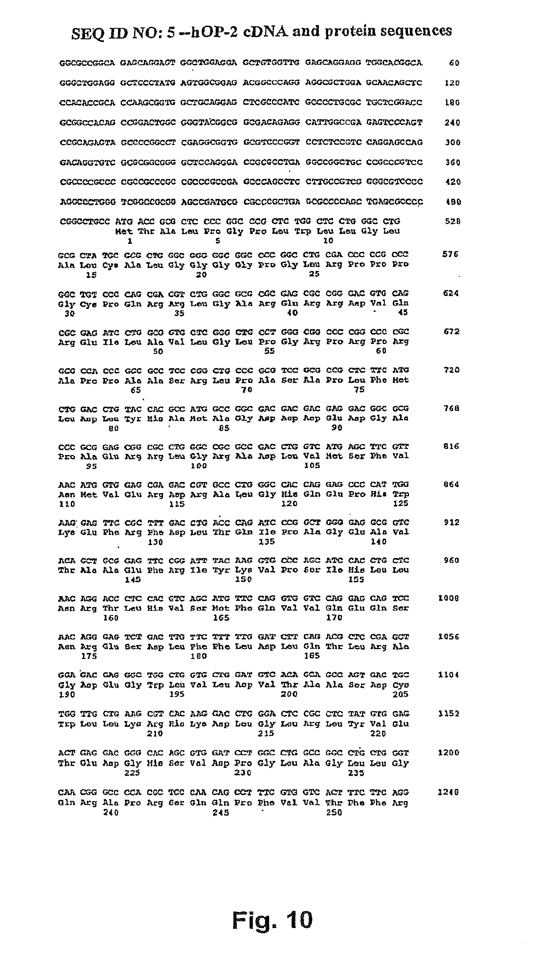

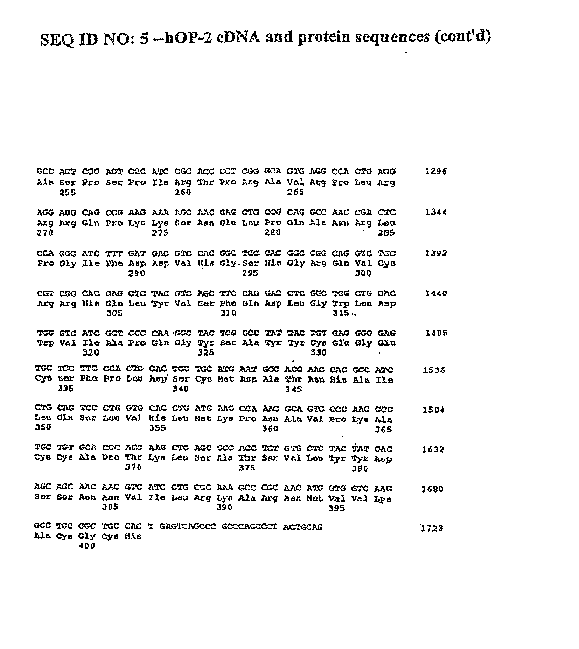

FIGURE 10 depicts human OP-2 cDNA and protein sequences (SEQ ID NO:5).

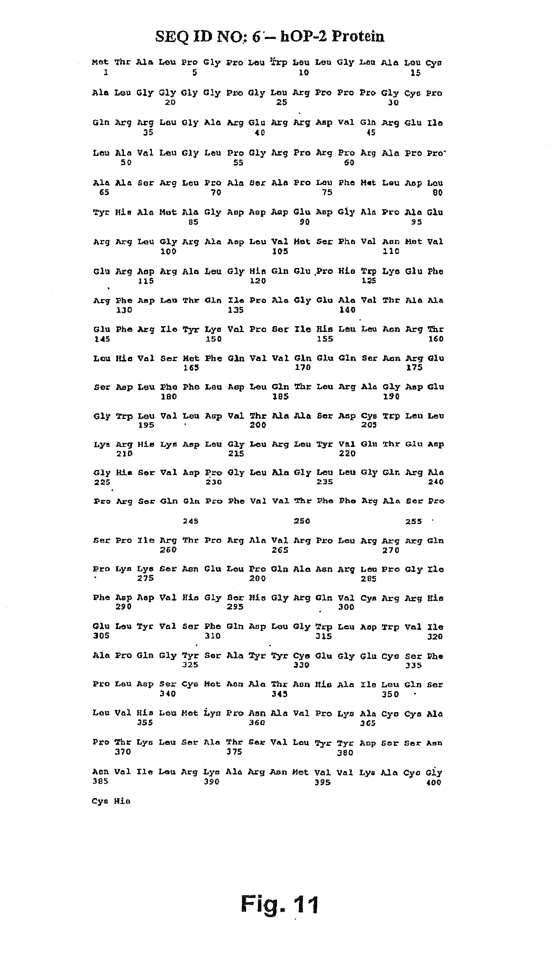

FIGURE 11 depicts a human OP-2 protein sequences (SEQ ID NO:6).

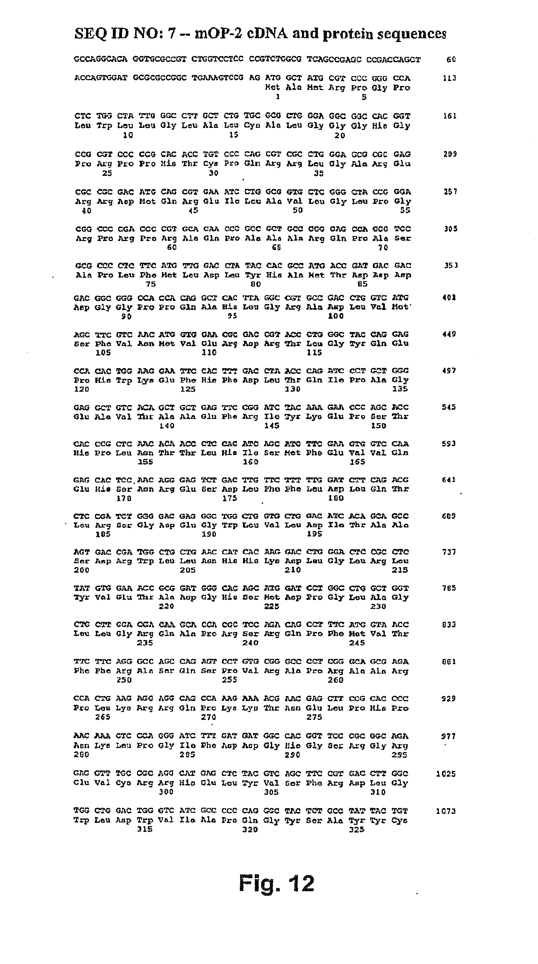

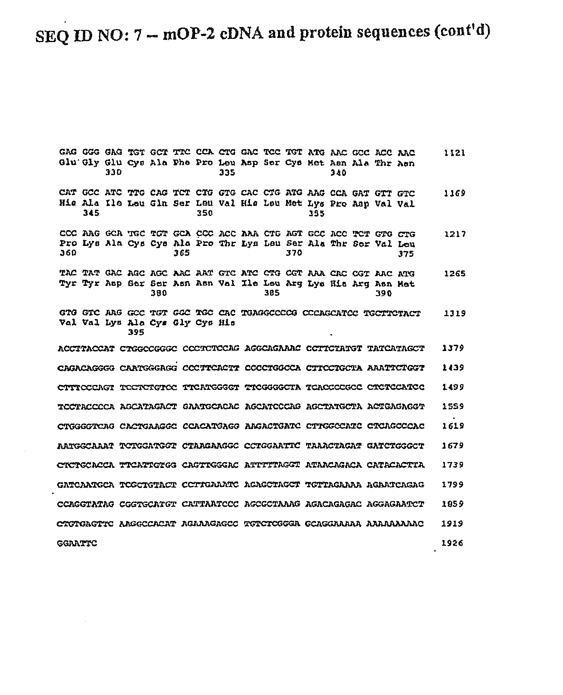

FIGURE 12 depicts murine OP-2 cDNA and protein sequences (SEQ ID NO:7).

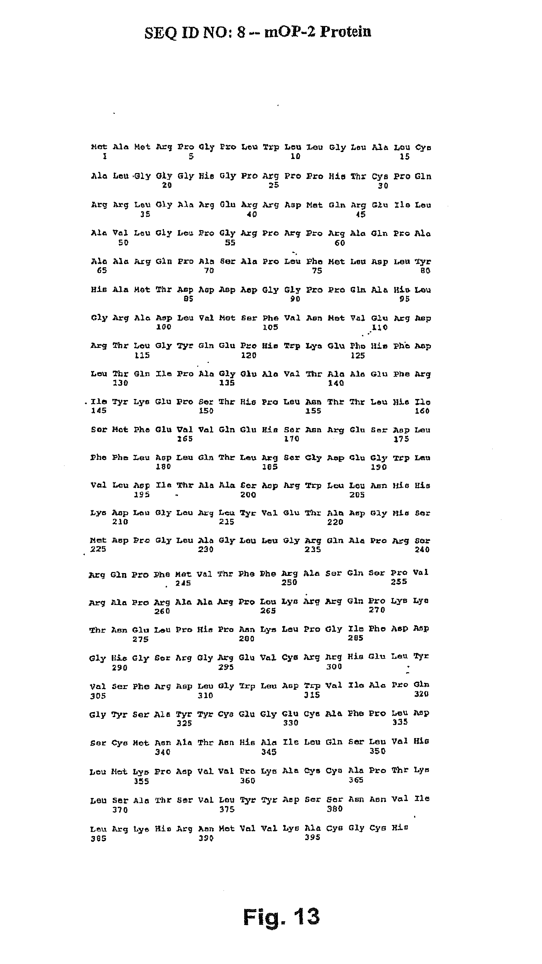

FIGURE 13 depicts a murine OP-2 protein sequence (SEQ ID NO:8).

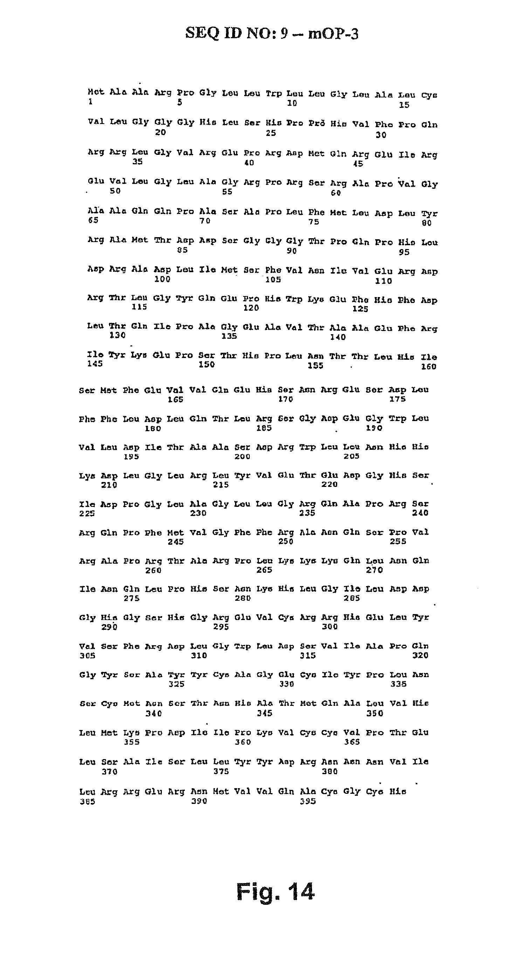

FIGURE 14 depicts a murine OP-3 protein sequence (SEQ ID NO:9).

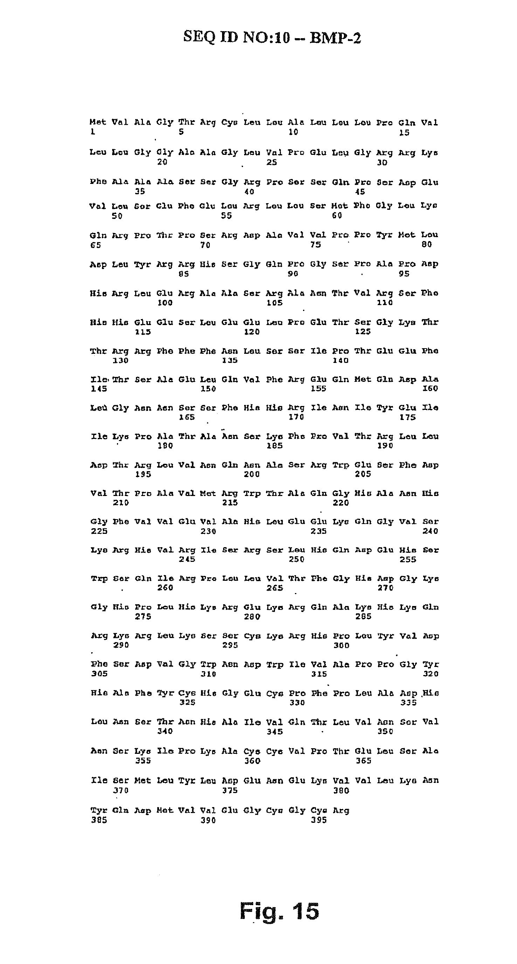

FIGURE 15 depicts a BMP-2 protein sequence (SEQ ID NO:10).

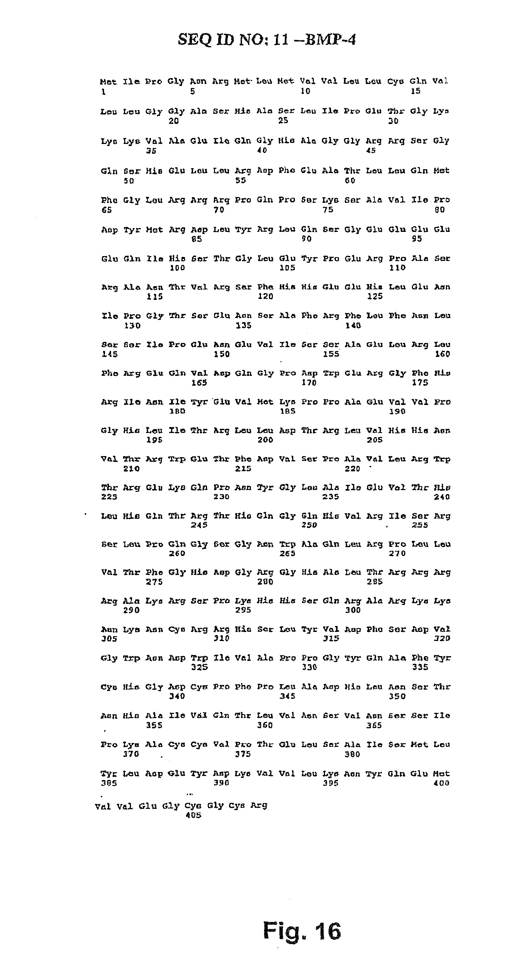

FIGURE 16 depicts a BMP-4 protein sequence (SEQ ID NO:11).

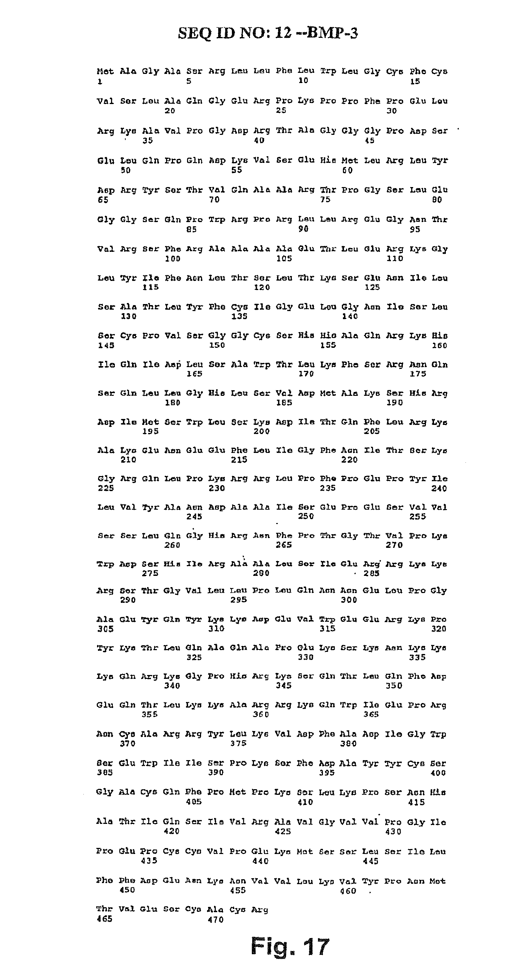

FIGURE 17 depicts a BMP-3 protein sequence (SEQ ID NO: 12).

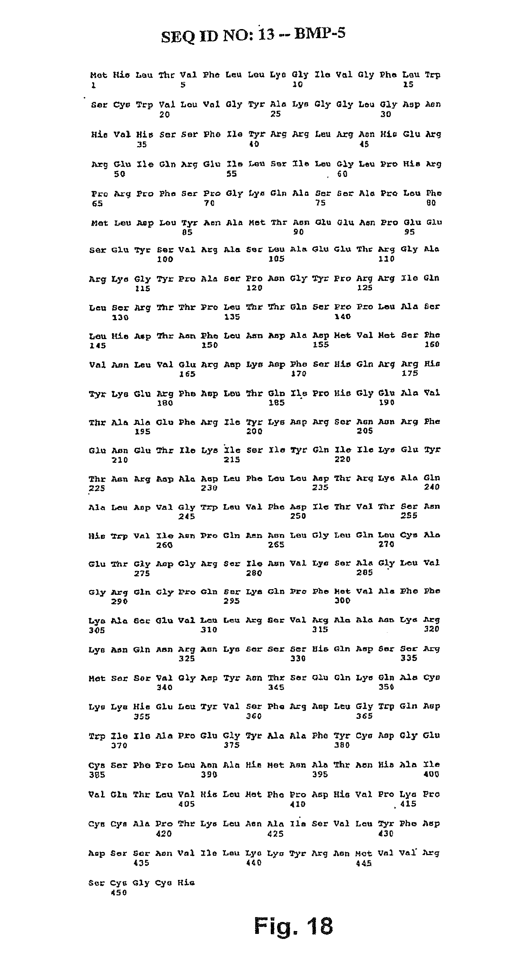

FIGURE 18 depicts a BMP-5 protein sequence (SEQ ID NO: 13).

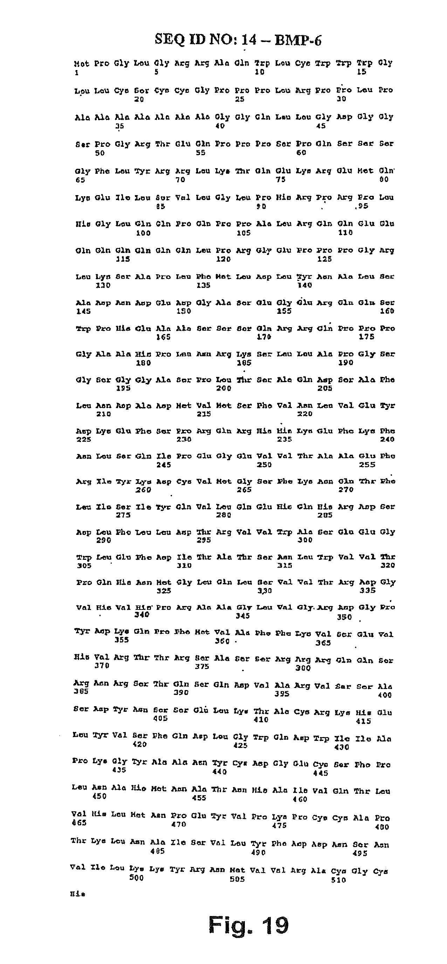

FIGURE 19 depicts a BMP-6 protein sequence (SEQ ID NO:14).

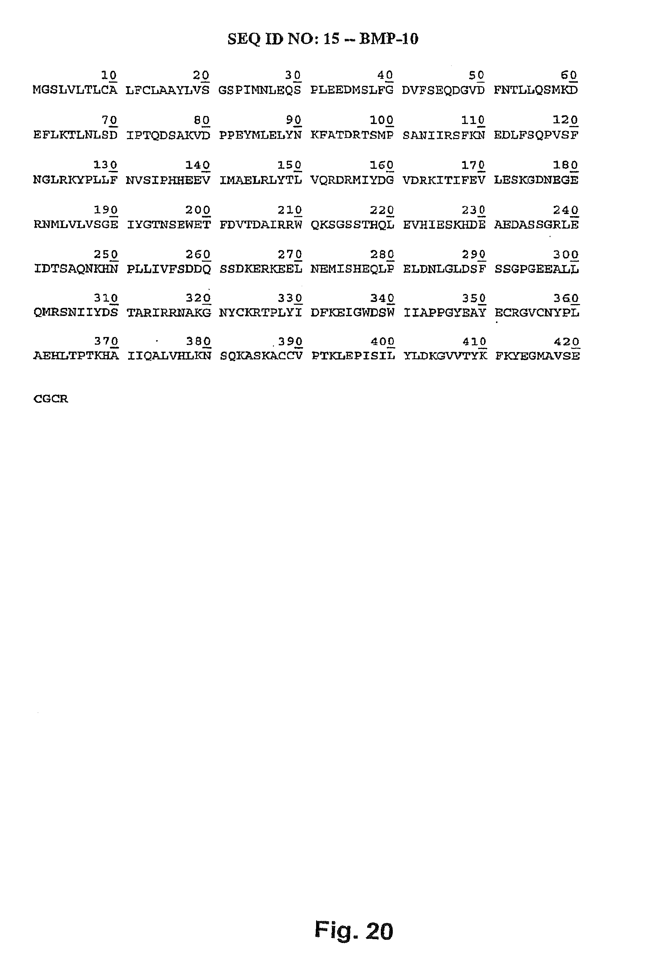

FIGURE 20 depicts a BMP-10 protein sequence (SEQ ID NO:15).

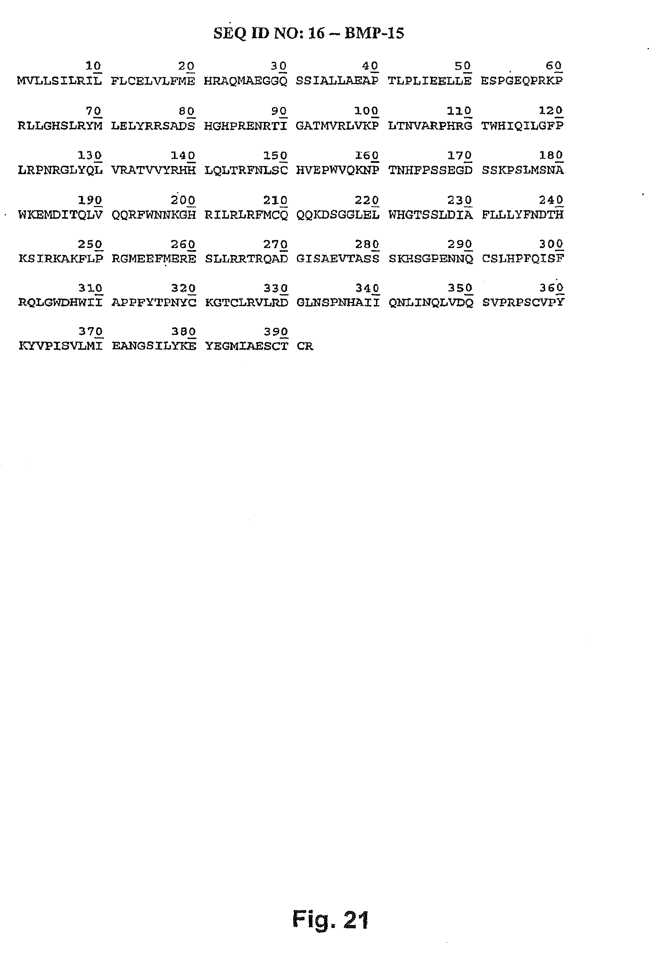

FIGURE 21 depicts a BMP-15 protein sequence (SEQ ID NO:16).

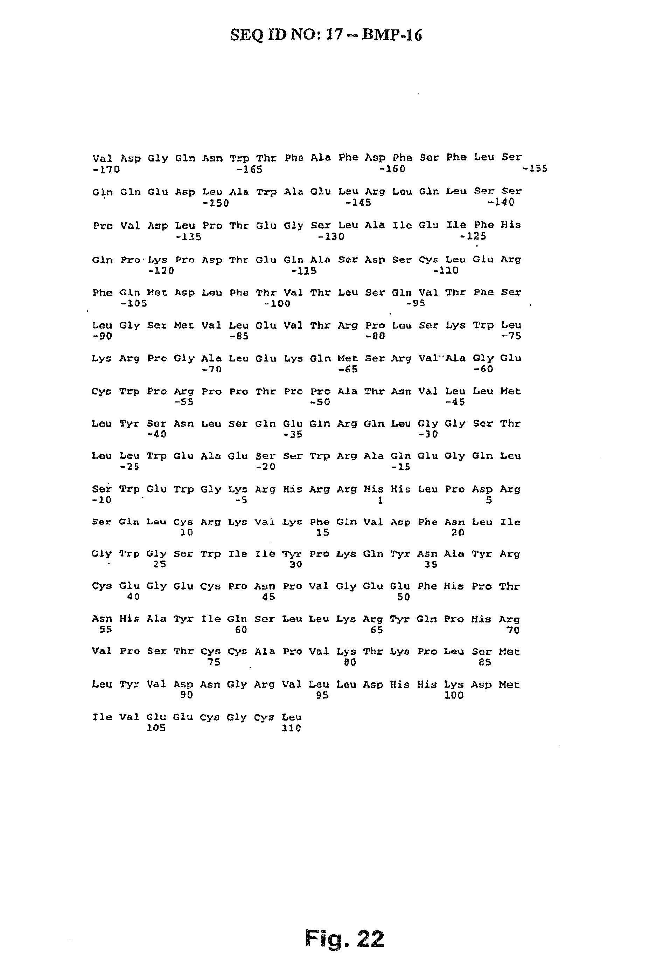

FIGURE 22 depicts a BMP-16 protein sequence (SEQ ID NO: 17).

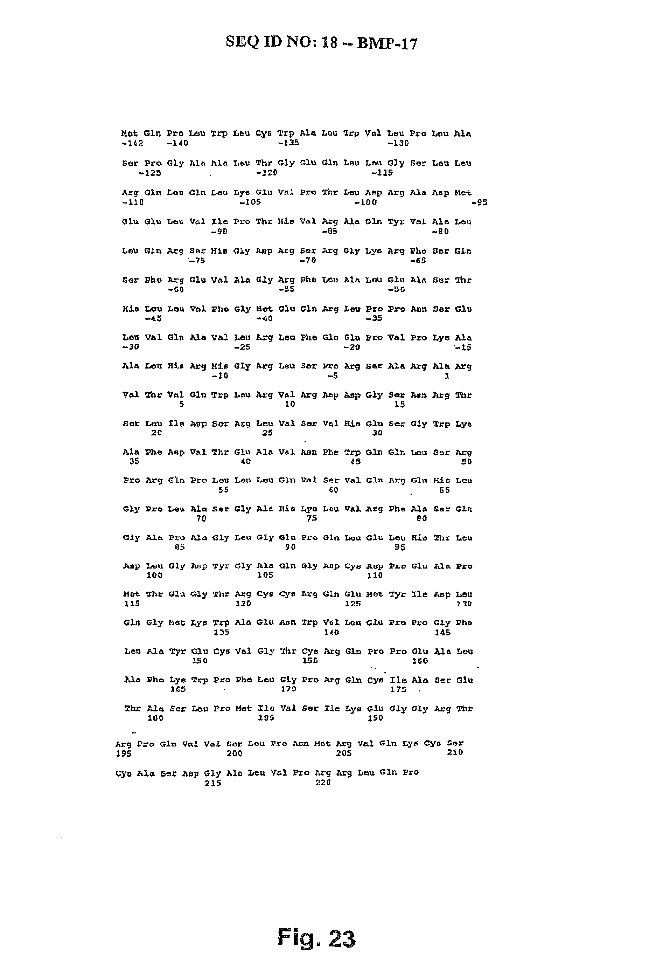

FIGURE 23 depicts a BMP-17 protein sequence (SEQ ID NO:18).

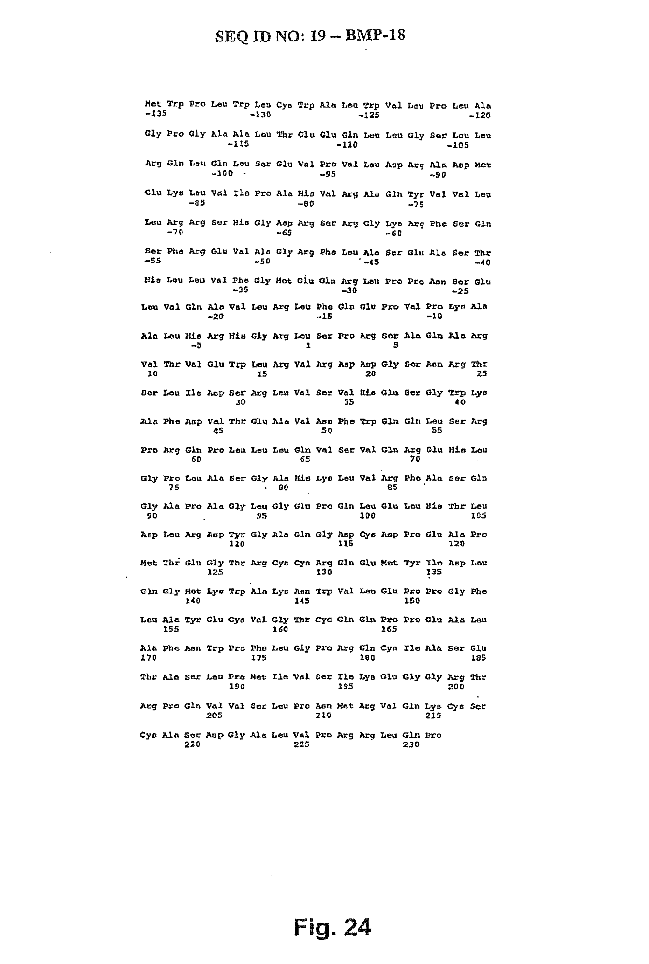

FIGURE 24 depicts a BMP-18 protein sequence (SEQ ID NO: 19).

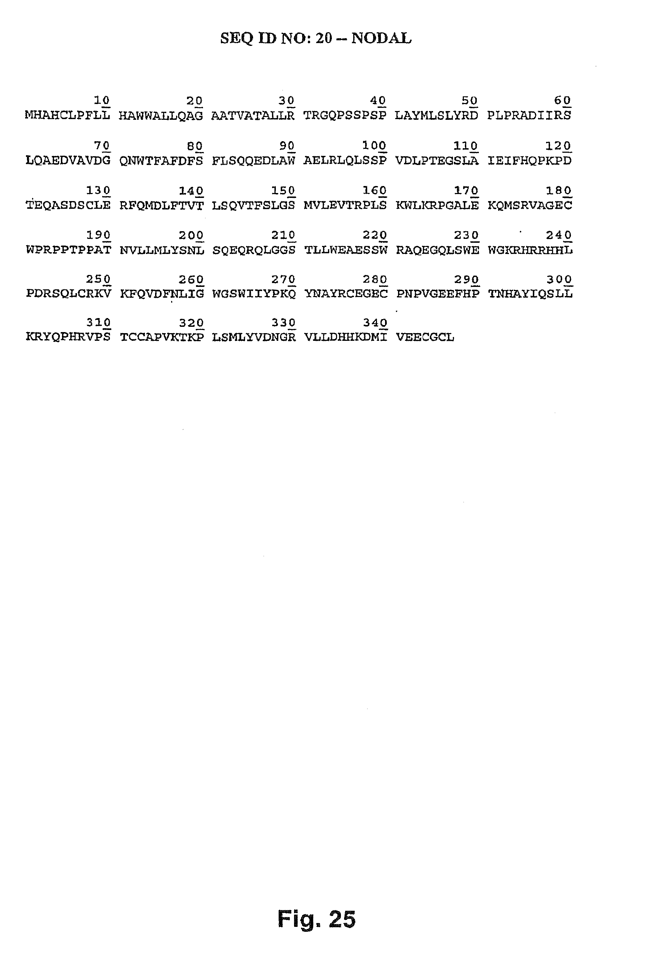

FIGURE 25 depicts a NODAL protein sequence (SEQ ID NO:20).

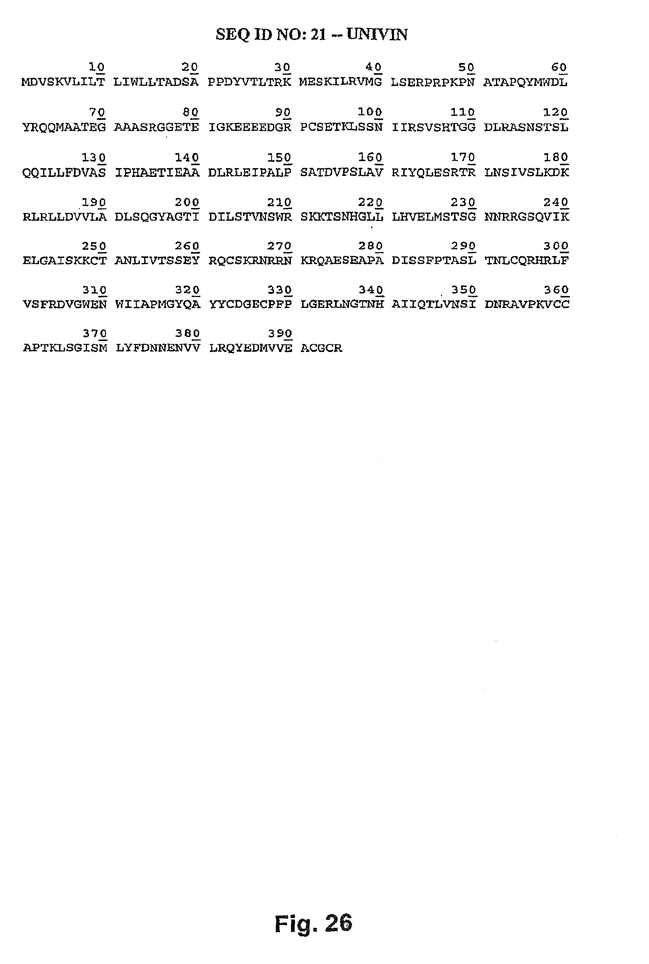

FIGURE 26 depicts a UNIVIN protein sequence (SEQ ID NO:21).

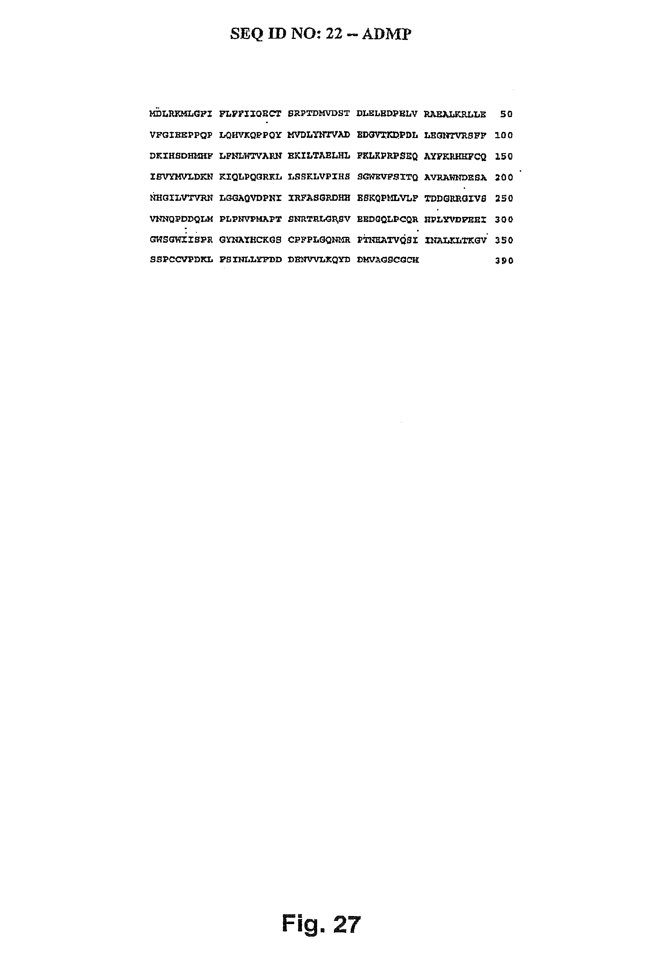

FIGURE 27 depicts a ADMP protein sequence (SEQ ID NO:22).

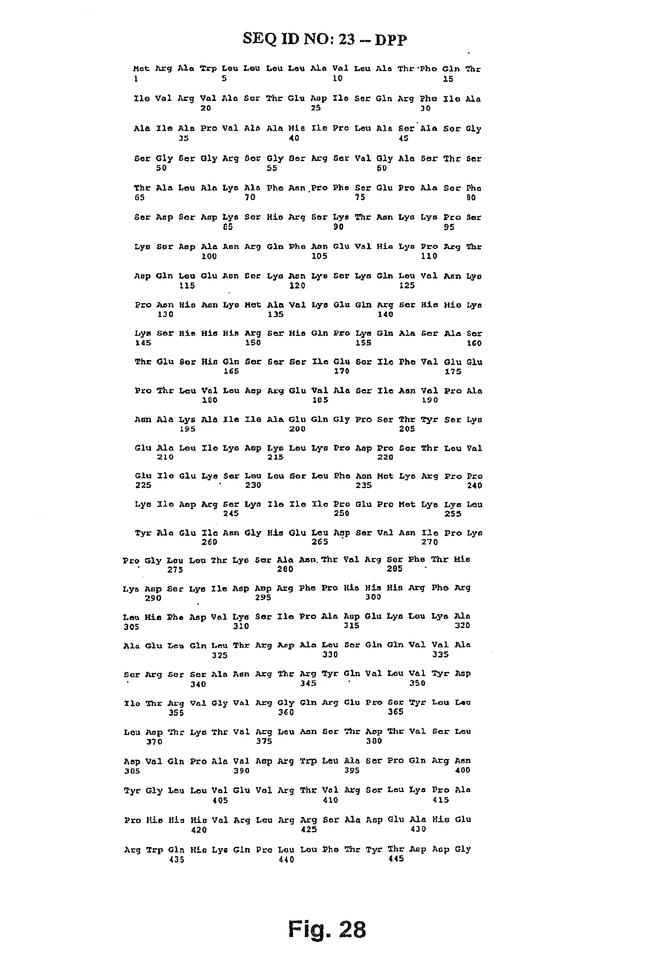

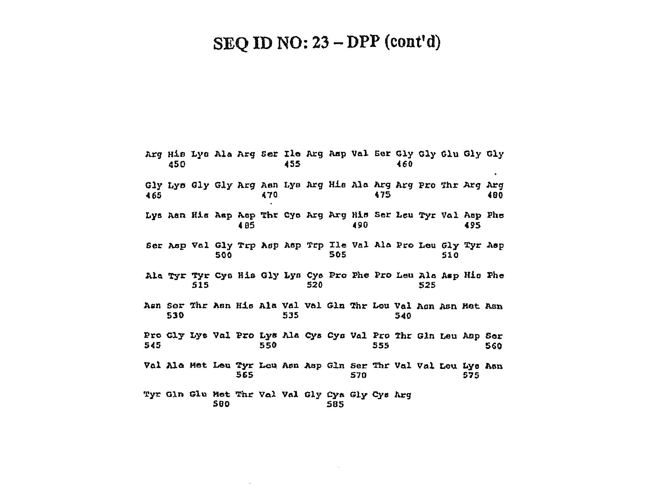

FIGURE 28 depicts a DPP protein sequence (SEQ ID NO:23).

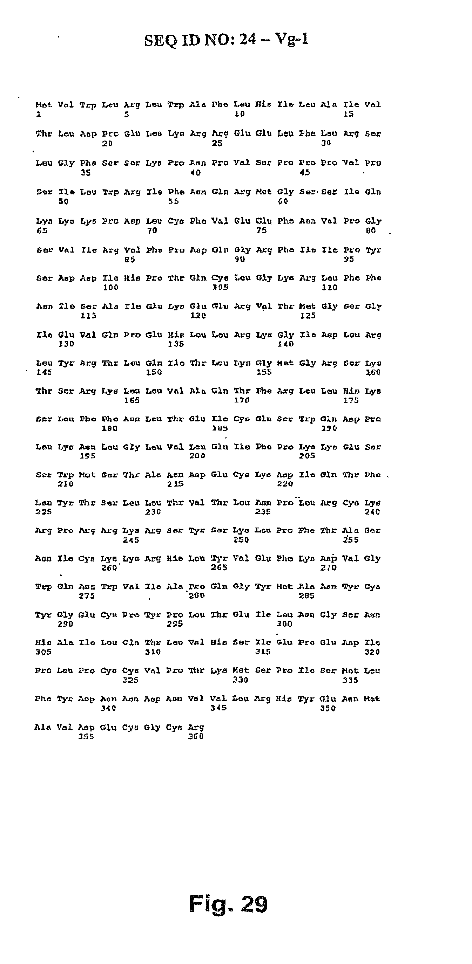

FIGURE 29 depicts a VG-1 protein sequence (SEQ ID NO:24).

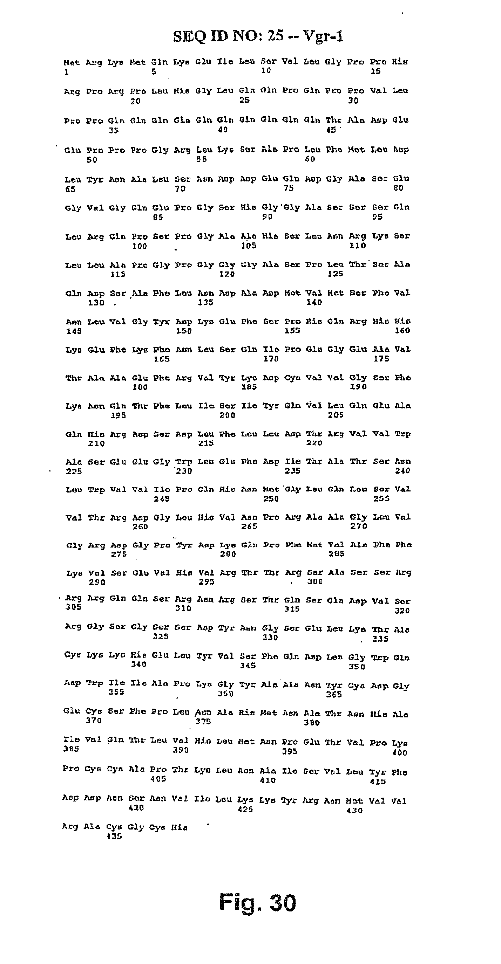

FIGURE 30 depicts a Vgr-1 protein sequence (SEQ ID NO:25).

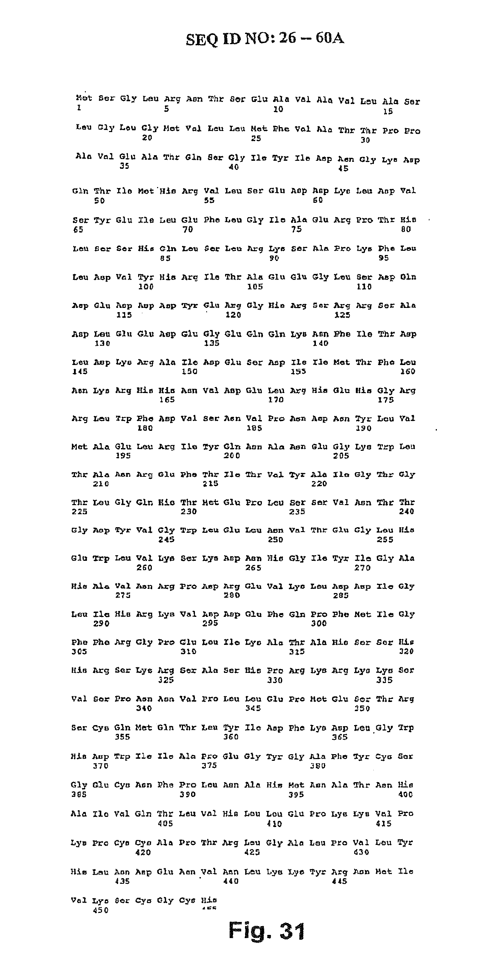

FIGURE 31 depicts a 60A protein sequence (SEQ ID NO:26).

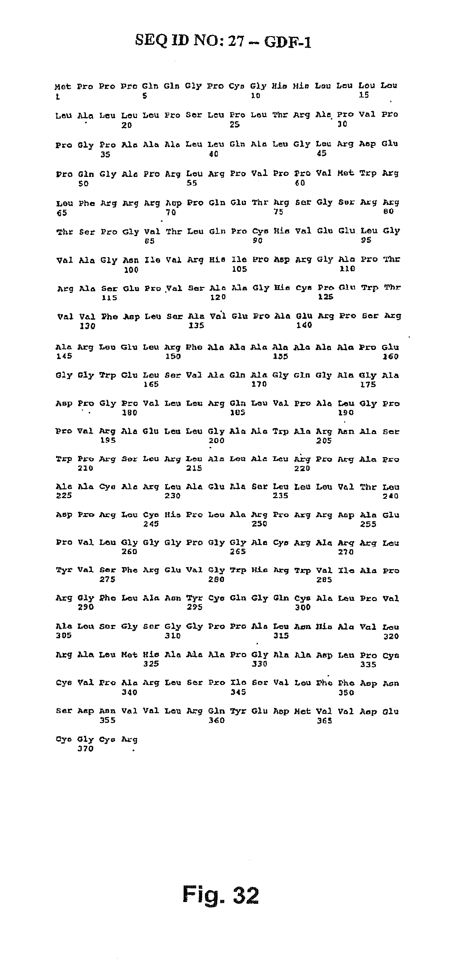

FIGURE 32 depicts a GDF-1 protein sequence (SEQ ID NO:27).

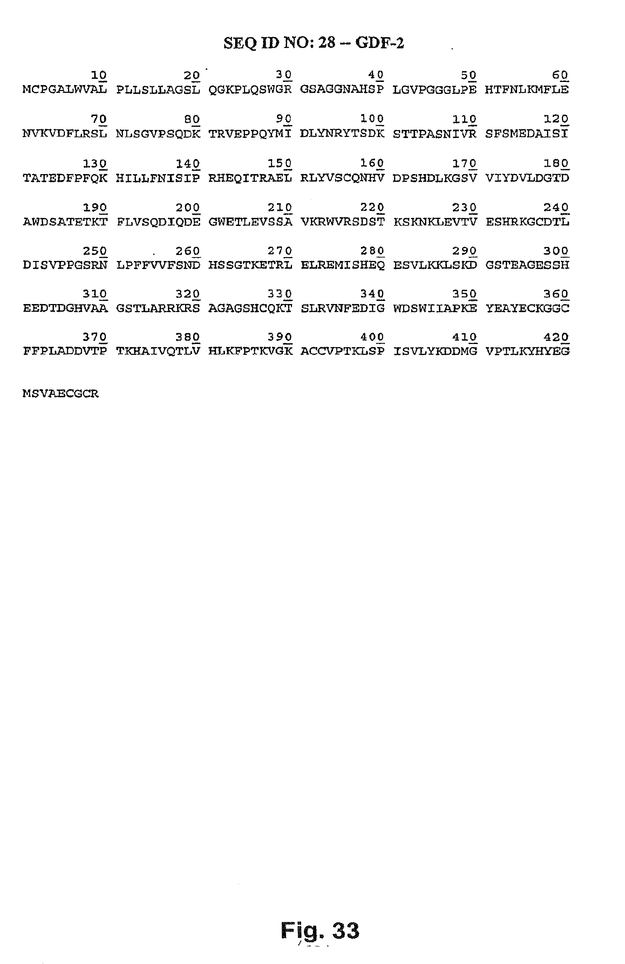

FIGURE 33 depicts a GDF-2 protein sequence (SEQ ID NO:28).

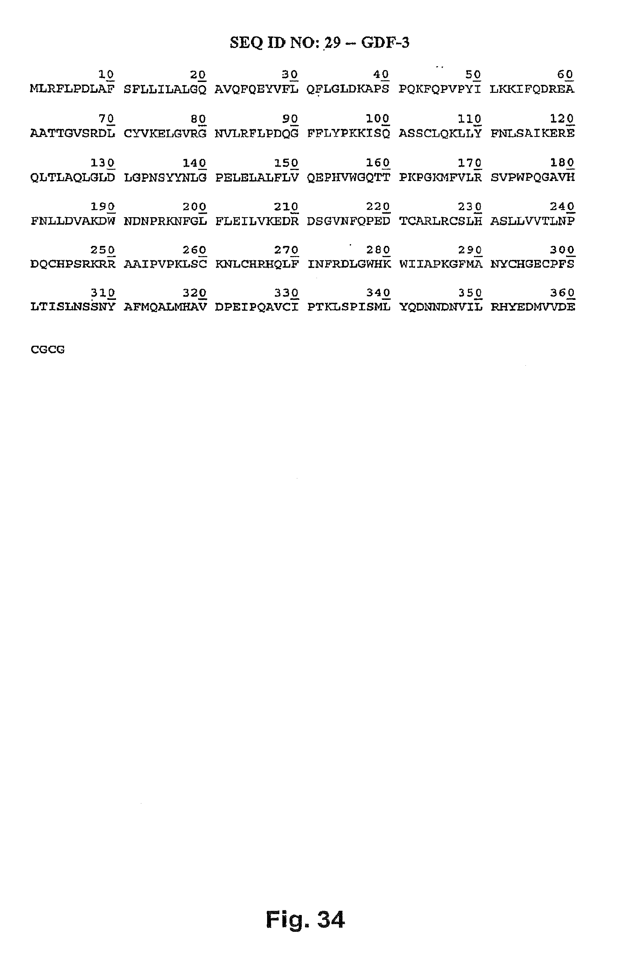

FIGURE 34 depicts a GDF-3 protein sequence (SEQ ID NO:29).

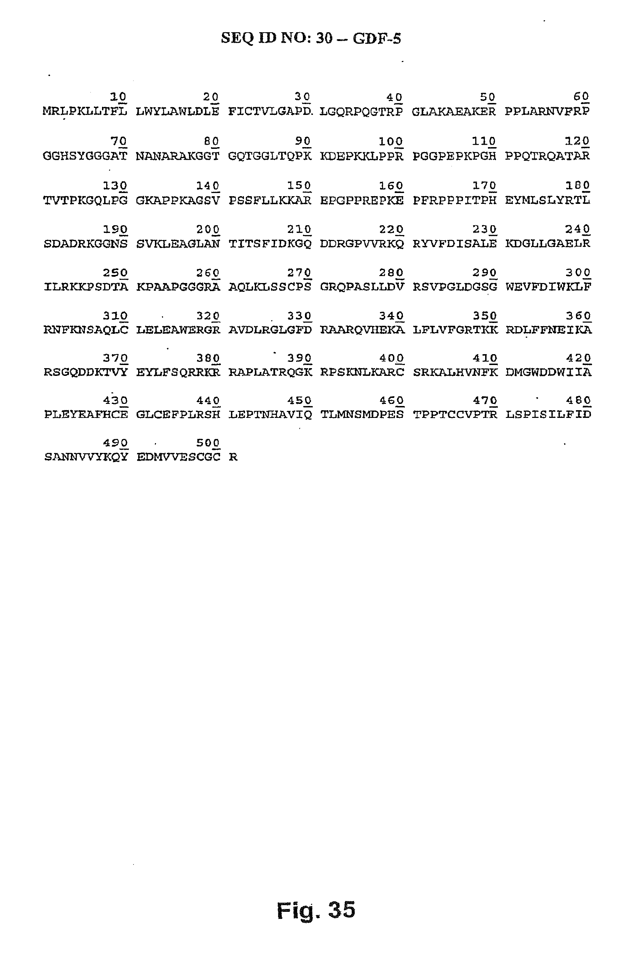

FIGURE 35 depicts a GDF-5 protein sequence (SEQ ID NO:30).

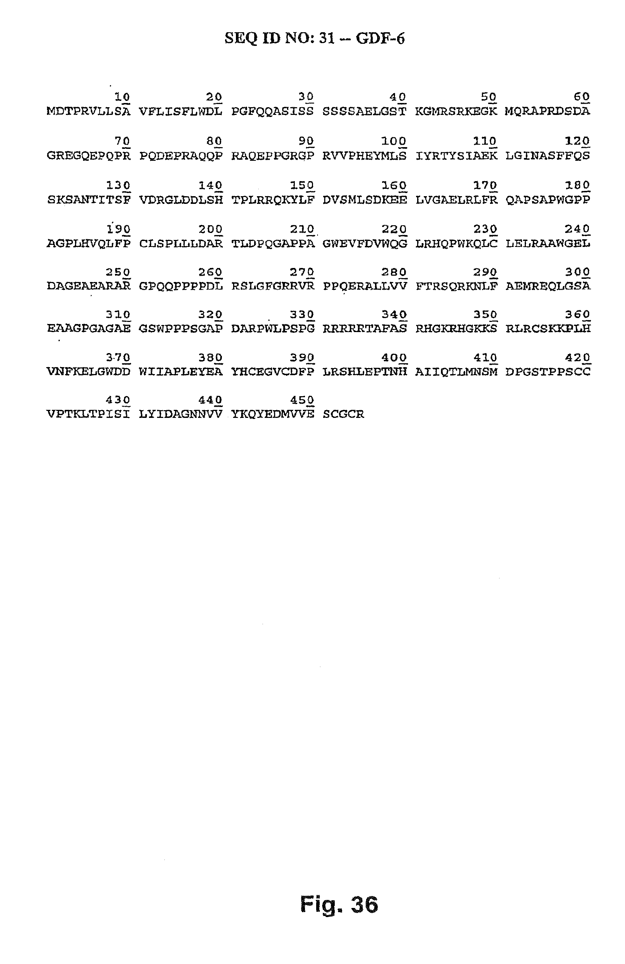

FIGURE 36 depicts a GDF-6 protein sequence (SEQ ID NO:31).

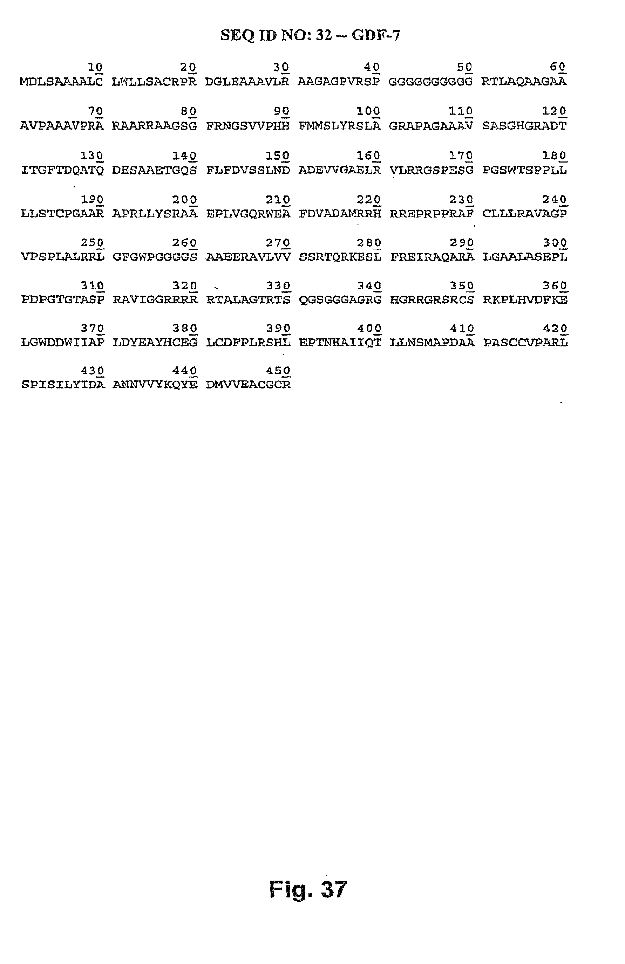

FIGURE 37 depicts a GDF-7 protein sequence (SEQ ID NO:32).

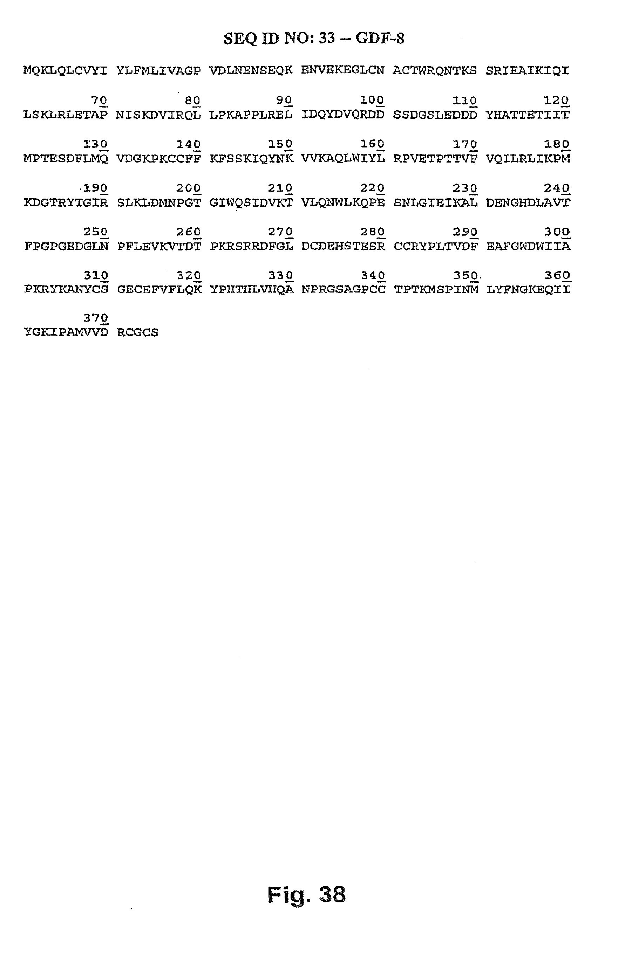

FIGURE 38 depicts a GDF-8 protein sequence (SEQ ID NO:33).

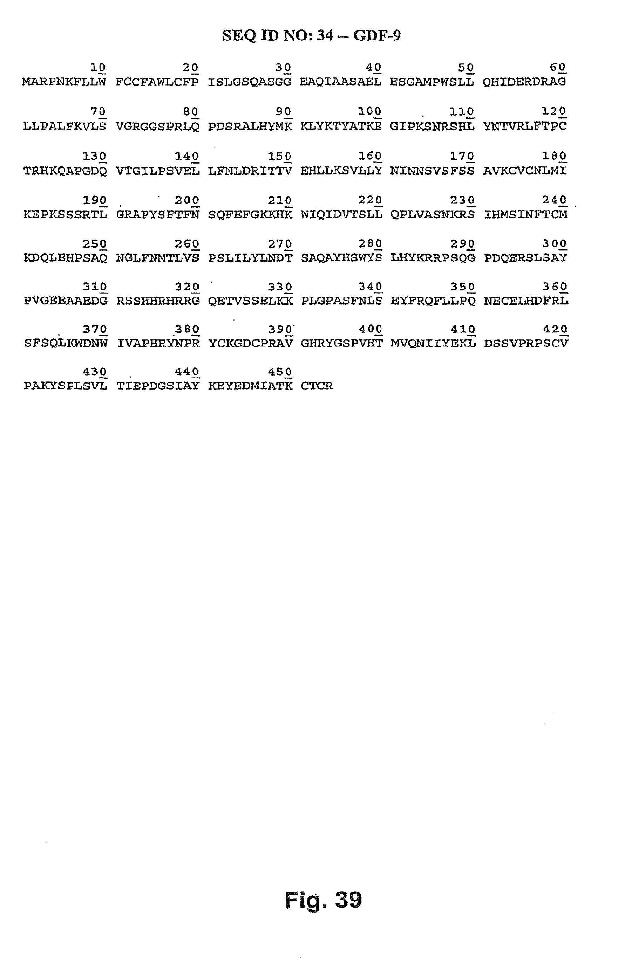

FIGURE 39 depicts a GDF-9 protein sequence (SEQ ID NO:34).

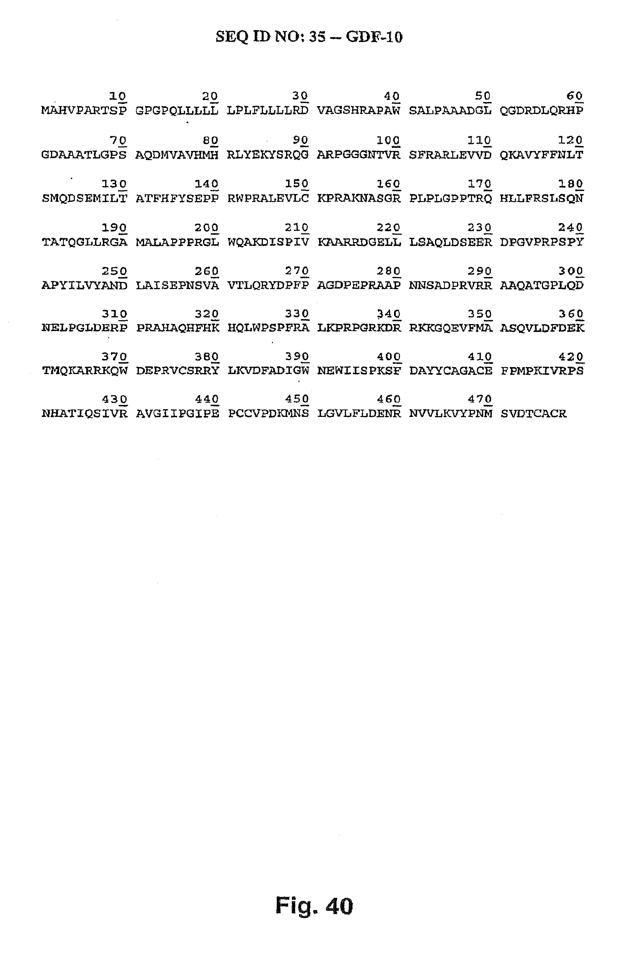

FIGURE 40 depicts a GDF-10 protein sequence (SEQ ID NO:35).

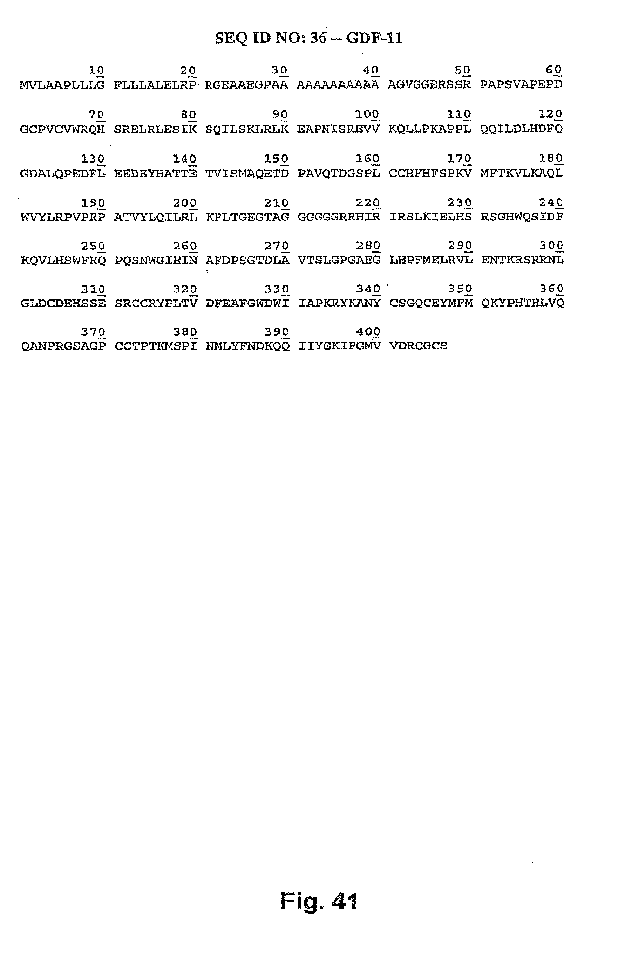

FIGURE 41 depicts a GDF-11 protein sequence (SEQ ID NO:36).

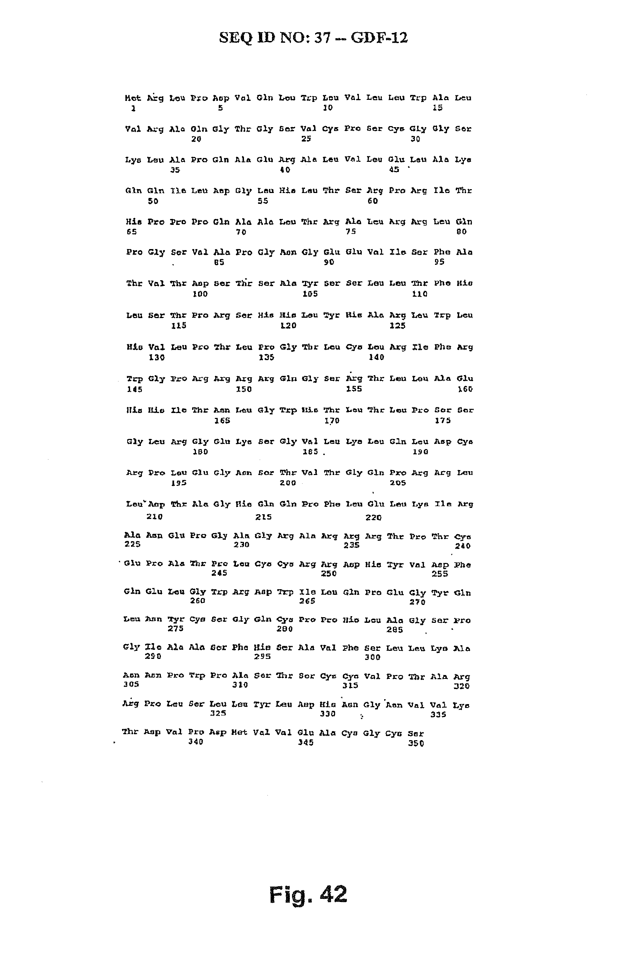

FIGURE 42 depicts a GDF-12 protein sequence (SEQ ID NO:37).

### DETAILED DESCRIPTION OF THE INVENTION

In order that the invention herein described may be fully understood, the following detailed description is set forth.

Unless defined otherwise, all technical and scientific terms used herein have the same meaning as those commonly understood by one of ordinary skill in the art to which this invention belongs. Although methods and materials similar or equivalent to those described herein can be used in the practice or testing of the present invention, suitable methods and materials are described below. The materials, methods and examples are illustrative only, and are not intended to be limiting. All publications, patents and other documents mentioned herein are incorporated by reference in their entirety.

Throughout this specification, the word "comprise" or variations such as "comprises" or "comprising" will be understood to imply the inclusion of a stated integer or groups of integers but not the exclusion of any other integer or group of integers.

In order to further define the invention, the following terms and definitions are provided herein.

The term "cartilage" refers to a type of connective tissue that contains chondrocytes or chondrocyte-like cells (having many, but not all characteristics of chondrocytes) and intercellular material (e.g., Types I, II, IX and XI collagen), proteoglycans (e.g., chondroitin sulfate, keratan sulfate, and dermatan sulfate proteoglycans) and other proteins. Cartilage includes articular and non-articular cartilage.

"Articular cartilage," also referred to as hyaline cartilage, refers to an avascular, non-mineralized connective tissue, which covers the articulating surfaces of bones in joints and serves as a friction reducing interface between two opposing bone surfaces. Articular cartilage allows movement in joints without direct bone-to-bone contact. Articular cartilage has no tendency to ossification. The cartilage surface appears smooth and pearly macroscopically, and is finely granular under high power magnification. Articular cartilage derives nutrients partly from the vessels of the neighboring synovial membrane and partly from the vessels of the bone it covers. Articular cartilage is associated with the presence of Type II and Type IX collagen and various well-characterized proteoglycans, and with the absence of Type X collagen, which is associated with endochondral bone formation. For a detailed description of articular cartilage microstructure, see, for example, Aydelotte and Kuettner, Conn. Tiss. Res., 18, p. 205 (1988); Zanetti et al., J. Cell Biol., 101, p. 53 (1985); and Poole et al., J. Anat., 138, p. 13 (1984).

"Non-articular cartilage" refers to cartilage that does not cover articulating surfaces and includes fibrocartilage (including interarticular fibrocartilage, fibrocartilaginous disc, connecting fibrocartilage and circumferential fibrocartilage) and elastic cartilage. In fibrocartilage, the micropolysaccharide network is interlaced with prominent collagen bundles, and the chondrocytes are more widely scattered than in hyaline or articular cartilage. Interarticular fibrocartilage is found in joints which are exposed to concussion and subject to frequent movement, e.g., the meniscus of the knee. Examples of such joints include but are not limited to the temporo-mandibular, sterno-clavicular, acromio-clavicular, wrist and knee joints. Secondary cartilaginous joints are formed by discs of fibrocartilage. Such fibrocartilaginous discs, which adhere closely to both of the opposed surfaces, are composed of concentric rings of fibrous tissue, with cartilaginous laminae interposed. An example of such fibrocartilaginous disc is the intervertebral disc of the spine. Connecting fibrocartilage is interposed between the bony surfaces of those joints, which allow for slight mobility as between the bodies of the vertebrae and between the pubic bones. Circumferential fibrocartilage surrounds the margin of some of the articular cavities, such as the cotyloid cavity of the hip and the glenoid cavity of the shoulder.

Elastic cartilage contains fibers of collagen that are histologically similar to elastin fibers. Such cartilage is found in the auricle of the external ear, the eustachian tubes, the cornicula laryngis and the epiglottis. As with all cartilage, elastic cartilage also contains chondrocytes and a matrix, the latter being pervaded in every direction, by a network of yellow elastic fibers, branching and anastomosing in all directions except immediately around each cell, where there is a variable amount of non-fibrillated, hyaline, intercellular substance.

The term "synovial fluid" refers to a thin, lubricating substance within the synovial cavity that reduces friction within the joint.

The term "defect" or "defect site", refers to a disruption of chondral or osteochondral tissue. A defect can assume the configuration of a "void", which is understood to mean a three-dimensional defect such as, for example, a gap, cavity, hole or other substantial disruption in the structural integrity of chondral or osteochondral tissue. A defect can also be a detachment of the cartilage from its point of attachment to the bone or ligaments. In certain embodiments, the defect is such that it is incapable of endogenous or spontaneous repair. A defect can be the result of accident, disease, and/or surgical manipulation. For example, cartilage defects may be the result of trauma to a joint such as a displacement of torn meniscus tissue into the joint. Cartilage defects may be also be the result of degenerative joint diseases such as osteoarthritis.

The term "repair" refers to new cartilage formation which is sufficient to at least partially fill the void or structural discontinuity at the defect site. Repair does not, however, mean, or otherwise necessitate, a process of complete healing or a treatment, which is 100% effective at restoring a defect to its pre-defect physiological/structural/mechanical state.

The term "therapeutically effective amount" refers to an amount effective to repair, regenerate, promote, accelerate, prevent degradation, or form cartilage tissue.

The term "patient" refers to an animal including a mammal (e.g., a human).

The term "pharmaceutically acceptable carrier of adjuvant" refers to a non-toxic carrier or adjuvant that may be administered to a patient, together with a soluble morphogenic protein complex of this invention, and which does not destroy the pharmacological activity thereof.

The term "morphogenic protein" refers to a protein having morphogenic activity. Preferably a morphogenic protein of this invention comprises at least one polypeptide belonging to the BMP protein family. Morphogenic proteins include osteogenic proteins. Morphogenic proteins may be capable of inducing progenitor cells to proliferate and/or to initiate differentiation pathways that lead to cartilage, bone, tendon, ligament or other types of tissue formation depending on local environmental cues, and thus morphogenic proteins may behave differently in different surroundings. For example, a morphogenic protein may induce bone tissue at one treatment site and cartilage tissue at a different treatment site.

The term "bone morphogenic protein (BMP)" refers to a protein belonging to the BMP family of the TGF-β superfamily of proteins (BMP family) based on DNA and amino acid sequence homology. A protein belongs to the BMP family according to this invention when it has at least 50% amino acid sequence identity with at least one known BMP family member within the conserved C-terminal cysteine-rich domain, which characterizes the BMP protein family. Preferably, the protein has at least 70% amino acid sequence identity with at least one known BMP family member within the conserved C-terminal cysteine rich domain. Members of the BMP family may have less than 50% DNA or amino acid sequence identity overall. Osteogenic protein as defined herein also is competent to induce articular cartilage formation at an appropriate in vivo avascular locus.

The term "amino acid sequence homology" is understood to include both amino acid sequence identity and similarity. Homologous sequences share identical and/or similar amino acid residues, where similar residues are conservative substitutions for, or "allowed point mutations" of, corresponding amino acid residues in an aligned reference sequence. Thus, a candidate polypeptide sequence that shares 70% amino acid homology with a reference sequence is one in which any 70% of the aligned residues are either identical to, or are conservative substitutions of, the corresponding residues in a reference sequence. Certain particularly preferred morphogenic polypeptides share at least 60%, and preferably 70% amino acid sequence identity with the C-terminal 102-106 amino acids, defining the conserved seven-cysteine domain of human OP-1 and related proteins.

Amino acid sequence homology can be determined by methods well known in the art. For instance, to determine the percent homology of a candidate amino acid sequence to the sequence of the seven-cysteine domain, the two sequences are first aligned. The alignment can be made with, e.g., the dynamic programming algorithm described in Needleman et al., J. Mal. Biol., 48, pp. 443 (1970), and the Align Program, a commercial software package produced by DNAstar, Inc. The teachings by both sources are incorporated by reference herein. An initial alignment can be refined by comparison to a multi-sequence alignment of a family of related proteins. Once the alignment is made and refined, a percent homology score is calculated. The aligned amino acid residues of the two sequences are compared sequentially for their similarity to each other. Similarity factors include similar size, shape and electrical charge. One particularly preferred method of determining amino acid similarities is the PAM250 matrix described in Dayhoff et al., Atlas of Protein Sequence and Structure, 5, pp. 345-352 (1978 & Supp.), which is incorporated herein by reference. A similarity score is first calculated as the sum of the aligned pair wise amino acid similarity scores. Insertions and deletions are ignored for the purposes of percent homology and identity. Accordingly, gap penalties are not used in this calculation. The raw score is then normalized by dividing it by the geometric mean of the scores of the candidate sequence and the seven-cysteine domain. The geometric mean is the square root of the product of these scores. The normalized raw score is the percent homology.

The term "conservative substitutions" refers to residues that are physically or functionally similar to the corresponding reference residues. That is, a conservative substitution and its reference residue have similar size, shape, electric charge, chemical properties including the ability to form covalent or hydrogen bonds, or the like. Preferred conservative substitutions are those fulfilling the criteria defined for an accepted point mutation in Dayhoff *et al., supra.* Examples of conservative substitutions are substitutions within the following groups: (a) valine, glycine; (b) glycine, alanine; (c) valine, isoleucine, leucine; (d) aspartic acid, glutamic acid; (e) asparagine, glutamine; (f) serine, threonine; (g) lysine, arginine, methionine; and (h) phenylalanine, tyrosine. The term "conservative variant" or "conservative variation" also includes the use of a substituting amino acid residue in place of an amino acid residue in a given parent amino acid sequence, where antibodies specific for the parent sequence are also specific for, i.e., "cross-react" or "immuno-react" with, the resulting substituted polypeptide sequence.

The term "osteogenic protein (OP)" refers to a morphogenic protein that is capable of inducing a progenitor cell to form cartilage and/or bone. The bone may be intramembranous bone or endochondral bone. Most osteogenic proteins are members of the BMP protein family and are thus also BMPs. As described elsewhere herein, the class of proteins is typified by human osteogenic protein (hOP-1). Other osteogenic proteins useful in the practice of the invention include osteogenically active forms of OP-1, OP-2, OP-3, BMP-2, BMP-3, BMP-4, BMP-5, BMP-6, BMP-8, BMP-9, BMP-10, BMP-11, BMP-12, BMP-13, BMP-15, BMP-16, BMP-17, BMP-18, DPP, Vg1, Vgr-1, 60A protein, GDF-1, GDF-2, GDF-3, GDF-5, GDF-6, GDF-7, GDF-8, GDF-9, GDF-10, GDF-11, GDF-12, CDMP-1, CDMP-2, CDMP-3, UNIVIN, NODAL, SCREW, ADMP or NEURAL, or conservative substitution variants or fragments thereof. Osteogenic proteins suitable for use with applicants' invention can be identified by means of routine experimentation using the art-recognized bioassay described by Reddi and Sampath (Sampath et al., Proc. Natl. Acad. Sci., 84, pp. 7109-13, incorporated herein by reference).

### Methods and Compositions For Cartilage Growth and Repair

The morphogenic compositions of this invention may be used for cartilage repair (e.g., at a joint, meniscus or intervertebral disc). The morphogenic compositions comprising a soluble morphogenic protein complex disclosed herein will permit the physician to treat a variety of tissue injuries, tissue degenerative or disease conditions and disorders that can be ameliorated or remedied by localized, stimulated tissue regeneration or repair.

The invention provides methods and compositions for treating cartilage tissue injuries and cartilage degenerative diseases or disorders including but not limited to osteoarthritis, chondral defects, osteochondral defects, meniscus tears, ACL injuries and disc degeneration.

In some embodiments, the invention provides methods and compositions for repairing or regenerating cartilage in a patient. The invention also provides methods and compositions for producing cartilage, promoting cartilage growth accelerating cartilage formation and preventing cartilage degradation in a patient.

In some embodiments, the methods of the present invention comprise the step of administering into the cartilage a composition comprising a therapeutically effective amount of a soluble morphogenic protein complex. This method involves contacting the cartilage tissue with the soluble morphogenic protein complex. For example, in one embodiment, the soluble morphogenic protein complex composition is administered directly into the cartilage tissue (e.g., an injection into the cartilage tissue). For example, the soluble morphogenic protein complex composition may be injected into a meniscus or an intervertebral disc. In some embodiments, the methods of the present invention comprise the step of administering the soluble morphogenic protein complex composition directly into a joint, for example, an articular joint, such as a knee, hip, shoulder, ankle, elbow, or knuckle. For example, a therapeutically effective amount of a soluble morphogenic protein complex can be administered into the synovial fluid surrounding the cartilage in a joint. In some embodiments, the cartilage is articular cartilage. In other embodiments, the cartilage is non-articular cartilage. In some embodiments, the non-articular cartilage includes but is not limited to intervertebral disc, interarticular meniscus, trachea, ear, nose, rib and larynx. In a preferred embodiment the non-articular cartilage is an intervertebral disc. In another preferred embodiment, the non-articular cartilage is a meniscus. In some embodiments, the area surrounding the cartilage is synovial fluid.

In some embodiments, the morphogenic protein in the composition used in the methods of the present invention includes but is not limited to OP-1, OP-2, OP-3, BMP-2, BMP-3, BMP-4, BMP-5, BMP-6, BMP-8, BMP-9, BMP-10, BMP-11, BMP-12, BMP-13, BMP-15, BMP-16, BMP-17, BMP-18, DPP, Vg1, Vgr, 60A protein, GDF-1, GDF-2, GDF-3, GDF-5, GDF-6, GDF-7, GDF-8, GDF-9, GDF-10, GDF-11, GDF-12, CDMP-1, CDMP-2, CDMP-3, NODAL, UNIVIN, SCREW, ADMP, NEURAL, or conservative substitution variants or fragments thereof. In a preferred embodiment, the morphogenic protein is OP-1, BMP-5, BMP-6, GDF-5, GDF-6, GDF-7, CDMP-1, CDMP-2 or CDMP-3. In a more preferred embodiment, the morphogenic protein is OP-1.

In other embodiments, the morphogenic protein comprises an amino acid sequence having at least 70% homology with the C-terminal 102-106 amino acids, including the conserved seven cysteine domain, of human OP-1, said morphogenic protein being capable of inducing repair of the cartilage defect.

### Useful Soluble Morphogenic Protein Complexes--Protein Considerations

Among the morphogenic proteins useful in this invention are the BMPs (BMP-1 to BMP-18), which were isolated primarily based on sequence homology. All but BMP-1 remain classified as members of the BMP family of morphogenic proteins (Ozkaynak et al., EMBO J., 9, pp. 2085-93 (1990)). Other useful morphogenic proteins are amino acid sequence-related proteins such as DPP (from Drosophila), Vgl (from Xenopus), Vgr-1 (from mouse, see U.S. Pat. No. 5,011,691 to Oppermann et al.), GDF-1 (from mouse, see Lee (1991) PNAS 88:4250-4254), 60A protein (from Drosophila, Seq. ID No. 24, see Wharton et al. (1991) PNAS 88:9214-9218), and OP-3. (See, e.g., Massagué, Annu. Rev. Cell Biol., 6, pp. 597-641 (1990), incorporated herein by reference).

The C-terminal domains of BMP-3, BMP-5, BMP-6, and OP-1 (BMP-7) are about 60% identical to that of BMP-2, and the C-terminal domains of BMP-6 and OP-1 are 87% identical. BMP-6 is likely the human homolog of the murine Vgr-1 (Lyons et al., Proc. Natl. Acad. Sci. U.S.A., 86, pp. 4554-59 (1989)); the two proteins are 92% identical overall at the amino acid sequence level (U. S. Patent No. 5,459,047, incorporated herein by reference). BMP-6 is 58% identical to the *Xenopus* Vg-1 product.

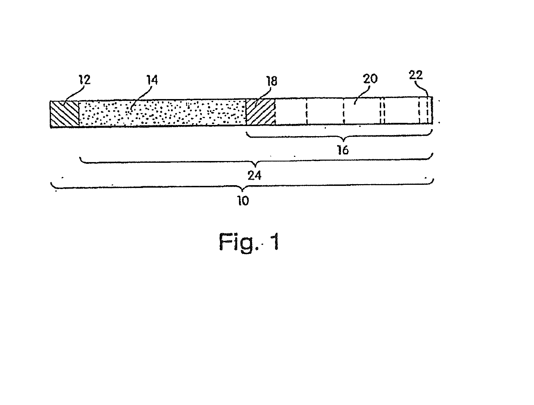

The members of this family, which are a subclass of the TGF-ß superfamily of proteins, share characteristic structural features, represented schematically in FIGURE 1, as well as substantial amino acid sequence homology in their C-terminal domains, including a conserved seven cysteine structure. As illustrated in the figure, the proteins are translated as a precursor polypeptide sequence 10, having an N-terminal signal peptide sequence 12, (the "pre pro" region, indicated in the figure by cross-hatching), typically less than about 30 residues, followed by a "pro" region 14, indicated in the figure by stippling, and which is cleaved to yield the mature sequence 16. The mature sequence comprises both the conserved C-terminal seven cysteine domain 20, and an N-terminal sequence 18, referred to herein as an N-terminal extension, and which varies significantly in sequence between the various morphogenic proteins. Cysteines are represented in the figure by vertical hatched lines 22. The polypeptide, chains dimerize and these dimers typically are stabilized by at least one interchain disulfide bond linking the two polypeptide chain subunits.

The signal peptide is cleaved rapidly upon translation, at a cleavage site that can be predicted in a given sequence using the method of Von Heijne ((1986) Nucleic Acids Research 14:4683-4691.) The "pro" form of the protein subunit, 24, in FIGURE 1, includes both the pro domain and the mature domain, peptide bonded together. Typically, this pro form is cleaved while the protein is still within the cell, and the pro domain remains noncovalently associated with the mature form of the subunit to form a soluble species that appears to be the primary form secreted from cultured mammalian cells. Typically, previous purification techniques utilized denaturing conditions that disassociated the complex.

Another characteristic of the BMP protein family members is their apparent ability to dimerize. Several bone-derived osteogenic proteins (OPs) and BMPs are found as homo- and heterodimers in their active forms. The ability of OPs and BMPs to form heterodimers may confer additional or altered morphogenic inductive capabilities on morphagenic proteins. Heterodimers may exhibit qualitatively or quantitatively different binding affinities than homodimers for OP and BMP receptor molecules. Altered binding affinities may in turn lead to differential activation of receptors that mediate different signaling pathways, which may ultimately lead to different biological activities or outcomes. Altered binding affinities could also be manifested in a tissue or cell type-specific manner, thereby inducing only particular progenitor cell types to undergo proliferation and/or differentiation.

Other soluble forms of morphogenic proteins secreted from mammalian cells include dimers of the pro forms of these proteins, wherein the pro region is not cleaved from the mature domain, and "hemi-dimers", wherein one subunit comprises a pro form of the polypeptide chain subunit and the other subunit comprises the cleaved mature form of the polypeptide chain subunit (including truncated forms thereof), preferably noncovalently associated with a cleaved pro domain.

The isolated pro domain typically has a substantial hydrophobic character, as determined both by analysis of the sequence and by characterization of its properties in solution. The isolated pro regions alone typically are not significantly soluble in aqueous solutions, and require the presence of denaturants, e.g., detergents, urea, guanidine HCl, and the like, and/or one or more carrier proteins. Accordingly, without being limited to any given theory, the non-covalent association of the cleaved pro region with the mature morphogenic protein dimeric species likely involves interaction of a hydrophobic portion of the pro region with a corresponding hydrophobic region on the dimeric species, the interaction of which effectively protects or "hides" an otherwise exposed hydrophobic region of the mature dimer from exposure to aqueous environments, enhancing the affinity of the mature dimer species for aqueous solutions.

Useful pro domains include the full length pro regions described below, as well as various truncated forms hereof, particularly truncated forms cleaved at proteolytic Arg-Xaa-Xaa-Arg cleavage sites. For example, in OP-1, possible pro sequences include sequences defined by residues 30-292 (full length form); 48-292; and 158-292. Soluble OP-1 complex stability is enhanced when the pro region comprises the full length form rather than a truncated form, such as the 48-292 truncated form, in that residues 30-47 show sequence homology to the N-terminal portions of other morphogenic proteins, and are believed to have particular utility in enhancing complex stability for all morphogenic proteins. Accordingly, currently preferred pro sequences are those encoding the full length form of the pro region for a given morphogenic protein (see below). Other pro sequences contemplated to have utility include biosynthetic pro sequences, particularly those that incorporate a sequence derived from the N-terminal portion of one or more morphogenic protein pro sequences.

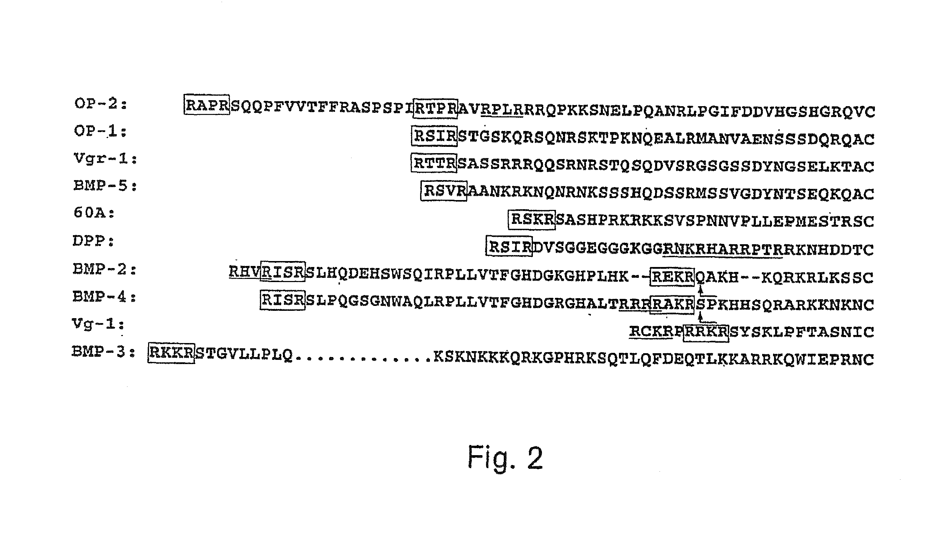

Table 1, below, describes the various preferred morphogenic proteins identified to date, including their nomenclature as used herein, the sequences defining the various regions of the subunit sequences, their SEQ ID references, and publication sources for their nucleic acid and amino acid sequences. The disclosure of these publications is incorporated herein by reference. The mature protein sequences defined are the longest anticipated forms of these sequences. As described above, shorter, truncated forms of these sequences also are contemplated. Preferably, truncated mature sequences include at least 10 amino acids of the N-terminal extension. FIGURE 2 lists the N-terminal extensions for a number of the preferred morphogenic protein sequences described below. Arg-Xaa-Xaa-Arg cleavage sites that may yield truncated sequences of the mature subunit form are boxed or underlined in the figure.

**TABLE 1**

| | |
|---|---|
| "OP-1" | Refers generically to the group of morphogenically active proteins expressed from part or all of a DNA sequence encoding OP-1 protein, including allelic and species variants thereof, e.g., human OP-1 ("hOP-1"), or mouse OP-1 ("mOP-1"). The cDNA sequences and the amino acids encoding the full length proteins are provided in SEQ ID NOs. 1 and 2 (hOP1) and SEQ ID NOs. 3 and 4 (mOP1.). The mature proteins are defined by residues 293-431 (hOP1) and 292-430 (mOP1), wherein the conserved seven cysteine skeleton is defined by residues 330-431 and 329-430, respectively, and the N-terminal extensions are defined by residues 293-329 and 292-329, respectively. The "pro" regions of the proteins, cleaved to yield the mature, morphogenically active proteins, are defined essentially by residues 30-292 (hOP1) and residues 30-291 (mOP1). |
| "OP-2" | Refers generically to the group of active proteins expressed from part or all of a DNA sequence encoding OP-2 protein, including allelic and species variants thereof, e.g., human OP-2 ("hOP-2") or mouse OP-2 ("mOP-2".) The full length proteins are provided in SEQ ID NOs. 5 and 6 (hOP2) and SEQ ID NOs. 7 and 8 (mOP2). The mature proteins are defined essentially by residues 264-402 (hOP2) and 261-399 (mOP2), wherein the conserved seven cysteine skeleton is defined by residues 301-402 and 298-399, respectively, and the N-terminal extensions are defined by residues 264-300 and 261-297, respectively. The "pro" regions of the proteins, cleaved to yield the mature, morphogenically active proteins likely are defined essentially by residues 18-263 (hOP2) and residues 18-260 (mOP2). (Another cleavage site also occurs 21 residues upstream for both OP-2 proteins). |
| "OP-3" | Refers generically to the group of active proteins expressed from part or all of a DNA sequence encoding OP-3 protein, including allelic and species variants thereof, e.g., mouse OP-3 ("mOP-3"). The full length protein is provided in SEQ ID NO: 9. The mature protein is defined essentially by residues 261-399 or 264-399, where in the conserved seven cysteine skeleton is defined by residues 298-399 and the N-terminal extension is defined by residues 264-297 or 261-297. The "pro" region of the protein, cleaved to yield the mature, morphogenically active proteins likely is defined essentially by residues 20-262. |
| "BMP2/BMP4" | Refers to protein sequences encoded by the human BMP2 and BMP4 genes. The amino acid sequence for the full length proteins, referred to in the literature as BMP2A and BMP2B, or BMP2 and BMP4, appear in SEQ ID NOs. 10 and 11, respectively, and in Wozney, et al. (1988) Science 242: 1528-1534. The pro domain for BMP2 (BMP2A) likely includes residues 25-248 or 25-282; the mature protein, residues 249-396 or 283-396, of which residues 249-296/283-296 define the N-terminal extension and 295-396 define the C-terminal domain. The pro domain for BMP4 (BMP2B) likely includes residues 25-256 or 25-292; the mature protein, residues 257-408 or 293-408, of which 257-307/293-307 define the N-terminal extension, and 308-408 define the C-terminal domain. |
| "BMP3" | Refers to protein sequences encoded by the human BMP3 gene. The amino acid sequence for the full length protein, including the mature form and the pro region, appears in SEQ ID NO: 12 and in Wozney et al. (1988) Science 242: 1528-1534. The pro domain likely extends from the signal peptide cleavage site to residue 290; the mature protein likely is defined by residues 291-472, wherein residues 291-370 define the N-terminal extension and residues 371-472 define the C-terminus. |
| "BMP5" | Refers to protein sequences encoded by the human BMP5 gene. The amino acid sequence for the full length protein, including the mature form and the pro region, appears in SEQ ID NO: 13 and in Celeste, et al. (1990) PNAS 87: 9843-9847. The pro domain likely extends from the signal peptide cleavage site to residue 316; the mature protein likely is defined by residues 317-454, where residues 317-352 define the N-terminus and residues 352-454 define the C-terminus. |
| "BMP6" | Refers to protein sequences encoded by the human BMP6 gene. The amino acid sequence for the full length protein, including the mature form and the pro region, appears in SEQ ID NO: 14 and in Celeste, et al. (1990) PNAS 87: 9843-5847. The pro domain likely includes extends from the signal peptide cleavage site to residue 374; the mature sequence likely includes residues 375-513, where residues 375-411 define the N-terminus and residues 412-513 define the C-terminus. |
| "BMP10" | Refers to protein sequences encoded by the human BMP 10 gene. The amino acid sequence for the full length protein, including the mature form and the pro region, appears in SEQ ID NO: 15 and in WO94/26893. The pro domain likely includes extends from the signal peptide cleavage site to residue 316; the mature sequence likely includes residues 317-424, where residues 317-321 define the N-terminus and residues 322-424 define the C-terminus. |
| "BMP15" | Refers to protein sequences encoded by the human BMP15 gene. The amino acid sequence for the full length protein, including the mature form and the pro region, appears in SEQ ID NO: 16 and in WO96/367I0. The pro domain likely includes extends from the signal peptide cleavage site to residue 267; the mature sequence likely includes residues 268-392, where residues 268-290 define the N-terminus and residues 291-392 define the C-terminus. |
| "GDF-9b" | |
| "BMP16" | Refers to protein sequences encoded by the human BMP16 gene. The amino acid sequence for the full length protein, including the mature form and the pro region, appears in SEQ ID NO: 17 and in WO98/12322 |
| "BMP17" | Refers to protein sequences encoded by the human BMP 17 gene. The amino acid sequence for the full length protein, including the mature form and the pro region, appears in SEQ ID NO: 18 and in U.S. 6,027,917. |
| "BMP18" | Refers to protein sequences encoded by the human BMP 18 gene. The amino acid sequence for the full length protein, including the mature form and the pro region, appears in SEQ ID NO: 19 and in U.S. 6,027,917. |
| "NODAL" | Refers to protein sequences encoded by the human NODAL gene. The amino acid sequence for the full length protein, including the mature form and the pro region, appears in SEQ ID NO: 20 and in http://www.expasy.org/uniprot/Q96S42. The pro domain likely includes extends from the signal peptide cleavage site to residue 237; the mature sequence likely includes residues 238-347, where residues 238-247 define the N-terminus and residues 239-347 define the C-terminus. |
| "UNIVIN" | Refers to protein sequences encoded by the *Strongylcentrotus purpuratus* UNIVIN gene. The amino acid sequence for the full length protein, including the mature form and the pro region, appears in SEQ ID NO: 21 and in http://ca.expasy.org/uniprot/P48970. The pro domain likely includes extends from the signal peptide cleavage site to residue 272; the mature sequence likely includes residues 273-395, where residues 273-294 define the N-terminus and residues 295-395 define the C-terminus. |
| "ADMP" | Refers to protein sequences encoded by the *Xenopus* ADMP gene. The amino acid sequence for the full length protein, including the mature form and the pro region, appears in SEQ ID NO: 22 and in Moos, et al., Development 121, 4293-4301 (1995). The pro domain likely includes extends from the signal peptide cleavage site to residue 278; the mature sequence likely includes residues 279-390, where residues 279-287 define the N-terminus and residues 288-390 define the C-terminus. |
| "DPP" | Refers to protein sequences encoded by the Drosophila DPP gene. The amino acid sequence for the full length protein, including the mature form and the pro region, appears in SEQ ID NO. 23 and in Padgett, et al (1987) Nature 325: 81-84. The pro domain likely extends from the signal peptide cleavage site to residue 456; the mature protein likely is defined by residues 457-588, where residues 457-586 define the N-terminal extension and 487-588 define the C-terminal domain. |
| "Vgl" | Refers to protein sequences encoded by the *Xenopus* Vgl gene. The amino acid sequence for the full length protein, including the mature form and the pro region, appears in SEQ ID NO. 24 and in Weeks (1987) Cell 51: 861-867. The pro domain likely extends from the signal peptide cleavage site to residue 246; the mature protein likely is defined by residues 247-360, where residues 247-258 define the N-terminal extension, and residues 259-360 define the C-terminal domain. |
| "Vgr-1" | Refers to protein sequences encoded by the murine Vgr-1 gene. The amino acid sequence for the full length protein, including the mature form and the pro region, appears in SEQ ID NO: 25 and in Lyons, et al, (1989) PNAS 86: 4554-4558. The pro domain likely extends from the signal peptide cleavage site to residue 299; the mature protein likely is defined by residues 300-438, where residues 300-336 define the N-terminal extension and residues 337-438 define the C-terminus. |
| "60A" | Refers to protein sequences encoded by the Drosophila 60A gene. The amino acid sequence for the full length protein appears in SEQ ID NO: 26 and in Wharton et al. (1991) PNAS 88:9214-9218) The pro domain likely extends from the signal peptide cleavage site to residue 324; the mature protein likely is defined by residues 325-455, wherein residues 325-353 define the N-terminal extension and residues 354-455 define the C-terminus. |
| "GDF-1" | Refers to protein sequences encoded by the human GDF-1 gene The cDNA and encoded amino sequence for the full length protein is provided in SEQ ID NO: 27 and Lee (1991) PNAS 88:4250-4254. The pro domain likely extends from the signal peptide cleavage site to residue 214; the mature protein likely is defined by residues 215-372, where residues 215-256 define the N-terminal extension and residues 257-372 define the C-terminus. |
| "GDF-2" | Refer to protein sequences encoded by the human GDF-2 gene. The cDNA and encoded amino sequence for the full length protein is provided in SEQ ID NO: 28 and WO95/3383 The pro domain likely extends from the signal peptide cleavage site to residue 319; the mature protein likely is defined by residues 320-429, where residues 320-326 define the N-terminal extension and residues 327-429 define the C-terminus. |
| "BMP-9" | |
| "GDF-3" | Refers to protein sequences encoded by the human GDF-3 gene. The cDNA and encoded amino sequence for the full length protein is provided in SEQ ID NO: 29. The pro domain likely extends from the signal peptide cleavage site to residue 250; the mature protein likely is defined by residues 251-364, where residues 251-259 define the N-terminal extension and residues 260-364 define the C-terminus. |
| "GDF-5" | Refer to protein sequences encoded by the human GDF-5 gene. The cDNA and encoded amino sequence for the full length protein is provided in SEQ ID NO: 30 and in WO94/15949, WO96/14335 and WO93/16099. The pro domain likely extends from the signal peptide cleavage site to residue 381; the mature protein likely is defined by residues 382-501, where residues 382-399 define the N-terminal extension and residues 400-501 define the C-terminus. |
| "CDMP-1" | |
| "BMP-14" | |
| "GDF-6" | Refers to protein sequences encoded by the human GDF-6 gene. The cDNA and encoded amino sequence for the full length protein is provided in SEQ ID NO: 31 and in WO95/01801, WO96/14335 and WO95/10635. The pro domain likely extends from the signal peptide cleavage site to residue 335; the mature protein likely is defined by residues 336-455, where residues 336-353 define the N-terminal extension and residues 354-455 define the C-terminus. |
| "CDMP-2" | |
| "BMP-13" | |
| "GDF-7" | Refers to protein sequences encoded by the human GDF-7 gene. The cDNA and encoded amino sequence for the full length protein is provided in SEQ ID NO: 32 and WO95/10802 and WO95/10635. The pro domain likely extends from the signal peptide cleavage site to residue 321; the mature protein likely is defined by residues 322-450, where residues 322-348 define the N-terminal extension and residues 349-450 define the C-terminus. |
| "BMP-12" | |
| "CDMP-3" | |
| "GDF-8" | Refers to protein sequences encoded by the human GDF-8 gene. The cDNA and encoded amino sequence for the full length protein is provided in SEQ ID NO: 33 and in WO94/21681. The pro domain likely extends from the signal peptide cleavage site to residue 266; the mature protein likely is defined by residues 267-375, where residues 267-271 define the N-terminal extension and residues 272-375 define the C-terminus. |
| "GDF-9" | Refers to protein sequences encoded by the human GDF-9 gene. The cDNA and encoded amino sequence for the full length protein is provided in SEQ ID NO: 34 and in WO94/15966 The pro domain likely extends from the signal peptide cleavage site to residue 319; the mature protein likely is defined by residues 320-456, where residues 320-352 define the N-terminal extension and residues 353-455 define the C-terminus. |
| "GDF-10" | Refers to protein sequences encoded by the human GDF-10 gene. The cDNA and encoded amino sequence for the full length protein is provided in SEQ ID NO: 35 and in WO95/10539 The pro domain likely extends from the signal peptide cleavage site to residue 368; the mature protein likely is defined by residues 369-478, where residues 369-375 define the N-terminal extension and residues 376-478 define the C-terminus. |
| "BMP-3b" | |
| "GDF-11" | Refers to protein sequences encoded by the human GDF-11 gene. The cDNA and encoded amino sequence for the full length protein is provided in SEQ ID NO: 36 and in WO96/01845. The pro domain likely extends from the signal peptide cleavage site to residue 298; the mature protein likely is defined by residues 299-407, where residues 299-303 define the N-terminal extension and residues 304-407 define the C-terminus. |
| "GDF-12" | Refers to protein sequences encoded by the human GDF-12 gene. The cDNA and encoded amino sequence for the full length protein is provided in SEQ ID NO: 37 and in US Patent No. 5,929,213. The pro domain likely extends from the signal peptide cleavage site to residue 231; the mature protein likely is defined by residues 232-350, where residues 232-239 define the N-terminal extension and residues 240-350 define the C-terminus. |

Note that the OP-2 and OP-3 proteins have an additional cysteine residue in the C-terminal region (e.g., see residue 338 in these sequences), in addition to the conserved cysteine skeleton in common with the other proteins in this family. The GDF-1 protein has a four amino acid insert within the conserved skeleton ("Gly-Gly-Pro-Pro") but this insert likely does not interfere with the relationship of the cysteines in the folded structure. In addition, the CBMP2 proteins are missing one amino acid residue within the cysteine skeleton.

The dimeric morphogenic protein species are inactive when reduced, but are active as oxidized homodimers and when oxidized in combination with other morphogenic proteins of this invention. Thus, in one embodiment, a morphogenic protein useful in a soluble morphogenic protein complex is a dimeric protein comprising a pair of polypeptide chains, wherein each polypeptide chain has less than 200 amino acids and comprises at least the C-terminal six, preferably seven cysteine skeleton defined by residues 335-431 of OP-1 SEQ ID NO: 2, including functionally equivalent arrangements of these cysteines (e.g., amino acid insertions or deletions which alter the linear arrangement of the cysteines in the sequence but not their relationship in the folded structure), such that, when the polypeptide chains are folded, the dimeric protein species comprising the pair of polypeptide chains has the appropriate three-dimensional structure, including the appropriate intra- or inter-chain disulfide bonds such that the protein is capable of acting as a morphogen as defined herein. The solubility of these structures is improved when the mature dimeric form of a morphogen, in accordance with the invention, is complexed with at least one, and preferably two, pro domains.

Functionally equivalent sequences include functionally equivalent arrangements of cysteine residues disposed within the reference sequence, including amino acid insertions or deletions which alter the linear arrangement of these cysteines, but do not materially impair their relationship in the folded structure of the dimeric morphogenic protein, including their ability to form such intra- or inter-chain disulfide bonds as may be necessary for morphogenic activity. Functionally equivalent sequences further include those wherein one or more amino acid residues differs from the corresponding residue of a reference sequence, e.g., the C-terminal seven cysteine domain (also referred to herein as the conserved seven cysteine skeleton) of human OP-1, provided that this difference does not destroy bone morphogenic activity. Accordingly, conservative substitutions of corresponding amino acids in the reference sequence are preferred. Amino acid residues that are conservative substitutions for corresponding residues in a reference sequence are those that are physically or functionally similar to the corresponding reference residues, e.g., that have similar size, shape, electric charge, chemical properties including the ability to form covalent or hydrogen bonds, or the like. Particularly preferred conservative substitutions are those fulfilling the criteria defined for an accepted point mutation in Dayhoff *et al., supra,* the teachings of which are incorporated by reference herein.

The osteogenic protein OP-1 has been described (see, *e.g.*, Oppermann et al., U. S. Patent No. 5,354,557, incorporated herein by reference). Natural-sourced osteogenic protein in its mature, native form is a glycosylated dimer typically having an apparent molecular weight of about 30-36 kDa as determined by SDS-PAGE. When reduced, the 30 kDa protein gives rise to two glycosylated peptide subunits having apparent molecular weights of about 16 kDa and 18 kDa. In the reduced state, the protein has no detectable osteogenic activity. The unglycosylated protein, which also has osteogenic activity, has an apparent molecular weight of about 27 kDa. When reduced, the 27 kDa protein gives rise to two unglycosylated polypeptides, having molecular weights of about 14 kDa to 16 kDa, capable of inducing endochondral bone formation in a mammal. Osteogenic proteins may include forms having varying glycosylation patterns, varying N-termini, and active truncated or mutated forms of native protein. As described above, particularly useful sequences include those comprising the C-terminal 96 or 102 amino acid sequences of DPP (from Drosophila), Vg1 (from Xenopus), Vgr-1 (from mouse), the OP-1 and OP-2 proteins,(see U.S. Pat. No. 5,011,691 and Oppermann *et al.,* incorporated herein by reference), as well as the proteins referred to as BMP-2, BMP-3, BMP-4 (see WO88/00205, U.S. Patent No. 5,013,649 and WO91/18098, incorporated herein by reference), BMP-5 and BMP-6 (see WO90/11366, PCT/US90/01630, incorporated herein by reference), BMP-8 and BMP-9.

Preferred morphogenic and osteogenic proteins of this invention comprise at least one polypeptide including, but not limited to OP-1 (BMP-7), OP-2, OP-3, COP-1, COP-3, COP-4, COP-5, COP-7, COP-16, BMP-2, BMP-3, BMP-3b, BMP-4, BMP-5, BMP-6, BMP-9, BMP-10, BMP-11, BMP-12, BMP-13, BMP-14, BMP-15, BMP-16, BMP-17, BMP-18, GDF-1, GDF-2, GDF-3, GDF-5, GDF-6, GDF-7, GDF-8, GDF-9, GDF-10, GDF-11, GDF-12, MP121, CDMP-1, CDMP-2, CDMP-3, dorsalin-1, DPP, Vg-1, Vgr-1, 60A protein, NODAL, UNIVIN, SCREW, ADMP, NEURAL, and amino acid sequence variants and homologs thereof, including species homologs, thereof. More preferably, the morphogenic protein includes, but is not limited to OP-1, OP-2, OP-3, BMP-2, BMP-3, BMP-4, BMP-5, BMP-6, BMP-8, BMP-9, BMP-10, BMP-11, BMP-12, BMP-13, BMP-15, BMP-16, BMP-17, BMP-18, DPP, Vg1, Vgr, 60A protein, GDF-1, GDF-2, GDF-3, GDF-5, GDF-6, GDF-7, GDF-8, GDF-9, GDF-10, GDF-11, GDF-12, CDMP-1, CDMP-2, CDMP-3, NODAL, UNIVIN, SCREW, ADMP, NEURAL, and amino acid sequence variants thereof. Even more preferably, the morphogenic protein comprises at least one polypeptide selected from OP-1 (BMP-7), BMP-2, BMP-4, BMP-5, BMP-6, GDF-5, GDF-6, GDF-7, CDMP-1, CDMP-2 or CDMP-3; more preferably, OP-1 (BMP-7) BMP-5, BMP-6, GDF-5, GDF-6, GDF-7, CDMP-1, CDMP-2 or CDMP-3; and most preferably, OP-1 (BMP-7).

In some embodiments, the osteogenic protein comprises an amino acid sequence having at least 70% homology with the C-terminal 102-106 amino acids, including the conserved seven cysteine domain, of human OP-1, said osteogenic protein being capable of inducing repair of the cartilage defect.

Publications disclosing these sequences, as well as their chemical and physical properties, include: OP-1 and OP-2 (U.S. Patent No. 5,011,691; U.S. Patent No. 5,266,683; Ozkaynak et al., EMBO J., 9, pp. 2085-2093 (1990); OP-3 (WO94/10203 (PCT US93/10520)); BMP-2, BMP-3, BMP-4, (WO88/00205; Wozney et al. Science, 242, pp. 1528-1534 (1988)); BMP-5 and BMP-6, (Celeste et al., PNAS, 87, 9843-9847 (1991)); Vgr-1 (Lyons et al., PNAS, 86, pp. 4554-4558 (1989)); DPP (Padgett et al. Nature, 325, pp. 81-84 (1987)); Vg-1 (Weeks, Cell, 51, pp. 861-867 (1987)); BMP-9 (WO95/33830 (PCT/US95/07084); BMP-10 (WO94/26893 (PCT/US94/05290); BMP-11 (WO94/26892 (PCT/US94/05288); BMF-12 (WO95/16035 (PCT/US94/14030); BMP-13 (WO95/16035 (PCT/US94/14030); GDF-1 (WO92/00382 (PCT/US91/04096) and Lee et al. PNAS, 88, pp. 4250-4254 (1991); GDF-8 (WO94/21681 (PCT/US94/03019); GDF-9 (WO94/15966 (PCT/US94/00685); GDF-10 (WO95/10539 (PCT/US94/11440); GDF-11 (WO96/01845 (PCT/US95/08543); BMP-15 (WO96/36710 (PCT/US96/06540); MP-121 (WO96/01316 (PCT/EP95/02552); GDF-5 (CDMP-1, MP52) (WO94/15949 (PCT/US94/00657) and WO96/14335 (PCT/US94/12814) and WO93/16099 (PCT/EP93/00350)); GDF-6 (CDMP-2, BMP13) (WO95/01801 (PCT/US94/07762) and WO96/14335 and WO95/10635 (PCT/US94/14030)); GDF-7 (CDMP-3, BMP12) (WO95/10802 (PCT/US94/07799) and WO95/10635 (PCT/US94/14030)); BMP-17 and BMP-18 (U.S. Patent No. 6,027,917). The above publications are incorporated herein by reference.

The family of bone morphogenic polypeptides useful in the present invention, and members thereof, can be defined by a generic amino acid sequence. For example, Generic Sequence 7 (SEQ ID NO: 5) and Generic Sequence 8 (SEQ ID NO: 6) are 96 and 102 amino acid sequences, respectively, and accommodate the homologies shared among preferred protein family members identified to date, including at least OP-1, OP-2, OP-3, CBMP-2A, CBMP-2B, BMP-3, 60A, DPP, Vg1, BMP-5, BMP-6, Vgr-1, and GDF-1. The amino acid sequences for these proteins are described herein and/or in the art, as summarized above. The generic sequences include both the amino acid identity shared by these sequences in the C-terminal domain, defined by the six and seven cysteine skeletons (Generic Sequences 7 and 8, respectively), as well as alternative residues for the variable positions within the sequence. The generic sequences provide an appropriate cysteine skeleton where inter- or intramolecular disulfide bonds can form, and contain certain critical amino acids likely to influence the tertiary structure of the folded proteins. In addition, the generic sequences allow for an additional cysteine at position 36 (Generic Sequence 7) or position 41 (Generic Sequence 8), thereby encompassing the morphogenically active sequences of OP-2 and OP-3. Generic Sequence 7 (SEQ ID NO: 38) 
Generic Sequence 7 (SEQ ID NO: 38)
Leu Xaa Xaa Xaa Phe Xaa Xaa
1               5
Xaa Gly Trp Xaa Xaa Xaa Xaa Xaa Xaa Pro
        10                  15
Xaa Xaa Xaa Xaa Ala Xaa Tyr Cys Xaa Gly
        20                  25
Xaa Cys Xaa Xaa Pro Xaa Xaa Xaa Xaa Xaa
        30                  35
Xaa Xaa Xaa Asn His Ala Xaa Xaa Xaa Xaa
        40                  45
Xaa Xaa Xaa Xaa Xaa Xaa Xaa Xaa Xaa Xaa
        50                  55
Xaa Xaa Xaa Cys Cys Xaa Pro Xaa Xaa Xaa
        60                  65
Xaa Xaa Xaa Xaa Xaa Leu Xaa Xaa Xaa Xaa
        70                  75
Xaa Xaa Xaa Val Xaa Leu Xaa Xaa Xaa Xaa
        80                  85
Xaa Met Xaa Val Xaa Xaa Cys Xaa Cys Xaa
        90                  95
 wherein each Xaa independently is selected from a group of one or more specified amino acids defined as follows: "res." means "residue" and Xaa at res.2 = (Tyr or Lys); Xaa at res.3 = Val or Ile); Xaa at res.4 = (Ser, Asp or Glu); Xaa at res.6 = (Arg, Gln, Ser, Lys or Ala); Xaa at res.7 = (Asp or Glu); Xaa at res. 8 = (Leu, Val or Ile); Xaa at res. 11 = (Gln, Leu, Asp, His, Asn or Ser); Xaa at res.12 = (Asp, Arg, Asn or Glu); Xaa at res.13 = (Trp or Ser); Xaa at res.14 = {Ile or Val); Xaa at res. 15 = (Ile or Val); Xaa at res.16 (Ala or Ser); Xaa at res.18 = (Glu, Gln, Leu, Lys, Pro or Arg); Xaa at res.19 = (Gly or Ser); Xaa at res.20 = (Tyr or Phe); Xaa at res.21 = (Ala, Ser, Asp, Met, His, Gln, Leu or Gly); Xaa at res.23 = (Tyr, Asn or Phe); Xaa at res.26 = (Glu, His, Tyr, Asp, Gln, Ala or Ser); Xaa at res.28 = (Glu, Lys, Asp, Gln or Ala); Xaa at res.30 = (Ala, Ser, Pro, Gln, Ile or Asn); Xaa at res.31 = (Phe, Leu or Tyr); Xaa at res.33 = (Leu, Val or Met); Xaa at res.34 = (Asn, Asp, Ala, Thr or Pro); Xaa at res.35 = (Ser, Asp, Glu, Leu, Ala or Lys); Xaa at res.36 = (Tyr, Cys, His, Ser or Ile); Xaa at res.37 = (Met, Phe, Gly or Leu); Xaa at res.38 = (Asn, Ser or Lys); Xaa at res.39 = (Ala, Ser, Gly or Pro); Xaa at res.40 = (Thr, Leu or Ser); Xaa at res.44 = (Ile, Val or Thr); Xaa at res.45 = (Val, Leu, Met or Ile); Xaa at res.46 = (Gln or Arg); Xaa at res.47 = (Thr, Ala or Ser); Xaa at res.48 = (Leu or Ile); Xaa at res.49 = (Val or Met); Xaa at res.50 = (His, Asn or Arg); Xaa at res.51 = (Phe, Leu, Asn, Ser, Ala or Val); Xaa at res.52 =(Ile, Met, Asn, Ala, Val, Gly or Leu); Xaa at res.53 = (Asn, Lys, Ala, Glu, Gly or Phe); Xaa at res.54 = (Pro, Ser or Val); Xaa at res.55 = (Glu, Asp, Asn, Gly, Val, Pro or Lys); Xaa at res.56 = (Thr, Ala, Val, Lys, Asp, Tyr, Ser, Gly, Ile or His); Xaa at res.57 = (Val, Ala or Ile); Xaa at res.58 = (Pro or Asp); Xaa at res.59 = (Lys, Leu or Glu); Xaa at res.60 = (Pro, Val or Ala); Xaa at res.63 = (Ala or Val); Xaa at res.65 = (Thr, Ala or Glu); Xaa at res.66 = (Gln, Lys, Arg or Glu); Xaa at res.67 = (Leu, Met or Val); Xaa at res.68 = (Asn, Ser, Asp or Gly); Xaa at res.69 =(Ala, Pro or Ser); Xaa at res.70 = (Ile, Thr, Val or Leu); Xaa at res.71 = (Ser, Ala or Pro); Xaa at res.72 = (Val, Leu, Met or Ile); Xaa at res.74 = (Tyr or Phe); Xaa at res.75 = (Phe, Tyr, Leu or His); Xaa at res.76 = (Asp, Asn or Leu); Xaa at res.77 = (Asp, Glu, Asn, Arg or Ser); Xaa at res.78 = (Ser, Gln, Asn, Tyr or Asp); Xaa at res.79 = (Ser, Asn, Asp, Glu or Lys); Xaa at res.80 = (Asn, Thr or Lys); Xaa at res. 82 = (Ile, Val or Asn); Xaa at res.84 = (Lys or Arg); Xaa at res.85 = (Lys, Asn, Gln, His, Arg or Val); Xaa at res.86 = (Tyr, Glu or His); Xaa at res.87 = (Arg, Gln, Glu or Pro); Xaa at res.88 = (Asn, Glu, Trp or Asp); Xaa at res.90 = (Val, Thr, Ala or Ile); Xaa at res.92 = (Arg, Lys, Val, Asp, Gln or Glu); Xaa at res.93 = (Ala, Gly, Glu or Ser); Xaa at res.95 = (Gly or Ala) and Xaa at res.97 = (His or Arg).

Generic Sequence 8 (SEQ ID NO: 39) includes all of Generic Sequence 7 and in addition includes the following sequence (SEQ ID NO: 40) at its N-terminus:
SEQ ID NO: 40 
Cys Xaa Xaa Xaa Xaa
1               5
Accordingly, beginning with residue 7, each "Xaa" in Generic Sequence 8 is a specified amino acid defined as for Generic Sequence 7, with the distinction that each residue number described for Generic Sequence 7 is shifted by five in Generic Sequence 8. Thus, "Xaa at res.2 =(Tyr or Lys)" in Generic Sequence 7 refers to Xaa at res. 7 in Generic Sequence 8. In Generic Sequence 8, Xaa at res.2 = (Lys, Arg, Ala or Gln); Xaa at res.3 = (Lys, Arg or Met); Xaa at res.4 = (His, Arg or Gln); and Xaa at res. 5 = (Glu, Ser, His, Gly, Arg, Pro, Thr, or Tyr).

In another embodiment, useful osteogenic proteins include those defined by Generic Sequences 9 and 10, defined as follows.

Specifically, Generic Sequences 9 and 10 are composite amino acid sequences of the following proteins: human OP-1, human OP-2, human OP-3, human BMP-2, human BMP-3, human BMP-4, human BMP-5, human BMP-6, human BMP-8, human BMP-9, human BMP 10, human BMP-11, Drosophila 60A, Xenopus Vg-1, sea urchin UNIVIN, human CDMP-1 (mouse GDF-5), human CDMP-2 (mouse GDF-6, human BMP-13), human CDMP-3 (mouse GDF-7, human BMP-12), mouse GDF-3, human GDF-1, mouse GDF-1, chicken DORSALIN, dpp, Drosophila SCREW, mouse NODAL, mouse GDF-8, human GDF-8, mouse GDF-9, mouse GDF-10, human GDF-11, mouse GDF-11, human BMP-15, and rat BMP3b. Like Generic Sequence 7, Generic Sequence 9 is a 96 amino acid sequence that accommodates the C-terminal six cysteine skeleton and, like Generic Sequence 8, Generic Sequence 10 is a 102 amino acid sequence which accommodates the seven cysteine skeleton. 
Generic Sequence 9 (SEQ ID NO: 41)
Xaa Xaa Xaa Xaa Xaa Xaa Xaa Xaa Xaa Xaa
1               5                   10
Xaa Xaa Xaa Xaa Xaa Xaa Pro Xaa Xaa Xaa
                15                  20
Xaa Xaa Xaa Xaa Cys Xaa Gly Xaa Cys Xaa
                25                  30
Xaa Xaa Xaa Xaa Xaa Xaa Xaa Xaa Xaa Xaa
                35                  40
Xaa Xaa Xaa Xaa Xaa Xaa Xaa Xaa Xaa Xaa
                45                  50
Xaa Xaa Xaa Xaa Xaa Xaa Xaa Xaa Xaa Xaa
                55                  60
Xaa Cys Xaa Pro Xaa Xaa Xaa Xaa Xaa Xaa
                65                  70
Xaa Xaa Leu Xaa Xaa Xaa Xaa Xaa Xaa Xaa
                75                  80
Xaa Xaa Xaa Xaa Xaa Xaa Xaa Xaa Xaa Xaa
                85                  90
Xaa Xaa Xaa Cys Xaa Cys Xaa
                95
 wherein each Xaa is independently selected from a group of one or more specified amino acids defined as follows: "res." means "residue" and Xaa at res. 1 = (Phe, Leu or Glu); Xaa at res. 2 = (Tyr, Phe, His, Arg, Thr, Lys, Gln, Val or Glu); Xaa at res. 3 = (Val, Ile, Leu or Asp); Xaa at res. 4 = (Ser, Asp, Glu, Asn or Phe); Xaa at res. 5 = (Phe or Glu); Xaa at res. 6 = (Arg, Gln, Lys, Ser, Glu, Ala or Asn); Xaa at res. 7 = (Asp, Glu, Leu, Ala or Gln); Xaa at res. 8 = (Leu, Val, Met, Ile or Phe); Xaa at res. 9 = (Gly, His or Lys); Xaa at res. 10 = (Trp or Met); Xaa at res. 11 = (Gln, Leu, His, Glu, Asn, Asp, Ser or Gly); Xaa at res. 12 = (Asp, Asn, Ser, Lys, Arg, Glu or His); Xaa at res. 13 = (Trp or Ser); Xaa at res. 14 = (Ile or Val); Xaa at res. 15 = (Ile or Val); Xaa at res. 16 = (Ala, Ser, Tyr or Trp); Xaa at res. 18 = (Glu, Lys, Gln, Met, Pro, Leu, Arg, His or Lys); Xaa at res. 19 = (Gly, Glu, Asp, Lys, Ser, Gln, Arg or Phe); Xaa at res. 20 = (Tyr or Phe); Xaa at res. 21 = (Ala, Ser, Gly, Met, Gln, His, Glu, Asp, Leu, Asn, Lys or Thr); Xaa at res. 22 = (Ala or Pro); Xaa at res. 23 = (Tyr, Phe, Asn, Ala or Arg); Xaa at res. 24 = (Tyr, His, Glu, Phe or Arg); Xaa at res. 26 = (Glu, Asp, Ala, Ser, Tyr, His, Lys, Arg, Gln or Gly); Xaa at res. 28 = (Glu, Asp, Leu, Val, Lys, Gly, Thr, Ala or Gln); Xaa at res. 30 = (Ala, Ser, Ile, Asn, Pro, Glu, Asp, Phe, Gln or Leu); Xaa at res. 31= (Phe, Tyr, Leu, Asn, Gly or Arg); Xaa at res. 32 = (Pro, Ser, Ala or Val); Xaa at res. 33 = (Leu, Met, Glu, Phe or Val); Xaa at res. 34 = (Asn, Asp, Thr, Gly, Ala, Arg, Leu or Pro); Xaa at res. 35 = (Ser, Ala, Glu, Asp, Thr, Leu, Lys, Gln or His); Xaa at res. 36 = (Tyr, His, Cys, Ile, Arg, Asp, Asn, Lys, Ser, Glu or Gly); Xaa at res. 37 = (Met, Leu, Phe, Val, Gly or Tyr); Xaa at res. 38 = (Asn, Glu, Thr, Pro, Lys, His, Gly, Met, Val or Arg); Xaa at res. 39 = (Ala, Ser, Gly, Pro or Phe); Xaa at res. 40 = (Thr, Ser, Leu, Pro, His or Met); Xaa at res. 41 = (Asn, Lys, Val, Thr or Gln); Xaa at res. 42 = (His, Tyr or Lys); Xaa at res. 43 = (Ala, Thr, Leu or Tyr); Xaa at res. 44 = (Ile, Thr, Val, Phe, Tyr, Met or Pro); Xaa at res. 45 = (Val, Leu, Met, Ile or His); Xaa at res. 46 = (Gln, Arg or Thr); Xaa at res. 47 = (Thr, Ser, Ala, Asn or His); Xaa at res. 48 = (Leu, Asn or Ile); Xaa at res. 49 = (Val, Met, Leu, Pro or Ile); Xaa at res. 50 = (His, Asn, Arg, Lys, Tyr or Gln); Xaa at res. 51 = (Phe, Leu, Ser, Asn, Met, Ala, Arg, Glu, Gly or Gln); Xaa at res. 52 = (Ile, Met, Leu, Val, Lys, Gln, Ala or Tyr); Xaa at res. 53 = (Asn, Phe, Lys, Glu, Asp, Ala, Gln, Gly, Leu or Val); Xaa at res. 54 = (Pro, Asn, Ser, Val or Asp); Xaa at res. 55 = (Glu, Asp, Asn, Lys, Arg, Ser, Gly, Thr, Gln, Pro or His); Xaa at res. 56 = (Thr, His, Tyr, Ala, Ile, Lys, Asp, Ser, Gly or Arg); Xaa at res. 57 = (Val, Ile, Thr, Ala, Leu or Ser); Xaa at res. 58 = (Pro, Gly, Ser, Asp or Ala); Xaa at res. 59 = (Lys, Leu, Pro, Ala, Ser, Glu, Arg or Gly); Xaa at res. 60 = (Pro, Ala, Val, Thr or Ser); Xaa at res. 61 = (Cys, Val or Ser); Xaa at res. 63 = (Ala, Val or Thr); Xaa at res. 65 = (Thr, Ala, Glu, Val, Gly, Asp or Tyr); Xaa at res. 66 = (Gln, Lys, Glu, Arg or Val); Xaa at res. 67 = (Leu, Met, Thr or Tyr); Xaa at res. 68 = (Asn, Ser, Gly, Thr, Asp, Glu, Lys or Val); Xaa at res. 69 = (Ala, Pro, Gly or Ser); Xaa at res. 70 = (Ile, Thr, Leu or Val); Xaa at res. 71 = (Ser, Pro, Ala, Thr, Asn or Gly); Xaa at res. 2 = (Val, Ile, Leu or Met); Xaa at res. 74 = (Tyr, Phe, Arg, Thr, Tyr or Met); Xaa at res. 75 = (Phe, Tyr, His, Leu, Ile, Lys, Gln or Val); Xaa at res. 76 = (Asp, Leu, Asn or Glu); Xaa at res. 77 = (Asp, Ser, Arg, Asn, Glu, Ala, Lys, Gly or Pro); Xaa at res. 78 = (Ser, Asn, Asp, Tyr, Ala, Gly, GIn, Met, Glu, Asn or Lys); Xaa at res. 79 = (Ser, Asn, Glu, Asp, Val, Lys, Gly, Gln or Arg); Xaa at res. 80 = (Asn, Lys, Thr, Pro, Val, Ile, Arg, Ser or Gln); Xaa at res. 81 = (Val, Ile, Thr or Ala); Xaa at res. 82 = (Ile, Asn, Val, Leu, Tyr, Asp or Ala); Xaa at res. 83 = (Leu, Tyr, Lys or Ile); Xaa at res. 84 = (Lys, Arg, Asn, Tyr, Phe, Thr, Glu or Gly); Xaa at res. 85 = (Lys, Arg, His, Gln, Asn, Glu or Val); Xaa at res. 86 = (Tyr, His, Glu or Ile); Xaa at res. 87 = (Arg, Glu, Gln, Pro or Lys); Xaa at res. 88 = (Asn, Asp, Ala, Glu, Gly or Lys); Xaa at res. 89 = (Met or Ala); Xaa at res. 90 = (Val, Ile, Ala, Thr, Ser or Lys); Xaa at res 91 = (Val or Ala); Xaa at res. 92 = (Arg, Lys, Gln, Asp, Glu, Val, Ala, Ser or Thr); Xaa at res. 93 = (Ala, Ser, Glu, Gly, Arg or Thr); Xaa at res. 95 = (Gly, Ala or Thr); Xaa at res. 97 = (His, Arg, Gly, Leu or Ser). Further, after res. 53 in rBMP3b and mGDF-10 there is an Ile; after res. 54 in GDF-1 there is a T ; after res. 54 in BMP3 there is a V; after res. 78 in BMP-8 and Dorsalin there is a G; after res. 37 in hGDF-1 there is Pro, Gly, Gly, Pro.

Generic Sequence 10 (SEQ ID NO: 42) includes all of Generic Sequence 9 (SEQ ID NO: 41) and in addition includes the following sequence (SEQ ID NO: 40) at its N-terminus:
SEQ ID NO: 40 
Cys Xaa Xaa Xaa Xaa
1               5
Accordingly, beginning with residue 6, each "Xaa" in Generic Sequence 10 is a specified amino acid defined as for Generic Sequence 9, with the distinction that each residue number described for Generic Sequence 9 is shifted by five in Generic Sequence 10. Thus, "Xaa at res. 1 = (Tyr, Phe, His, Arg, Thr, Lys, Gln, Val or Glu)" in Generic Sequence 9 refers to Xaa at res. 6 in Generic Sequence 10. In Generic Sequence 10, Xaa at res. 2 = (Lys, Arg, Gln, Ser, His, Glu, Ala, or Cys); Xaa at res. 3 = (Lys, Arg, Met, Lys, Thr, Leu, Tyr, or Ala); Xaa at res. 4 = (His, Gln, Arg, Lys, Thr, Leu, Val, Pro, or Tyr); and Xaa at res. 5 = (Gln, Thr, His, Arg, Pro, Ser, Ala, GIn, Asn, Tyr, Lys, Asp, or Leu).

Non-native osteogenic proteins have been synthesized using a series of consensus DNA sequences (U.S. Patent No. 5,324,819, incorporated herein by reference). These consensus sequences were designed based on partial amino acid sequence data obtained from natural osteogenic products and on their observed homologies with other genes reported in the literature having a presumed or demonstrated developmental function.

Several of the biosynthetic consensus sequences (called consensus osteogenic proteins or "COPs") have been expressed as fusion proteins in prokaryotes (see, e.g., U.S. Pat. No. 5,011,691, incorporated herein by reference. These include COP-1, COP-3, COP-4, COP-5, COP-7 and COP-16, as well as other proteins known in the art. Purified fusion proteins may be cleaved, refolded, implanted in an established animal model and shown to have bone- and/or cartilage-inducing activity. The currently preferred synthetic osteogenic proteins comprise two synthetic amino acid sequences designated COP-5 (SEQ. ID NO: 43) and COP-7 (SEQ. ID NO: 44).

Oppermann et al., U. S. Patent Nos. 5,011,691 and 5,324,819, which are incorporated herein by reference, describe the amino acid sequences of COP-5 and COP-7 as shown below:
COP5 LYVDFS-DVGWDDWIVAPPGYQAFYCHGECPFPLAD
COP7 LYVDFS-DVGWNDWIVAPPGYHAFYCHGECPFPLAD
COP5 HFNSTN--H-AVVQTLVNSVNSKI--PKACCVPTELSA
COP7 HLNSTN--H-AVVQTLVNSVNSKI--PKACCVPTELSA
COP5 ISMLYLDENEKVVLKYNQEMVVEGCGCR
COP7 ISMLYLDENEKVVLKYNQEMVVEGCGCR

In these amino acid sequences, the dashes (-) are used as fillers only to line up comparable sequences in related proteins. Differences between the aligned amino acid sequences are highlighted.

The DNA and amino acid sequences of these and other BMP family members are published and may be used by those of skill in the art to determine whether a newly identified protein belongs to the BMP family. New BMP-related gene products are expected by analogy to possess at least one morphogenic activity and thus classified as a BMP.

In one preferred embodiment of this invention, the morphogenic protein comprises a pair of subunits disulfide bonded to produce a dimeric species, wherein at least one of the subunits comprises a recombinant peptide belonging to the BMP protein family. In another preferred embodiment of this invention, the morphogenic protein comprises a pair of subunits that produce a dimeric species formed through non-covalent interactions, wherein at least one of the subunits comprises a recombinant peptide belonging to the BMP protein family. Non-covalent interactions include Van der Waals, hydrogen bond, hydrophobic and electrostatic interactions. The dimeric species may be a homodimer or heterodimer and is capable of inducing cell proliferation and/or tissue formation.

In certain preferred embodiments, morphogenic proteins useful herein include those in which the amino acid sequences comprise a sequence sharing at least 70% amino acid sequence homology or "similarity", and preferably 75%, 80%, 85%, 90%, 95%, or 98% homology or similarity, with a reference morphogenic protein selected from the foregoing naturally occurring proteins. Preferably, the reference protein is human OP-1, and the reference sequence thereof is the C-terminal seven cysteine domain present in osteogenically active forms of human OP-1, residues 330-431 of SEQ ID NO: 2. In certain embodiments, a polypeptide suspected of being functionally equivalent to a reference morphogen polypeptide is aligned therewith using the method of Needleman, *et al., supra,* implemented conveniently by computer programs such as the Align program (DNAstar, Inc.). As noted above, internal gaps and amino acid insertions in the candidate sequence are ignored for purposes of calculating the defined relationship, conventionally expressed as a level of amino acid sequence homology or identity, between the candidate and reference sequences. "Amino acid sequence homology" is understood herein to include both amino acid sequence identity and similarity. Homologous sequences share identical and/or similar amino acid residues, where similar residues are conservation substitutions for, or "allowed point mutations" of, corresponding amino acid residues in an aligned reference sequence. Thus, a candidate polypeptide sequence that shares 70% amino acid homology with a reference sequence is one in which any 70% of the aligned residues are either identical to, or are conservative substitutions of, the corresponding residues in a reference sequence. In a currently preferred embodiment, the reference sequence is OP-1. Morphogenic proteins useful herein accordingly include allelic, phylogenetic counterpart and other variants of the preferred reference sequence, whether naturally-occurring or biosynthetically produced (*e.g.*, including "muteins" or "mutant proteins"), as well as novel members of the general morphogenic family of proteins, including those set forth and identified above. Certain particularly preferred morphogenic polypeptides share at least 60% amino acid identity with the preferred reference sequence of human OP-1, still more preferably at least 65%, 70%, 75%, 80%, 85%, 90%, 95%, or 98% amino acid identity therewith.

In another embodiment, useful osteogenic proteins include those sharing the conserved seven cysteine domain and sharing at least 70% amino acid sequence homology (similarity) within the C-terminal active domain, as defined herein. In still another embodiment, the osteogenic proteins of the invention can be defined as osteogenically active proteins having any one of the generic sequences defined herein, including OPX (SEQ ID NO: 45) and Generic Sequences 7 (SEQ ID NO: 38) and 8 (SEQ ID NO: 39), or Generic Sequences 9 (SEQ ID NO: 41) and 10 (SEQ ID NO: 42).

As noted above, certain currently preferred bone morphogenic polypeptide sequences useful in this invention have greater than 60% identity, preferably greater than 65%, 70%, 75%, 80%, 85%, 90%, 95%, or 98% identity, with the amino acid sequence defining the preferred reference sequence of hOP-1. These particularly preferred sequences include allelic and phylogenetic counterpart variants of the OP-1 and OP-2 proteins, including the Drosophila 60A protein. Accordingly, in certain particularly preferred embodiments, useful morphogenic proteins include active proteins comprising pairs of polypeptide chains within the generic amino acid sequence herein referred to as "OPX" (SEQ ID NO: 45), which defines the seven cysteine skeleton and accommodates the homologies between several identified variants of OP-1 and OP-2. As described therein, each Xaa at a given position independently is selected from the residues occurring at the corresponding position in the C-terminal sequence of mouse or human OP-1 or OP-2. 
Cys Xaa Xaa His Glu Leu Tyr Val Ser Phe Xaa Asp Leu Gly Trp Xaa Asp Trp
1               5                   10                  15
Xaa Ile Ala Pro Xaa Gly Tyr Xaa Ala Tyr Tyr Cys Glu Gly Glu Cys Xaa Phe Pro
    20                  25                  30                  35
 
Leu Xaa Ser Xaa Met Asn Ala Thr Asn His Ala Ile Xaa Gln Xaa Leu Val His Xaa
        40                  45                  50                  55
Xaa Xaa Pro Xaa Xaa Val Pro Lys Xaa Cys Cys Ala Pro Thr Xaa Leu Xaa Ala
            60                  65                  70
Xaa Ser Val Leu Tyr Xaa Asp Xaa Ser Xaa Asn Val Ile Leu Xaa Lys Xaa Arg
 75                  80                  85                  90
Asn Met Val Val Xaa Ala Cys Gly Cys His
        95                  100
 wherein Xaa at res. 2 = (Lys or Arg); Xaa at res. 3 = (Lys or Arg); Xaa at res. 11 = (Arg or Gln); Xaa at res. 16 = (Gln or Leu); Xaa at res. 19 = (Ile or Val); Xaa at res. 23 = (Glu or Gln); Xaa at res. 26 = (Ala or Ser); Xaa at res. 35 = (Ala or Ser); Xaa at res. 39 = (Asn or Asp); Xaa at res. 41 = (Tyr or Cys); Xaa at res. 50 = (Val or Leu); Xaa at res. 52 = (Ser or Thr); Xaa at res. 56 = (Phe or Leu); Xaa at res. 57 = (Ile or Met); Xaa at res. 58 = (Asn or Lys); Xaa at res. 60 = (Glu, Asp or Asn); Xaa at res. 61 = (Thr, Ala or Val); Xaa at res. 65 = (Pro or Ala); Xaa at res. 71 = (Gln or Lys); Xaa at res. 73 = (Asn or Ser); Xaa at res. 75 = (Ile or Thr); Xaa at res. 80 = (Phe or Tyr); Xaa at res. 82 = (Asp or Ser); Xaa at res. 84 = (Ser or Asn); Xaa at res. 89 = (Lys or Arg); Xaa at res. 91 = (Tyr or His); and Xaa at res. 97 = (Arg or Lys).

### II. Recombinant Production of Soluble Morphogenic Protein Complexes

Soluble morphogenic protein complexes can be produced from eukaryotic host cells, preferably mammalian cells, using standard recombinant expression techniques as described in U.S. Patent 6,395,883, incorporated herein by reference. An exemplary protocol currently preferred, is provided below, using a particular vector construct and chinese hamster ovary (CHO) cell line. Those skilled in the art will appreciate that other expression systems are contemplated to be useful, including other vectors and other cell systems, and the invention is not intended to be limited to soluble morphogenic protein complexes produced only by the method detailed hereinbelow. Similar results to those described herein have been observed using recombinant expression systems developed for COS and BSC cells.

Morphogenic protein DNA encoding the precursor sequence is subcloned into an insertion site of a suitable, commercially available pUC-type vector (e.g., pUC-19, ATCC #37254, Rockville, Md., along with a suitable promoter/enhancer sequences and 3' termination sequences. Useful DNA sequences include the published sequences encoding these proteins, and/or synthetic constructs. Currently preferred promoter/enhancer sequences are the CMV promoter (human cytomegalovirus major intermediate--early promoter) and the mouse mammary tumor virus promoter (mMTV) boosted by the rous sarcoma virus LTR enhancer sequence (e.g., from Clonetech, Inc., Palo Alto). Expression also may be further enhanced using transactivating enhancer sequences. The plasmid also contains DHFR as an amplifiable marker, under SV40 early promoter control (ATCC #37148). Transfection, cell culturing, gene amplification and protein expression conditions are standard conditions, well known in the art, such as are described, for example in Ausubel et al., ed., Current Protocols in Molecular Biology, John Wiley & Sons, NY (1989). Briefly, transfected cells are cultured in medium containing 0.1-0.5% dialyzed fetal calf serum (FCS) and stably transfected high expression cell lines are obtained by subcloning and evaluated by standard Western or Northem blot. Southern blots also are used to assess the state of integrated sequences and the extent of their copy number amplification.

A currently preferred expression vector contains the DHFR gene, under SV40 early promoter control, as both a selection marker and as an inducible gene amplifier. The DNA sequence for DHFR is well characterized in the art, and is available commercially, For example, a suitable vector may be generated from pMAM-neo (Clontech, Inc., Palo Alto, Calif.) by replacing the neo gene (BamHI digest) with an SphI-BamHI, or a PvuII-BamHI fragment from pSV5-DHFR (ATCC #37148), which contains the DHFR gene under SV40 early promoter control. A BamHI site can be engineered at the SphI or PvuII site using standard techniques (e.g., by linker insertion or site-directed mutagenesis) to allow insertion of the fragment into the vector backbone. The morphogenic protein DNA can be inserted into the polylinker site downstream of the MMTV-LTR sequence (mouse mammary tumor virus LTR). The CMV promoter sequence then may be inserted into the expression vector (e.g., from pCDM8, Invitrogen, Inc.) The SV40 early promoter, which drives DHFR expression, preferably is modified in these vectors to reduce the level of DHFR mRNA produced.

The currently preferred mammalian cell line is a CHO Chinese hamster ovary, cell line, and the preferred procedure for establishing a stable morphogenic protein production cell line with high expression levels comprises transfecting a stable CHO cell line, preferably CHO-DXB11, with the expression vector described above, isolating clones with high morphogenic protein expression levels, and subjecting these clones to cycles of subcloning using a limited dilution method described below to obtain a population of high expression clones. Subcloning preferably is performed in the absence of MTX to identify stable high expression clones which do not require addition of MTX to the growth media for morphogenic protein production.

In the subcloning protocol cells are seeded on ten 100 mm petri dishes at a cell density of either 50 or 100 cells per plate, with or preferably without MTX in the culture media. After 14 days of growth, clones are isolated using cloning cylinders and standard procedures, and cultured in 24-well plates. Clones then are screened for morphogenic protein expression by Western immunoblots using standard procedures, and Morphogenic protein expression levels compared to parental lines. Cell line stability of high expression subclones then is determined by monitoring morphogenic protein expression levels over multiple cell passages (e.g., four or five passages).

### III. Isolation of Soluble Morphogenic Protein Complex from Conditioned Media or Body Fluid

Morphogenic proteins are expressed from mammalian cells as soluble complexes. Typically, however the complex is disassociated during purification, generally by exposure to denaturants often added to the purification solutions, such as detergents, alcohols, organic solvents, chaotropic agents and compounds added to reduce the pH of the solution. Provided below is a currently preferred protocol for purifying the soluble proteins from conditioned media (or, optionally, a body fluid such as serum, cerebro-spinal or peritoneal fluid), under non-denaturing conditions. The method is rapid, reproducible and yields isolated soluble morphogenic protein complexes in substantially pure form.

Soluble morphogenic protein complexes can be isolated from conditioned media using a simple, three step chromatographic protocol performed in the absence of denaturants. The protocol involves running the media (or body fluid) over an affinity column, followed by ion exchange and gel filtration chromatographies. The affinity column described below is a Zn-IMAC column. The present protocol has general applicability to the purification of a variety of morphogenic proteins, all of which are anticipated to be isolatable using only minor modifications of the protocol described below. An alternative protocol also envisioned to have utility an immunoaffinity column, created using standard procedures and, for example, using antibody specific for a given Morphogenic protein pro domain (complexed, for example, to a protein A-conjugated Sepharose column). Protocols for developing immunoaffinity columns are well described in the art, (see, for example, Guide to Protein Purification, M. Deutscher, ed., Academic Press, San Diego, 1990, particularly sections VII and XI).

In this experiment OP-1 was expressed in CHO cells as described above. The CHO cell conditioned media containing 0.5% FBS was initially purified using Immobilized Metal-Ion Affinity Chromatography (IMAC). The soluble OP-1 complex from conditioned media binds very selectively to the Zn-IMAC resin and a high concentration of imidazole (50 mM imidazole, pH 8.0) is required for the effective elution of the bound complex. The Zn-IMAC step separates the soluble OP-1 from the bulk of the contaminating serum proteins that elute in the flow through and 35 mM imidazole wash fractions. The Zn-IMAC purified soluble OP-1 is next applied to an S-Sepharose cation-exchange column equilibrated in 20 mM NaPO₄ (pH 7.0) with 50 mM NaCl. This S-Sepharose step serves to further purify and concentrate the soluble OP-1 complex in preparation for the following gel filtration step. The protein was applied to a Sephacryl S-200HR column equilibrated in TBS. Using substantially the same protocol, soluble morphogenic proteins also may be isolated from one or more body fluids, including serum, cerebro-spinal fluid or peritoneal fluid.

IMAC was performed using Chelating-Sepharose (Pharmacia) that had been charged with three column volumes of 0.2 M ZnSO₄. The conditioned media was titrated to pH 7.0 and applied directly to the ZN-IMAC resin equilibrated in 20 mM HEPES (pH 7.0) with 500 mM NaCl. The Zn-IMAC resin was loaded with 80 mL of starting conditioned media per mL of resin. After loading the column was washed with equilibration buffer and most of the contaminating proteins were eluted with 35 mM imidazole (pH 7.0) in equilibration buffer. The soluble OP-1 complex is then eluted with 50 mM imidazole (pH 8.0) in 20 mM HEPES and 500 mM NaCl.

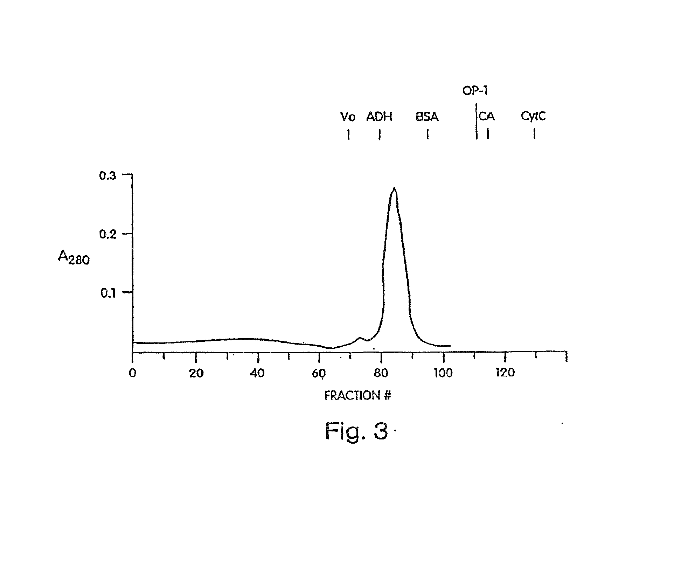

The 50 mM imidazole eluate containing the soluble OP-1 complex was diluted with nine volumes of 20 mM NaPO₄ (pH 7.0) and applied to an S-Sepharose (Pharmacia) column equilibrated in 20 mM NaPO₄ (pH 7.0) with 50 mM NaCl. The S-Sepharose resin was loaded with an equivalent of 800 mL of starting conditioned media per mL of resin. After loading the S-Sepharose column was washed with equilibration buffer and eluted with 100 mM NaCl followed by 300 mM and 500 mM NaCl in 20 mM NaPO₄ (pH 7.0). The 300 mM NaCl pool was further purified using gel filtration chromatography. Fifty mls of the 300 mm NaCl eluate was applied to a 5.0x90 cm Sephacryl S-200HR (Pharmacia) equilibrated in Tris buffered saline (TBS), 50 mM Tris, 150 mM NaCl (pH 7.4). The column was eluted at a flow rate of 5 mL/minute collecting 10 mL fractions. The apparent molecular of the soluble OP-1 was determined by comparison to protein molecular weight standards (alcohol dehydrogenase (ADH, 150 kDa), bovine serum albumin (BSA, 68 kDa), carbonic anhydrase (CA, 30 kDa) and cytochrome C (cyt C, 12.5 kDa) (see FIGURE 3). The purity of the S-200 column fractions was determined by separation on standard 15% polyacrylamide SDS gels stained with coomassie blue. The identity of the mature OP-1 and the pro-domain was determined by N-terminal sequence analysis after separation of the mature OP-1 from the pro-domain using standard reverse phase C18 HPLC.

FIGURE 3 shows the absorbance profile at 280 nm. The soluble OP-1 complex elutes with an apparent molecular weight of 110 kDa. This agrees well with the predicted composition of the soluble OP-1 complex with one mature OP-1 dimer (35-36 kDa) associated with two pro-domains (39 kDa each). Purity of the final complex can be verified by running the appropriate fraction in a reduced 15% polyacrylamide gel.

The complex components can be verified by running the complex-containing fraction from the S-200 or S-200HR columns over a reverse phase C18 HPLC column and eluting in an acetonitrile gradient (in 0.1 % TFA), using standard procedures. The complex is dissociated by this step, and the pro domain and mature species elute as separate species. These separate species then can be subjected to N-terminal sequencing using standard procedures (see, for example, Guide to Protein Purification, M. Deutscher, ed., Academic Press, San Diego, 1990, particularly pp. 602-613), and the identity of the isolated 36 kD, 39 kDa proteins confirmed as mature morphogenic protein and isolated, cleaved pro domain, respectively. N-terminal sequencing of the isolated pro domain from mammalian cell produced OP-1 revealed 2 forms of the pro region, the intact form (beginning at residue 30 of Seq. ID No. 1) and a truncated form, (beginning at residue 48 of Seq. ID No. 1). N-terminal sequencing of the polypeptide subunit of the isolated mature species reveals a range of N-termini for the mature sequence, beginning at residues 293, 300, 313, 315, 316, and 318, of SEQ ID NO: 1, all of which are active as demonstrated by the standard bone induction assay.

### V. In Vitro Soluble Morphogenic Protein Complex Formation

As an alternative to purifying soluble complexes from culture media or a body fluid, soluble complexes may be formulated from purified pro domains and mature dimeric species. Successful complex formation apparently requires association of the components under denaturing conditions sufficient to relax the folded structure of these molecules, without affecting disulfide bonds. Preferably, the denaturing conditions mimic the environment of an intracellular vesicle sufficiently such that the cleaved pro domain has an opportunity to associate with the mature dimeric species under relaxed folding conditions. The concentration of denaturant in the solution then is decreased in a controlled, preferably step-wise manner, so as to allow proper refolding of the dimer and pro regions while maintaining the association of the pro domain with the dimer. Useful denaturants include 4-6M urea or guanidine hydrochloride (GuHCl), in buffered solutions of pH 4-10, preferably pH 6-8. The soluble complex then is formed by controlled dialysis or dilution into a solution having a final denaturant concentration of less than 0.1-2M urea or GuHCl, preferably 1-2 M urea of GuHCl, which then preferably can be diluted into a physiological buffer. Protein purification/renaturing procedures and considerations are well described in the art, and details for developing a suitable renaturing protocol readily can be determined by one having ordinary skill in the art. One useful text one the subject is Guide to Protein Purification, M. Deutscher, ed., Academic Press, San Diego, 1990, particularly section V. Complex formation also may be aided by addition of one or more chaperone proteins.

### VI. Stability of Soluble Morphogenic protein Complexes

The stability of the highly purified soluble morphogenic protein complex in a physiological buffer, e.g., tris-buffered saline (TBS) and phosphate-buffered saline (PBS), can be enhanced by any of a number of means. Currently preferred is by means of a pro region that comprises at least the first 18 amino acids of the pro sequence (e.g., residues 30-47 of Seq. ID NO. 2 for OP-1), and preferably is the full length pro region. Residues 30-47 show sequence homology to the N-terminal portion of other morphogenic proteins and are believed to have particular utility in enhancing complex stability for all morphogenic proteins. Other useful means for enhancing the stability of soluble morphogenic protein complexes include three classes of additive. These additives include basic amino acids (e.g., L-arginine, lysine and betaine); nonionic detergents (e.g., Tween 80 or Nonidet P-120); and carrier proteins (e.g., serum albumin and casein). These additives include 1-100 mM, preferably 10-70 mM, including 50 mM, basic amino acid; 0.01-1.0%, preferably 0.05-0.2%, including 0.1% (v/v) nonionic detergent; and 0.01-1.0%, preferably 0.05-0.2%, including 0.1 % (w/v) carrier protein.

### VII. Activity of Soluble Morphogenic Protein Complex

Association of the pro domain with the mature dimeric species does not interfere with the morphogenic activity of the protein in vivo as demonstrated by different activity assays. Specifically, soluble OP-1 complex provided in a standard rat osteopenia model induces significant increase in bone growth and osteocalcin production (see Table 2, below), in a manner analogous to the results obtained using mature morphogenic protein.

The assay is analogous to the osteoporosis model described in U.S. Ser. No. 923,780, now abandoned in favor of continuation application Ser. No. 08/432,883 now abandoned, but uses aged female rats rather than ovariectomized animals. Briefly, young or aged female rats (Charles River Labs, 115-145, and 335-460 g body weight, respectively) were dosed daily for 7 days by intravenous tail injection, with either 20 µg/Kg body weight soluble OP-1, or 100 µg/Kg body weight soluble OP-1. Control groups of young and aged female rats were dosed only with tris-buffered saline (TBS). Water and food were provided to all animals ad libitum. After 14 days, animals were sacrificed, and new bone growth measured by standard histometric procedures. Osteocalcin concentrations in serum also were measured. No detrimental effects of morphogenic protein administration were detected as determined by changes in animal body or organ weight or by hematology profiles.

**TABLE 2**

| No. Animals | Animal Group | Bone Area (B.Ar/T. Ar) | Osteocalcin (ng/ml) |
|---|---|---|---|
| 4 | Control | 5.50 ± 0.64 | 11.89 ± 4.20 |
| 5 | Aged female,20 µg/Kg sol. OP-1 | 7.68 ± 0.63** | 22.24 ± 2.28** |
| 5 | Aged female, 100 µg/Kg sol. OP-1 | 9.82 ± 3.31* | 20.87 ± 6.14* |

| | | | |
|---|---|---|---|
| *P < 0.05 **P < 0.01 | | | |

Similar experiments performed using soluble OP-1 complex in the osteoporosis model described in U.S. Ser. No. 923,780, now abandoned in favor of continuation application Ser. No. 08/432,883 now abandoned and incorporated hereinabove by reference using ovariectomized rats also show no detrimental effect using the complex form.

Both mature and soluble morphogenic protein also can induce CAM (cell adhesion molecule) expression, as described in copending U.S. Ser. No. 07/922,813, filed Jul. 31, 1992, now abandoned in favor of continuation application Ser. No. 08/260,675 pending the disclosure of which is incorporated hereinabove by reference.

Briefly, and as described therein, induction ofN-CAM isoforms (N-CAM-180, N-CAM-140 and N-CAM-120) can be monitored by reaction with the commercially available antibody mAb H28.123 (Sigma Co., St. Louis) and standard Western blot analysis (see, for example, Molecular Cloning, A Laboratory Manual, Sambrook et al. eds. Cold Spring Harbor Press, New York, 1989, particularly Section 18). Incubation of a growing culture of transformed cells of neuronal origin, NG148-15 cells (ATCC, Rockville, Md.) with either mature morphogenic protein dimers or soluble morphogenic protein complexes (10-100 ng/ml, preferably at least 40 ng/ml) induces a redifferentiation of these cells back to a morphology characteristic of untransformed neurons, including specific induction and/or enhanced expression of all 3 N-CAM isoforms. In the experiment, cells were subcultured on poly-L-lysine coated 6-well plates and grown in chemically defined medium for 2 days before the experiment. Fresh aliquots of morphogenic protein were added (2.5 µl) daily.

### Pharmaceutical Compositions

The pharmaceutical compositions comprising a soluble morphogenic protein complex may be in a variety of forms. These include, for example, solid, semisolid and liquid dosage forms such as powders, tablets, pills, suppositories, liquid solutions, suspensions, gels, putty, pastes, emulsions and infusible solutions. The preferred form depends on the intended mode of administration and the therapeutic application and may be selected by one skilled in the art. Modes of administration may include oral, parenteral, intramuscular, intraperitoneal, intra-articular, subcutaneous, intravenous, intralesional, surgical implantation or topical administration. The compositions may be formulated in dosage forms appropriate for each route of administration. In some embodiments, the pharmaceutical compositions of this invention will be administered into the site (i.e., directly into the cartilage) in need of tissue regeneration or repair. In other embodiments, the pharmaceutical compositions of this invention will be administered in the vicinity of the site in need of tissue regeneration or repair. For example, in some embodiments, the pharmaceutical compositions of this invention may be administered into the area surrounding the cartilage (e.g., the synovial fluid) in need of repair (i.e. a joint). In other embodiments, the pharmaceutical compositions of this invention may be administered directly into the cartilage tissue (e.g., a meniscus or an intervertebral disc).

In a preferred embodiment, the soluble morphogenic protein complex composition is administered directly into the site of cartilage damage or degradation, such as an articular joint or joint capsule, in an acceptable fluid carrier. The composition can be administered once or multiple times, with an administration frequency, as is known in the art, that optimizes tissue healing or repair. The composition can, for example, be administered daily, weekly, monthly, semimonthly, bimonthly, quarterly, biyearly or yearly.

The pharmaceutical compositions comprising a soluble morphogenic protein complex may, for example, be placed into sterile, isotonic formulations with or without cofactors which stimulate uptake or stability. The formulation is preferably liquid, or may be lyophilized powder. For example, the soluble morphogenic protein complex may be diluted with a formulation buffer. The solution can be lyophilized, stored under refrigeration and reconstituted prior to administration with sterile Water-For-Injection (USP).

The compositions also will preferably include conventional pharmaceutically acceptable carriers well known in the art (see, e.g., Remington's Pharmaceutical Sciences, 16th Ed., Mac Publishing Company (1980)). Such pharmaceutically acceptable carriers may include other medicinal agents, carriers, genetic carriers, adjuvants, excipients, etc., such as human serum albumin or plasma preparations. Preferably, the carrier is isotonic with the blood or synovial fluid of the patient. Examples of such carrier vehicles include water, saline, Ringer's solution, buffered aqueous solutions, hyaluronan, hyaluronic acid and dextrose solution. Non-aqueous vehicles such as fixed oils and ethyl oleate are also useful herein. The compositions are preferably in the form of a unit dose and will usually be administered as a dose regimen that depends on the particular tissue treatment.

In some embodiments, the compositions of this invention are sustained release formulations, slow delivery formulations, or formulations whereby the soluble morphogenic protein complex clearance is delayed. There are numerous delivery materials available for preparing these compositions. They include, but are not limited to, microspheres of polylactic/polyglycolic acid polymers, liposomes, collagen, polyethylene glycol (PEG), hyaluronic acid/fibrin matrices, hyaluronic acid, fibrin, chitosan, gelatin, SABER™ System (sucrose acetate isobutyrate (SAIB)), DURIN™ (biodegradabale polymer for drug loaded implants), MIGRODUR™ (biodegradable polymers/microencapsulation) and DUROS™ (mini-osmotic pump). In some embodiments, the soluble morphogenic protein complex is covalently linked to the delivery material.

The compositions of this invention comprise a soluble morphogenic protein complex dispersed in a biocompatible carrier material that functions as a suitable delivery system for the.compounds. Suitable examples of sustained release carriers include semipermeable polymer matrices. Implantable or microcapsular sustained release matrices include polylactides (U.S. Patent No. 3,773,319; EP 58,481), copolymers of L-glutamic acid and gamma-ethyl-L-glutamate (Sidman et al., Biopolymers, 22, pp. 547-56 (1985)); poly(2-hydroxyethyl-methacrylate), ethylene vinyl acetate (Langer et al., J. Biomed. Mater. Res., 15, pp. 167-277 (1981); Langer, Chem. Tech., 12, pp. 98-105 (1982)) orpoly-D-(-)-3hydroxybutyric acid (EP 133,988), polylactic acid, poly glycolic acid or polymers of the above.

The pharmaceutical compositions of this invention may also be administered using, for example, microspheres, liposomes, other microparticulate delivery systems or sustained release formulations placed in, near, or otherwise in communication with affected tissues, the fluids bathing those tissues (e.g., synovial fluid) or bloodstream bathing those tissues.

Liposomes containing a soluble morphogenic protein complex of this invention can be prepared by well-known methods (See, e.g. DE 3,218,121; Epstein et al., Proc. Natl. Acad. Sci. U.S.A., 82, pp. 3688-92 (1985); Hwang et al., Proc. Natl. Acad. Sci. U.S.A., 77, pp. 4030-34 (1980); U.S. Patent Nos. 4,485,045 and 4,544,545). Ordinarily the liposomes are of the small (about 200-800 Angstroms) unilamellar type in which the lipid content is greater than about 30 mol.% cholesterol. The proportion of cholesterol is selected to control the optimal rate of soluble morphogenic protein complex release.

The soluble morphogenic protein complexes of this invention may also be attached to liposomes containing other biologically active molecules such as immunosuppressive agents, cytokines, etc., to modulate the rate and characteristics of tissue induction. Attachment of soluble morphogenic protein complexes to liposomes may be accomplished by any known cross-linking agent such as heterobifunctional cross-linking agents that have been widely used to couple toxins or chemotherapeutic agents to antibodies for targeted delivery. Conjugation to liposomes can also be accomplished using the carbohydrate-directed cross-linking reagent 4-(4-maleimidophenyl) butyric acid hydrazide (MPBH) (Duzgunes et al., J. Cell. Biochem. Abst. Suppl. 16E 77 (1992)).

The soluble morphogenic protein complexes of this invention may also be glycosylated. Glycosylation is the modification of a protein by addition of one or more oligosaccharide groups. There are usually two types of glycosylation: O-linked oligosaccharides are attached to serine or threonine residues while N-linked oligosaccharides are attached to asparagine residues when they are part of the sequence Asn-X-Ser/Thr, where X can be any amino acid except proline. Glycosylation can dramatically affect the physical properties of proteins and can also be important in protein stability, secretion, half-life, and subcellular localization. In some embodiments, the soluble morphogenic protein complexes of the present invention comprise N-linked oligosaccharaides. In other embodiments, the soluble morphogenic protein complexes of this invention comprise O-linked oligosaccharides. In yet other embodiments, the soluble morphogenic protein complexes of this inventions comprise both N-linked and O-linked oligosaccharides. In some embodiments, the glycosylation pattern of the soluble morphogenic protein complex may be modified to control the carbohydrate composition of the glycoprotein.

One skilled in the art may create a biocompatible, and or biodegradable formulation of choice to promote tissue induction.

A successful carrier for soluble morphogenic protein complexes should perform several important functions. It should act as a slow release delivery system of soluble morphogenic protein complex or delay clearance of the soluble morphogenic protein complex, and protect the soluble morphogenic protein complex from non-specific proteolysis.

In addition, selected materials must be biocompatible *in vivo* and preferably biodegradable. Polylactic acid (PLA), polyglycolic acid (PGA), and various combinations have different dissolution rates *in vivo.*

The carrier may also take the form of a hydrogel. When the carrier material comprises a hydrogel, it refers to a three dimensional network of crosslinked hydrophilic polymers in the form of a gel substantially composed of water, preferably but not limited to gels being greater than 90% water. Hydrogel can carry a net positive or net negative charge, or may be neutral. A typical net negative charged hydrogel is alginate. Hydrogels carrying a net positive charge may be typified by extracellular matrix components such as collagen and laminin. Examples of commercially available extracellular matrix components include Matrigel™ and Vitrogen™. An example of a net neutral hydrogel is highly crosslinked polyethylene oxide, or polyvinyalcohol.

Various growth factors, cytokines, hormones, trophic agents and therapeutic compositions including antibiotics and chemotherapeutic agents, enzymes, enzyme inhibitors and other bioactive agents also may be adsorbed onto or dispersed within the carrier material comprising the soluble morphogenic protein complex, and will also be released over time and is slowly absorbed.

Dosage levels of between about 1 µg and about 1000 µg per day, preferably between 3 µg and 50 µg per day of the soluble morphogenic protein complex are useful in cartilage repair and regeneration. As the skilled artisan will appreciate, lower or higher doses than those recited may be required. Specific dosage and treatment regimens for any particular patient will depend upon a variety of factors, including the activity of the specific soluble morphogenic protein complex employed, the age, body weight, general health status, sex, diet, time of administration, rate of excretion, drug combination, the severity of the tissue damage and the judgment of the treating physician.

### EXAMPLE 1: Dog Model Repair of Osteochondral Defects

12 adult male bred for purpose dogs will undergo surgery. Both hindlimbs will be prepped and draped in sterile fashion. A medial parapatellar incision approximately four centimeters in length will be made. The patella will be retracted laterally to expose the femoral condyle. In the right medial condyle, a 5.0 mm diameter defect extending through the cartilage layer and penetrating the subchondral bone to a depth of 6 mm will be created in the central load bearing region of the femoral condyle with a specially designed or modified 5.0 mm drill bit. The animals will be divided into two groups of 6 animals each. In the first group, after copious irrigation with saline to remove debris and spilled marrow cells, the appropriate time release soluble OP-1 complex will be applied to the synovial fluid surrounding the defect. In the first group of 6 animals, the right defects will receive the time release soluble OP-1 complex. The left limb of all animals will serve as a control receiving control beads (0% OP-1).

The second group of 6 animals will receive no OP-1 treatment at the time of surgery. At 3 days post surgery, the appropriate time release soluble OP-1 complex formulation will be injected into the synovial fluid surrounding the joint with the defect. In 6 animals, time release soluble OP-1 complex will be injected into the synovial fluid around the right defect. The left limb of all animals will serve as a control receiving control beads (0% OP-1).

The animals will be sacrificed at 16 weeks post-surgery. At sacrifice, the distal femurs will be retrieved *en bloc* and the defect sites will be evaluated histologically and grossly based on the scheme of Moran et al (J. Bone Joint Surg. 74B:659-667, 1992) that has been used in previous investigations.

Radiographs of the hindlimbs will be obtained preoperatively, immediately postoperative, and at postoperative week 16. The preoperative radiographs will be used to assure that no pre-existing abnormalities are present and to verify skeletal maturity. Post-operative radiographs will be used to assess defect placement. Sacrifice radiographs will be used to assess the rate of healing and restoration of the subchondral bone and the articulating surface. Radiographs will be obtained within one week of the evaluation date.

Gross pathological examination of the carcasses will be conducted immediately after sacrifice. The distal femurs will be immediately harvested en *bloc* and stored in saline soaked towels and placed in labeled plastic bags. High power photographs of the defect sites will be taken and carefully labeled.

Soft tissues will be meticulously dissected away from the defect site and the proximal end of the femur will be removed. On a water cooled diamond cut saw each defect site will be isolated for histologic evaluation.

Specimens will be fixed by immersion in 4% paraformaldehyde solution and prepared for decalcified histologic processing. Three sections from three levels will be cut from each block. Levels 1 and 3 will be closest to the defect perimeter. Level 2 will be located at the defect center. Three sections from each level will be stained with toluidine blue and Safranin O and fast green. Sections will be graded based on the scheme of Moran et al. (J. Bone Joint Surg. 74B:659-667, 1992).

### EXAMPLE 2: Sheep Model of Regeneration of Chondral Defects By Intra-articular Administration af OP-1 in Time-Release Microspheres

18 adult bred for purpose sheep will undergo surgery. With a specially designed instrument, a 10mm chondral defect will be created in the left hindlimb knee of 18 sheep on the weight bearing condyle surface, 2mm deep up to the calcified layer (exposition of blood will be pronounced as a failure). The right knees of all animals will remain untouched to serve as a control.

Group 1 (6 animals): At postoperative day 3, the left knee of each animal will receive an intra-articular injection of a 250µl suspension containing 57mg of control 0.3% microspheres without soluble OP-1 complex.

Group 2 (6 animals): At postoperative day 3, the left knee of each animal will receive an intra-articular injection of a 250µl suspension containing 57mg of 0.3% microspheres containing 170µg of soluble OP-1 complex.

Group 3 (6 animals): At postoperative day 3 and at postoperative week 6, the left knee of each animal will receive an intra-articular injection of a 250µl suspension containing 57mg of 0.3% microspheres containing 170µg of soluble OP-1 complex.

Arthroscopic evaluation of the knees will be performed at postoperative weeks 3 and 6 on all the animals. NMR/MRI scans will be performed at postoperative week 3 and 6. Mechanical testing of the knees will also be performed periodically.

All animals will be sacrificed at 3 months postoperative. After sacrifice, histology, histomorphometry, immunostaining, and in situ hybridization for specific articular chondrocyte markers will be performed.

### EXAMPLE 3: Sheep Model for Prevention of Osteoarthritis

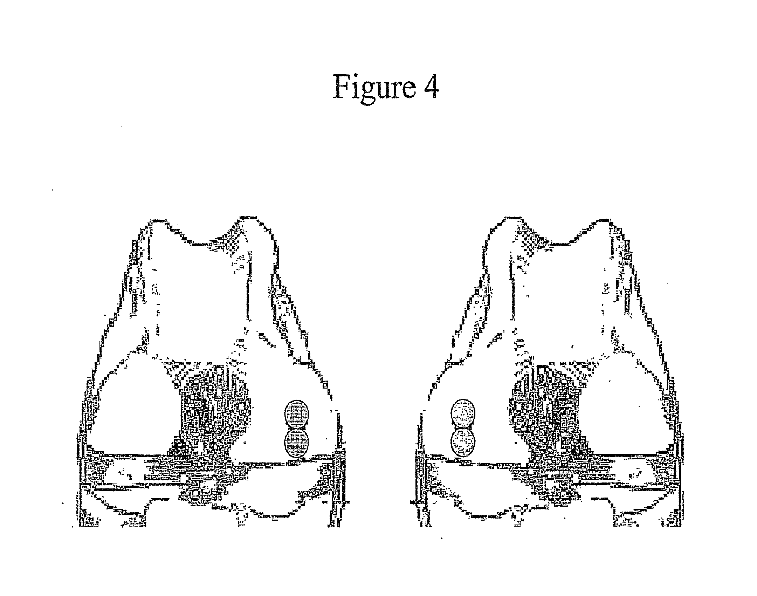

Sheep are used as a model for osteoarthritis because it has been demonstrated that progressive osteoarthritis occurs in these animals after a single injury impact. Twelve adult female crossbred sheep that are acclimatized for 14 days will be used in this study. All sheep will receive general anesthesia and using aseptic techniques, a 3 cm arthrotomy will be used to allow access to both femorotibial joints. A spring loaded mechanical device will be used to create bilateral impact injuries to the weight bearing region of the median femoral condyle (30 Mpa, 6mm diameter x 2) (see FIGURE 4). After a routine closure of these incisions, the sheep will receive an intra-articular injection in each knee of soluble OP-1 complex in a vehicle or vehicle alone. Two experimental groups (N=6) will be used. Group A will received 0.3 ml of soluble OP-1 complex intra-articularly in one knee at the time of surgery (day 0) and one week later (day 7). Day 0 injections were administered immediately after the surgical incision is closed. Group B will receive soluble OP-1 complex in one knee on day 0, 7, 14, 21, 28, and 35. Synovial fluid will be aspirated before injection of the soluble OP-1 complex and vehicle to allow measurement of leukocyte numbers and total protein as indicators of inflammation.

The sheep will be sacrificed 12 weeks postoperatively for detailed assessment (paravital staining, TUNEL staining, histopathology, cartilage, sulfated GAG analysis, biomechanical indentation testing) of the articular tissues.

### EXAMPLE 4: Sheep Model for Therapeutic Effect of OP-1 after Intra-Articular Injection

This study will use N=12 adult female 1.5-2.5 year old crossbred sheep that are acclimatized for 14 days and pass a health status assessment before entry into the study. Under general anesthesia and using aseptic technique, all sheep will receive standardized 30 MPa impact injuries to both (left and right) medial femoral condyles by a 3 cm minimally invasive arthrotomy. Three weeks postoperatively the sheep will be sedated with diazepam (10mg/kg) and ketamine (3-5mg/kg) to allow aseptic preparation of knee for synoviocentesis and injection of test article, placebo or physiologic saline into the medial femorotibial joint according the to Table 3.

**Table 3**

| | | **Week** | | **0** | **3** | **4** | **8** | **12** | **16** |
|---|---|---|---|---|---|---|---|---|---|
| **Dose** | **Group** | # **animals** | **# Knees** | **Surgery** | **intra-articular injection** | | | | |
| two doses 3 & 4 weeks post injury | Test-L | 9 | 9 | Impact Injury | Sol OP-1 Cmplx /P | Sol OP-1 Cmplx /P | | | sacrifice |
| | Placebo-R | | 9 | | Placebo | Placebo | | | |
| saline controls | Saline control-R | 3 | 6 | Impact Injury | Saline | Saline | | | sacrifice |
| | Saline control-L | | | | None | None | | | |
| **Total animals in study** | | **12** | | | | | | | |
| Synovial Fluid Aspirate | | | | x | x | x | x | x | x |

All sheep will receive bilateral medial femoral condyle injuries. In the first group of nine sheep, one knee will receive the test article and the contralateral knee will receive a placebo consisting of the vehicle alone. Knee treatments will be allocated by a complete block design. A second group of three sheep will receive physiologic saline USP as a control for the effect of the placebo.

The study will follow the following procedure set forth in Table 4:

**Table 4**

| | |
|---|---|
| Day -14 to day -1 | Preconditioning, health maintenance program, foot trimming, Q-fever test |
| Week 0 | Surgery and impact injury to both knees of sheep. |
| Week 3, 4 | Synovial fluid collection. |
| | Synovial fluid harvested and soluble OP-1 complex and placebo injected into respective joints. |
| Week 8, 12 | Synovial fluid harvested using aseptic technique and sedation. Freeze 2 aliquots synovial fluid (200 uL each) and process one fresh EDTA aliquot for total leukocyte count, differential counts and total protein determination. |
| Week 16 | Sacrifice all sheep. Harvest synovial fluid and tissues for detailed assessments |

### EXAMPLE 5: Guinea Pig and Rabbit Models of Osteoarthritis

The Hartley guinea pig (spontaneous) and rabbit ACL-resection (induced) osteoarthritis models will be utilized. Fourteen guinea pigs of either 3, 6 or 9 months of age will be injected in the right knee with a phosphate buffered saline (PBS) solution containing 50 µg soluble OP-1 complex at 3-week intervals for a period of 12 weeks. The left knee will serve as an untreated control.

In ten New Zealand White rabbits, the left ACL will be resected and receive either an injection into the joint of 100 µg soluble OP-1 complex in a PBS solution or a control solution at 3-week intervals during a 12-week evaluation period. The right knee will serve as a non ACL- resected nontreated control in all animals.

All animals in both models will be evaluated for gross appearance and histologic evidence of arthritic changes using a modified Mankin scale to grade the severity of degeneration.

### EXAMPLE 6: Sheep Model of Meniscus Healing

A hole (6mm diameter) and a longitudinal tear (2cm long) sutured by non-resorbable thread will be created in each medial meniscus of both knees of sheep. There will be two treatment groups: soluble OP-1 complex and a control group with no treatment other than the surgically created defect. In the treatment group, the soluble OP-1 complex will be injected into the joint space just prior to closing the incision and will then be injected into the joint space 7 days after surgery.

6, 12 and 26 weeks after treatment, the animals will be euthanized. After euthanasia, the meniscus will be removed and cut in two parts, the anterior, longitudinal sutured tear and the posterior, with the hole. The sections will be stained with Masson's Trichrome and safranin O. Immunohistochemistry of the meniscus may also be performed using specific antibodies to detect collagen I, II, VI, S 100, proteases MMP 1.

A section of meniscus will be separated, embedded in OCT and frozen in liquid nitrogen. Sections obtained with a cryostat will be collected, homogenized and RNA prepared using Trizol reagent. RT-PCR will be performed to study gene expression of various markers including type I, type II, type II collagen and aggrecan as markers for extracellular matrix, TGF-β and IGF-2 as growth factors, MMP-1, MMP-3 and TIMP-1 as matrix degrading enzymes, and finally cyclin A, Bel-2, BAX and caspase 3 as markers for proliferating and apoptotic state of cells. Other joint tissue will also be inspected and compared to controls for any gross differences which may be caused by soluble OP-1 complex.

### EXAMPLE 7: Sheep Model of Disc Repair and Regeneration

Experimental induction of controlled outer annular defects in sheep initiates a sequence of events which closely reproduces, pathologically and biochemically, the evolution of disc degeneration in man. Compositional changes include an alteration in the amount of, and the types of collagens synthesized by cells of the lesion site (Kaapa et al 1994a, b, 1995, Kaapa E. et at (1995) Collagen synthesis and types I, III, IV, and VI collagens in an animal model of disc degeneration, Spine 20, 59-67; Kaapa E et al., (1994) Collagens in the injured porcine intervertebral disc, J. Orthop. Res. 12. 93-102; and Kaapa E et al., (1994) Proteoglycan chemistry in experimentally injured porcine intervertebral disk, J. Spin. Dis. 7, 296-306) loss of large high buoyant density aggrecan type proteoglycans and an elevation in levels of the small DS substituted proteoglycans decorin and biglycan in the injured disc (Melrose J. et al, (1992) A longitudinal study of the matrix changes induced in the intervertebral disc by surgical damage to the annulus fibrosus, J Orthop Res 10:665-676; Melrose J. et al., (1997) Topographical variation in the catabolism of aggrecan in an ovine annular lesion model of experimental disc degeneration J Spinal Disord 10:55-67; and Melrose J. et al., (1997) Elevated synthesis of biglycan and decorin in an ovine annular lesion model of experimental disc degeneration, Eur Spine J 6:376-84). Changes in the vascular supply to the cartilaginous end plate (CEP) (Moore RJ et at., (1992) Changes in endplate vascularity after an outer anulus tear in the sheep, Spine 17:874-878) and remodelling of vertebral bone adjacent to experimental annular defects (Moore RJ, et al. (1996) Remodeling of vertebral bone after outer anular injury in sheep, Spine 21:936-940.), changes in the biomechanical competence of "repaired" lesion affected discs (Latham JM et al., (1994) Mechanical consequences of annular tears and subsequent intervertebral disc degeneration, J Clin Biomech 9:211-9), and osteoarthritic changes in spinal facet joints (Moore RJ et al., (1999) Osteoarthrosis of the facet joints resulting from anular rim lesions in sheep lumbar discs, Spine, 24:519-525) as a consequence of disc degeneration have also been noted.

### A. The ovine annular lesion model

The sheep will be fasted for 24 h prior to surgery and anaesthesia will be induced with an intravenous injection of Ig thiopentone. A lateral plain X-ray film will be taken to verify normal lumbar spine anatomy. General anaesthesia will be maintained after endotracheal intubation by 2.5% halothane and monitored by pulse oximetry and end tidal CO₂ measurement. The left flank from the ribs to the iliac crest will be prepared for sterile surgery. The sheep will receive an intramuscular injection of 1200 mg penicillin. A skin incision will be made on the left side immediately anterior to the transverse processes of the spine and the lumbar spine will be exposed by blunt dissection using an anterior muscle-splitting technique. The vascular and neural anatomy will be respected and bleeding will be controlled by direct pressure or electrocautery as required.

A total of twelve two year old sheep will receive controlled annular lesions in their L1-L2, L3-L4 and L5-L6 discs by incision through the left anterolateral annulus fibrosus parallel and adjacent to the cranial endplate using a #11 scalpel blade to create a lesion measuring 4 mm long x 5 mm deep. The intervening lumbar discs (L2-L3, L4-L5) will not be incised.

The incised discs will receive one of 3 therapies, (I) no treatment, (II) vehicle or (III) soluble OP-1 complex in vehicle. In all sheep the L3-L4 disc will receive an annular lesion with no treatment. In 4 sheep the L1-L2 discs will be treated with vehicle only and the L5-L6 disc will be treated with soluble OP-1 complex plus vehicle. In the remaining 4 sheep the treatments in the L1-L2 and L5-L6 discs will be reversed to avoid any potential outcome bias associated with spinal level. A non-operated disc must remain between treated discs to allow for adequate anchorage of FSUs in subsequent mechanical testing (see below). A wire suture will be used to identify the craniad operated level for later identification purposes both in X-rays and for morphological identification. Three additional non-operated animals will also be used as controls for the biomechanical study.

Degeneration following annular incision is well established in the sheep (Osti OL et al., (1990) Volvo Award for Basic Science, Annulus tears and intervertebral disc degeneration. An experimental study using an animal model, Spine 15:762-7) and can be expected to show the earliest radiographic and histochemical evidence after 12 weeks. Three months after induction of the annular lesions the sheep will be killed by intravenous injection of 6.5 g sodium pentobarbitone and the lumbar spines will be radiographed to evaluate disc calcification, excised and processed for biomechanical (n=8) and histochemical (n=4) analyses, and, after the biomechanical testing the same discs will be zonally dissected for compositional analyses.

### B. Compositional analysis of disc tissues

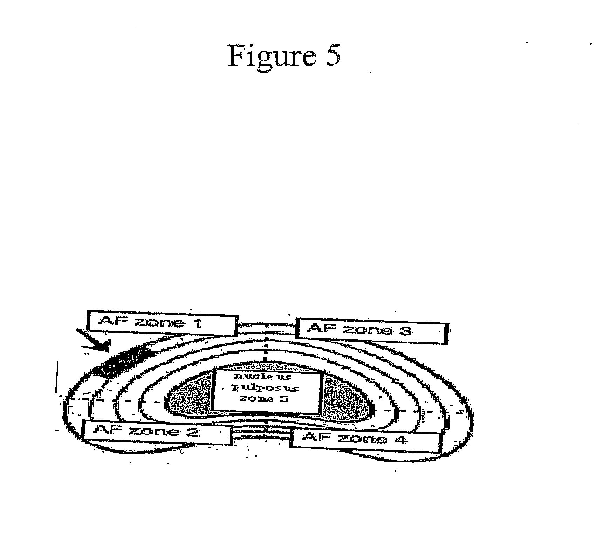

Intervertebral disc tissues will be zonally dissected into annular quadrants and nucleus pulposus as depicted in FIGURE 5.

### C. Determination of Proteoglycan and Collagen Contents of Disc Tissues

Samples of annulus fibrosus and nucleus pulposus will be finely diced over ice and representative portions of each tissue zone of known wet weight will be freeze dried to constant weight. The difference between the starting and final weights of the tissues will provide their water contents. Triplicate portions (1-2 mg) of the dried tissues will be hydrolyzed in 6M HCl at 110°C for 16 h and aliquots of the neutralized digests assayed for hydroxyproline as a measure of the tissue collagen content (Melrose J et al., (1992) A longitudinal study of the matrix changes induced in the intervertebral disc by surgical damage to the annulus fibrous, J Orthop Res 10:665-676; Melrose J et al., (1994a) Proteoglycan heterogeneity in the normal adult ovine intervertebral disc, Matrix 14:61-75; Melrose J et al., (1994b) Variation in the composition of the ovine intervertebral disc with spinal level and in its constituent proteoglycans, Vet Comp Orthop Traum 7:70-76; Melrose J et al., (1991) The influence of scoliosis and ageing on proteoglycan heterogeneity in the human intervertebral disc J Orthop Res 9:68-77; and Melrose J et al., (1996) Intervertebral disc reconstitution after chemonucleolysis with chymopapain is dependent on dosage: an experimental study in beagle dogs Spine 21:9-17). Triplicate portions of dried tissues (∼2 mg) will also be digested with papain and aliquots of the solubilized tissue assayed for sulphated glycosaminoglycan using the metachromatic dye 1, 9-dimethylmethylene blue as a measure of tissue proteoglycan (see Melrose et al 1991, 1992, 1994, 1996, supra).

### D. Histochemical and Immunohistochemical Analyses

Spinal motion segments that are designated for histochemical analysis will be isolated by cutting through the cranial and caudal vertebral bodies close to the cartilaginous endplates using a bone saw. Entire disc specimens including the adjacent vertebral body segments will be fixed en bloc in either 10% neutral buffered formalin or Histochoice^{®} for 56 h and decalcified in several changes of 10% formic acid in 5% NBF for 2 weeks with constant agitation until complete decalcification is confirmed using a Faxitron HP43855A X-ray cabinet (Hewlett Packard, McMinnville, USA).

Sagittal slices (5 mm thick) of the decalcified disc-vertebral body specimens will be dehydrated through graded ethanol solutions by standard histological methods and embedded in paraffin wax. Paraffin sections 4 µm thick will be prepared for histochemical staining and mounted on Superfrost Plus glass microscope slides (Menzel-Glaser) and dried at 85°C for 30 min then at 55°C overnight. The sections will be deparaffinized in xylene (4 changes x 2 min) and rehydrated through graded ethanol washes (100-70% v/v) to water.

Three sections from all blocks will be stained with haematoxylin and eosin. These sections will be coded and examined by an independent histopathologist who will compare the histological characteristics of those levels that receive annular incision only with those that are incised and receive soluble OP-1 complex. A four-point semi-quantitative grading system will be used to assess the microscopic features. Collagen architecture will also be examined in sections stained with Masson's trichrome and picro-sirius red using polarized light microscopy.

The immunohistochemistry procedures will be performed using a Sequenza cassette and disposable Coverplate immunostaining system as described earlier (Melrose J et al., (2002) Perlecan, the Multi-domain Proteoglycan of Basement Membrane is also a Prominent Pericellular Component of Hypertrophic Chondrocytes of Ovine Vertebral Growth Plate and Cartilaginous End Plate Cartilage, Histochem. Cell Biol. 118, 269-280; Melrose J et al., (2002) Increased nerve and blood-vessel in-growth associated with proteoglycan depletion in an ovine annular lesion model of experimental disc degeneration, Spine 27, 1278-85; Melrose J et al., (2002) Comparison of the morphology and growth characteristics of intervertebral disc cells, synovial fibroblasts and articular chondrocytes in monolayer and alginate bead cultures, Eur. Spine J. 12, 57-65; Melrose J et at. (2001) Differential expression of proteoglycan epitopes and growth characteristics of ovine intervertebral disc cells grown in alginate beads, Cells Tissues Organs 168:137-146; Melrose J et al., (2003) Perlecan, the multi domain HS-proteoglycan af basement membranes is a prominent extracellular and pericellular component of the cartilaginous vertebral body rudiments, vertebral growth plates and intervertebral discs of the developing human spinal column, J Histochem Cytochem 51:1331-1341; Melrose J et al., (2000) Differential Expression of Proteoglycan epitopes by ovine intervertebral disc cells grown in alginate bead culture, J. Anat. 197:189-198; Melrose J et al., (2002) Spatial and Temporal Localisation of Transforming Growth Factor-β, Fibroblast Growth Factor-2, Osteonectin and Identification of Cells Expressing α-Smooth Muscle Actin in the Injured Annulus Fibrosus: Implications for Extracellular Matrix Repair, Spine 27:1756-1764; and Knox S et al., (2002) Not all perlecans are created equal : interactions with fibroblast growth factor-2 (FGF-2) and FGF receptors, J. Biol. Chem. 277:14657-14665). Endogenous peroxidase activity will be initially blocked by incubating the tissue sections with 3% H₂O₂. This will be followed by pre-digestion of the tissue sections with combinations af chondraitinase ABC (0.25 U/ml) in 20 mM Tris-acetate buffer pH 8.0 for 1 h at 37°C, bovine testicular hyaluronidase 1000 U/ml for 1 h at 37°C in phosphate buffer pH 5.0, followed by three washes in 20 mM Tris-HCl pH 7.2 0.5M NaCl (TBS) or proteinase-K (DAKO S3020) for 6 min at room temperature to expose antigenic epitopes. The tissues will then be blocked for 1 h in 20% normal swine serum and be probed with a number of primary antibodies to large and small proteoglycans and collagens (Table 5). Negative control sections will also be processed either omitting primary antibody or substituting an irrelevant isotype matched primary antibody for the authentic primary antibody of interest. Horseradish peroxidase or alkaline phosphatase conjugated secondary antibodies will be used for detection using 0.05% 3, 3'-diaminobenzidene dihydrochloride and 0.03% H₂O₂ in TBS or Nova RED substrates. The stained slides will be examined by bright-field microscopy and photographed using a Leica MPS 60 photomicroscope digital camera system.

**Table 5**

| | |
|---|---|
| Primary antibodies to proteoglycan and collagen core protein epitopes | |

| **Primary Antibody epitope** | **Clone (isotype)** |
|---|---|
| **Large Proteoglycans** | |
| Aggrecan | AD 11-2A9 (IgG) |
| Perlecan | A76 (Ip-G₁) |
| Versican | AlSIDIDI (IgG) |

| **Small proteoglycans** | |
|---|---|
| Decorin | 6-B-6 (IgG) |
| Biglycan | LF-96 (rabbit IgG) |
| Fibromodulin | Rabbit polyclonal |

| **Collagen** | |
|---|---|
| Typel I | 18H5 (I_{S}G₁) |
| Type II | 11-4CII (IgG₁) |
| Type IV | CIV-22 (IgG₁) |
| Type VI | Rabbit polyclonal |
| Type X | Mouse polyclonal |

### E. Biomechanical assessment of spinal motion segments

Non-destructive biomechanical range of motion (ROM) analysis will be conducted on each functional spinal unit (FSU) in various planes of motion (flexion-extension, lateral bending, compression and torsion). Each FSU comprises two adjacent vertebrae, the intervening disc and associated ligaments.

A specially designed jig, based on that developed by Callaghan and McGill, allows pure torsion and bending moments to be applied to each FSU while maintaining a constant axial load. This combined loading is a close simulation of the physiological loads experienced by the spine in-vivo.

Four FSUs will be tested: non-operated control levels; levels that are incised; levels that are incised and treated with soluble OP-1 complex and vehicle and levels that were incised and treated with vehicle alone. Each FSU will be mounted in two aluminum alloy cups and secured with cold cure dental cement. Care will be taken to ensure that the intervertebral disc is aligned with the cups. Prior to the commencement of testing each FSU will be preloaded to a stress of 0.5 MPa until a reproducible state of hydration is achieved. This is used as the baseline prior to each test. The preload stress of 0.5 MPa simulates relaxed standing and is based on *in-vivo* measurement of intradiscal pressure (Wilke H-J et al., (1999) New in vivo measurements of pressures in the intervertebral disc in daily life, Spine 24:755-62). A ± 5 Nm torsional load and ± 1Nm flexion-extension, lateral bending load will be applied over 10 cycles whilst under a constant 0.5 MPa axial load. A cyclic axial load (0-1000N over 20 cycles) will be applied to investigate the axial compression response of the IVD.

### EXAMPLE 8: The Effect Of OP-1 On Chondral And Microfracture Treated Cartilage Defects In A Goat Model

This study will evaluate the effects of soluble OP-1 complex on the amount and composition of the reparative tissue induced by a microfracture procedure in a goat model. A total of 24 adult male goats (ages 1.5 to 3 years) weighing approximately 25 kg will be used. Prior to surgery, the knee joints will be roentgenographically examined to exclude animals with degenerative joint disease or other noted orthopedic problems. One 8mm (on a side) square chondral defect (cartilage removed down to tidemark-the calcified cartilage layer) will be produced in the trochlear groove of the right knees (stifle joints) of all animals. In 12 of the goats this chondral defect will serve as the site to the treated (Groups IA and IB (see table 6 below). The right knee joints of 22 of the animals will then undergo microfracture treatment (Groups IIA and IIB). 16 microfracture holes will be produced using a pick of approximately 1 mm diameter.

Immediately postoperative, soluble OP-1 complex will be injected into the synovial fluid of the joint. At seven days, a second injection will be administered. In 6 of the animals in the chondral defect group (IB) and in 6 of the animals in the microfracture group (IIB) only vehicle will be delivered.

**Table 6**

| **Group** | **Type of Lesion** | **Treatment** (+ **or - Sol. OP-1 Cmplx)** | **Sample Size** |
|---|---|---|---|
| IA | Chondral | + | 6 |
| IB | Chondral | - | 6 |
| IIA | Microfracture | + | 6 |
| IIB | Microfracture | - | 6 |

All animals will be sacrificed 16 weeks after surgery. All of the sites will be prepared for histomorphometric evaluation. One histological section from the center portion of each defect will be evaluated. The total area and the percentages of specific tissue types (articular cartilage, hyaline cartilage, fibrocartilage and fibrous tissue) filling the original chondral defect region will be determined using a grid in the eyepiece of the microscope. Well-accepted histological criteria for tissue types will be employed (see, e.g., Wang Q., et al. Healing of defects in canine articular cartilage: distribution of nonvascular alpha smooth muscle actin-containing cells, Wound Repair Regen. 8, pp. 145-158 (2000); Breinan HA, et al., Healing of canine articular cartilage defects treated with microfracture, a type II collagen matrix, or cultured autologous chondrocytes, J. Orthop. Res. 18, pp. 781-789 (2000); and Breinan, HA, et al., Effect of cultured autologous chondrocytes on repair of chondral defects in a canine model, J. Bone Joint Surg. 79A, pp. 1439-1451 (1997)).
Furthermore, the present invention relates to the following items:
1. A method of repairing a cartilage defect in a patient comprising the step of administering into the cartilage or into the area surrounding the cartilage a composition comprising a therapeutically effective amount of an isolated soluble morphogenic protein complex comprising:
   (a) a morphogenic protein; and
   (b) a morphogenic protein pro region isolated from a morphogenic protein, or a conservative substitution variant or a fragment of said pro region, wherein said pro region or variant or fragment is noncovalently linked to the morphogenic protein, and wherein said complex is more soluble in an aqueous solvent than said morphogenic protein alone.
2. The method of item 1, wherein the cartilage is selected from articular and non-articular cartilage.
3. The method of item 2, wherein the non-articular cartilage is selected from the group consisting of a meniscus and an intervertebral disc.
4. The method of item 1, wherein the area surrounding the cartilage is synovial fluid.
5. The method of any one of items 1-4, wherein the morphogenic protein is a dimer.
6. The method of any one of item s 1-5, wherein the morphogenic protein is selected from the group consisting of OP-1, OP-2, OP-3, BMP-2, BMP-3, BMP-4, BMP-5, BMP-6, BMP-8, BMP-9, BMP-10, BMP-11, BMP-12, BMP-13, BMP-15, BMP-16, BMP-17, BMP-18, DPP, Vg1, Vgr-1, 60A protein, GDF-1, GDF-2, GDF-3, GDF-5, GDF-6, GDF-7, GDF-8, GDF-9, GDF-10, GDF-11, GDF-12, CDMP-1, CDMP-2, CDMP-3, NODAL, UNIVIN, SCREW, ADMP, NEURAL, and conservative substitution variants and fragments thereof.
7. The method of item 6, wherein the morphogenic protein is selected from the group consisting of OP-1, BMP-5, BMP-6, GDF-5, GDF-6, GDF-7, CDMP-1, CDMP-2, CDMP-3, BMP-12, and BMP-13.
8. The method of item 7, wherein the morphogenic protein is OP-1.
9. The method of any one of item s 1-5, wherein the morphogenic protein comprises an amino acid sequence having at least 70% homology with the C-terminal 102-106 amino acids, including the conserved seven cysteine domain, of human OP-1, said morphogenic protein being capable of inducing repair of the cartilage defect.
10. The method of any one of item s 1-9, wherein the morphogenic protein pro region comprises a pro region amino acid sequence selected from the group consisting of OP-1, OP-2, OP-3, BMP-2, BMP-3, BMP-4, BMP-5, BMP-6, BMP-8, BMP-9, BMP-10, BMP-11, BMP-12, BMP-13, BMP-15, BMP-16, BMP-17, BMP-18, DPP, Vg1, Vgr, 60A protein, GDF-1, GDF-2, GDF-3, GDF-5, GDF-6, GDF-7, GDF-8, GDF-9, GDF-10, GDF-11, GDF-12, CDMP-1, CDMP-2, CDMP-3, NODAL, UNIVIN, SCREW, ADMP, NEURAL, and conservative substitution variants and fragments thereof.
11. The method of item 10, wherein the morphogenic protein pro region comprises a pro region amino acid sequence selected from the group consisting of OP-1, BMP-5, BMP-6, GDF-5, GDF-6, GDF-7, CDMP-1, CDMP-2, CDMP-3, BMP-12, and BMP-13, and conservative substitution variants and fragments thereof.
12. The method of item 11, wherein the morphogenic protein pro region comprises a pro region amino acid sequence of OP-1 or a conservative substitution variant and fragment thereof.
13. A method of regenerating or producing cartilage in a patient comprising the step of administering into the cartilage or the area surrounding the cartilage a composition comprising a therapeutically effective amount of an isolated soluble morphogenic protein complex comprising:
   (a) a morphogenic protein; and
   (b) a morphogenic protein pro region isolated from a morphogenic protein, or a conservative substitution variant or a fragment of said pro region, wherein said pro region or variant or fragment is noncovalently linked to the morphogenic protein, and wherein said complex is more soluble in an aqueous solvent than said morphogenic protein alone.
14. The method of item 13, wherein the cartilage is selected from articular and non-articular cartilage.
15. The method of item 14, wherein the non-articular cartilage is selected from the group consisting of a meniscus and an intervertebral disc.
16. The method of item 13, wherein the area surrounding the cartilage is synovial fluid.
17. The method of any one of item s 13-16, wherein the morphogenic protein is a dimer.
18. The method of any one of item s 13-17, wherein the morphogenic protein is selected from the group consisting of OP-1, OP-2, OP-3, BMP-2, BMP-3, BMP-4, BMP-5, BMP-6, BMP-8, BMP-9, BMP-10, BMP-11, BMP-12, BMP-13, BMP-15, BMP-16, BMP-17, BMP-18, DPP, Vg1, Vgr-1, 60A protein, GDF-1, GDF-2, GDF-3, GDF-5, GDF-6, GDF-7, GDF-8, GDF-9, GDF-10, GDF-11, GDF-12, CDMP-1, CDMP-2, CDMP-3, NODAL, UNIVIN, SCREW, ADMP, NEURAL, and conservative substitution variants and fragments thereof.
19. The method of item 18, wherein the morphogenic protein is selected from the group consisting of OP-1, BMP-5, BMP-6, GDF-5, GDF-6, GDF-7, CDMP-1, CDMP-2, CDMP-3, BMP-12, and BMP-13.
20. The method of item 19, wherein the morphogenic protein is OP-1.
21. The method of any one of items 13-17, wherein the morphogenic protein comprises an amino acid sequence having at least 70% homology with the C-terminal 102-106 amino acids, including the conserved seven cysteine domain, of human OP-1, said morphogenic protein being capable of inducing repair of the cartilage defect.
22. The method of any one of items 13-21, wherein the morphogenic protein pro region comprises a pro region amino acid sequence selected from the group consisting of OP-1, OP-2, OP-3, BMP-2, BMP-3, BMP-4, BMP-5, BMP-6, BMP-8, BMP-9, BMP-10, BMP-11, BMP-12, BMP-13, BMP-15, BMP-16, BMP-17, BMPs-18, DPP, Vg1, Vgr, 60A protein, GDF-1, GDF-2, GDF-3, GDF-5, GDF-6, GDF-7, GDF-8, GDF-9, GDF-10, GDF-11, GDF-12, CDMP-1, CDMP-2, CDMP-3, NODAL, UNIVIN, SCREW, ADMP, NEURAL, and conservative substitution variants and fragments thereof.
23. The method of item 22, wherein the morphogenic protein pro region comprises a pro region amino acid sequence selected from the group consisting of OP-1, BMP-5, BMP-6, GDF-5, GDF-6, GDF-7, CDMP-1, CDMP-2, CDMP-3, BMP-12, and BMP-13, and conservative substitution variants and fragments thereof.
24. The method of item 23, wherein the morphogenic protein pro region comprises a pro region amino acid sequence of OP-1 or a conservative substitution variant and fragment thereof.
25. A method of promoting cartilage growth or accelerating cartilage formation in a patient comprising the step of administering into the cartilage or into the area surrounding the cartilage a composition comprising a therapeutically effective amount of an isolated soluble morphogenic protein complex comprising:
   (a) a morphogenic protein; and
   (b) a morphogenic protein pro region isolated from a morphogenic protein, or a conservative substitution variant or a fragment of said pro region, wherein said pro region or variant or fragment is noncovalently linked to the morphogenic protein, and wherein said complex is more soluble in an aqueous solvent than said morphogenic protein alone.
26. The method of item 25, wherein the cartilage is selected from articular and non-articular cartilage.
27. The method of item 26, wherein the non-articular cartilage is selected from the group consisting of a meniscus and an intervertebral disc.
28. The method of item 25, wherein the area surrounding the cartilage is synovial fluid.
29. The method of any one of item s 25-28, wherein the morphogenic protein is a dimer.
30. The method of any one of items 25-29, wherein the morphogenic protein is selected from the group consisting of OP-1, OP-2, OP-3, BMP-2, BMP-3, BMP-4, BMP-5, BMP-6, BMP-8, BMP-9, BMP-10, BMP-11, BMP-12, BMP-13, BMP-15, BMP-16, BMP-17, BMP-18, DPP, Vg1, Vgr-1, 60A protein, GDF-1, GDF-2, GDF-3, GDF-5, GDF-6, GDF-7, GDF-8, GDF-9, GDF-10, GDF-11, GDF-12, CDMP-1, CDMP-2, CDMP-3, NODAL, UNIVIN, SCREW, ADMP, NEURAL, and conservative substitution variants and fragments thereof.
31. The method of item 30, wherein the morphogenic protein is selected from the group consisting of OP-1, BMP-5, BMP-6, GDF-5, GDF-6, GDF-7, CDMP-1, CDMP-2, CDMP-3, BMP-12, and BMP-13.
32. The method of item 31, wherein the morphogenic protein is OP-1.
33. The method of any one of items 25-29, wherein the morphogenic protein comprises an amino acid sequence having at least 70% homology with the C-terminal 102-106 amino acids, including the conserved seven cysteine domain, of human OP-1, said morphogenic protein being capable of inducing repair of the cartilage defect.
34. The method of any one of items 25-33, wherein the morphogenic protein pro region comprises a pro region amino acid sequence selected from the group consisting of OP-1, OP-2, OP-3, BMP-2, BMP-3, BMP-4, BMP-5, BMP-6, BMP-8, BMP-9, BMP-10, BMP-11, BMP-12, BMP-13, BMP-15, BMP-16, BMP-17, BMP-18, DPP, Vg1, Vgr, 60A protein, GDF-1, GDF-2, GDF-3, GDF-5, GDF-6, GDF-7, GDF-8, GDF-9, GDF-10, GDF-11, GDF-12, CDMP-1, CDMP-2, CDMP-3, NODAL, UNIVIN, SCREW, ADMP, NEURAL, and conservative substitution variants and fragments thereof.
35. The method of item 34, wherein the morphogenic protein pro region comprises a pro region amino acid sequence selected from the group consisting of OP-1, BMP-5, BMP-6, GDF-5, GDF-6, GDF-7, CDMP-1, CDMP-2, CDMP-3, BMP-12, and BMP-13, and conservative substitution variants and fragments thereof.
36. The method of item 35, wherein the morphogenic protein pro region comprises a pro region amino acid sequence of OP-1 or a conservative substitution variant and fragment thereof.
37. A method of preventing cartilage degradation or treating cartilage injury or degenerative disease or disorder in a patient comprising the step of administering into the cartilage or into the area surrounding the cartilage a composition comprising a therapeutically effective amount of an isolated soluble morphogenic protein complex comprising:
   (a) a morphogenic protein; and
   (b) a morphogenic protein pro region isolated from a morphogenic protein, or a conservative substitution variant or a fragment of said pro region, wherein said pro region or variant or fragment is noncovalently linked to the morphogenic protein, and wherein said complex is more soluble in an aqueous solvent than said morphogenic protein alone.
38. The method of item 37, wherein the cartilage is selected from articular and non-articular cartilage.
39. The method of item 38, wherein the non-articular cartilage is selected from the group consisting of a meniscus and an intervertebral disc.
40. The method of item 3 7, wherein the area surrounding the cartilage is synovial fluid.
41. The method of any one of items 37-40, wherein the morphogenic protein is a dimer.
42. The method of any one of items 37-41, wherein the morphogenic protein is selected from the group consisting of OP-1, OP-2, OP-3, BMP-2, BMP-3, BMP-4, BMP-5, BMP-6, BMP-8, BMP-9, BMP-10, BMP-11, BMP-12, BMP-13, BMP-15, BMP-16, BMP-17, BMP-18, DPP, Vg1, Vgr-1, 60A protein, GDF-1, GDF-2, GDF-3, GDF-5, GDF-6, GDF-7, GDF-8, GDF-9, GDF-10, GDF-11, GDF-12, CDMP-1, CDMP-2, CDMP-3, NODAL, UNIVIN, SCREW, ADMP, NEURAL, and conservative substitution variants and fragments thereof.
43. The method of item 42, wherein the morphogenic protein is selected from the group consisting of OP-1, BMP-5, BMP-6, GDF-5, GDF-6, GDF-7, CDMP-1, CDMP-2, CDMP-3, BMP-12, and BMP-13.
44. The method of item 43, wherein the morprhogenic protein is OP-1.
45. The method of any one of items 37-41, wherein the morphogenic protein comprises an amino acid sequence having at least 70% homology with the C-terminal 102-106 amino acids, including the conserved seven cysteine domain, of human OP-1, said morphogenic protein being capable of inducing repair of the cartilage defect.
46. The method of any one of items 37-45, wherein the morphogenic protein pro region comprises a pro region amino acid sequence selected from the group consisting of OP-1, OP-2, OP-3, BMP-2, BMP-3, BMP-4, BMP-5, BMP-6, BMP-8, BMP-9, BMP-10, BMP-11, BMP-12, BMP-13, BMP-15, BMP-16, BMP-17, BMP-18, DPP, Vg1, Vgr, 60A protein, GDF-1, GDF-2, GDF-3, GDF-5, GDF-6, GDF-7, GDF-8, GDF-9, GDF-10, GDF-11, GDF-12, CDMP-1, CDMP-2, CDMP-3, NODAL, UNIVIN, SCREW, ADMP, NEURAL, and conservative substitution variants and fragments thereof.
47. The method of item 46, wherein the morphogenic protein pro region comprises a pro region amino acid sequence selected from the group consisting of OP-1, BMP-5, BMP-6, GDF-5, GDF-6, GDF-7, CDMP-1, CDMP-2, CDMP-3, BMP-12, and BMP-13, and conservative substitution variants and fragments thereof.
48. The method of item 47, wherein the morphogenic protein pro region comprises a pro region amino acid sequence of OP-1 or a conservative substitution variant and fragment thereof.
49. A method of treating cartilage tissue injury or degenerative disease or disorder in a patient comprising the step of administering to the patient a composition comprising a therapeutically effective amount of an isolated soluble morphogenic protein complex comprising:
   (a) a morphogenic protein; and
   (b) a morphogenic protein pro region isolated from a morphogenic protein, or a conservative substitution variant or a fragment of said pro region, wherein said pro region or variant or fragment is noncovalently linked to the morphogenic protein, and wherein said complex is more soluble in an aqueous solvent than said morphogenic protein alone.
50. The method of item 49, wherein the cartilage tissue injury is selected from the group consisting of meniscus tear meniscus tears, chondral voids and defects, osteochondral voids and defects and ACL injury.
51. The method of item 49, wherein the cartilage degenerative disease or disorder is selected from the group consisting of osteoarthritis and intervertebral disc degeneration.
52. The method of any one of item s 49-51, wherein the morphogenic protein is a dimer.
53. The method of any one of item 49-52, wherein the morphogenic protein is selected from the group consisting of OP-1, OP-2, OP-3, BMP-2, BMP-3, BMP-4, BMP-5, BMP-6, BMP-8, BMP-9, BMP-10, BMP-11, BMP-12, BMP-13, BMP-15, BMP-16, BMP-17, BMP-18, DPP, Vg1, Vgr-1, 60A protein, GDF-1, GDF-2, GDF-3, GDF-5, GDF-6, GDF-7, GDF-8, GDF-9, GDF-10, GDF-11, GDF-12, CDMP-1, CDMP-2, CDMP-3, NODAL, UNIVIN, SCREW, ADMP, NEURAL, and conservative substitution variants and fragments thereof.
54. The method of item 53, wherein the morphogenic protein is selected from the group consisting of OP-1, BMP-5, BMP-6, GDF-5, GDF-6, GDF-7, CDMP-1, CDMP-2, CDMP-3, BMP-12, and BMP-13.
55. The method of item 16, wherein the morphogenic protein is OP-1.
56. The method of any one of item s 49-52, wherein the morphogenic protein comprises an amino acid sequence having at least 70% homology with the C-terminal 102-106 amino acids, including the conserved seven cysteine domain, of human OP-1, said morphogenic protein being capable of inducing repair of the cartilage defect.
57. The method of any one of items 49-56, wherein the morphogenic protein pro region comprises a pro region amino acid sequence selected from the group consisting of OP-1, OP-2, OP-3, BMP-2, BMP-3, BMP-4, BMP-5, BMP-6, BMP-8, BMP-9, BMP-10, BMP-11, BMP-12, BMP-13, BMP-15, BMP-16, BMP-17, BMP-18, DPP, Vg1, Vgr, 60A protein, GDF-1, GDF-2, GDF-3, GDF-5, GDF-6, GDF-7, GDF-8, GDF-9, GDF-10, GDF-11, GDF-12, CDMP-1, CDMP-2, CDMP-3, NODAL, UNIVIN, SCREW, ADMP, NEURAL, and conservative substitution variants and fragments thereof.
58. The method of item 57, wherein the morphogenic protein pro region comprises a pro region amino acid sequence selected from the group consisting of OP-1, BMP-5, BMP-6, GDF-5, GDF-6, GDF-7, CDMP-1, CDMP-2, CDMP-3, BMP-12, and BMP-13, and conservative substitution variants and fragments thereof.
59. The method of item 58, wherein the morphogenic protein pro region comprises a pro region amino acid sequence of OP-1 or a conservative substitution variant and fragment thereof.
60. The method of item 51, wherein the composition is administered into the osteoarthritic defect site or into the area surrounding the osteoarthritic defect site.
61. The method of item 51, wherein the composition is administered into the intervertebral disc degeneration defect site or into the area surrounding the intervertebral disc degeneration defect site.

## Claims

1. A composition comprising a therapeutically effective amount of an isolated soluble morphogenic protein complex comprising:
(a) a morphogenic protein; and
(b) a morphogenic protein pro region isolated from a morphogenic protein, or a conservative substitution variant or a fragment of said pro region, wherein said pro region or variant or fragment is noncovalently linked to the morphogenic protein, and wherein said complex is more soluble in an aqueous solvent than said morphogenic protein alone,
for use in repairing a cartilage defect in a patient, wherein said composition is to be administered into the cartilage or into the area surrounding the cartilage.

2. A composition comprising a therapeutically effective amount of an isolated soluble morphogenic protein complex comprising:
(a) a morphogenic protein; and
(b) a morphogenic protein pro region isolated from a morphogenic protein, or a conservative substitution variant or a fragment of said pro region, wherein said pro region or variant or fragment is noncovalently linked to the morphogenic protein, and wherein said complex is more soluble in an aqueous solvent than said morphogenic protein alone,
for use in regenerating or producing cartilage in a patient, wherein said composition is to be administered into the cartilage or into the area surrounding the cartilage.

3. A composition comprising a therapeutically effective amount of an isolated soluble morphogenic protein complex comprising:
(a) a morphogenic protein; and
(b) a morphogenic protein pro region isolated from a morphogenic protein, or a conservative substitution variant or a fragment of said pro region, wherein said pro region or variant or fragment is noncovalently linked to the morphogenic protein, and wherein said complex is more soluble in an aqueous solvent than said morphogenic protein alone,
for use in promoting cartilage growth or accelerating cartilage formation in a patient, wherein said composition is to be administered into the cartilage or into the area surrounding the cartilage.

4. A composition comprising a therapeutically effective amount of an isolated soluble morphogenic protein complex comprising:
(a) a morphogenic protein; and
(b) a morphogenic protein pro region isolated from a morphogenic protein, or a conservative substitution variant or a fragment of said pro region, wherein said pro region or variant or fragment is noncovalently linked to the morphogenic protein, and wherein said complex is more soluble in an aqueous solvent than said morphogenic protein alone
for use in preventing cartilage degradation in a patient, wherein said composition is to be administered into the cartilage or into the area surrounding the cartilage.

5. A composition comprising a therapeutically effective amount of an isolated soluble morphogenic protein complex comprising:
(a) a morphogenic protein; and
(b) a morphogenic protein pro region isolated from a morphogenic protein, or a conservative substitution variant or a fragment of said pro region, wherein said pro region or variant or fragment is noncovalently linked to the morphogenic protein, and wherein said complex is more soluble in an aqueous solvent than said morphogenic protein alone
for use in treating cartilage injury or degenerative disease or disorder in a patient, wherein said composition is to be administered into the cartilage or into the area surrounding the cartilage.

6. The composition of any one of claims 1-5, wherein the cartilage is selected from articular and non-articular cartilage.

7. The composition of claim 6, wherein the non-articular cartilage is selected from the group consisting of a meniscus and an intervertebral disc.

8. The composition of any one of claims 1-5, wherein the area surrounding the cartilage is synovial fluid.

9. The composition of any one of claims 1 to 8, wherein the morphogenic protein is a dimer.

10. The composition of any one of claims 1 to 9, wherein the morphogenic protein is selected from the group consisting of OP-1, OP-2, OP-3, BMP-2, BMP-3, BMP-4, BMP-5, BMP-6, BMP-8, BMP-9, BMP-10, BMP-11, BMP-12, BMP-13, BMP-15, BMP-16, BMP-17, BMP-18, DPP, Vgl, Vgr, 60A protein, GDF-1, GDF-2, GDF-3, GDF-5, GDF-6, GDF-7, GDF-8, GDF-9, GDF-10, GDF-11, GDF-12, CDMP-1, CDMP-2, CDMP-3, NODAL, UNIVIN, SCREW, ADMP, NEURAL, and conservative substitution variants and fragments thereof.

11. The composition of any one of claims 1 to 9, wherein the morphogenic protein comprises an amino acid sequence having at least 70% homology with the C-terminal 102-106 amino acids, including the conserved seven cysteine domain, of human OP-1, said morphogenic protein being capable of inducing repair of the cartilage defect.

12. The composition of any one of claims 1 to 11, wherein the morphogenic protein pro region comprises a pro region amino acid sequence selected from the group consisting of OP-1, OP-2, OP-3, BMP-2, BMP-3, BMP-4, BMP-5, BMP-6, BMP-8, BMP-9, BMP-10, BMP-11, BMP-12, BMP-13, BMP-15, BMP-16, BMP-17, BMP-18, DPP, Vgl, Vgr, 60A protein, GDF-1, GDF-2, GDF-3, GDF-5, GDF-6, GDF-7, GDF-8, GDF-9, GDF-10, GDF-11, GDF-12, CDMP-1, CDMP-2, CDMP-3, NODAL, UNIVIN, SCREW, ADMP, NEURAL, and conservative substitution variants and fragments thereof.

13. The composition of any one of claims 5 to 12, wherein said cartilage injury is selected from the group consistion of meniscus tears, chondral voids or defects, osteochondral voids or defect and ACL injury.

14. The composition of any one of claims 5 to 12, wherein said cartilage degenerative disease or disorder is selected from the group consisting of osteoarthritis and disc degeneration.


SEQUENCE LISTING

<110> STRYKER CORPORATION

<120> METHODS OF TREATING CARTILAGE DEFECTS USING A SOLUBLE
      MORPHOGENIC PROTEIN COMPLEX

<130> P3355 EP/1 S3

<150> EP 07 79 5187.9
<151> 2007-05-17

<150> US 60/801,551
<151> 2006-05-17

<160> 58

<170> PatentIn Ver. 3.3

<210> 1
<211> 1017
<212> DNA
<213> Homo sapiens

<220>
<221> CDS
<222> (49)..(1017)

<400> 1
ggtgcgggcc cggagcccgg agcccgggta gcgcgtagag ccggcgcg atg cac gtg   57
                                                     Met His Val
                                                      1

cgc tca ctg cga gct gcg gcg ccg cac agc ttc gtg gcg ctc tgg gca   105
Arg Ser Leu Arg Ala Ala Ala Pro His Ser Phe Val Ala Leu Trp Ala
      5                  10                  15

ccc ctg ttc ctg ctg cgc tcc gcc ctg gcc gac ttc agc ctg gac aac   153
Pro Leu Phe Leu Leu Arg Ser Ala Leu Ala Asp Phe Ser Leu Asp Asn
 20                  25                  30                  35

gag gtg cac tcg agc ttc atc cac cgg cgc ctc cgc agc cag gag cgg   201
Glu Val His Ser Ser Phe Ile His Arg Arg Leu Arg Ser Gln Glu Arg
                 40                  45                  50

cgg gag atg cag cgc gag atc ctc tcc att ttg ggc ttg ccc cac cgc   249
Arg Glu Met Gln Arg Glu Ile Leu Ser Ile Leu Gly Leu Pro His Arg
             55                  60                  65

ccg cgc ccg cac ctc cag ggc aag cac aac tcg gca ccc atg ttc atg   297
Pro Arg Pro His Leu Gln Gly Lys His Asn Ser Ala Pro Met Phe Met
         70                  75                  80

ctg gac ctg tac aac gcc atg gcg gtg gag gag ggc ggc ggg ccc ggc   345
Leu Asp Leu Tyr Asn Ala Met Ala Val Glu Glu Gly Gly Gly Pro Gly
     85                  90                  95

ggc cag ggc ttc tcc tac ccc tac aag gcc gtc ttc agt acc cag ggc   393
Gly Gln Gly Phe Ser Tyr Pro Tyr Lys Ala Val Phe Ser Thr Gln Gly
100                 105                 110                 115

ccc cct ctg gcc agc ctg caa gat agc cat ttc ctc acc gac gcc gac   441
Pro Pro Leu Ala Ser Leu Gln Asp Ser His Phe Leu Thr Asp Ala Asp
                120                 125                 130

atg gtc atg agc ttc gtc aac ctc gtg gaa cat gac aag gaa ttc ttc     489
Met Val Met Ser Phe Val Asn Leu Val Glu His Asp Lys Glu Phe Phe
            135                 140                 145

cac cca cgc tac cac cat cga gag ttc cgg ttt gat ctt tcc aag atc     537
His Pro Arg Tyr His His Arg Glu Phe Arg Phe Asp Leu Ser Lys Ile
        150                 155                 160

cca gaa ggg gaa gct gtc acg gca gcc gaa ttc cgg atc tac aag gac     585
Pro Glu Gly Glu Ala Val Thr Ala Ala Glu Phe Arg Ile Tyr Lys Asp
    165                 170                 175

tac atc cgg gaa cgc ttc gac aat gag acg ttc cgg atc agc gtt tat     633
Tyr Ile Arg Glu Arg Phe Asp Asn Glu Thr Phe Arg Ile Ser Val Tyr
180                 185                 190                 195

cag gtg ctc cag gag cac ttg ggc agg gaa tcg gat ctc ttc ctg ctc     681
Gln Val Leu Gln Glu His Leu Gly Arg Glu Ser Asp Leu Phe Leu Leu
                200                 205                 210

gac agc cgt acc ctc tgg gcc tcg gag gag ggc tgg ctg gtg ttt gac     729
Asp Ser Arg Thr Leu Trp Ala Ser Glu Glu Gly Trp Leu Val Phe Asp
            215                 220                 225

atc aca gcc acc agc aac cac tgg gtg gtc aat ccg cgg cac aac ctg     777
Ile Thr Ala Thr Ser Asn His Trp Val Val Asn Pro Arg His Asn Leu
        230                 235                 240

ggc ctg cag ctc tcg gtg gag acg ctg gat ggg cag agc atc aac ccc     825
Gly Leu Gln Leu Ser Val Glu Thr Leu Asp Gly Gln Ser Ile Asn Pro
    245                 250                 255

aag ttg gcg ggc ctg att ggg cgg cac ggg ccc cag aac aag cag ccc     873
Lys Leu Ala Gly Leu Ile Gly Arg His Gly Pro Gln Asn Lys Gln Pro
260                 265                 270                 275

ttc atg gtg gct ttc ttc aag gcc acg gag gtc cac ttc cgc agc atc     921
Phe Met Val Ala Phe Phe Lys Ala Thr Glu Val His Phe Arg Ser Ile
                280                 285                 290

cgg tcc acg ggg agc aaa cag cgc agc cag aac cgc tcc aag acg ccc     969
Arg Ser Thr Gly Ser Lys Gln Arg Ser Gln Asn Arg Ser Lys Thr Pro
            295                 300                 305

aag aac cag gaa gcc ctg cgg atg gcc aac gtg gca gag aac agc agc     1017
Lys Asn Gln Glu Ala Leu Arg Met Ala Asn Val Ala Glu Asn Ser Ser
        310                 315                 320


<210> 2
<211> 431
<212> PRT
<213> Homo sapiens

<400> 2
Met His Val Arg Ser Leu Arg Ala Ala Ala Pro His Ser Phe Val Ala
  1               5                  10                  15

Leu Trp Ala Pro Leu Phe Leu Leu Arg Ser Ala Leu Ala Asp Phe Ser
             20                  25                  30

Leu Asp Asn Glu Val His Ser Ser Phe Ile His Arg Arg Leu Arg Ser
         35                  40                  45

Gln Glu Arg Arg Glu Met Gln Arg Glu Ile Leu Ser Ile Leu Gly Leu
        50                  55                  60

Pro His Arg Pro Arg Pro His Leu Gln Gly Lys His Asn Ser Ala Pro
65                  70                  75                  80

Met Phe Met Leu Asp Leu Tyr Asn Ala Met Ala Val Glu Glu Gly Gly
                85                  90                  95

Gly Pro Gly Gly Gln Gly Phe Ser Tyr Pro Tyr Lys Ala Val Phe Ser
            100                 105                 110

Thr Gln Gly Pro Pro Leu Ala Ser Leu Gln Asp Ser His Phe Leu Thr
        115                 120                 125

Asp Ala Asp Met Val Met Ser Phe Val Asn Leu Val Glu His Asp Lys
    130                 135                 140

Glu Phe Phe His Pro Arg Tyr His His Arg Glu Phe Arg Phe Asp Leu
145                 150                 155                 160

Ser Lys Ile Pro Glu Gly Glu Ala Val Thr Ala Ala Glu Phe Arg Ile
                165                 170                 175

Tyr Lys Asp Tyr Ile Arg Glu Arg Phe Asp Asn Glu Thr Phe Arg Ile
            180                 185                 190

Ser Val Tyr Gln Val Leu Gln Glu His Leu Gly Arg Glu Ser Asp Leu
        195                 200                 205

Phe Leu Leu Asp Ser Arg Thr Leu Trp Ala Ser Glu Glu Gly Trp Leu
    210                 215                 220

Val Phe Asp Ile Thr Ala Thr Ser Asn His Trp Val Val Asn Pro Arg
225                 230                 235                 240

His Asn Leu Gly Leu Gln Leu Ser Val Glu Thr Leu Asp Gly Gln Ser
                245                 250                 255

Ile Asn Pro Lys Leu Ala Gly Leu Ile Gly Arg His Gly Pro Gln Asn
            260                 265                 270

Lys Gln Pro Phe Met Val Ala Phe Phe Lys Ala Thr Glu Val His Phe
        275                 280                 285

Arg Ser Ile Arg Ser Thr Gly Ser Lys Gln Arg Ser Gln Asn Arg Ser
    290                 295                 300

Lys Thr Pro Lys Asn Gln Glu Ala Leu Arg Met Ala Asn Val Ala Glu
305                 310                 315                 320

Asn Ser Ser Ser Asp Gln Arg Gln Ala Cys Lys Lys His Glu Leu Tyr
                325                 330                 335

Val Ser Phe Arg Asp Leu Gly Trp Gln Asp Trp Ile Ile Ala Pro Glu
            340                 345                 350

Gly Tyr Ala Ala Tyr Tyr Cys Glu Gly Glu Cys Ala Phe Pro Leu Asn
        355                 360                 365

Ser Tyr Met Asn Ala Thr Asn His Ala Ile Val Gln Thr Leu Val His
    370                 375                 380

Phe Ile Asn Pro Glu Thr Val Pro Lys Pro Cys Cys Ala Pro Thr Gln
385                 390                 395                 400

Leu Asn Ala Ile Ser Val Leu Tyr Phe Asp Asp Ser Ser Asn Val Ile
                405                 410                 415

Leu Lys Lys Tyr Arg Asn Met Val Val Arg Ala Cys Gly Cys His
            420                 425                 430

<210> 3
<211> 1873
<212> DNA
<213> Mus musculus

<220>
<221> CDS
<222> (104)..(1393)

<400> 3
ctgcagcaag tgacctcggg tcgtggaccg ctgccctgcc ccctccgctg ccacctgggg 60

cggcgcgggc ccggtgcccc ggatcgcgcg tagagccggc gcg atg cac gtg cgc   115
                                                Met His Val Arg
                                                1

tcg ctg cgc gct gcg gcg cca cac agc ttc gtg gcg ctc tgg gcg cct   163
Ser Leu Arg Ala Ala Ala Pro His Ser Phe Val Ala Leu Trp Ala Pro
5                   10                  15                  20

ctg ttc ttg ctg cgc tcc gcc ctg gcc gat ttc agc ctg gac aac gag   211
Leu Phe Leu Leu Arg Ser Ala Leu Ala Asp Phe Ser Leu Asp Asn Glu
                25                  30                  35

gtg cac tcc agc ttc atc cac cgg cgc ctc cgc agc cag gag cgg cgg   259
Val His Ser Ser Phe Ile His Arg Arg Leu Arg Ser Gln Glu Arg Arg
            40                  45                  50

gag atg cag cgg gag atc ctg tcc atc tta ggg ttg ccc cat cgc ccg   307
Glu Met Gln Arg Glu Ile Leu Ser Ile Leu Gly Leu Pro His Arg Pro
        55                  60                  65

cgc ccg cac ctc cag gga aag cat aat tcg gcg ccc atg ttc atg ttg   355
Arg Pro His Leu Gln Gly Lys His Asn Ser Ala Pro Met Phe Met Leu
    70                  75                  80

gac ctg tac aac gcc atg gcg gtg gag gag agc ggg ccg gac gga cag   403
Asp Leu Tyr Asn Ala Met Ala Val Glu Glu Ser Gly Pro Asp Gly Gln
85                  90                  95                  100

ggc ttc tcc tac ccc tac aag gcc gtc ttc agt acc cag ggc ccc cct   451
Gly Phe Ser Tyr Pro Tyr Lys Ala Val Phe Ser Thr Gln Gly Pro Pro
                105                 110                 115

tta gcc agc ctg cag gac agc cat ttc ctc act gac gcc gac atg gtc   499
Leu Ala Ser Leu Gln Asp Ser His Phe Leu Thr Asp Ala Asp Met Val
            120                 125                 130

atg agc ttc gtc aac cta gtg gaa cat gac aaa gaa ttc ttc cac cct   547
Met Ser Phe Val Asn Leu Val Glu His Asp Lys Glu Phe Phe His Pro
        135                 140                 145

cga tac cac cat cgg gag ttc cgg ttt gat ctt tcc aag atc ccc gag   595
Arg Tyr His His Arg Glu Phe Arg Phe Asp Leu Ser Lys Ile Pro Glu
    150                 155                 160

ggc gaa cgg gtg acc gca gcc gaa ttc agg atc tat aag gac tac atc 643
Gly Glu Arg Val Thr Ala Ala Glu Phe Arg Ile Tyr Lys Asp Tyr Ile
165                 170                 175                 180

cgg gag cga ttt gac aac gag acc ttc cag atc aca gtc tat cag gtg 691
Arg Glu Arg Phe Asp Asn Glu Thr Phe Gln Ile Thr Val Tyr Gln Val
                185                 190                 195

ctc cag gag cac tca ggc agg gag tcg gac ctc ttc ttg ctg gac agc 739
Leu Gln Glu His Ser Gly Arg Glu Ser Asp Leu Phe Leu Leu Asp Ser
            200                 205                 210

cgc acc atc tgg gct tct gag gag ggc tgg ttg gtg ttt gat atc aca 787
Arg Thr Ile Trp Ala Ser Glu Glu Gly Trp Leu Val Phe Asp Ile Thr
        215                 220                 225

gcc acc agc aac cac tgg gtg gtc aac cct cgg cac aac ctg ggc tta 835
Ala Thr Ser Asn His Trp Val Val Asn Pro Arg His Asn Leu Gly Leu
    230                 235                 240

cag ctc tct gtg gag acc ctg gat ggg cag agc atc aac ccc aag ttg 883
Gln Leu Ser Val Glu Thr Leu Asp Gly Gln Ser Ile Asn Pro Lys Leu
245                 250                 255                 260

gca ggc ctg att gga cgg cat gga ccc cag aac aag caa ccc ttc atg 931
Ala Gly Leu Ile Gly Arg His Gly Pro Gln Asn Lys Gln Pro Phe Met
                265                 270                 275

gtg gcc ttc ttc aag gcc acg gaa gtc cat ctc cgt agt atc cgg tcc 979
Val Ala Phe Phe Lys Ala Thr Glu Val His Leu Arg Ser Ile Arg Ser
            280                 285                 290

acg ggg ggc aag cag cgc agc cag aat cgc tcc aag acg cca aag aac 1027
Thr Gly Gly Lys Gln Arg Ser Gln Asn Arg Ser Lys Thr Pro Lys Asn
        295                 300                 305

caa gag gcc ctg agg atg gcc agt gtg gca gaa aac agc agc agt gac 1075
Gln Glu Ala Leu Arg Met Ala Ser Val Ala Glu Asn Ser Ser Ser Asp
    310                 315                 320

cag agg cag gcc tgc aag aaa cat gag ctg tac gtc agc ttc cga gac 1123
Gln Arg Gln Ala Cys Lys Lys His Glu Leu Tyr Val Ser Phe Arg Asp
325                 330                 335                 340

ctt ggc tgg cag gac tgg atc att gca cct gaa ggc tat gct gcc tac 1171
Leu Gly Trp Gln Asp Trp Ile Ile Ala Pro Glu Gly Tyr Ala Ala Tyr
                345                 350                 355

tac tgt gag gga gag tgc gcc ttc cct ctg aac tcc tac atg aac gcc 1219
Tyr Cys Glu Gly Glu Cys Ala Phe Pro Leu Asn Ser Tyr Met Asn Ala
            360                 365                 370

acc aac cac gcc atc gtc cag aca ctg gtt cac ttc atc aac cca gac 1267
Thr Asn His Ala Ile Val Gln Thr Leu Val His Phe Ile Asn Pro Asp
        375                 380                 385

aca gta ccc aag ccc tgc tgt gcg ccc acc cag ctc aac gcc atc tct 1315
Thr Val Pro Lys Pro Cys Cys Ala Pro Thr Gln Leu Asn Ala Ile Ser
    390                 395                 400

gtc ctc tac ttc gac gac agc tct aat gtc atc ctg aag aag tac aga 1363
Val Leu Tyr Phe Asp Asp Ser Ser Asn Val Ile Leu Lys Lys Tyr Arg
405                 410                 415                 420

aac atg gtg gtc cgg gcc tgt ggc tgc cac tagctcttcc tgagaccctg 1413
Asn Met Val Val Arg Ala Cys Gly Cys His
                425                 430

acctttgcgg ggccacacct ttccaaatct tcgatgtctc accatctaag tctctcactg 1473

cccaccttgg cgaggagaac agaccaacct ctcctgagcc ttccctcacc tcccaaccgg 1533

aagcatgtaa gggttccaga aacctgagcg tgcagcagct gatgagcgcc ctttccttct 1593

ggcacgtgac ggacaagatc ctaccagcta ccacagcaaa cgcctaagag caggaaaaat 1653

gtctgccagg aaagtgtcca gtgtccacat ggcccctggc gctctgagtc tttgaggagt 1713

aatcgcaagc ctcgttcagc tgcagcagaa ggaagggctt agccagggtg ggcgctggcg 1773

tctgtgttga agggaaacca agcagaagcc actgtaatga tatgtcacaa taaaacccat 1833

gaatgaaaaa aaaaaaaaaa aaaaaaaaaa aaaagaattc 1873

<210> 4
<211> 430
<212> PRT
<213> Mus musculus

<400> 4
Met His Val Arg Ser Leu Arg Ala Ala Ala Pro His Ser Phe Val Ala
1               5                   10                  15

Leu Trp Ala Pro Leu Phe Leu Leu Arg Ser Ala Leu Ala Asp Phe Ser
            20                  25                  30

Leu Asp Asn Glu Val His Ser Ser Phe Ile His Arg Arg Leu Arg Ser
        35                  40                  45

Gln Glu Arg Arg Glu Met Gln Arg Glu Ile Leu Ser Ile Leu Gly Leu
    50                  55                  60

Pro His Arg Pro Arg Pro His Leu Gln Gly Lys His Asn Ser Ala Pro
65                  70                  75                  80

Met Phe Met Leu Asp Leu Tyr Asn Ala Met Ala Val Glu Glu Ser Gly
                85                  90                  95

Pro Asp Gly Gln Gly Phe Ser Tyr Pro Tyr Lys Ala Val Phe Ser Thr
            100                 105                 110

Gln Gly Pro Pro Leu Ala Ser Leu Gln Asp Ser His Phe Leu Thr Asp
        115                 120                 125

Ala Asp Met Val Met Ser Phe Val Asn Leu Val Glu His Asp Lys Glu
    130                 135                 140

Phe Phe His Pro Arg Tyr His His Arg Glu Phe Arg Phe Asp Leu Ser
145                 150                 155                 160

Lys Ile Pro Glu Gly Glu Arg Val Thr Ala Ala Glu Phe Arg Ile Tyr
                165                 170                 175

Lys Asp Tyr Ile Arg Glu Arg Phe Asp Asn Glu Thr Phe Gln Ile Thr
            180                 185                 190

Val Tyr Gln Val Leu Gln Glu His Ser Gly Arg Glu Ser Asp Leu Phe
        195                 200                 205

Leu Leu Asp Ser Arg Thr Ile Trp Ala Ser Glu Glu Gly Trp Leu Val
    210                 215                 220

Phe Asp Ile Thr Ala Thr Ser Asn His Trp Val Val Asn Pro Arg His
225                 230                 235                 240

Asn Leu Gly Leu Gln Leu Ser Val Glu Thr Leu Asp Gly Gln Ser Ile
                245                 250                 255

Asn Pro Lys Leu Ala Gly Leu Ile Gly Arg His Gly Pro Gln Asn Lys
            260                 265                 270

Gln Pro Phe Met Val Ala Phe Phe Lys Ala Thr Glu Val His Leu Arg
        275                 280                 285

Ser Ile Arg Ser Thr Gly Gly Lys Gln Arg Ser Gln Asn Arg Ser Lys
    290                 295                 300

Thr Pro Lys Asn Gln Glu Ala Leu Arg Met Ala Ser Val Ala Glu Asn
305                 310                 315                 320

Ser Ser Ser Asp Gln Arg Gln Ala Cys Lys Lys His Glu Leu Tyr Val
                325                 330                 335

Ser Phe Arg Asp Leu Gly Trp Gln Asp Trp Ile Ile Ala Pro Glu Gly
            340                 345                 350

Tyr Ala Ala Tyr Tyr Cys Glu Gly Glu Cys Ala Phe Pro Leu Asn Ser
        355                 360                 365

Tyr Met Asn Ala Thr Asn His Ala Ile Val Gln Thr Leu Val His Phe
    370                 375                 380

Ile Asn Pro Asp Thr Val Pro Lys Pro Cys Cys Ala Pro Thr Gln Leu
385                 390                 395                 400

Asn Ala Ile Ser Val Leu Tyr Phe Asp Asp Ser Ser Asn Val Ile Leu
                405                 410                 415

Lys Lys Tyr Arg Asn Met Val Val Arg Ala Cys Gly Cys His
            420                 425                 430

<210> 5
<211> 1723
<212> DNA
<213> Homo sapiens

<220>
<221> CDS
<222> (490)..(1695)

<400> 5
ggcgccggca gagcaggagt ggctggagga gctgtggttg gagcaggagg tggcacggca 60

gggctggagg gctccctatg agtggcggag acggcccagg aggcgctgga gcaacagctc 120

ccacaccgca ccaagcggtg gctgcaggag ctcgcccatc gcccctgcgc tgctcggacc 180

gcggccacag ccggactggc gggtacggcg gcgacagagg cattggccga gagtcccagt 240

ccgcagagta gccccggcct cgaggcggtg gcgtcccggt cctctccgtc caggagccag 300

gacaggtgtc gcgcggcggg gctccaggga ccgcgcctga ggccggctgc ccgcccgtcc 360

cgccccgccc cgccgcccgc cgcccgccga gcccagcctc cttgccgtcg gggcgtcccc 420

aggccctggg tcggccgcgg agccgatgcg cgcccgctga gcgccccagc tgagcgcccc 480

cggcctgcc atg acc gcg ctc ccc ggc ccg ctc tgg ctc ctg ggc ctg gcg 531
Met Thr Ala Leu Pro Gly Pro Leu Trp Leu Leu Gly Leu Ala
1               5                   10

cta tgc gcg ctg ggc ggg ggc ggc ccc ggc ctg cga ccc ccg ccc ggc 579
Leu Cys Ala Leu Gly Gly Gly Gly Pro Gly Leu Arg Pro Pro Pro Gly
15                  20                  25                  30

tgt ccc cag cga cgt ctg ggc gcg cgc gag cgc cgg gac gtg cag cgc 627
Cys Pro Gln Arg Arg Leu Gly Ala Arg Glu Arg Arg Asp Val Gln Arg
                35                  40                  45

gag atc ctg gcg gtg ctc ggg ctg cct ggg cgg ccc cgg ccc cgc gcg 675
Glu Ile Leu Ala Val Leu Gly Leu Pro Gly Arg Pro Arg Pro Arg Ala
            50                  55                  60

cca ccc gcc gcc tcc cgg ctg ccc gcg tcc gcg ccg ctc ttc atg ctg 723
Pro Pro Ala Ala Ser Arg Leu Pro Ala Ser Ala Pro Leu Phe Met Leu
        65                  70                  75

gac ctg tac cac gcc atg gcc ggc gac gac gac gag gac ggc gcg ccc 771
Asp Leu Tyr His Ala Met Ala Gly Asp Asp Asp Glu Asp Gly Ala Pro
    80                  85                  90

gcg gag cgg cgc ctg ggc cgc gcc gac ctg gtc atg agc ttc gtt aac 819
Ala Glu Arg Arg Leu Gly Arg Ala Asp Leu Val Met Ser Phe Val Asn
95                  100                 105                 110

atg gtg gag cga gac cgt gcc ctg ggc cac cag gag ccc cat tgg aag 867
Met Val Glu Arg Asp Arg Ala Leu Gly His Gln Glu Pro His Trp Lys
                115                 120                 125

gag ttc cgc ttt gac ctg acc cag atc ccg gct ggg gag gcg gtc aca 915
Glu Phe Arg Phe Asp Leu Thr Gln Ile Pro Ala Gly Glu Ala Val Thr
            130                 135                 140

gct gcg gag ttc cgg att tac aag gtg ccc agc atc cac ctg ctc aac 963
Ala Ala Glu Phe Arg Ile Tyr Lys Val Pro Ser Ile His Leu Leu Asn
        145                 150                 155

agg acc ctc cac gtc agc atg ttc cag gtg gtc cag gag cag tcc aac 1011
Arg Thr Leu His Val Ser Met Phe Gln Val Val Gln Glu Gln Ser Asn
    160                 165                 170

agg gag tct gac ttg ttc ttt ttg gat ctt cag acg ctc cga gct gga 1059
Arg Glu Ser Asp Leu Phe Phe Leu Asp Leu Gln Thr Leu Arg Ala Gly
175                 180                 185                 190

gac gag ggc tgg ctg gtg ctg gat gtc aca gca gcc agt gac tgc tgg 1107
Asp Glu Gly Trp Leu Val Leu Asp Val Thr Ala Ala Ser Asp Cys Trp
                195                 200                 205

ttg ctg aag cgt cac aag gac ctg gga ctc cgc ctc tat gtg gag act 1155
Leu Leu Lys Arg His Lys Asp Leu Gly Leu Arg Leu Tyr Val Glu Thr
            210                 215                 220

gag gac ggg cac agc gtg gat cct ggc ctg gcc ggc ctg ctg ggt caa 1203
Glu Asp Gly His Ser Val Asp Pro Gly Leu Ala Gly Leu Leu Gly Gln
        225                 230                 235

cgg gcc cca cgc tcc caa cag cct ttc gtg gtc act ttc ttc agg gcc     1251
Arg Ala Pro Arg Ser Gln Gln Pro Phe Val Val Thr Phe Phe Arg Ala
    240                 245                 250

agt ccg agt ccc atc cgc acc cct cgg gca gtg agg cca ctg agg agg     1299
Ser Pro Ser Pro Ile Arg Thr Pro Arg Ala Val Arg Pro Leu Arg Arg
255                 260                 265                 270

agg cag ccg aag aaa agc aac gag ctg ccg cag gcc aac cga ctc cca     1347
Arg Gln Pro Lys Lys Ser Asn Glu Leu Pro Gln Ala Asn Arg Leu Pro
                275                 280                 285

ggg atc ttt gat gac gtc cac ggc tcc cac ggc cgg cag gtc tgc cgt     1395
Gly Ile Phe Asp Asp Val His Gly Ser His Gly Arg Gln Val Cys Arg
            290                 295                 300

cgg cac gag ctc tac gtc agc ttc cag gac ctc ggc tgg ctg gac tgg     1443
Arg His Glu Leu Tyr Val Ser Phe Gln Asp Leu Gly Trp Leu Asp Trp
        305                 310                 315

gtc atc gct ccc caa ggc tac tcg gcc tat tac tgt gag ggg gag tgc     1491
Val Ile Ala Pro Gln Gly Tyr Ser Ala Tyr Tyr Cys Glu Gly Glu Cys
    320                 325                 330

tcc ttc cca ctg gac tcc tgc atg aat gcc acc aac cac gcc atc ctg     1539
Ser Phe Pro Leu Asp Ser Cys Met Asn Ala Thr Asn His Ala Ile Leu
335                 340                 345                 350

cag tcc ctg gtg cac ctg atg aag cca aac gca gtc ccc aag gcg tgc     1587
Gln Ser Leu Val His Leu Met Lys Pro Asn Ala Val Pro Lys Ala Cys
                355                 360                 365

tgt gca ccc acc aag ctg agc gcc acc tct gtg ctc tac tat gac agc     1635
Cys Ala Pro Thr Lys Leu Ser Ala Thr Ser Val Leu Tyr Tyr Asp Ser
            370                 375                 380

agc aac aac gtc atc ctg cgc aaa gcc cgc aac atg gtg gtc aag gcc     1683
Ser Asn Asn Val Ile Leu Arg Lys Ala Arg Asn Met Val Val Lys Ala
        385                 390                 395

tgc ggc tgc cac tgagtcagcc cgcccagccc tactgcag                      1723
Cys Gly Cys His
    400


<210> 6
<211> 402
<212> PRT
<213> Homo sapiens

<400> 6
Met Thr Ala Leu Pro Gly Pro Leu Trp Leu Leu Gly Leu Ala Leu Cys
1               5                   10                  15

Ala Leu Gly Gly Gly Gly Pro Gly Leu Arg Pro Pro Pro Gly Cys Pro
            20                  25                  30

Gln Arg Arg Leu Gly Ala Arg Glu Arg Arg Asp Val Gln Arg Glu Ile
        35                  40                  45

Leu Ala Val Leu Gly Leu Pro Gly Arg Pro Arg Pro Arg Ala Pro Pro
    50                  55                  60

Ala Ala Ser Arg Leu Pro Ala Ser Ala Pro Leu Phe Met Leu Asp Leu

65                  70                  75                  80

Tyr His Ala Met Ala Gly Asp Asp Asp Glu Asp Gly Ala Pro Ala Glu
                85                  90                  95

Arg Arg Leu Gly Arg Ala Asp Leu Val Met Ser Phe Val Asn Met Val
            100                 105                 110

Glu Arg Asp Arg Ala Leu Gly His Gln Glu Pro His Trp Lys Glu Phe
        115                 120                 125

Arg Phe Asp Leu Thr Gln Ile Pro Ala Gly Glu Ala Val Thr Ala Ala
    130                 135                 140

Glu Phe Arg Ile Tyr Lys Val Pro Ser Ile His Leu Leu Asn Arg Thr
145                 150                 155                 160

Leu His Val Ser Met Phe Gln Val Val Gln Glu Gln Ser Asn Arg Glu
                165                 170                 175

Ser Asp Leu Phe Phe Leu Asp Leu Gln Thr Leu Arg Ala Gly Asp Glu
            180                 185                 190

Gly Trp Leu Val Leu Asp Val Thr Ala Ala Ser Asp Cys Trp Leu Leu
        195                 200                 205

Lys Arg His Lys Asp Leu Gly Leu Arg Leu Tyr Val Glu Thr Glu Asp
    210                 215                 220

Gly His Ser Val Asp Pro Gly Leu Ala Gly Leu Leu Gly Gln Arg Ala
225                 230                 235                 240

Pro Arg Ser Gln Gln Pro Phe Val Val Thr Phe Phe Arg Ala Ser Pro
                245                 250                 255

Ser Pro Ile Arg Thr Pro Arg Ala Val Arg Pro Leu Arg Arg Arg Gln
            260                 265                 270

Pro Lys Lys Ser Asn Glu Leu Pro Gln Ala Asn Arg Leu Pro Gly Ile
        275                 280                 285

Phe Asp Asp Val His Gly Ser His Gly Arg Gln Val Cys Arg Arg His
    290                 295                 300

Glu Leu Tyr Val Ser Phe Gln Asp Leu Gly Trp Leu Asp Trp Val Ile
305                 310                 315                 320

Ala Pro Gln Gly Tyr Ser Ala Tyr Tyr Cys Glu Gly Glu Cys Ser Phe
                325                 330                 335

Pro Leu Asp Ser Cys Met Asn Ala Thr Asn His Ala Ile Leu Gln Ser
            340                 345                 350

Leu Val His Leu Met Lys Pro Asn Ala Val Pro Lys Ala Cys Cys Ala
        355                 360                 365

Pro Thr Lys Leu Ser Ala Thr Ser Val Leu Tyr Tyr Asp Ser Ser Asn
    370                 375                 380

Asn Val Ile Leu Arg Lys Ala Arg Asn Met Val Val Lys Ala Cys Gly
385                 390                 395                 400

Cys His

<210> 7
<211> 1926
<212> DNA
<213> Mus musculus

<220>
<221> CDS
<222> (93)..(1289)

<400> 7
gccaggcaca ggtgcgccgt ctggtcctcc ccgtctggcg tcagccgagc ccgaccagct 60

accagtggat gcgcgccggc tgaaagtccg ag atg gct atg cgt ccc ggg cca 113
Met Ala Met Arg Pro Gly Pro
1               5

ctc tgg cta ttg ggc ctt gct ctg tgc gcg ctg gga ggc ggc cac ggt 161
Leu Trp Leu Leu Gly Leu Ala Leu Cys Ala Leu Gly Gly Gly His Gly
        10                  15                  20

ccg cgt ccc ccg cac acc tgt ccc cag cgt cgc ctg gga gcg cgc gag 209
Pro Arg Pro Pro His Thr Cys Pro Gln Arg Arg Leu Gly Ala Arg Glu
    25                  30                  35

cgc cgc gac atg cag cgt gaa atc ctg gcg gtg ctc ggg cta ccg gga 257
Arg Arg Asp Met Gln Arg Glu Ile Leu Ala Val Leu Gly Leu Pro Gly
40                  45                  50                  55

cgg ccc cga ccc cgt gca caa ccc gcc gct gcc cgg cag cca gcg tcc 305
Arg Pro Arg Pro Arg Ala Gln Pro Ala Ala Ala Arg Gln Pro Ala Ser
                60                  65                  70

gcg ccc ctc ttc atg ttg gac cta tac cac gcc atg acc gat gac gac 353
Ala Pro Leu Phe Met Leu Asp Leu Tyr His Ala Met Thr Asp Asp Asp
            75                  80                  85

gac ggc ggg cca cca cag gct cac tta ggc cgt gcc gac ctg gtc atg 401
Asp Gly Gly Pro Pro Gln Ala His Leu Gly Arg Ala Asp Leu Val Met
        90                  95                  100

agc ttc gtc aac atg gtg gaa cgc gac cgt acc ctg ggc tac cag gag 449
Ser Phe Val Asn Met Val Glu Arg Asp Arg Thr Leu Gly Tyr Gln Glu
    105                 110                 115

cca cac tgg aag gaa ttc cac ttt gac cta acc cag atc cct gct ggg 497
Pro His Trp Lys Glu Phe His Phe Asp Leu Thr Gln Ile Pro Ala Gly
120                 125                 130                 135

gag gct gtc aca gct gct gag ttc cgg atc tac aaa gaa ccc agc acc 545
Glu Ala Val Thr Ala Ala Glu Phe Arg Ile Tyr Lys Glu Pro Ser Thr
                140                 145                 150

cac ccg ctc aac aca acc ctc cac atc agc atg ttc gaa gtg gtc caa 593
His Pro Leu Asn Thr Thr Leu His Ile Ser Met Phe Glu Val Val Gln
            155                 160                 165

gag cac tcc aac agg gag tct gac ttg ttc ttt ttg gat ctt cag acg 641
Glu His Ser Asn Arg Glu Ser Asp Leu Phe Phe Leu Asp Leu Gln Thr
        170                 175                 180

ctc cga tct ggg gac gag ggc tgg ctg gtg ctg gac atc aca gca gcc 689
Leu Arg Ser Gly Asp Glu Gly Trp Leu Val Leu Asp Ile Thr Ala Ala
    185                 190                 195

agt gac cga tgg ctg ctg aac cat cac aag gac ctg gga ctc cgc ctc 737
Ser Asp Arg Trp Leu Leu Asn His His Lys Asp Leu Gly Leu Arg Leu
200                 205                 210                 215

tat gtg gaa acc gcg gat ggg cac agc atg gat cct ggc ctg gct ggt 785
Tyr Val Glu Thr Ala Asp Gly His Ser Met Asp Pro Gly Leu Ala Gly
                220                 225                 230

ctg ctt gga cga caa gca cca cgc tcc aga cag cct ttc atg gta acc 833
Leu Leu Gly Arg Gln Ala Pro Arg Ser Arg Gln Pro Phe Met Val Thr
            235                 240                 245

ttc ttc agg gcc agc cag agt cct gtg cgg gcc cct cgg gca gcg aga 881
Phe Phe Arg Ala Ser Gln Ser Pro Val Arg Ala Pro Arg Ala Ala Arg
        250                 255                 260

cca ctg aag agg agg cag cca aag aaa acg aac gag ctt ccg cac ccc 929
Pro Leu Lys Arg Arg Gln Pro Lys Lys Thr Asn Glu Leu Pro His Pro
    265                 270                 275

aac aaa ctc cca ggg atc ttt gat gat ggc cac ggt tcc cgc ggc aga 977
Asn Lys Leu Pro Gly Ile Phe Asp Asp Gly His Gly Ser Arg Gly Arg
280                 285                 290                 295

gag gtt tgc cgc agg cat gag ctc tac gtc agc ttc cgt gac ctt ggc 1025
Glu Val Cys Arg Arg His Glu Leu Tyr Val Ser Phe Arg Asp Leu Gly
                300                 305                 310

tgg ctg gac tgg gtc atc gcc ccc cag ggc tac tct gcc tat tac tgt 1073
Trp Leu Asp Trp Val Ile Ala Pro Gln Gly Tyr Ser Ala Tyr Tyr Cys
            315                 320                 325

gag ggg gag tgt gct ttc cca ctg gac tcc tgt atg aac gcc acc aac 1121
Glu Gly Glu Cys Ala Phe Pro Leu Asp Ser Cys Met Asn Ala Thr Asn
        330                 335                 340

cat gcc atc ttg cag tct ctg gtg cac ctg atg aag cca gat gtt gtc 1169
His Ala Ile Leu Gln Ser Leu Val His Leu Met Lys Pro Asp Val Val
    345                 350                 355

ccc aag gca tgc tgt gca ccc acc aaa ctg agt gcc acc tct gtg ctg 1217
Pro Lys Ala Cys Cys Ala Pro Thr Lys Leu Ser Ala Thr Ser Val Leu
360                 365                 370                 375

tac tat gac agc agc aac aat gtc atc ctg cgt aaa cac cgt aac atg 1265
Tyr Tyr Asp Ser Ser Asn Asn Val Ile Leu Arg Lys His Arg Asn Met
                380                 385                 390

gtg gtc aag gcc tgt ggc tgc cac tgaggccccg cccagcatcc tgcttctact 1319
Val Val Lys Ala Cys Gly Cys His
            395

accttaccat ctggccgggc ccctctccag aggcagaaac ccttctatgt tatcatagct 1379

cagacagggg caatgggagg cccttcactt cccctggcca cttcctgcta aaattctggt 1439

ctttcccagt tcctctgtcc ttcatggggt ttcggggcta tcaccccgcc ctctccatcc 1499

tcctacccca agcatagact gaatgcacac agcatcccag agctatgcta actgagaggt 1559

ctggggtcag cactgaaggc ccacatgagg aagactgatc cttggccatc ctcagcccac 1619

aatggcaaat tctggatggt ctaagaaggc cctggaattc taaactagat gatctgggct 1679

ctctgcacca ttcattgtgg cagttgggac atttttaggt ataacagaca catacactta 1739

gatcaatgca tcgctgtact ccttgaaatc agagctagct tgttagaaaa agaatcagag 1799

ccaggtatag cggtgcatgt cattaatccc agcgctaaag agacagagac aggagaatct 1859

ctgtgagttc aaggccacat agaaagagcc tgtctcggga gcaggaaaaa aaaaaaaaac 1919

ggaattc 1926

<210> 8
<211> 399
<212> PRT
<213> Mus musculus

<400> 8
Met Ala Met Arg Pro Gly Pro Leu Trp Leu Leu Gly Leu Ala Leu Cys
1               5                   10                  15

Ala Leu Gly Gly Gly His Gly Pro Arg Pro Pro His Thr Cys Pro Gln
            20                  25                  30

Arg Arg Leu Gly Ala Arg Glu Arg Arg Asp Met Gln Arg Glu Ile Leu
        35                  40                  45

Ala Val Leu Gly Leu Pro Gly Arg Pro Arg Pro Arg Ala Gln Pro Ala
    50                  55                  60

Ala Ala Arg Gln Pro Ala Ser Ala Pro Leu Phe Met Leu Asp Leu Tyr
65                  70                  75                  80

His Ala Met Thr Asp Asp Asp Asp Gly Gly Pro Pro Gln Ala His Leu
                85                  90                  95

Gly Arg Ala Asp Leu Val Met Ser Phe Val Asn Met Val Glu Arg Asp
            100                 105                 110

Arg Thr Leu Gly Tyr Gln Glu Pro His Trp Lys Glu Phe His Phe Asp
        115                 120                 125

Leu Thr Gln Ile Pro Ala Gly Glu Ala Val Thr Ala Ala Glu Phe Arg
    130                 135                 140

Ile Tyr Lys Glu Pro Ser Thr His Pro Leu Asn Thr Thr Leu His Ile
145                 150                 155                 160

Ser Met Phe Glu Val Val Gln Glu His Ser Asn Arg Glu Ser Asp Leu
                165                 170                 175

Phe Phe Leu Asp Leu Gln Thr Leu Arg Ser Gly Asp Glu Gly Trp Leu
            180                 185                 190

Val Leu Asp Ile Thr Ala Ala Ser Asp Arg Trp Leu Leu Asn His His
        195                 200                 205

Lys Asp Leu Gly Leu Arg Leu Tyr Val Glu Thr Ala Asp Gly His Ser
    210                 215                 220

Met Asp Pro Gly Leu Ala Gly Leu Leu Gly Arg Gln Ala Pro Arg Ser
225                 230                 235                 240

Arg Gln Pro Phe Met Val Thr Phe Phe Arg Ala Ser Gln Ser Pro Val
                245                 250                 255

Arg Ala Pro Arg Ala Ala Arg Pro Leu Lys Arg Arg Gln Pro Lys Lys
                260                 265                 270

Thr Asn Glu Leu Pro His Pro Asn Lys Leu Pro Gly Ile Phe Asp Asp
            275                 280                 285

Gly His Gly Ser Arg Gly Arg Glu Val Cys Arg Arg His Glu Leu Tyr
    290                 295                 300

Val Ser Phe Arg Asp Leu Gly Trp Leu Asp Trp Val Ile Ala Pro Gln
305                 310                 315                 320

Gly Tyr Ser Ala Tyr Tyr Cys Glu Gly Glu Cys Ala Phe Pro Leu Asp
                325                 330                 335

Ser Cys Met Asn Ala Thr Asn His Ala Ile Leu Gln Ser Leu Val His
            340                 345                 350

Leu Met Lys Pro Asp Val Val Pro Lys Ala Cys Cys Ala Pro Thr Lys
        355                 360                 365

Leu Ser Ala Thr Ser Val Leu Tyr Tyr Asp Ser Ser Asn Asn Val Ile
    370                 375                 380

Leu Arg Lys His Arg Asn Met Val Val Lys Ala Cys Gly Cys His
385                 390                 395

<210> 9
<211> 399
<212> PRT
<213> Mus musculus

<400> 9
Met Ala Ala Arg Pro Gly Leu Leu Trp Leu Leu Gly Leu Ala Leu Cys
  1               5                  10                  15

Val Leu Gly Gly Gly His Leu Ser His Pro Pro His Val Phe Pro Gln
                 20                  25                  30

Arg Arg Leu Gly Val Arg Glu Pro Arg Asp Met Gln Arg Glu Ile Arg
         35                  40                  45

Glu Val Leu Gly Leu Ala Gly Arg Pro Arg Ser Arg Ala Pro Val Gly
     50                  55                  60

Ala Ala Gln Gln Pro Ala Ser Ala Pro Leu Phe Met Leu Asp Leu Tyr
 65                  70                  75                  80

Arg Ala Met Thr Asp Asp Ser Gly Gly Gly Thr Pro Gln Pro His Leu
                 85                  90                  95

Asp Arg Ala Asp Leu Ile Met Ser Phe Val Asn Ile Val Glu Arg Asp
                100                 105                 110

Arg Thr Leu Gly Tyr Gln Glu Pro His Trp Lys Glu Phe His Phe Asp
            115                 120                 125

Leu Thr Gln Ile Pro Ala Gly Glu Ala Val Thr Ala Ala Glu Phe Arg
    130                 135                 140

Ile Tyr Lys Glu Pro Ser Thr His Pro Leu Asn Thr Thr Leu His Ile
145                 150                 155                 160

Ser Met Phe Glu Val Val Gln Glu His Ser Asn Arg Glu Ser Asp Leu
                165                 170                 175

Phe Phe Leu Asp Leu Gln Thr Leu Arg Ser Gly Asp Glu Gly Trp Leu
            180                 185                 190

Val Leu Asp Ile Thr Ala Ala Ser Asp Arg Trp Leu Leu Asn His His
        195                 200                 205

Lys Asp Leu Gly Leu Arg Leu Tyr Val Glu Thr Glu Asp Gly His Ser
    210                 215                 220

Ile Asp Pro Gly Leu Ala Gly Leu Leu Gly Arg Gln Ala Pro Arg Ser
225                 230                 235                 240

Arg Gln Pro Phe Met Val Gly Phe Phe Arg Ala Asn Gln Ser Pro Val
                245                 250                 255

Arg Ala Pro Arg Thr Ala Arg Pro Leu Lys Lys Lys Gln Leu Asn Gln
            260                 265                 270

Ile Asn Gln Leu Pro His Ser Asn Lys His Leu Gly Ile Leu Asp Asp
        275                 280                 285

Gly His Gly Ser His Gly Arg Glu Val Cys Arg Arg His Glu Leu Tyr
    290                 295                 300

Val Ser Phe Arg Asp Leu Gly Trp Leu Asp Ser Val Ile Ala Pro Gln
305                 310                 315                 320

Gly Tyr Ser Ala Tyr Tyr Cys Ala Gly Glu Cys Ile Tyr Pro Leu Asn
                325                 330                 335

Ser Cys Met Asn Ser Thr Asn His Ala Thr Met Gln Ala Leu Val His
            340                 345                 350

Leu Met Lys Pro Asp Ile Ile Pro Lys Val Cys Cys Val Pro Thr Glu
        355                 360                 365

Leu Ser Ala Ile Ser Leu Leu Tyr Tyr Asp Arg Asn Asn Asn Val Ile
    370                 375                 380

Leu Arg Arg Glu Arg Asn Met Val Val Gln Ala Cys Gly Cys His
385                 390                 395


<210> 10
<211> 396
<212> PRT
<213> Homo sapiens

<400> 10
Met Val Ala Gly Thr Arg Cys Leu Leu Ala Leu Leu Leu Pro Gln Val
1               5                   10                  15

Leu Leu Gly Gly Ala Ala Gly Leu Val Pro Glu Leu Gly Arg Arg Lys
            20                  25                  30

Phe Ala Ala Ala Ser Ser Gly Arg Pro Ser Ser Gln Pro Ser Asp Glu
        35                  40                  45

Val Leu Ser Glu Phe Glu Leu Arg Leu Leu Ser Met Phe Gly Leu Lys
    50                  55                  60

Gln Arg Pro Thr Pro Ser Arg Asp Ala Val Val Pro Pro Tyr Met Leu

65                  70                  75                  80

Asp Leu Tyr Arg Arg His Ser Gly Gln Pro Gly Ser Pro Ala Pro Asp
                85                  90                  95

His Arg Leu Glu Arg Ala Ala Ser Arg Ala Asn Thr Val Arg Ser Phe
            100                 105                 110

His His Glu Glu Ser Leu Glu Glu Leu Pro Glu Thr Ser Gly Lys Thr
        115                 120                 125

Thr Arg Arg Phe Phe Phe Asn Leu Ser Ser Ile Pro Thr Glu Glu Phe
    130                 135                 140

Ile Thr Ser Ala Glu Leu Gln Val Phe Arg Glu Gln Met Gln Asp Ala
145                 150                 155                 160

Leu Gly Asn Asn Ser Ser Phe His His Arg Ile Asn Ile Tyr Glu Ile
                165                 170                 175

Ile Lys Pro Ala Thr Ala Asn Ser Lys Phe Pro Val Thr Arg Leu Leu
            180                 185                 190

Asp Thr Arg Leu Val Asn Gln Asn Ala Ser Arg Trp Glu Ser Phe Asp
        195                 200                 205

Val Thr Pro Ala Val Met Arg Trp Thr Ala Gln Gly His Ala Asn His
    210                 215                 220

Gly Phe Val Val Glu Val Ala His Leu Glu Glu Lys Gln Gly Val Ser
225                 230                 235                 240

Lys Arg His Val Arg Ile Ser Arg Ser Leu His Gln Asp Glu His Ser
                245                 250                 255

Trp Ser Gln Ile Arg Pro Leu Leu Val Thr Phe Gly His Asp Gly Lys
            260                 265                 270

Gly His Pro Leu His Lys Arg Glu Lys Arg Gln Ala Lys His Lys Gln
        275                 280                 285

Arg Lys Arg Leu Lys Ser Ser Cys Lys Arg His Pro Leu Tyr Val Asp
    290                 295                 300

Phe Ser Asp Val Gly Trp Asn Asp Trp Ile Val Ala Pro Pro Gly Tyr
305                 310                 315                 320

His Ala Phe Tyr Cys His Gly Glu Cys Pro Phe Pro Leu Ala Asp His
                325                 330                 335

Leu Asn Ser Thr Asn His Ala Ile Val Gln Thr Leu Val Asn Ser Val
            340                 345                 350

Asn Ser Lys Ile Pro Lys Ala Cys Cys Val Pro Thr Glu Leu Ser Ala
        355                 360                 365

Ile Ser Met Leu Tyr Leu Asp Glu Asn Glu Lys Val Val Leu Lys Asn
    370                 375                 380

Tyr Gln Asp Met Val Val Glu Gly Cys Gly Cys Arg
385                 390                 395

<210> 11
<211> 408

<212> PRT
<213> Homo sapiens

<400> 11


<210> 12
<211> 472
<212> PRT
<213> Homo sapiens

<400> 12
Met Ala Gly Ala Ser Arg Leu Leu Phe Leu Trp Leu Gly Cys Phe Cys
1               5                   10                  15

Val Ser Leu Ala Gln Gly Glu Arg Pro Lys Pro Pro Phe Pro Glu Leu
            20                  25                  30

Arg Lys Ala Val Pro Gly Asp Arg Thr Ala Gly Gly Gly Pro Asp Ser
        35                  40                  45

Glu Leu Gln Pro Gln Asp Lys Val Ser Glu His Met Leu Arg Leu Tyr
    50                  55                  60

Asp Arg Tyr Ser Thr Val Gln Ala Ala Arg Thr Pro Gly Ser Leu Glu
65                  70                  75                  80

Gly Gly Ser Gln Pro Trp Arg Pro Arg Leu Leu Arg Glu Gly Asn Thr
                85                  90                  95

Val Arg Ser Phe Arg Ala Ala Ala Ala Glu Thr Leu Glu Arg Lys Gly
            100                 105                 110

Leu Tyr Ile Phe Asn Leu Thr Ser Leu Thr Lys Ser Glu Asn Ile Leu
        115                 120                 125

Ser Ala Thr Leu Tyr Phe Cys Ile Gly Glu Leu Gly Asn Ile Ser Leu
    130                 135                 140

Ser Cys Pro Val Ser Gly Gly Cys Ser His His Ala Gln Arg Lys His
145                 150                 155                 160

Ile Gln Ile Asp Leu Ser Ala Trp Thr Leu Lys Phe Ser Arg Asn Gln
                165                 170                 175

Ser Gln Leu Leu Gly His Leu Ser Val Asp Met Ala Lys Ser His Arg
            180                 185                 190

Asp Ile Met Ser Trp Leu Ser Lys Asp Ile Thr Gln Phe Leu Arg Lys
        195                 200                 205

Ala Lys Glu Asn Glu Glu Phe Leu Ile Gly Phe Asn Ile Thr Ser Lys
    210                 215                 220

Gly Arg Gln Leu Pro Lys Arg Arg Leu Pro Phe Pro Glu Pro Tyr Ile
225                 230                 235                 240

Leu Val Tyr Ala Asn Asp Ala Ala Ile Ser Glu Pro Glu Ser Val Val
                245                 250                 255

Ser Ser Leu Gln Gly His Arg Asn Phe Pro Thr Gly Thr Val Pro Lys
            260                 265                 270

Trp Asp Ser His Ile Arg Ala Ala Leu Ser Ile Glu Arg Arg Lys Lys
        275                 280                 285

Arg Ser Thr Gly Val Leu Leu Pro Leu Gln Asn Asn Glu Leu Pro Gly
    290                 295                 300

Ala Glu Tyr Gln Tyr Lys Lys Asp Glu Val Trp Glu Glu Arg Lys Pro
305                 310                 315                 320

Tyr Lys Thr Leu Gln Ala Gln Ala Pro Glu Lys Ser Lys Asn Lys Lys
                325                 330                 335

Lys Gln Arg Lys Gly Pro His Arg Lys Ser Gln Thr Leu Gln Phe Asp
            340                 345                 350

Glu Gln Thr Leu Lys Lys Ala Arg Arg Lys Gln Trp Ile Glu Pro Arg
        355                 360                 365

Asn Cys Ala Arg Arg Tyr Leu Lys Val Asp Phe Ala Asp Ile Gly Trp
    370                 375                 380

Ser Glu Trp Ile Ile Ser Pro Lys Ser Phe Asp Ala Tyr Tyr Cys Ser
385                 390                 395                 400

Gly Ala Cys Gln Phe Pro Met Pro Lys Ser Leu Lys Pro Ser Asn His
                405                 410                 415

Ala Thr Ile Gln Ser Ile Val Arg Ala Val Gly Val Val Pro Gly Ile
            420                 425                 430

Pro Glu Pro Cys Cys Val Pro Glu Lys Met Ser Ser Leu Ser Ile Leu
        435                 440                 445

Phe Phe Asp Glu Asn Lys Asn Val Val Leu Lys Val Tyr Pro Asn Met
    450                 455                 460

Thr Val Glu Ser Cys Ala Cys Arg
465                 470


<210> 13
<211> 453
<212> PRT
<213> Homo sapiens

<400> 13
Met His Leu Thr Val Phe Leu Leu Lys Gly Ile Val Gly Phe Leu Trp
  1               5                  10                  15

Ser Cys Trp Val Leu Val Gly Tyr Ala Lys Gly Gly Leu Gly Asp Asn
             20                  25                  30

His Val His Ser Ser Phe Ile Tyr Arg Arg Leu Arg Asn His Glu Arg
         35                  40                  45

Arg Glu Ile Gln Arg Glu Ile Leu Ser Ile Leu Gly Leu Pro His Arg
50                  55                  60

Pro Arg Pro Phe Ser Pro Gly Lys Gln Ala Ser Ser Ala Pro Leu Phe
65                  70                  75                  80

Met Leu Asp Leu Tyr Asn Ala Met Thr Asn Glu Glu Asn Pro Glu Glu
                85                  90                  95

Ser Glu Tyr Ser Val Arg Ala Ser Leu Ala Glu Glu Thr Arg Gly Ala
            100                 105                 110

Arg Lys Gly Tyr Pro Ala Ser Pro Asn Gly Tyr Pro Arg Arg Ile Gln
        115                 120                 125

Leu Ser Arg Thr Thr Pro Leu Thr Thr Gln Ser Pro Pro Leu Ala Ser
    130                 135                 140

Leu His Asp Thr Asn Phe Leu Asn Asp Ala Asp Met Val Met Ser Phe
145                 150                 155                 160

Val Asn Leu Val Glu Arg Asp Lys Asp Phe Ser His Gln Arg Arg His
                165                 170                 175

Tyr Lys Glu Arg Phe Asp Leu Thr Gln Ile Pro His Gly Glu Ala Val
            180                 185                 190

Thr Ala Ala Glu Phe Arg Ile Tyr Lys Asp Arg Ser Asn Asn Arg Phe
        195                 200                 205

Glu Asn Glu Thr Ile Lys Ile Ser Ile Tyr Gln Ile Ile Lys Glu Tyr
    210                 215                 220

Thr Asn Arg Asp Ala Asp Leu Phe Leu Leu Asp Thr Arg Lys Ala Gln
225                 230                 235                 240

Ala Leu Asp Val Gly Trp Leu Val Phe Asp Ile Thr Val Thr Ser Asn
                245                 250                 255

His Trp Val Ile Asn Pro Gln Asn Asn Leu Gly Leu Gln Leu Cys Ala
            260                 265                 270

Glu Thr Gly Asp Gly Arg Ser Ile Asn Val Lys Ser Ala Gly Leu Val
        275                 280                 285

Gly Arg Gln Gly Pro Gln Ser Lys Gln Pro Phe Met Val Ala Phe Phe
    290                 295                 300

Lys Ala Ser Glu Val Leu Leu Arg Ser Val Arg Ala Ala Asn Lys Arg
305                 310                 315                 320

Lys Asn Gln Asn Arg Asn Lys Ser Ser Ser His Gln Asp Ser Ser Arg
                325                 330                 335

Met Ser Ser Val Gly Asp Tyr Asn Thr Ser Glu Gln Lys Gln Ala Cys
            340                 345                 350

Lys Lys His Glu Leu Tyr Val Ser Phe Arg Asp Leu Gly Trp Gln Asp
        355                 360                 365

Trp Ile Ile Ala Pro Glu Gly Tyr Ala Ala Phe Tyr Cys Asp Gly Glu
    370                 375                 380

Cys Ser Phe Pro Leu Asn Ala His Met Asn Ala Thr Asn His Ala Ile
385                 390                 395                 400

Val Gln Thr Leu Val His Leu Met Phe Pro Asp His Val Pro Lys Pro
                405                 410                 415

Cys Cys Ala Pro Thr Lys Leu Asn Ala Ile Ser Val Leu Tyr Phe Asp
            420                 425                 430

Asp Ser Ser Asn Val Ile Leu Lys Lys Tyr Arg Asn Met Val Val Arg
        435                 440                 445

Ser Cys Gly Cys His
    450

<210> 14
<211> 513
<212> PRT
<213> Homo sapiens

<400> 14

<210> 15
<211> 424
<212> PRT
<213> Homo sapiens

<400> 15
Met Gly Ser Leu Val Leu Thr Leu Cys Ala Leu Phe Cys Leu Ala Ala
1               5                   10                  15

Tyr Leu Val Ser Gly Ser Pro Ile Met Asn Leu Glu Gln Ser Pro Leu

            20                  25                  30

Glu Glu Asp Met Ser Leu Phe Gly Asp Val Phe Ser Glu Gln Asp Gly
        35                  40                  45

Val Asp Phe Asn Thr Leu Leu Gln Ser Met Lys Asp Glu Phe Leu Lys
    50                  55                  60

Thr Leu Asn Leu Ser Asp Ile Pro Thr Gln Asp Ser Ala Lys Val Asp
65                  70                  75                  80

Pro Pro Glu Tyr Met Leu Glu Leu Tyr Asn Lys Phe Ala Thr Asp Arg
                85                  90                  95

Thr Ser Met Pro Ser Ala Asn Ile Ile Arg Ser Phe Lys Asn Glu Asp
            100                 105                 110

Leu Phe Ser Gln Pro Val Ser Phe Asn Gly Leu Arg Lys Tyr Pro Leu
        115                 120                 125

Leu Phe Asn Val Ser Ile Pro His His Glu Glu Val Ile Met Ala Glu
    130                 135                 140

Leu Arg Leu Tyr Thr Leu Val Gln Arg Asp Arg Met Ile Tyr Asp Gly
145                 150                 155                 160

Val Asp Arg Lys Ile Thr Ile Phe Glu Val Leu Glu Ser Lys Gly Asp
                165                 170                 175

Asn Glu Gly Glu Arg Asn Met Leu Val Leu Val Ser Gly Glu Ile Tyr
            180                 185                 190

Gly Thr Asn Ser Glu Trp Glu Thr Phe Asp Val Thr Asp Ala Ile Arg
        195                 200                 205

Arg Trp Gln Lys Ser Gly Ser Ser Thr His Gln Leu Glu Val His Ile
    210                 215                 220

Glu Ser Lys His Asp Glu Ala Glu Asp Ala Ser Ser Gly Arg Leu Glu
225                 230                 235                 240

Ile Asp Thr Ser Ala Gln Asn Lys His Asn Pro Leu Leu Ile Val Phe
                245                 250                 255

Ser Asp Asp Gln Ser Ser Asp Lys Glu Arg Lys Glu Glu Leu Asn Glu
            260                 265                 270

Met Ile Ser His Glu Gln Leu Pro Glu Leu Asp Asn Leu Gly Leu Asp
        275                 280                 285

Ser Phe Ser Ser Gly Pro Gly Glu Glu Ala Leu Leu Gln Met Arg Ser
    290                 295                 300

Asn Ile Ile Tyr Asp Ser Thr Ala Arg Ile Arg Arg Asn Ala Lys Gly
305                 310                 315                 320

Asn Tyr Cys Lys Arg Thr Pro Leu Tyr Ile Asp Phe Lys Glu Ile Gly
                325                 330                 335

Trp Asp Ser Trp Ile Ile Ala Pro Pro Gly Tyr Glu Ala Tyr Glu Cys
            340                 345                 350

Arg Gly Val Cys Asn Tyr Pro Leu Ala Glu His Leu Thr Pro Thr Lys
        355                 360                 365

His Ala Ile Ile Gln Ala Leu Val His Leu Lys Asn Ser Gln Lys Ala
        370                 375                 380

Ser Lys Ala Cys Cys Val Pro Thr Lys Leu Glu Pro Ile Ser Ile Leu
385                 390                 395                 400

Tyr Leu Asp Lys Gly Val Val Thr Tyr Lys Phe Lys Tyr Glu Gly Met
                405                 410                 415

Ala Val Ser Glu Cys Gly Cys Arg
            420

<210> 16
<211> 392
<212> PRT
<213> Homo sapiens

<400> 16


<210> 17
<211> 280
<212> PRT
<213> Homo sapiens

<400> 17
Val Asp Gly Gln Asn Trp Thr Phe Ala Phe Asp Phe Ser Phe Leu Ser
1               5                   10                  15

Gln Gln Glu Asp Leu Ala Trp Ala Glu Leu Arg Leu Gln Leu Ser Ser
            20                  25                  30

Pro Val Asp Leu Pro Thr Glu Gly Ser Leu Ala Ile Glu Ile Phe His
        35                  40                  45

Gln Pro Lys Pro Asp Thr Glu Gln Ala Ser Asp Ser Cys Leu Glu Arg
    50                  55                  60

Phe Gln Met Asp Leu Phe Thr Val Thr Leu Ser Gln Val Thr Phe Ser
65                  70                  75                  80

Leu Gly Ser Met Val Leu Glu Val Thr Arg Pro Leu Ser Lys Trp Leu
                85                  90                  95

Lys Arg Pro Gly Ala Leu Glu Lys Gln Met Ser Arg Val Ala Gly Glu
            100                 105                 110

Cys Trp Pro Arg Pro Pro Thr Pro Pro Ala Thr Asn Val Leu Leu Met
        115                 120                 125

Leu Tyr Ser Asn Leu Ser Gln Glu Gln Arg Gln Leu Gly Gly Ser Thr
    130                 135                 140

Leu Leu Trp Glu Ala Glu Ser Ser Trp Arg Ala Gln Glu Gly Gln Leu
145                 150                 155                 160

Ser Trp Glu Trp Gly Lys Arg His Arg Arg His His Leu Pro Asp Arg
                165                 170                 175

Ser Gln Leu Cys Arg Lys Val Lys Phe Gln Val Asp Phe Asn Leu Ile
            180                 185                 190

Gly Trp Gly Ser Trp Ile Ile Tyr Pro Lys Gln Tyr Asn Ala Tyr Arg
        195                 200                 205

Cys Glu Gly Glu Cys Pro Asn Pro Val Gly Glu Glu Phe His Pro Thr
    210                 215                 220

Asn His Ala Tyr Ile Gln Ser Leu Leu Lys Arg Tyr Gln Pro His Arg
225                 230                 235                 240

Val Pro Ser Thr Cys Cys Ala Pro Val Lys Thr Lys Pro Leu Ser Met
                245                 250                 255

Leu Tyr Val Asp Asn Gly Arg Val Leu Leu Asp His His Lys Asp Met
            260                 265                 270

Ile Val Glu Glu Cys Gly Cys Leu
        275                 280


<210> 18
<211> 366
<212> PRT
<213> Homo sapiens

<400> 18


<210> 19
<211> 366
<212> PRT
<213> Homo sapiens

<400> 19
Met Trp Pro Leu Trp Leu Cys Trp Ala Leu Trp Val Leu Pro Leu Ala
1               5                   10                  15

Gly Pro Gly Ala Ala Leu Thr Glu Glu Gln Leu Leu Gly Ser Leu Leu
            20                  25                  30

Arg Gln Leu Gln Leu Ser Glu Val Pro Val Leu Asp Arg Ala Asp Met
        35                  40                  45

Glu Lys Leu Val Ile Pro Ala His Val Arg Ala Gln Tyr Val Val Leu
    50                  55                  60

Leu Arg Arg Ser His Gly Asp Arg Ser Arg Gly Lys Arg Phe Ser Gln
65                  70                  75                  80

Ser Phe Arg Glu Val Ala Gly Arg Phe Leu Ala Ser Glu Ala Ser Thr
                85                  90                  95

His Leu Leu Val Phe Gly Met Glu Gln Arg Leu Pro Pro Asn Ser Glu
            100                 105                 110

Leu Val Gln Ala Val Leu Arg Leu Phe Gln Glu Pro Val Pro Lys Ala

        115                 120                 125

Ala Leu His Arg His Gly Arg Leu Ser Pro Arg Ser Ala Gln Ala Arg
    130                 135                 140

Val Thr Val Glu Trp Leu Arg Val Arg Asp Asp Gly Ser Asn Arg Thr
145                 150                 155                 160

Ser Leu Ile Asp Ser Arg Leu Val Ser Val His Glu Ser Gly Trp Lys
                165                 170                 175

Ala Phe Asp Val Thr Glu Ala Val Asn Phe Trp Gln Gln Leu Ser Arg
            180                 185                 190

Pro Arg Gln Pro Leu Leu Leu Gln Val Ser Val Gln Arg Glu His Leu
        195                 200                 205

Gly Pro Leu Ala Ser Gly Ala His Lys Leu Val Arg Phe Ala Ser Gln
    210                 215                 220

Gly Ala Pro Ala Gly Leu Gly Glu Pro Gln Leu Glu Leu His Thr Leu
225                 230                 235                 240

Asp Leu Arg Asp Tyr Gly Ala Gln Gly Asp Cys Asp Pro Glu Ala Pro
                245                 250                 255

Met Thr Glu Gly Thr Arg Cys Cys Arg Gln Glu Met Tyr Ile Asp Leu
            260                 265                 270

Gln Gly Met Lys Trp Ala Lys Asn Trp Val Leu Glu Pro Pro Gly Phe
        275                 280                 285

Leu Ala Tyr Glu Cys Val Gly Thr Cys Gln Gln Pro Pro Glu Ala Leu
    290                 295                 300

Ala Phe Asn Trp Pro Phe Leu Gly Pro Arg Gln Cys Ile Ala Ser Glu
305                 310                 315                 320

Thr Ala Ser Leu Pro Met Ile Val Ser Ile Lys Glu Gly Gly Arg Thr
                325                 330                 335

Arg Pro Gln Val Val Ser Leu Pro Asn Met Arg Val Gln Lys Cys Ser
            340                 345                 350

Cys Ala Ser Asp Gly Ala Leu Val Pro Arg Arg Leu Gln Pro
        355                 360                 365

<210> 20
<211> 347
<212> PRT
<213> Homo sapiens

<400> 20
Met His Ala His Cys Leu Pro Phe Leu Leu His Ala Trp Trp Ala Leu
1               5                   10                  15

Leu Gln Ala Gly Ala Ala Thr Val Ala Thr Ala Leu Leu Arg Thr Arg
            20                  25                  30

Gly Gln Pro Ser Ser Pro Ser Pro Leu Ala Tyr Met Leu Ser Leu Tyr
        35                  40                  45

Arg Asp Pro Leu Pro Arg Ala Asp Ile Ile Arg Ser Leu Gln Ala Glu
    50                  55                  60

Asp Val Ala Val Asp Gly Gln Asn Trp Thr Phe Ala Phe Asp Phe Ser
65                  70                  75                  80

Phe Leu Ser Gln Gln Glu Asp Leu Ala Trp Ala Glu Leu Arg Leu Gln
                85                  90                  95

Leu Ser Ser Pro Val Asp Leu Pro Thr Glu Gly Ser Leu Ala Ile Glu
            100                 105                 110

Ile Phe His Gln Pro Lys Pro Asp Thr Glu Gln Ala Ser Asp Ser Cys
        115                 120                 125

Leu Glu Arg Phe Gln Met Asp Leu Phe Thr Val Thr Leu Ser Gln Val
    130                 135                 140

Thr Phe Ser Leu Gly Ser Met Val Leu Glu Val Thr Arg Pro Leu Ser
145                 150                 155                 160

Lys Trp Leu Lys Arg Pro Gly Ala Leu Glu Lys Gln Met Ser Arg Val
                165                 170                 175

Ala Gly Glu Cys Trp Pro Arg Pro Pro Thr Pro Pro Ala Thr Asn Val
            180                 185                 190

Leu Leu Met Leu Tyr Ser Asn Leu Ser Gln Glu Gln Arg Gln Leu Gly
        195                 200                 205

Gly Ser Thr Leu Leu Trp Glu Ala Glu Ser Ser Trp Arg Ala Gln Glu
    210                 215                 220

Gly Gln Leu Ser Trp Glu Trp Gly Lys Arg His Arg Arg His His Leu
225                 230                 235                 240

Pro Asp Arg Ser Gln Leu Cys Arg Lys Val Lys Phe Gln Val Asp Phe
                245                 250                 255

Asn Leu Ile Gly Trp Gly Ser Trp Ile Ile Tyr Pro Lys Gln Tyr Asn
            260                 265                 270

Ala Tyr Arg Cys Glu Gly Glu Cys Pro Asn Pro Val Gly Glu Glu Phe
        275                 280                 285

His Pro Thr Asn His Ala Tyr Ile Gln Ser Leu Leu Lys Arg Tyr Gln
    290                 295                 300

Pro His Arg Val Pro Ser Thr Cys Cys Ala Pro Val Lys Thr Lys Pro
305                 310                 315                 320

Leu Ser Met Leu Tyr Val Asp Asn Gly Arg Val Leu Leu Asp His His
                325                 330                 335

Lys Asp Met Ile Val Glu Glu Cys Gly Cys Leu
            340                 345


<210> 21
<211> 395
<212> PRT
<213> Strongylocentrotus purpuratus

<400> 21
Met Asp Val Ser Lys Val Leu Ile Leu Thr Leu Ile Trp Leu Leu Thr
1               5                   10                  15

Ala Asp Ser Ala Pro Pro Asp Tyr Val Thr Leu Thr Arg Lys Met Glu
            20                  25                  30

Ser Lys Ile Leu Arg Val Met Gly Leu Ser Glu Arg Pro Arg Pro Lys
        35                  40                  45

Pro Asn Ala Thr Ala Pro Gln Tyr Met Trp Asp Leu Tyr Arg Gln Gln
    50                  55                  60

Met Ala Ala Thr Glu Gly Ala Ala Ala Ser Arg Gly Gly Glu Thr Glu
65                  70                  75                  80

Ile Gly Lys Glu Glu Glu Glu Asp Gly Arg Pro Cys Ser Glu Thr Lys
                85                  90                  95

Leu Ser Ser Asn Ile Ile Arg Ser Val Ser His Thr Gly Gly Asp Leu
            100                 105                 110

Arg Ala Ser Asn Ser Thr Ser Leu Gln Gln Ile Leu Leu Phe Asp Val
        115                 120                 125

Ala Ser Ile Pro His Ala Glu Thr Ile Glu Ala Ala Asp Leu Arg Leu
    130                 135                 140

Glu Ile Pro Ala Leu Pro Ser Ala Thr Asp Val Pro Ser Leu Ala Val
145                 150                 155                 160

Arg Ile Tyr Gln Leu Glu Ser Arg Thr Arg Leu Asn Ser Ile Val Ser
                165                 170                 175

Leu Lys Asp Lys Arg Leu Arg Leu Leu Asp Val Val Leu Ala Asp Leu
            180                 185                 190

Ser Gln Gly Tyr Ala Gly Thr Ile Asp Ile Leu Ser Thr Val Asn Ser
        195                 200                 205

Trp Arg Ser Lys Lys Thr Ser Asn His Gly Leu Leu Leu His Val Glu
    210                 215                 220

Leu Met Ser Thr Ser Gly Asn Asn Arg Arg Gly Ser Gln Val Ile Lys
225                 230                 235                 240

Glu Leu Gly Ala Ile Ser Lys Lys Cys Thr Ala Asn Leu Ile Val Thr
                245                 250                 255

Ser Ser Glu Tyr Arg Gln Cys Ser Lys Arg Asn Arg Arg Asn Lys Arg
            260                 265                 270

Gln Ala Glu Ser Glu Ala Pro Ala Asp Ile Ser Ser Phe Pro Thr Ala
        275                 280                 285

Ser Leu Thr Asn Leu Cys Gln Arg His Arg Leu Phe Val Ser Phe Arg
    290                 295                 300

Asp Val Gly Trp Glu Asn Trp Ile Ile Ala Pro Met Gly Tyr Gln Ala
305                 310                 315                 320

Tyr Tyr Cys Asp Gly Glu Cys Pro Phe Pro Leu Gly Glu Arg Leu Asn
                325                 330                 335

Gly Thr Asn His Ala Ile Ile Gln Thr Leu Val Asn Ser Ile Asp Asn
            340                 345                 350

Arg Ala Val Pro Lys Val Cys Cys Ala Pro Thr Lys Leu Ser Gly Ile
        355                 360                 365

Ser Met Leu Tyr Phe Asp Asn Asn Glu Asn Val Val Leu Arg Gln Tyr
    370                 375                 380

Glu Asp Met Val Val Glu Ala Cys Gly Cys Arg
385                 390                 395

<210> 22
<211> 390
<212> PRT
<213> Xenopus sp.

<400> 22
Met Asp Leu Arg Lys Met Leu Gly Phe Ile Phe Leu Phe Phe Ile Ile
1               5                   10                  15

Gln Glu Cys Thr Ser Arg Pro Thr Asp Met Val Asp Ser Thr Asp Leu
            20                  25                  30

Glu Leu Glu Asp Pro Glu Leu Val Arg Ala Glu Ala Leu Lys Arg Leu
        35                  40                  45

Leu Glu Val Phe Gly Ile Glu Glu Pro Pro Gln Pro Leu Gln His Val
    50                  55                  60

Lys Gln Pro Pro Gln Tyr Met Val Asp Leu Tyr Asn Thr Val Ala Asp
65                  70                  75                  80

Glu Asp Gly Val Thr Lys Asp Pro Asp Leu Leu Glu Gly Asn Thr Val
                85                  90                  95

Arg Ser Phe Phe Asp Lys Ile His Ser Asp His Met His Phe Leu Phe
            100                 105                 110

Asn Leu Trp Thr Val Ala Arg Asn Glu Lys Ile Leu Thr Ala Glu Leu
        115                 120                 125

His Leu Phe Lys Leu Lys Pro Arg Pro Ser Glu Gln Ala Tyr Phe Lys
    130                 135                 140

Arg His His Phe Cys Gln Ile Ser Val Tyr Met Val Leu Asp Lys Asn
145                 150                 155                 160

Lys Ile Gln Leu Pro Gln Gly Arg Lys Leu Leu Ser Ser Lys Leu Val
                165                 170                 175

Pro Ile His Ser Ser Gly Trp Glu Val Phe Ser Ile Thr Gln Ala Val
            180                 185                 190

Arg Ala Trp Asn Asp Glu Ser Ala Asn His Gly Ile Leu Val Thr Val
        195                 200                 205

Arg Asn Leu Gly Gly Ala Gln Val Asp Pro Asn Ile Ile Arg Phe Ala
    210                 215                 220

Ser Gly Arg Asp His His Glu Ser Lys Gln Pro Met Leu Val Leu Phe
225                 230                 235                 240

Thr Asp Asp Gly Arg Arg Gly Ile Val Ser Val Asn Asn Gln Pro Asp
                245                 250                 255

Asp Gln Leu Met Pro Leu Pro Asn Val Pro Met Ala Pro Thr Ser Asn
            260                 265                 270

Arg Thr Arg Leu Gly Arg Ser Val Glu Glu Asp Gly Gln Leu Pro Cys
        275                 280                 285

Gln Arg His Pro Leu Tyr Val Asp Phe Glu Glu Ile Gly Trp Ser Gly
    290                 295                 300

Trp Ile Ile Ser Pro Arg Gly Tyr Asn Ala Tyr His Cys Lys Gly Ser
305                 310                 315                 320

Cys Pro Phe Pro Leu Gly Gln Asn Met Arg Pro Thr Asn His Ala Thr
                325                 330                 335

Val Gln Ser Ile Ile Asn Ala Leu Lys Leu Thr Lys Gly Val Ser Ser
            340                 345                 350

Pro Cys Cys Val Pro Asp Lys Leu Phe Ser Ile Asn Leu Leu Tyr Phe
        355                 360                 365

Asp Asp Asp Glu Asn Val Val Leu Lys Gln Tyr Asp Asp Met Val Ala
    370                 375                 380

Gly Ser Cys Gly Cys His
385                 390


<210> 23
<211> 588
<212> PRT
<213> Drosophila sp.

<400> 23
Met Arg Ala Trp Leu Leu Leu Leu Ala Val Leu Ala Thr Phe Gln Thr
  1               5                  10                  15

Ile Val Arg Val Ala Ser Thr Glu Asp Ile Ser Gln Arg Phe Ile Ala
             20                  25                  30

Ala Ile Ala Pro Val Ala Ala His Ile Pro Leu Ala Ser Ala Ser Gly
         35                  40                  45

Ser Gly Ser Gly Arg Ser Gly Ser Arg Ser Val Gly Ala Ser Thr Ser
     50                  55                  60

Thr Ala Leu Ala Lys Ala Phe Asn Pro Phe Ser Glu Pro Ala Ser Phe
 65                  70                  75                  80

Ser Asp Ser Asp Lys Ser His Arg Ser Lys Thr Asn Lys Lys Pro Ser
                 85                  90                  95

Lys Ser Asp Ala Asn Arg Gln Phe Asn Glu Val His Lys Pro Arg Thr
            100                 105                 110

Asp Gln Leu Glu Asn Ser Lys Asn Lys Ser Lys Gln Leu Val Asn Lys
        115                 120                 125

Pro Asn His Asn Lys Met Ala Val Lys Glu Gln Arg Ser His His Lys
    130                 135                 140

Lys Ser His His His Arg Ser His Gln Pro Lys Gln Ala Ser Ala Ser
145                 150                 155                 160

Thr Glu Ser His Gln Ser Ser Ser Ile Glu Ser Ile Phe Val Glu Glu
                165                 170                 175

Pro Thr Leu Val Leu Asp Arg Glu Val Ala Ser Ile Asn Val Pro Ala

180                 185                 190

Asn Ala Lys Ala Ile Ile Ala Glu Gln Gly Pro Ser Thr Tyr Ser Lys
        195                 200                 205

Glu Ala Leu Ile Lys Asp Lys Leu Lys Pro Asp Pro Ser Thr Leu Val
    210                 215                 220

Glu Ile Glu Lys Ser Leu Leu Ser Leu Phe Asn Met Lys Arg Pro Pro
225                 230                 235                 240

Lys Ile Asp Arg Ser Lys Ile Ile Ile Pro Glu Pro Met Lys Lys Leu
                245                 250                 255

Tyr Ala Glu Ile Asn Gly His Glu Leu Asp Ser Val Asn Ile Pro Lys
            260                 265                 270

Pro Gly Leu Leu Thr Lys Ser Ala Asn Thr Val Arg Ser Phe Thr His
        275                 280                 285

Lys Asp Ser Lys Ile Asp Asp Arg Phe Pro His His His Arg Phe Arg
    290                 295                 300

Leu His Phe Asp Val Lys Ser Ile Pro Ala Asp Glu Lys Leu Lys Ala
305                 310                 315                 320

Ala Glu Leu Gln Leu Thr Arg Asp Ala Leu Ser Gln Gln Val Val Ala
                325                 330                 335

Ser Arg Ser Ser Ala Asn Arg Thr Arg Tyr Gln Val Leu Val Tyr Asp
            340                 345                 350

Ile Thr Arg Val Gly Val Arg Gly Gln Arg Glu Pro Ser Tyr Leu Leu
        355                 360                 365

Leu Asp Thr Lys Thr Val Arg Leu Asn Ser Thr Asp Thr Val Ser Leu
    370                 375                 380

Asp Val Gln Pro Ala Val Asp Arg Trp Leu Ala Ser Pro Gln Arg Asn
385                 390                 395                 400

Tyr Gly Leu Leu Val Glu Val Arg Thr Val Arg Ser Leu Lys Pro Ala
                405                 410                 415

Pro His His His Val Arg Leu Arg Arg Ser Ala Asp Glu Ala His Glu
            420                 425                 430

Arg Trp Gln His Lys Gln Pro Leu Leu Phe Thr Tyr Thr Asp Asp Gly
        435                 440                 445

Arg His Lys Ala Arg Ser Ile Arg Asp Val Ser Gly Gly Glu Gly Gly
    450                 455                 460

Gly Lys Gly Gly Arg Asn Lys Arg His Ala Arg Arg Pro Thr Arg Arg
465                 470                 475                 480

Lys Asn His Asp Asp Thr Cys Arg Arg His Ser Leu Tyr Val Asp Phe
                485                 490                 495

Ser Asp Val Gly Trp Asp Asp Trp Ile Val Ala Pro Leu Gly Tyr Asp
            500                 505                 510

Ala Tyr Tyr Cys His Gly Lys Cys Pro Phe Pro Leu Ala Asp His Phe
        515                 520                 525

Asn Ser Thr Asn His Ala Val Val Gln Thr Leu Val Asn Asn Met Asn
        530                 535                 540

Pro Gly Lys Val Pro Lys Ala Cys Cys Val Pro Thr Gln Leu Asp Ser
545                 550                 555                 560

Val Ala Met Leu Tyr Leu Asn Asp Gln Ser Thr Val Val Leu Lys Asn
                565                 570                 575

Tyr Gln Glu Met Thr Val Val Gly Cys Gly Cys Arg
            580                 585


<210> 24
<211> 360
<212> PRT
<213> Mus musculus

<400> 24
Met Val Trp Leu Arg Leu Trp Ala Phe Leu His Ile Leu Ala Ile Val
  1               5                  10                  15

Thr Leu Asp Pro Glu Leu Lys Arg Arg Glu Glu Leu Phe Leu Arg Ser
             20                  25                  30

Leu Gly Phe Ser Ser Lys Pro Asn Pro Val Ser Pro Pro Pro Val Pro
         35                  40                  45

Ser Ile Leu Trp Arg Ile Phe Asn Gln Arg Met Gly Ser Ser Ile Gln
     50                  55                  60

Lys Lys Lys Pro Asp Leu Cys Phe Val Glu Glu Phe Asn Val Pro Gly
 65                  70                  75                  80

Ser Val Ile Arg Val Phe Pro Asp Gln Gly Arg Phe Ile Ile Pro Tyr
                 85                  90                  95

Ser Asp Asp Ile His Pro Thr Gln Cys Leu Gly Lys Arg Leu Phe Phe
            100                 105                 110

Asn Ile Ser Ala Ile Glu Lys Glu Glu Arg Val Thr Met Gly Ser Gly
        115                 120                 125

Ile Glu Val Gln Pro Glu His Leu Leu Arg Lys Gly Ile Asp Leu Arg
    130                 135                 140

Leu Tyr Arg Thr Leu Gln Ile Thr Leu Lys Gly Met Gly Arg Ser Lys
145                 150                 155                 160

Thr Ser Arg Lys Leu Leu Val Ala Gln Thr Phe Arg Leu Leu His Lys
                165                 170                 175

Ser Leu Phe Phe Asn Leu Thr Glu Ile Cys Gln Ser Trp Gln Asp Pro
            180                 185                 190

Leu Lys Asn Leu Gly Leu Val Leu Glu Ile Phe Pro Lys Lys Glu Ser
        195                 200                 205

Ser Trp Met Ser Thr Ala Asn Asp Glu Cys Lys Asp Ile Gln Thr Phe
    210                 215                 220

Leu Tyr Thr Ser Leu Leu Thr Val Thr Leu Asn Pro Leu Arg Cys Lys
225                 230                 235                 240

Arg Pro Arg Arg Lys Arg Ser Tyr Ser Lys Leu Pro Phe Thr Ala Ser

245                 250                 255

Asn Ile Cys Lys Lys Arg His Leu Tyr Val Glu Phe Lys Asp Val Gly
            260                 265                 270

Trp Gln Asn Trp Val Ile Ala Pro Gln Gly Tyr Met Ala Asn Tyr Cys
        275                 280                 285

Tyr Gly Glu Cys Pro Tyr Pro Leu Thr Glu Ile Leu Asn Gly Ser Asn
    290                 295                 300

His Ala Ile Leu Gln Thr Leu Val His Ser Ile Glu Pro Glu Asp Ile
305                 310                 315                 320

Pro Leu Pro Cys Cys Val Pro Thr Lys Met Ser Pro Ile Ser Met Leu
                325                 330                 335

Phe Tyr Asp Asn Asn Asp Asn Val Val Leu Arg His Tyr Glu Asn Met
            340                 345                 350

Ala Val Asp Glu Cys Gly Cys Arg
        355                 360

<210> 25
<211> 438
<212> PRT
<213> Mus musculus

<400> 25
Met Arg Lys Met Gln Lys Glu Ile Leu Ser Val Leu Gly Pro Pro His
1               5                   10                  15

Arg Pro Arg Pro Leu His Gly Leu Gln Gln Pro Gln Pro Pro Val Leu
            20                  25                  30

Pro Pro Gln Gln Gln Gln Gln Gln Gln Gln Gln Gln Thr Ala Asp Glu
        35                  40                  45

Glu Pro Pro Pro Gly Arg Leu Lys Ser Ala Pro Leu Phe Met Leu Asp
    50                  55                  60

Leu Tyr Asn Ala Leu Ser Asn Asp Asp Glu Glu Asp Gly Ala Ser Glu
65                  70                  75                  80

Gly Val Gly Gln Glu Pro Gly Ser His Gly Gly Ala Ser Ser Ser Gln
                85                  90                  95

Leu Arg Gln Pro Ser Pro Gly Ala Ala His Ser Leu Asn Arg Lys Ser
            100                 105                 110

Leu Leu Ala Pro Gly Pro Gly Gly Gly Ala Ser Pro Leu Thr Ser Ala
        115                 120                 125

Gln Asp Ser Ala Phe Leu Asn Asp Ala Asp Met Val Met Ser Phe Val
    130                 135                 140

Asn Leu Val Gly Tyr Asp Lys Glu Phe Ser Pro His Gln Arg His His
145                 150                 155                 160

Lys Glu Phe Lys Phe Asn Leu Ser Gln Ile Pro Glu Gly Glu Ala Val
                165                 170                 175

Thr Ala Ala Glu Phe Arg Val Tyr Lys Asp Cys Val Val Gly Ser Phe
            180                 185                 190

Lys Asn Gln Thr Phe Leu Ile Ser Ile Tyr Gln Val Leu Gln Glu Ala
        195                 200                 205

Gln His Arg Asp Ser Asp Leu Phe Leu Leu Asp Thr Arg Val Val Trp
    210                 215                 220

Ala Ser Glu Glu Gly Trp Leu Glu Phe Asp Ile Thr Ala Thr Ser Asn
225                 230                 235                 240

Leu Trp Val Val Ile Pro Gln His Asn Met Gly Leu Gln Leu Ser Val
                245                 250                 255

Val Thr Arg Asp Gly Leu His Val Asn Pro Arg Ala Ala Gly Leu Val
            260                 265                 270

Gly Arg Asp Gly Pro Tyr Asp Lys Gln Pro Phe Met Val Ala Phe Phe
        275                 280                 285

Lys Val Ser Glu Val His Val Arg Thr Thr Arg Ser Ala Ser Ser Arg
    290                 295                 300

Arg Arg Gln Gln Ser Arg Asn Arg Ser Thr Gln Ser Gln Asp Val Ser
305                 310                 315                 320

Arg Gly Ser Gly Ser Ser Asp Tyr Asn Gly Ser Glu Leu Lys Thr Ala
                325                 330                 335

Cys Lys Lys His Glu Leu Tyr Val Ser Phe Gln Asp Leu Gly Trp Gln
            340                 345                 350

Asp Trp Ile Ile Ala Pro Lys Gly Tyr Ala Ala Asn Tyr Cys Asp Gly
        355                 360                 365

Glu Cys Ser Phe Pro Leu Asn Ala His Met Asn Ala Thr Asn His Ala
    370                 375                 380

Ile Val Gln Thr Leu Val His Leu Met Asn Pro Glu Thr Val Pro Lys
385                 390                 395                 400

Pro Cys Cys Ala Pro Thr Lys Leu Asn Ala Ile Ser Val Leu Tyr Phe
                405                 410                 415

Asp Asp Asn Ser Asn Val Ile Leu Lys Lys Tyr Arg Asn Met Val Val
            420                 425                 430

Arg Ala Cys Gly Cys His
        435


<210> 26
<211> 455
<212> PRT
<213> Drosophila sp.

<400> 26
Met Ser Gly Leu Arg Asn Thr Ser Glu Ala Val Ala Val Leu Ala Ser
  1               5                  10                  15

Leu Gly Leu Gly Met Val Leu Leu Met Phe Val Ala Thr Thr Pro Pro
                 20                  25                  30

Ala Val Glu Ala Thr Gln Ser Gly Ile Tyr Ile Asp Asn Gly Lys Asp
         35                  40                  45

Gln Thr Ile Met His Arg Val Leu Ser Glu Asp Asp Lys Leu Asp Val
50                  55                  60

Ser Tyr Glu Ile Leu Glu Phe Leu Gly Ile Ala Glu Arg Pro Thr His
65                  70                  75                  80

Leu Ser Ser His Gln Leu Ser Leu Arg Lys Ser Ala Pro Lys Phe Leu
                85                  90                  95

Leu Asp Val Tyr His Arg Ile Thr Ala Glu Glu Gly Leu Ser Asp Gln
            100                 105                 110

Asp Glu Asp Asp Asp Tyr Glu Arg Gly His Arg Ser Arg Arg Ser Ala
        115                 120                 125

Asp Leu Glu Glu Asp Glu Gly Glu Gln Gln Lys Asn Phe Ile Thr Asp
    130                 135                 140

Leu Asp Lys Arg Ala Ile Asp Glu Ser Asp Ile Ile Met Thr Phe Leu
145                 150                 155                 160

Asn Lys Arg His His Asn Val Asp Glu Leu Arg His Glu His Gly Arg
                165                 170                 175

Arg Leu Trp Phe Asp Val Ser Asn Val Pro Asn Asp Asn Tyr Leu Val
            180                 185                 190

Met Ala Glu Leu Arg Ile Tyr Gln Asn Ala Asn Glu Gly Lys Trp Leu
        195                 200                 205

Thr Ala Asn Arg Glu Phe Thr Ile Thr Val Tyr Ala Ile Gly Thr Gly
    210                 215                 220

Thr Leu Gly Gln His Thr Met Glu Pro Leu Ser Ser Val Asn Thr Thr
225                 230                 235                 240

Gly Asp Tyr Val Gly Trp Leu Glu Leu Asn Val Thr Glu Gly Leu His
                245                 250                 255

Glu Trp Leu Val Lys Ser Lys Asp Asn His Gly Ile Tyr Ile Gly Ala
            260                 265                 270

His Ala Val Asn Arg Pro Asp Arg Glu Val Lys Leu Asp Asp Ile Gly
        275                 280                 285

Leu Ile His Arg Lys Val Asp Asp Glu Phe Gln Pro Phe Met Ile Gly
    290                 295                 300

Phe Phe Arg Gly Pro Glu Leu Ile Lys Ala Thr Ala His Ser Ser His
305                 310                 315                 320

His Arg Ser Lys Arg Ser Ala Ser His Pro Arg Lys Arg Lys Lys Ser
                325                 330                 335

Val Ser Pro Asn Asn Val Pro Leu Leu Glu Pro Met Glu Ser Thr Arg
            340                 345                 350

Ser Cys Gln Met Gln Thr Leu Tyr Ile Asp Phe Lys Asp Leu Gly Trp
        355                 360                 365

His Asp Trp Ile Ile Ala Pro Glu Gly Tyr Gly Ala Phe Tyr Cys Ser
    370                 375                 380

Gly Glu Cys Asn Phe Pro Leu Asn Ala His Met Asn Ala Thr Asn His
385                 390                 395                 400

Ala Ile Val Gln Thr Leu Val His Leu Leu Glu Pro Lys Lys Val Pro
                405                 410                 415

Lys Pro Cys Cys Ala Pro Thr Arg Leu Gly Ala Leu Pro Val Leu Tyr
            420                 425                 430

His Leu Asn Asp Glu Asn Val Asn Leu Lys Lys Tyr Arg Asn Met Ile
        435                 440                 445

Val Lys Ser Cys Gly Cys His
    450                 455


<210> 27
<211> 372
<212> PRT
<213> Homo sapiens

<400> 27
Met Pro Pro Pro Gln Gln Gly Pro Cys Gly His His Leu Leu Leu Leu
  1               5                  10                  15

Leu Ala Leu Leu Leu Pro Ser Leu Pro Leu Thr Arg Ala Pro Val Pro
             20                  25                  30

Pro Gly Pro Ala Ala Ala Leu Leu Gln Ala Leu Gly Leu Arg Asp Glu
         35                  40                  45

Pro Gln Gly Ala Pro Arg Leu Arg Pro Val Pro Pro Val Met Trp Arg
     50                  55                  60

Leu Phe Arg Arg Arg Asp Pro Gln Glu Thr Arg Ser Gly Ser Arg Arg
 65                  70                  75                  80

Thr Ser Pro Gly Val Thr Leu Gln Pro Cys His Val Glu Glu Leu Gly
                 85                  90                  95

Val Ala Gly Asn Ile Val Arg His Ile Pro Asp Arg Gly Ala Pro Thr
            100                 105                 110

Arg Ala Ser Glu Pro Val Ser Ala Ala Gly His Cys Pro Glu Trp Thr
        115                 120                 125

Val Val Phe Asp Leu Ser Ala Val Glu Pro Ala Glu Arg Pro Ser Arg
    130                 135                 140

Ala Arg Leu Glu Leu Arg Phe Ala Ala Ala Ala Ala Ala Ala Pro Glu
145                 150                 155                 160

Gly Gly Trp Glu Leu Ser Val Ala Gln Ala Gly Gln Gly Ala Gly Ala
                165                 170                 175

Asp Pro Gly Pro Val Leu Leu Arg Gln Leu Val Pro Ala Leu Gly Pro
            180                 185                 190

Pro Val Arg Ala Glu Leu Leu Gly Ala Ala Trp Ala Arg Asn Ala Ser
        195                 200                 205

Trp Pro Arg Ser Leu Arg Leu Ala Leu Ala Leu Arg Pro Arg Ala Pro
    210                 215                 220

Ala Ala Cys Ala Arg Leu Ala Glu Ala Ser Leu Leu Leu Val Thr Leu
225                 230                 235                 240

Asp Pro Arg Leu Cys His Pro Leu Ala Arg Pro Arg Arg Asp Ala Glu
                245                 250                 255

Pro Val Leu Gly Gly Gly Pro Gly Gly Ala Cys Arg Ala Arg Arg Leu
            260                 265                 270

Tyr Val Ser Phe Arg Glu Val Gly Trp His Arg Trp Val Ile Ala Pro
        275                 280                 285

Arg Gly Phe Leu Ala Asn Tyr Cys Gln Gly Gln Cys Ala Leu Pro Val
    290                 295                 300

Ala Leu Ser Gly Ser Gly Gly Pro Pro Ala Leu Asn His Ala Val Leu
305                 310                 315                 320

Arg Ala Leu Met His Ala Ala Ala Pro Gly Ala Ala Asp Leu Pro Cys
                325                 330                 335

Cys Val Pro Ala Arg Leu Ser Pro Ile Ser Val Leu Phe Phe Asp Asn
            340                 345                 350

Ser Asp Asn Val Val Leu Arg Gln Tyr Glu Asp Met Val Val Asp Glu
        355                 360                 365

Cys Gly Cys Arg
    370


<210> 28
<211> 429
<212> PRT
<213> Homo sapiens

<400> 28


<210> 29
<211> 364
<212> PRT
<213> Homo sapiens

<400> 29

<210> 30
<211> 501
<212> PRT

<213> Homo sapiens

<400> 30
Met Arg Leu Pro Lys Leu Leu Thr Phe Leu Leu Trp Tyr Leu Ala Trp
1               5                   10                  15

Leu Asp Leu Glu Phe Ile Cys Thr Val Leu Gly Ala Pro Asp Leu Gly
            20                  25                  30

Gln Arg Pro Gln Gly Thr Arg Pro Gly Leu Ala Lys Ala Glu Ala Lys
        35                  40                  45

Glu Arg Pro Pro Leu Ala Arg Asn Val Phe Arg Pro Gly Gly His Ser
    50                  55                  60

Tyr Gly Gly Gly Ala Thr Asn Ala Asn Ala Arg Ala Lys Gly Gly Thr
65                  70                  75                  80

Gly Gln Thr Gly Gly Leu Thr Gln Pro Lys Lys Asp Glu Pro Lys Lys
                85                  90                  95

Leu Pro Pro Arg Pro Gly Gly Pro Glu Pro Lys Pro Gly His Pro Pro
            100                 105                 110

Gln Thr Arg Gln Ala Thr Ala Arg Thr Val Thr Pro Lys Gly Gln Leu
        115                 120                 125

Pro Gly Gly Lys Ala Pro Pro Lys Ala Gly Ser Val Pro Ser Ser Phe
    130                 135                 140

Leu Leu Lys Lys Ala Arg Glu Pro Gly Pro Pro Arg Glu Pro Lys Glu
145                 150                 155                 160

Pro Phe Arg Pro Pro Pro Ile Thr Pro His Glu Tyr Met Leu Ser Leu
                165                 170                 175

Tyr Arg Thr Leu Ser Asp Ala Asp Arg Lys Gly Gly Asn Ser Ser Val
            180                 185                 190

Lys Leu Glu Ala Gly Leu Ala Asn Thr Ile Thr Ser Phe Ile Asp Lys
        195                 200                 205

Gly Gln Asp Asp Arg Gly Pro Val Val Arg Lys Gln Arg Tyr Val Phe
    210                 215                 220

Asp Ile Ser Ala Leu Glu Lys Asp Gly Leu Leu Gly Ala Glu Leu Arg
225                 230                 235                 240

Ile Leu Arg Lys Lys Pro Ser Asp Thr Ala Lys Pro Ala Ala Pro Gly
                245                 250                 255

Gly Gly Arg Ala Ala Gln Leu Lys Leu Ser Ser Cys Pro Ser Gly Arg
            260                 265                 270

Gln Pro Ala Ser Leu Leu Asp Val Arg Ser Val Pro Gly Leu Asp Gly
        275                 280                 285

Ser Gly Trp Glu Val Phe Asp Ile Trp Lys Leu Phe Arg Asn Phe Lys
    290                 295                 300

Asn Ser Ala Gln Leu Cys Leu Glu Leu Glu Ala Trp Glu Arg Gly Arg
305                 310                 315                 320

Ala Val Asp Leu Arg Gly Leu Gly Phe Asp Arg Ala Ala Arg Gln Val
                325                 330                 335

His Glu Lys Ala Leu Phe Leu Val Phe Gly Arg Thr Lys Lys Arg Asp
            340                 345                 350

Leu Phe Phe Asn Glu Ile Lys Ala Arg Ser Gly Gln Asp Asp Lys Thr
        355                 360                 365

Val Tyr Glu Tyr Leu Phe Ser Gln Arg Arg Lys Arg Arg Ala Pro Leu
    370                 375                 380

Ala Thr Arg Gln Gly Lys Arg Pro Ser Lys Asn Leu Lys Ala Arg Cys
385                 390                 395                 400

Ser Arg Lys Ala Leu His Val Asn Phe Lys Asp Met Gly Trp Asp Asp
                405                 410                 415

Trp Ile Ile Ala Pro Leu Glu Tyr Glu Ala Phe His Cys Glu Gly Leu
            420                 425                 430

Cys Glu Phe Pro Leu Arg Ser His Leu Glu Pro Thr Asn His Ala Val
        435                 440                 445

Ile Gln Thr Leu Met Asn Ser Met Asp Pro Glu Ser Thr Pro Pro Thr
    450                 455                 460

Cys Cys Val Pro Thr Arg Leu Ser Pro Ile Ser Ile Leu Phe Ile Asp
465                 470                 475                 480

Ser Ala Asn Asn Val Val Tyr Lys Gln Tyr Glu Asp Met Val Val Glu
                485                 490                 495

Ser Cys Gly Cys Arg
            500



<210> 31
<211> 455
<212> PRT
<213> Homo sapiens

<400> 31

<210> 32

<211> 450
<212> PRT
<213> Homo sapiens

<400> 32
Met Asp Leu Ser Ala Ala Ala Ala Leu Cys Leu Trp Leu Leu Ser Ala
1               5                   10                  15

Cys Arg Pro Arg Asp Gly Leu Glu Ala Ala Ala Val Leu Arg Ala Ala
            20                  25                  30

Gly Ala Gly Pro Val Arg Ser Pro Gly Gly Gly Gly Gly Gly Gly Gly
        35                  40                  45

Gly Gly Arg Thr Leu Ala Gln Ala Ala Gly Ala Ala Ala Val Pro Ala
    50                  55                  60

Ala Ala Val Pro Arg Ala Arg Ala Ala Arg Arg Ala Ala Gly Ser Gly
65                  70                  75                  80

Phe Arg Asn Gly Ser Val Val Pro His His Phe Met Met Ser Leu Tyr
                85                  90                  95

Arg Ser Leu Ala Gly Arg Ala Pro Ala Gly Ala Ala Ala Val Ser Ala
            100                 105                 110

Ser Gly His Gly Arg Ala Asp Thr Ile Thr Gly Phe Thr Asp Gln Ala
        115                 120                 125

Thr Gln Asp Glu Ser Ala Ala Glu Thr Gly Gln Ser Phe Leu Phe Asp
    130                 135                 140

Val Ser Ser Leu Asn Asp Ala Asp Glu Val Val Gly Ala Glu Leu Arg
145                 150                 155                 160

Val Leu Arg Arg Gly Ser Pro Glu Ser Gly Pro Gly Ser Trp Thr Ser
                165                 170                 175

Pro Pro Leu Leu Leu Leu Ser Thr Cys Pro Gly Ala Ala Arg Ala Pro
            180                 185                 190

Arg Leu Leu Tyr Ser Arg Ala Ala Glu Pro Leu Val Gly Gln Arg Trp
        195                 200                 205

Glu Ala Phe Asp Val Ala Asp Ala Met Arg Arg His Arg Arg Glu Pro
    210                 215                 220

Arg Pro Pro Arg Ala Phe Cys Leu Leu Leu Arg Ala Val Ala Gly Pro
225                 230                 235                 240

Val Pro Ser Pro Leu Ala Leu Arg Arg Leu Gly Phe Gly Trp Pro Gly
                245                 250                 255

Gly Gly Gly Ser Ala Ala Glu Glu Arg Ala Val Leu Val Val Ser Ser
            260                 265                 270

Arg Thr Gln Arg Lys Glu Ser Leu Phe Arg Glu Ile Arg Ala Gln Ala
        275                 280                 285

Arg Ala Leu Gly Ala Ala Leu Ala Ser Glu Pro Leu Pro Asp Pro Gly
    290                 295                 300

Thr Gly Thr Ala Ser Pro Arg Ala Val Ile Gly Gly Arg Arg Arg Arg
305                 310                 315                 320

Arg Thr Ala Leu Ala Gly Thr Arg Thr Ser Gln Gly Ser Gly Gly Gly
                325                 330                 335

Ala Gly Arg Gly His Gly Arg Arg Gly Arg Ser Arg Cys Ser Arg Lys
            340                 345                 350

Pro Leu His Val Asp Phe Lys Glu Leu Gly Trp Asp Asp Trp Ile Ile
        355                 360                 365

Ala Pro Leu Asp Tyr Glu Ala Tyr His Cys Glu Gly Leu Cys Asp Phe
    370                 375                 380

Pro Leu Arg Ser His Leu Glu Pro Thr Asn His Ala Ile Ile Gln Thr
385                 390                 395                 400

Leu Leu Asn Ser Met Ala Pro Asp Ala Ala Pro Ala Ser Cys Cys Val
                405                 410                 415

Pro Ala Arg Leu Ser Pro Ile Ser Ile Leu Tyr Ile Asp Ala Ala Asn
            420                 425                 430

Asn Val Val Tyr Lys Gln Tyr Glu Asp Met Val Val Glu Ala Cys Gly
        435                 440                 445

Cys Arg
    450


<210> 33
<211> 375
<212> PRT
<213> Homo sapiens

<400> 33
Met Gln Lys Leu Gln Leu Cys Val Tyr Ile Tyr Leu Phe Met Leu Ile
  1               5                  10                  15

Val Ala Gly Pro Val Asp Leu Asn Glu Asn Ser Glu Gln Lys Glu Asn
             20                  25                  30

Val Glu Lys Glu Gly Leu Cys Asn Ala Cys Thr Trp Arg Gln Asn Thr
         35                  40                  45

Lys Ser Ser Arg Ile Glu Ala Ile Lys Ile Gln Ile Leu Ser Lys Leu
     50                  55                  60

Arg Leu Glu Thr Ala Pro Asn Ile Ser Lys Asp Val Ile Arg Gln Leu
 65                  70                  75                  80

Leu Pro Lys Ala Pro Pro Leu Arg Glu Leu Ile Asp Gln Tyr Asp Val
                 85                  90                  95

Gln Arg Asp Asp Ser Ser Asp Gly Ser Leu Glu Asp Asp Asp Tyr His
            100                 105                 110

Ala Thr Thr Glu Thr Ile Ile Thr Met Pro Thr Glu Ser Asp Phe Leu
        115                 120                 125

Met Gln Val Asp Gly Lys Pro Lys Cys Cys Phe Phe Lys Phe Ser Ser
    130                 135                 140

Lys Ile Gln Tyr Asn Lys Val Val Lys Ala Gln Leu Trp Ile Tyr Leu
145                 150                 155                 160

Arg Pro Val Glu Thr Pro Thr Thr Val Phe Val Gln Ile Leu Arg Leu

165                 170                 175

Ile Lys Pro Met Lys Asp Gly Thr Arg Tyr Thr Gly Ile Arg Ser Leu
            180                 185                 190

Lys Leu Asp Met Asn Pro Gly Thr Gly Ile Trp Gln Ser Ile Asp Val
        195                 200                 205

Lys Thr Val Leu Gln Asn Trp Leu Lys Gln Pro Glu Ser Asn Leu Gly
    210                 215                 220

Ile Glu Ile Lys Ala Leu Asp Glu Asn Gly His Asp Leu Ala Val Thr
225                 230                 235                 240

Phe Pro Gly Pro Gly Glu Asp Gly Leu Asn Pro Phe Leu Glu Val Lys
                245                 250                 255

Val Thr Asp Thr Pro Lys Arg Ser Arg Arg Asp Phe Gly Leu Asp Cys
            260                 265                 270

Asp Glu His Ser Thr Glu Ser Arg Cys Cys Arg Tyr Pro Leu Thr Val
        275                 280                 285

Asp Phe Glu Ala Phe Gly Trp Asp Trp Ile Ile Ala Pro Lys Arg Tyr
    290                 295                 300

Lys Ala Asn Tyr Cys Ser Gly Glu Cys Glu Phe Val Phe Leu Gln Lys
305                 310                 315                 320

Tyr Pro His Thr His Leu Val His Gln Ala Asn Pro Arg Gly Ser Ala
                325                 330                 335

Gly Pro Cys Cys Thr Pro Thr Lys Met Ser Pro Ile Asn Met Leu Tyr
            340                 345                 350

Phe Asn Gly Lys Glu Gln Ile Ile Tyr Gly Lys Ile Pro Ala Met Val
        355                 360                 365

Val Asp Arg Cys Gly Cys Ser
    370                 375


<210> 34
<211> 454
<212> PRT
<213> Homo sapiens

<400> 34
Met Ala Arg Pro Asn Lys Phe Leu Leu Trp Phe Cys Cys Phe Ala Trp
1               5                   10                  15

Leu Cys Phe Pro Ile Ser Leu Gly Ser Gln Ala Ser Gly Gly Glu Ala
            20                  25                  30

Gln Ile Ala Ala Ser Ala Glu Leu Glu Ser Gly Ala Met Pro Trp Ser
        35                  40                  45

Leu Leu Gln His Ile Asp Glu Arg Asp Arg Ala Gly Leu Leu Pro Ala
    50                  55                  60

Leu Phe Lys Val Leu Ser Val Gly Arg Gly Gly Ser Pro Arg Leu Gln
65                  70                  75                  80

Pro Asp Ser Arg Ala Leu His Tyr Met Lys Lys Leu Tyr Lys Thr Tyr
                85                  90                  95

Ala Thr Lys Glu Gly Ile Pro Lys Ser Asn Arg Ser His Leu Tyr Asn
            100                 105                 110

Thr Val Arg Leu Phe Thr Pro Cys Thr Arg His Lys Gln Ala Pro Gly
        115                 120                 125

Asp Gln Val Thr Gly Ile Leu Pro Ser Val Glu Leu Leu Phe Asn Leu
    130                 135                 140

Asp Arg Ile Thr Thr Val Glu His Leu Leu Lys Ser Val Leu Leu Tyr
145                 150                 155                 160

Asn Ile Asn Asn Ser Val Ser Phe Ser Ser Ala Val Lys Cys Val Cys
                165                 170                 175

Asn Leu Met Ile Lys Glu Pro Lys Ser Ser Ser Arg Thr Leu Gly Arg
            180                 185                 190

Ala Pro Tyr Ser Phe Thr Phe Asn Ser Gln Phe Glu Phe Gly Lys Lys
        195                 200                 205

His Lys Trp Ile Gln Ile Asp Val Thr Ser Leu Leu Gln Pro Leu Val
    210                 215                 220

Ala Ser Asn Lys Arg Ser Ile His Met Ser Ile Asn Phe Thr Cys Met
225                 230                 235                 240

Lys Asp Gln Leu Glu His Pro Ser Ala Gln Asn Gly Leu Phe Asn Met
                245                 250                 255

Thr Leu Val Ser Pro Ser Leu Ile Leu Tyr Leu Asn Asp Thr Ser Ala
            260                 265                 270

Gln Ala Tyr His Ser Trp Tyr Ser Leu His Tyr Lys Arg Arg Pro Ser
        275                 280                 285

Gln Gly Pro Asp Gln Glu Arg Ser Leu Ser Ala Tyr Pro Val Gly Glu
    290                 295                 300

Glu Ala Ala Glu Asp Gly Arg Ser Ser His His Arg His Arg Arg Gly
305                 310                 315                 320

Gln Glu Thr Val Ser Ser Glu Leu Lys Lys Pro Leu Gly Pro Ala Ser
                325                 330                 335

Phe Asn Leu Ser Glu Tyr Phe Arg Gln Phe Leu Leu Pro Gln Asn Glu
            340                 345                 350

Cys Glu Leu His Asp Phe Arg Leu Ser Phe Ser Gln Leu Lys Trp Asp
        355                 360                 365

Asn Trp Ile Val Ala Pro His Arg Tyr Asn Pro Arg Tyr Cys Lys Gly
    370                 375                 380

Asp Cys Pro Arg Ala Val Gly His Arg Tyr Gly Ser Pro Val His Thr
385                 390                 395                 400

Met Val Gln Asn Ile Ile Tyr Glu Lys Leu Asp Ser Ser Val Pro Arg
                405                 410                 415

Pro Ser Cys Val Pro Ala Lys Tyr Ser Pro Leu Ser Val Leu Thr Ile
            420                 425                 430

Glu Pro Asp Gly Ser Ile Ala Tyr Lys Glu Tyr Glu Asp Met Ile Ala

435                 440                 445

Thr Lys Cys Thr Cys Arg
    450

<210> 35
<211> 478
<212> PRT
<213> Homo sapiens

<400> 35
Met Ala His Val Pro Ala Arg Thr Ser Pro Gly Pro Gly Pro Gln Leu
1               5                   10                  15

Leu Leu Leu Leu Leu Pro Leu Phe Leu Leu Leu Leu Arg Asp Val Ala
            20                  25                  30

Gly Ser His Arg Ala Pro Ala Trp Ser Ala Leu Pro Ala Ala Ala Asp
        35                  40                  45

Gly Leu Gln Gly Asp Arg Asp Leu Gln Arg His Pro Gly Asp Ala Ala
    50                  55                  60

Ala Thr Leu Gly Pro Ser Ala Gln Asp Met Val Ala Val His Met His
65                  70                  75                  80

Arg Leu Tyr Glu Lys Tyr Ser Arg Gln Gly Ala Arg Pro Gly Gly Gly
                85                  90                  95

Asn Thr Val Arg Ser Phe Arg Ala Arg Leu Glu Val Val Asp Gln Lys
            100                 105                 110

Ala Val Tyr Phe Phe Asn Leu Thr Ser Met Gln Asp Ser Glu Met Ile
        115                 120                 125

Leu Thr Ala Thr Phe His Phe Tyr Ser Glu Pro Pro Arg Trp Pro Arg
    130                 135                 140

Ala Leu Glu Val Leu Cys Lys Pro Arg Ala Lys Asn Ala Ser Gly Arg
145                 150                 155                 160

Pro Leu Pro Leu Gly Pro Pro Thr Arg Gln His Leu Leu Phe Arg Ser
                165                 170                 175

Leu Ser Gln Asn Thr Ala Thr Gln Gly Leu Leu Arg Gly Ala Met Ala
            180                 185                 190

Leu Ala Pro Pro Pro Arg Gly Leu Trp Gln Ala Lys Asp Ile Ser Pro
        195                 200                 205

Ile Val Lys Ala Ala Arg Arg Asp Gly Glu Leu Leu Leu Ser Ala Gln
    210                 215                 220

Leu Asp Ser Glu Glu Arg Asp Pro Gly Val Pro Arg Pro Ser Pro Tyr
225                 230                 235                 240

Ala Pro Tyr Ile Leu Val Tyr Ala Asn Asp Leu Ala Ile Ser Glu Pro
                245                 250                 255

Asn Ser Val Ala Val Thr Leu Gln Arg Tyr Asp Pro Phe Pro Ala Gly
            260                 265                 270

Asp Pro Glu Pro Arg Ala Ala Pro Asn Asn Ser Ala Asp Pro Arg Val
        275                 280                 285

Arg Arg Ala Ala Gln Ala Thr Gly Pro Leu Gln Asp Asn Glu Leu Pro
    290                 295                 300

Gly Leu Asp Glu Arg Pro Pro Arg Ala His Ala Gln His Phe His Lys
305                 310                 315                 320

His Gln Leu Trp Pro Ser Pro Phe Arg Ala Leu Lys Pro Arg Pro Gly
                325                 330                 335

Arg Lys Asp Arg Arg Lys Lys Gly Gln Glu Val Phe Met Ala Ala Ser
            340                 345                 350

Gln Val Leu Asp Phe Asp Glu Lys Thr Met Gln Lys Ala Arg Arg Lys
        355                 360                 365

Gln Trp Asp Glu Pro Arg Val Cys Ser Arg Arg Tyr Leu Lys Val Asp
    370                 375                 380

Phe Ala Asp Ile Gly Trp Asn Glu Trp Ile Ile Ser Pro Lys Ser Phe
385                 390                 395                 400

Asp Ala Tyr Tyr Cys Ala Gly Ala Cys Glu Phe Pro Met Pro Lys Ile
                405                 410                 415

Val Arg Pro Ser Asn His Ala Thr Ile Gln Ser Ile Val Arg Ala Val
            420                 425                 430

Gly Ile Ile Pro Gly Ile Pro Glu Pro Cys Cys Val Pro Asp Lys Met
        435                 440                 445

Asn Ser Leu Gly Val Leu Phe Leu Asp Glu Asn Arg Asn Val Val Leu
    450                 455                 460

Lys Val Tyr Pro Asn Met Ser Val Asp Thr Cys Ala Cys Arg
465                 470                 475


<210> 36
<211> 407
<212> PRT
<213> Homo sapiens

<400> 36
Met Val Leu Ala Ala Pro Leu Leu Leu Gly Phe Leu Leu Leu Ala Leu
1               5                   10                  15

Glu Leu Arg Pro Arg Gly Glu Ala Ala Glu Gly Pro Ala Ala Ala Ala
            20                  25                  30

Ala Ala Ala Ala Ala Ala Ala Ala Ala Gly Val Gly Gly Glu Arg Ser
        35                  40                  45

Ser Arg Pro Ala Pro Ser Val Ala Pro Glu Pro Asp Gly Cys Pro Val
    50                  55                  60

Cys Val Trp Arg Gln His Ser Arg Glu Leu Arg Leu Glu Ser Ile Lys
65                  70                  75                  80

Ser Gln Ile Leu Ser Lys Leu Arg Leu Lys Glu Ala Pro Asn Ile Ser
                85                  90                  95

Arg Glu Val Val Lys Gln Leu Leu Pro Lys Ala Pro Pro Leu Gln Gln
            100                 105                 110

Ile Leu Asp Leu His Asp Phe Gln Gly Asp Ala Leu Gln Pro Glu Asp
        115                 120                 125

Phe Leu Glu Glu Asp Glu Tyr His Ala Thr Thr Glu Thr Val Ile Ser
    130                 135                 140

Met Ala Gln Glu Thr Asp Pro Ala Val Gln Thr Asp Gly Ser Pro Leu
145                 150                 155                 160

Cys Cys His Phe His Phe Ser Pro Lys Val Met Phe Thr Lys Val Leu
                165                 170                 175

Lys Ala Gln Leu Trp Val Tyr Leu Arg Pro Val Pro Arg Pro Ala Thr
            180                 185                 190

Val Tyr Leu Gln Ile Leu Arg Leu Lys Pro Leu Thr Gly Glu Gly Thr
        195                 200                 205

Ala Gly Gly Gly Gly Gly Gly Arg Arg His Ile Arg Ile Arg Ser Leu
    210                 215                 220

Lys Ile Glu Leu His Ser Arg Ser Gly His Trp Gln Ser Ile Asp Phe
225                 230                 235                 240

Lys Gln Val Leu His Ser Trp Phe Arg Gln Pro Gln Ser Asn Trp Gly
                245                 250                 255

Ile Glu Ile Asn Ala Phe Asp Pro Ser Gly Thr Asp Leu Ala Val Thr
            260                 265                 270

Ser Leu Gly Pro Gly Ala Glu Gly Leu His Pro Phe Met Glu Leu Arg
        275                 280                 285

Val Leu Glu Asn Thr Lys Arg Ser Arg Arg Asn Leu Gly Leu Asp Cys
    290                 295                 300

Asp Glu His Ser Ser Glu Ser Arg Cys Cys Arg Tyr Pro Leu Thr Val
305                 310                 315                 320

Asp Phe Glu Ala Phe Gly Trp Asp Trp Ile Ile Ala Pro Lys Arg Tyr
                325                 330                 335

Lys Ala Asn Tyr Cys Ser Gly Gln Cys Glu Tyr Met Phe Met Gln Lys
            340                 345                 350

Tyr Pro His Thr His Leu Val Gln Gln Ala Asn Pro Arg Gly Ser Ala
        355                 360                 365

Gly Pro Cys Cys Thr Pro Thr Lys Met Ser Pro Ile Asn Met Leu Tyr
    370                 375                 380

Phe Asn Asp Lys Gln Gln Ile Ile Tyr Gly Lys Ile Pro Gly Met Val
385                 390                 395                 400

Val Asp Arg Cys Gly Cys Ser
                405


<210> 37
<211> 350
<212> PRT
<213> Homo sapiens

<400> 37
Met Arg Leu Pro Asp Val Gln Leu Trp Leu Val Leu Leu Trp Ala Leu

1               5                   10                  15

Val Arg Ala Gln Gly Thr Gly Ser Val Cys Pro Ser Cys Gly Gly Ser
                20                  25                  30

Lys Leu Ala Pro Gln Ala Glu Arg Ala Leu Val Leu Glu Leu Ala Lys
            35                  40                  45

Gln Gln Ile Leu Asp Gly Leu His Leu Thr Ser Arg Pro Arg Ile Thr
    50                  55                  60

His Pro Pro Pro Gln Ala Ala Leu Thr Arg Ala Leu Arg Arg Leu Gln
65                  70                  75                  80

Pro Gly Ser Val Ala Pro Gly Asn Gly Glu Glu Val Ile Ser Phe Ala
                85                  90                  95

Thr Val Thr Asp Ser Thr Ser Ala Tyr Ser Ser Leu Leu Thr Phe His
            100                 105                 110

Leu Ser Thr Pro Arg Ser His His Leu Tyr His Ala Arg Leu Trp Leu
        115                 120                 125

His Val Leu Pro Thr Leu Pro Gly Thr Leu Cys Leu Arg Ile Phe Arg
    130                 135                 140

Trp Gly Pro Arg Arg Arg Arg Gln Gly Ser Arg Thr Leu Leu Ala Glu
145                 150                 155                 160

His His Ile Thr Asn Leu Gly Trp His Thr Leu Thr Leu Pro Ser Ser
                165                 170                 175

Gly Leu Arg Gly Glu Lys Ser Gly Val Leu Lys Leu Gln Leu Asp Cys
            180                 185                 190

Arg Pro Leu Glu Gly Asn Ser Thr Val Thr Gly Gln Pro Arg Arg Leu
        195                 200                 205

Leu Asp Thr Ala Gly His Gln Gln Pro Phe Leu Glu Leu Lys Ile Arg
    210                 215                 220

Ala Asn Glu Pro Gly Ala Gly Arg Ala Arg Arg Arg Thr Pro Thr Cys
225                 230                 235                 240

Glu Pro Ala Thr Pro Leu Cys Cys Arg Arg Asp His Tyr Val Asp Phe
                245                 250                 255

Gln Glu Leu Gly Trp Arg Asp Trp Ile Leu Gln Pro Glu Gly Tyr Gln
            260                 265                 270

Leu Asn Tyr Cys Ser Gly Gln Cys Pro Pro His Leu Ala Gly Ser Pro
        275                 280                 285

Gly Ile Ala Ala Ser Phe His Ser Ala Val Phe Ser Leu Leu Lys Ala
    290                 295                 300

Asn Asn Pro Trp Pro Ala Ser Thr Ser Cys Cys Val Pro Thr Ala Arg
305                 310                 315                 320

Arg Pro Leu Ser Leu Leu Tyr Leu Asp His Asn Gly Asn Val Val Lys
                325                 330                 335

Thr Asp Val Pro Asp Met Val Val Glu Ala Cys Gly Cys Ser
            340                 345                 350

<210> 38
<211> 97
<212> PRT
<213> Artificial Sequence

<220>
<223> Description of Artificial Sequence: Synthetic
      construct

<220>
<221> MOD_RES
<222> (2)..(2)
<223> Tyr or Lys

<220>
<221> MOD_RES
<222> (3)..(3)
<223> Val or Ile

<220>
<221> MOD_RES
<222> (4)..(4)
<223> Ser, Asp or Glu

<220>
<221> MOD_RES
<222> (6)..(6)
<223> Arg, Gln, Ser, Lys or Ala

<220>
<221> MOD_RES
<222> (7)..(7)
<223> Asp or Glu

<220>
<221> MOD_RES
<222> (8)..(8)
<223> Leu, Val or Ile

<220>
<221> MOD_RES
<222> (11)..(11)
<223> Gln, Leu, Asp, His, Asn or Ser

<220>
<221> MOD_RES
<222> (12)..(12)
<223> Asp, Arg, Asn or Glu

<220>
<221> MOD_RES
<222> (13)..(13)
<223> Trp or Ser

<220>
<221> MOD_RES
<222> (14)..(14)
<223> Ile or Val

<220>
<221> MOD_RES
<222> (15)..(15)
<223> Ile or Val

<220>
<221> MOD_RES
<222> (16)..(16)
<223> Ala or Ser

<220>
<221> MOD_RES
<222> (18)..(18)
<223> Glu, Gln, Leu, Lys, Pro or Arg

<220>
<221> MOD_RES
<222> (19)..(19)
<223> Gly or Ser

<220>
<221> MOD_RES
<222> (20)..(20)
<223> Tyr or Phe

<220>
<221> MOD_RES
<222> (21)..(21)
<223> Ala, Ser, Asp, Met, His, Gln, Leu or Gly

<220>
<221> MOD_RES
<222> (23)..(23)
<223> Tyr, Asn or Phe

<220>
<221> MOD_RES
<222> (26)..(26)
<223> Glu, His, Tyr, Asp, Gln, Ala or Ser

<220>
<221> MOD_RES
<222> (28)..(28)
<223> Glu, Lys, Asp, Gln or Ala

<220>
<221> MOD_RES
<222> (30)..(30)
<223> Ala, Ser, Pro, Gln, Ile or Asn

<220>
<221> MOD_RES
<222> (31)..(31)
<223> Phe, Leu or Tyr

<220>
<221> MOD_RES
<222> (33)..(33)
<223> Leu, Val or Met

<220>
<221> MOD_RES
<222> (34)..(34)
<223> Asn, Asp, Ala, Thr or Pro

<220>
<221> MOD_RES
<222> (35)..(35)
<223> Ser, Asp, Glu, Leu, Ala or Lys

<220>
<221> MOD_RES
<222> (36)..(36)
<223> Tyr, Cys, His, Ser or Ile

<220>
<221> MOD_RES
<222> (37)..(37)
<223> Met, Phe, Gly or Leu

<220>
<221> MOD_RES
<222> (38)..(38)
<223> Asn, Ser or Lys

<220>
<221> MOD_RES
<222> (39)..(39)
<223> Ala, Ser, Gly or Pro

<220>
<221> MOD_RES
<222> (40)..(40)
<223> Thr, Leu or Ser

<220>
<221> MOD_RES
<222> (44)..(44)
<223> Ile, Val or Thr

<220>
<221> MOD_RES
<222> (45)..(45)
<223> Val, Leu, Met or Ile

<220>
<221> MOD_RES
<222> (46)..(46)
<223> Gln or Arg

<220>
<221> MOD_RES
<222> (47)..(47)
<223> Thr, Ala or Ser

<220>
<221> MOD_RES
<222> (48)..(48)
<223> Leu or Ile

<220>
<221> MOD_RES
<222> (49)..(49)
<223> Val or Met

<220>
<221> MOD_RES
<222> (50)..(50)
<223> His, Asn or Arg

<220>
<221> MOD_RES
<222> (51)..(51)
<223> Phe, Leu, Asn, Ser, Ala or Val

<220>
<221> MOD_RES
<222> (52)..(52)
<223> Ile, Met, Asn, Ala, Val, Gly or Leu

<220>
<221> MOD_RES
<222> (53)..(53)
<223> Asn, Lys, Ala, Glu, Gly or Phe

<220>
<221> MOD_RES
<222> (54)..(54)
<223> Pro, Ser or Val

<220>
<221> MOD_RES
<222> (55)..(55)
<223> Glu, Asp, Asn, Gly, Val, Pro or Lys

<220>
<221> MOD_RES
<222> (56)..(56)
<223> Thr, Ala, Val, Lys, Asp, Tyr, Ser, Gly, Ile or His

<220>
<221> MOD_RES
<222> (57)..(57)
<223> Val, Ala or Ile

<220>
<221> MOD_RES
<222> (58)..(58)
<223> Pro or Asp

<220>
<221> MOD_RES
<222> (59)..(59)
<223> Lys, Leu or Glu

<220>
<221> MOD_RES
<222> (60)..(60)
<223> Pro, Val or Ala

<220>
<221> MOD_RES
<222> (63)..(63)
<223> Ala or Val

<220>
<221> MOD_RES
<222> (65)..(65)
<223> Thr, Ala or Glu

<220>
<221> MOD_RES
<222> (66)..(66)
<223> Gln, Lys, Arg or Glu

<220>
<221> MOD_RES
<222> (67)..(67)
<223> Leu, Met or Val

<220>
<221> MOD_RES
<222> (68)..(68)
<223> Asn, Ser, Asp or Gly

<220>
<221> MOD_RES
<222> (69)..(69)
<223> Ala, Pro or Ser

<220>
<221> MOD_RES
<222> (70)..(70)
<223> Ile, Thr, Val or Leu

<220>
<221> MOD_RES
<222> (71)..(71)
<223> Ser, Ala or Pro

<220>
<221> MOD_RES
<222> (72)..(72)
<223> Val, Leu, Met or Ile

<220>
<221> MOD_RES
<222> (74)..(74)
<223> Tyr or Phe

<220>
<221> MOD_RES
<222> (75)..(75)
<223> Phe, Tyr, Leu or His

<220>
<221> MOD_RES
<222> (76)..(76)
<223> Asp, Asn or Leu

<220>
<221> MOD_RES
<222> (77)..(77)
<223> Asp, Glu, Asn, Arg or Ser

<220>
<221> MOD_RES
<222> (78)..(78)
<223> Ser, Gln, Asn, Tyr or Asp

<220>
<221> MOD_RES
<222> (79)..(79)
<223> Ser, Asn, Asp, Glu or Lys

<220>
<221> MOD_RES
<222> (80)..(80)
<223> Asn, Thr or Lys

<220>
<221> MOD_RES
<222> (82)..(82)
<223> Ile, Val or Asn

<220>
<221> MOD_RES
<222> (84)..(84)
<223> Lys or Arg

<220>
<221> MOD_RES
<222> (85)..(85)
<223> Lys, Asn, Gln, His, Arg or Val

<220>
<221> MOD_RES
<222> (86)..(86)
<223> Tyr, Glu or His

<220>
<221> MOD_RES
<222> (87)..(87)
<223> Arg, Gln, Glu or Pro

<220>
<221> MOD_RES
<222> (88)..(88)
<223> Asn, Glu, Trp or Asp

<220>
<221> MOD_RES
<222> (90)..(90)
<223> Val, Thr, Ala or Ile

<220>
<221> MOD_RES
<222> (92)..(92)
<223> Arg, Lys, Val, Asp, Gln or Glu

<220>
<221> MOD_RES
<222> (93)..(93)
<223> Ala, Gly, Glu or Ser

<220>
<221> MOD_RES
<222> (95)..(95)
<223> Gly or Ala

<220>
<221> MOD_RES
<222> (97)..(97)
<223> His or Arg

<400> 38
Leu Xaa Xaa Xaa Phe Xaa Xaa Xaa Gly Trp Xaa Xaa Xaa Xaa Xaa Xaa
1               5                   10                  15

Pro Xaa Xaa Xaa Xaa Ala Xaa Tyr Cys Xaa Gly Xaa Cys Xaa Xaa Pro
            20                  25                  30

Xaa Xaa Xaa Xaa Xaa Xaa Xaa Xaa Asn His Ala Xaa Xaa Xaa Xaa Xaa
        35                  40                  45

Xaa Xaa Xaa Xaa Xaa Xaa Xaa Xaa Xaa Xaa Xaa Xaa Cys Cys Xaa Pro
    50                  55                  60

Xaa Xaa Xaa Xaa Xaa Xaa Xaa Xaa Leu Xaa Xaa Xaa Xaa Xaa Xaa Xaa
65                  70                  75                  80

Val Xaa Leu Xaa Xaa Xaa Xaa Xaa Met Xaa Val Xaa Xaa Cys Xaa Cys
                85                  90                  95

Xaa

<210> 39
<211> 102
<212> PRT
<213> Artificial Sequence

<220>
<223> Description of Artificial Sequence: Synthetic
      construct

<220>
<221> MOD_RES
<222> (2)..(2)
<223> Lys, Arg, Ala or Gln

<220>
<221> MOD_RES
<222> (3)..(3)
<223> Lys, Arg or Met

<220>
<221> MOD_RES
<222> (4)..(4)
<223> His, Arg or Gln

<220>
<221> MOD_RES
<222> (5)..(5)
<223> Glu, Ser, His, Gly, Arg, Pro, Thr or Tyr

<220>
<221> MOD_RES
<222> (7)..(7)
<223> Tyr or Lys

<220>
<221> MOD_RES
<222> (8)..(8)
<223> Val or Ile

<220>
<221> MOD_RES
<222> (9)..(9)
<223> Ser, Asp or Glu

<220>
<221> MOD_RES
<222> (11)..(11)
<223> Arg, Gln, Ser, Lys or Ala

<220>
<221> MOD_RES
<222> (12)..(12)
<223> Asp or Glu

<220>
<221> MOD_RES
<222> (13)..(13)

<223> Leu, Val or Ile

<220>
<221> MOD_RES
<222> (16)..(16)
<223> Gln, Leu, Asp, His, Asn or Ser

<220>
<221> MOD_RES
<222> (17)..(17)
<223> Asp, Arg, Asn or Glu

<220>
<221> MOD_RES
<222> (18)..(18)
<223> Trp or Ser

<220>
<221> MOD_RES
<222> (19)..(19)
<223> Ile or Val

<220>
<221> MOD_RES
<222> (20)..(20)
<223> Ile or Val

<220>
<221> MOD_RES
<222> (21)..(21)
<223> Ala or Ser

<220>
<221> MOD_RES
<222> (23)..(23)
<223> Glu, Gln, Leu, Lys, Pro or Arg

<220>
<221> MOD_RES
<222> (24)..(24)
<223> Gly or Ser

<220>
<221> MOD_RES
<222> (25)..(25)
<223> Tyr or Phe

<220>
<221> MOD_RES
<222> (26)..(26)
<223> Ala, Ser, Asp, Met, His, Gln, Leu or Gly

<220>
<221> MOD_RES
<222> (28)..(28)
<223> Tyr, Asn or Phe

<220>
<221> MOD_RES
<222> (31)..(31)
<223> Glu, His, Tyr, Asp, Gln, Ala or Ser

<220>
<221> MOD_RES
<222> (33)..(33)

<223> Glu, Lys, Asp, Gln or Ala

<220>
<221> MOD_RES
<222> (35)..(35)
<223> Ala, Ser, Pro, Gln, Ile or Asn

<220>
<221> MOD_RES
<222> (36)..(36)
<223> Phe, Leu or Tyr

<220>
<221> MOD_RES
<222> (38)..(38)
<223> Leu, Val or Met

<220>
<221> MOD_RES
<222> (39)..(39)
<223> Asn, Asp, Ala, Thr or Pro

<220>
<221> MOD_RES
<222> (40)..(40)
<223> Ser, Asp, Glu, Leu, Ala or Lys

<220>
<221> MOD_RES
<222> (41)..(41)
<223> Tyr, Cys, His, Ser or Ile

<220>
<221> MOD_RES
<222> (42)..(42)
<223> Met, Phe, Gly or Leu

<220>
<221> MOD_RES
<222> (43)..(43)
<223> Asn, Ser or Lys

<220>
<221> MOD_RES
<222> (44)..(44)
<223> Ala, Ser, Gly or Pro

<220>
<221> MOD_RES
<222> (45)..(45)
<223> Thr, Leu or Ser

<220>
<221> MOD_RES
<222> (49)..(49)
<223> Ile, Val or Thr

<220>
<221> MOD_RES
<222> (50)..(50)
<223> Val, Leu, Met or Ile

<220>
<221> MOD_RES
<222> (51)..(51)

<223> Gln or Arg

<220>
<221> MOD_RES
<222> (52)..(52)
<223> Thr, Ala or Ser

<220>
<221> MOD_RES
<222> (53)..(53)
<223> Leu or Ile

<220>
<221> MOD_RES
<222> (54)..(54)
<223> Val or Met

<220>
<221> MOD_RES
<222> (55)..(55)
<223> His, Asn or Arg

<220>
<221> MOD_RES
<222> (56)..(56)
<223> Phe, Leu, Asn, Ser, Ala or Val

<220>
<221> MOD_RES
<222> (57)..(57)
<223> Ile, Met, Asn, Ala, Val, Gly or Leu

<220>
<221> MOD_RES
<222> (58)..(58)
<223> Asn, Lys, Ala, Glu, Gly or Phe

<220>
<221> MOD_RES
<222> (59)..(59)
<223> Pro, Ser or Val

<220>
<221> MOD_RES
<222> (60)..(60)
<223> Glu, Asp, Asn, Gly, Val, Pro or Lys

<220>
<221> MOD_RES
<222> (61)..(61)
<223> Thr, Ala, Val, Lys, Asp, Tyr, Ser, Gly, Ile or His

<220>
<221> MOD_RES
<222> (62)..(62)
<223> Val, Ala or Ile

<220>
<221> MOD_RES
<222> (63)..(63)
<223> Pro or Asp

<220>
<221> MOD_RES
<222> (64)..(64)

<223> Lys, Leu or Glu

<220>
<221> MOD_RES
<222> (65)..(65)
<223> Pro, Val or Ala

<220>
<221> MOD_RES
<222> (68)..(68)
<223> Ala or Val

<220>
<221> MOD_RES
<222> (70)..(70)
<223> Thr, Ala or Glu

<220>
<221> MOD_RES
<222> (71)..(71)
<223> Gln, Lys, Arg or Glu

<220>
<221> MOD_RES
<222> (72)..(72)
<223> Leu, Met or Val

<220>
<221> MOD_RES
<222> (73)..(73)
<223> Asn, Ser, Asp or Gly

<220>
<221> MOD_RES
<222> (74)..(74)
<223> Ala, Pro or Ser

<220>
<221> MOD_RES
<222> (75)..(75)
<223> Ile, Thr, Val or Leu

<220>
<221> MOD_RES
<222> (76)..(76)
<223> Ser, Ala or Pro

<220>
<221> MOD_RES
<222> (77)..(77)
<223> Val, Leu, Met or Ile

<220>
<221> MOD_RES
<222> (79)..(79)
<223> Tyr or Phe

<220>
<221> MOD_RES
<222> (80)..(80)
<223> Phe, Tyr, Leu or His

<220>
<221> MOD_RES
<222> (81)..(81)

<223> Asp, Asn or Leu

<220>
<221> MOD_RES
<222> (82)..(82)
<223> Asp, Glu, Asn, Arg or Ser

<220>
<221> MOD_RES
<222> (83)..(83)
<223> Ser, Gln, Asn, Tyr or Asp

<220>
<221> MOD_RES
<222> (84)..(84)
<223> Ser, Asn, Asp, Glu or Lys

<220>
<221> MOD_RES
<222> (85)..(85)
<223> Asn, Thr or Lys

<220>
<221> MOD_RES
<222> (87)..(87)
<223> Ile, Val or Asn

<220>
<221> MOD_RES
<222> (89)..(89)
<223> Lys or Arg

<220>
<221> MOD_RES
<222> (90)..(90)
<223> Lys, Asn, Gln, His, Arg or Val

<220>
<221> MOD_RES
<222> (91)..(91)
<223> Tyr, Glu or His

<220>
<221> MOD_RES
<222> (92)..(92)
<223> Arg, Gln, Glu or Pro

<220>
<221> MOD_RES
<222> (93)..(93)
<223> Asn, Glu, Trp or Asp

<220>
<221> MOD_RES
<222> (95)..(95)
<223> Val, Thr, Ala or Ile

<220>
<221> MOD_RES
<222> (97)..(97)
<223> Arg, Lys, Val, Asp, Gln or Glu

<220>
<221> MOD_RES
<222> (98)..(98)

<223> Ala, Gly, Glu or Ser

<220>
<221> MOD_RES
<222> (100)..(100)
<223> Gly or Ala

<220>
<221> MOD_RES
<222> (102)..(102)
<223> His or Arg

<400> 39
Cys Xaa Xaa Xaa Xaa Leu Xaa Xaa Xaa Phe Xaa Xaa Xaa Gly Trp Xaa
1               5                   10                  15

Xaa Xaa Xaa Xaa Xaa Pro Xaa Xaa Xaa Xaa Ala Xaa Tyr Cys Xaa Gly
            20                  25                  30

Xaa Cys Xaa Xaa Pro Xaa Xaa Xaa Xaa Xaa Xaa Xaa Xaa Asn His Ala
        35                  40                  45

Xaa Xaa Xaa Xaa Xaa Xaa Xaa Xaa Xaa Xaa Xaa Xaa Xaa Xaa Xaa Xaa
    50                  55                  60

Xaa Cys Cys Xaa Pro Xaa Xaa Xaa Xaa Xaa Xaa Xaa Xaa Leu Xaa Xaa
65                  70                  75                  80

Xaa Xaa Xaa Xaa Xaa Val Xaa Leu Xaa Xaa Xaa Xaa Xaa Met Xaa Val
                85                  90                  95

Xaa Xaa Cys Xaa Cys Xaa
            100


<210> 40
<211> 5
<212> PRT
<213> Artificial Sequence

<220>
<223> Description of Artificial Sequence: Synthetic
      peptide

<220>
<221> MOD_RES
<222> (2)..(2)
<223> Lys, Arg, Ala or Gln

<220>
<221> MOD_RES
<222> (3)..(3)
<223> Lys, Arg or Met

<220>
<221> MOD_RES
<222> (4)..(4)
<223> His, Arg or Gln

<220>
<221> MOD_RES
<222> (5)..(5)
<223> Glu, Ser, His, Gly, Arg, Pro, Thr or Tyr

<400> 40

Cys Xaa Xaa Xaa Xaa
1               5

<210> 41
<211> 97
<212> PRT
<213> Artificial Sequence

<220>
<223> Description of Artificial Sequence: Synthetic
      construct

<220>
<221> MOD_RES
<222> (1)..(1)
<223> Phe, Leu or Glu

<220>
<221> MOD_RES
<222> (2)..(2)
<223> Tyr, Phe, His, Arg, Thr, Lys, Gln, Val or Glu

<220>
<221> MOD_RES
<222> (3)..(3)
<223> Val, Ile, Leu or Asp

<220>
<221> MOD_RES
<222> (4)..(4)
<223> Ser, Asp, Glu, Asn or Phe

<220>
<221> MOD_RES
<222> (5)..(5)
<223> Phe or Glu

<220>
<221> MOD_RES
<222> (6)..(6)
<223> Arg, Gln, Lys, Ser, Glu, Ala or Asn

<220>
<221> MOD_RES
<222> (7)..(7)
<223> Asp, Glu, Leu, Ala or Gln

<220>
<221> MOD_RES
<222> (8)..(8)
<223> Leu, Val, Met, Ile or Phe

<220>
<221> MOD_RES
<222> (9)..(9)
<223> Gly, His or Lys

<220>
<221> MOD_RES
<222> (10)..(10)
<223> Trp or Met

<220>
<221> MOD_RES

<222> (11)..(11)
<223> Gln, Leu, His, Glu, Asn, Asp, Ser or Gly

<220>
<221> MOD_RES
<222> (12)..(12)
<223> Asp, Asn, Ser, Lys, Arg, Glu or His

<220>
<221> MOD_RES
<222> (13)..(13)
<223> Trp or Ser

<220>
<221> MOD_RES
<222> (14)..(14)
<223> Ile or Val

<220>
<221> MOD_RES
<222> (15)..(15)
<223> Ile or Val

<220>
<221> MOD_RES
<222> (16)..(16)
<223> Ala, Ser, Tyr or Trp

<220>
<221> MOD_RES
<222> (18)..(18)
<223> Glu, Lys, Gln, Met, Pro, Leu, Arg, His or Lys

<220>
<221> MOD_RES
<222> (19)..(19)
<223> Gly, Glu, Asp, Lys, Ser, Gln, Arg or Phe

<220>
<221> MOD_RES
<222> (20)..(20)
<223> Tyr or Phe

<220>
<221> MOD_RES
<222> (21)..(21)
<223> Ala, Ser, Gly, Met, Gln, His, Glu, Asp, Leu, Asn, Lys
      or Thr

<220>
<221> MOD_RES
<222> (22)..(22)
<223> Ala or Pro

<220>
<221> MOD_RES
<222> (23)..(23)
<223> Tyr, Phe, Asn, Ala or Arg

<220>
<221> MOD_RES
<222> (24)..(24)
<223> Tyr, His, Glu, Phe or Arg

<220>

<221> MOD_RES
<222> (26)..(26)
<223> Glu, Asp, Ala, Ser, Tyr, His, Lys, Arg, Gln or Gly

<220>
<221> MOD_RES
<222> (28)..(28)
<223> Glu, Asp, Leu, Val, Lys, Gly, Thr, Ala or Gln

<220>
<221> MOD_RES
<222> (30)..(30)
<223> Ala, Ser, Ile, Asn, Pro, Glu, Asp, Phe, Gln or Leu

<220>
<221> MOD_RES
<222> (31)..(31)
<223> Phe, Tyr, Leu, Asn, Gly or Arg

<220>
<221> MOD_RES
<222> (32)..(32)
<223> Pro, Ser, Ala or Val

<220>
<221> MOD_RES
<222> (33)..(33)
<223> Leu, Met, Glu, Phe or Val

<220>
<221> MOD_RES
<222> (34)..(34)
<223> Asn, Asp, Thr, Gly, Ala, Arg, Leu or Pro

<220>
<221> MOD_RES
<222> (35)..(35)
<223> Ser, Ala, Glu, Asp, Thr, Leu, Lys, Gln or His

<220>
<221> MOD_RES
<222> (36)..(36)
<223> Tyr, His, Cys, Ile, Arg, Asp, Asn, Lys, Ser, Glu or Gly

<220>
<221> MOD_RES
<222> (37)..(37)
<223> Met, Leu, Phe, Val, Gly or Tyr

<220>
<221> MOD_RES
<222> (38)..(38)
<223> Asn, Glu, Thr, Pro, Lys, His, Gly, Met, Val or Arg

<220>
<221> MOD_RES
<222> (39)..(39)
<223> Ala, Ser, Gly, Pro or Phe

<220>
<221> MOD_RES
<222> (40)..(40)
<223> Thr, Ser, Leu, Pro, His or Met

<220>

<221> MOD_RES
<222> (41)..(41)
<223> Asn, Lys, Val, Thr or Gln

<220>
<221> MOD_RES
<222> (42)..(42)
<223> His, Tyr or Lys

<220>
<221> MOD_RES
<222> (43)..(43)
<223> Ala, Thr, Leu or Tyr

<220>
<221> MOD_RES
<222> (44)..(44)
<223> Ile, Thr, Val, Phe Tyr, Met or Pro

<220>
<221> MOD_RES
<222> (45)..(45)
<223> Val, Leu, Met, Ile or His

<220>
<221> MOD_RES
<222> (46)..(46)
<223> Gln, Arg or Thr

<220>
<221> MOD_RES
<222> (47)..(47)
<223> Thr, Ser, Ala, Asn or His

<220>
<221> MOD_RES
<222> (48)..(48)
<223> Leu, Asn or Ile

<220>
<221> MOD_RES
<222> (49)..(49)
<223> Val, Met, Leu, Pro or Ile

<220>
<221> MOD_RES
<222> (50)..(50)
<223> His, Asn, Arg, Lys, Tyr or Gln

<220>
<221> MOD_RES
<222> (51)..(51)
<223> Phe, Leu, Ser, Asn, Met, Ala, Arg, Glu, Gly or Gln

<220>
<221> MOD_RES
<222> (52)..(52)
<223> Ile, Met, Leu, Val, Lys, Gln, Ala or Tyr

<220>
<221> MOD_RES
<222> (53)..(53)
<223> Asn, Phe, Lys, Glu, Asp, Ala, Gln, Gly, Leu or Val

<220>

<221> MOD_RES
<222> (54)..(54)
<223> Pro, Asn, Ser, Val or Asp

<220>
<221> MOD_RES
<222> (55)..(55)
<223> Glu, Asp, Asn, Lys, Arg, Ser, Gly, Thr, Gln, Pro or His

<220>
<221> MOD_RES
<222> (56)..(56)
<223> Thr, His, Tyr, Ala, Ile, Lys, Asp, Ser, Gly or Arg

<220>
<221> MOD_RES
<222> (57)..(57)
<223> Val, Ile, Thr, Ala, Leu or Ser

<220>
<221> MOD_RES
<222> (58)..(58)
<223> Pro, Gly, Ser, Asp or Ala

<220>
<221> MOD_RES
<222> (59)..(59)
<223> Lys, Leu, Pro, Ala, Ser, Glu, Arg or Gly

<220>
<221> MOD_RES
<222> (60)..(60)
<223> Pro, Ala, Val, Thr or Ser

<220>
<221> MOD_RES
<222> (61)..(61)
<223> Cys, Val or Ser

<220>
<221> MOD_RES
<222> (63)..(63)
<223> Ala, Val or Thr

<220>
<221> MOD_RES
<222> (65)..(65)
<223> Thr, Ala, Glu, Val, Gly, Asp or Tyr

<220>
<221> MOD_RES
<222> (66)..(66)
<223> Gln, Lys, Glu, Arg or Val

<220>
<221> MOD_RES
<222> (67)..(67)
<223> Leu, Met, Thr or Tyr

<220>
<221> MOD_RES
<222> (68)..(68)
<223> Asn, Ser, Gly, Thr, Asp, Glu, Lys or Val

<220>

<221> MOD_RES
<222> (69)..(69)
<223> Ala, Pro, Gly or Ser

<220>
<221> MOD_RES
<222> (70)..(70)
<223> Ile, Thr, Leu or Val

<220>
<221> MOD_RES
<222> (71)..(71)
<223> Ser, Pro, Ala, Thr, Asn or Gly

<220>
<221> MOD_RES
<222> (72)..(72)
<223> Val, Ile, Leu or Met

<220>
<221> MOD_RES
<222> (74)..(74)
<223> Tyr, Phe, Arg, Thr, Tyr or Met

<220>
<221> MOD_RES
<222> (75)..(75)
<223> Phe, Tyr, His, Leu, Ile, Lys, Gln or Val

<220>
<221> MOD_RES
<222> (76)..(76)
<223> Asp, Leu, Asn or Glu

<220>
<221> MOD_RES
<222> (77)..(77)
<223> Asp, Ser, Arg, Asn, Glu, Ala, Lys, Gly or Pro

<220>
<221> MOD_RES
<222> (78)..(78)
<223> Ser, Asn, Glu, Asp, Val, Lys, Gly, Gln, Met, Glu, Asn
      or Lys

<220>
<221> MOD_RES
<222> (79)..(79)
<223> Ser, Asn, Glu, Asp, Val, Lys, Gly, Gln or Arg

<220>
<221> MOD_RES
<222> (80)..(80)
<223> Asn, Lys, Thr, Pro, Val, Ile, Arg, Ser or Gln

<220>
<221> MOD_RES
<222> (81)..(81)
<223> Val, Ile, Thr or Ala

<220>
<221> MOD_RES
<222> (82)..(82)
<223> Ile, Asn, Val, Leu, Tyr, Asp or Ala

<220>
<221> MOD_RES
<222> (83)..(83)
<223> Leu, Tyr, Lys or Ile

<220>
<221> MOD_RES
<222> (84)..(84)
<223> Lys, Arg, Asn, Tyr, Phe, Thr, Glu or Gly

<220>
<221> MOD_RES
<222> (85)..(85)
<223> Lys, Arg, His, Gln, Asn, Glu or Val

<220>
<221> MOD_RES
<222> (86)..(86)
<223> Tyr, His, Glu or Ile

<220>
<221> MOD_RES
<222> (87)..(87)
<223> Arg, Glu, Gln, Pro or Lys

<220>
<221> MOD_RES
<222> (88)..(88)
<223> Asn, Asp, Ala, Glu, Gly or Lys

<220>
<221> MOD_RES
<222> (89)..(89)
<223> Met or Ala

<220>
<221> MOD_RES
<222> (90)..(90)
<223> Val, Ile, Ala, Thr, Ser or Lys

<220>
<221> MOD_RES
<222> (91)..(91)
<223> Val or Ala

<220>
<221> MOD_RES
<222> (92)..(92)
<223> Arg, Lys, Gln, Asp, Glu, Val, Ala, Ser or Thr

<220>
<221> MOD_RES
<222> (93)..(93)
<223> Ala, Ser, Glu, Gly, Arg or Thr

<220>
<221> MOD_RES
<222> (95)..(95)
<223> Gly, Ala or Thr

<220>
<221> MOD_RES
<222> (97)..(97)
<223> His, Arg, Gly, Leu or Ser

<400> 41
Xaa Xaa Xaa Xaa Xaa Xaa Xaa Xaa Xaa Xaa Xaa Xaa Xaa Xaa Xaa Xaa
1               5                   10                  15

Pro Xaa Xaa Xaa Xaa Xaa Xaa Xaa Cys Xaa Gly Xaa Cys Xaa Xaa Xaa
            20                  25                  30

Xaa Xaa Xaa Xaa Xaa Xaa Xaa Xaa Xaa Xaa Xaa Xaa Xaa Xaa Xaa Xaa
        35                  40                  45

Xaa Xaa Xaa Xaa Xaa Xaa Xaa Xaa Xaa Xaa Xaa Xaa Xaa Cys Xaa Pro
    50                  55                  60

Xaa Xaa Xaa Xaa Xaa Xaa Xaa Xaa Leu Xaa Xaa Xaa Xaa Xaa Xaa Xaa
65                  70                  75                  80

Xaa Xaa Xaa Xaa Xaa Xaa Xaa Xaa Xaa Xaa Xaa Xaa Xaa Cys Xaa Cys
                85                  90                  95

Xaa

<210> 42
<211> 102
<212> PRT
<213> Artificial Sequence

<220>
<223> Description of Artificial Sequence: Synthetic
      construct

<220>
<221> MOD_RES
<222> (2)..(2)
<223> Lys, Arg, Gln, Ser, His, Glu, Ala or Cys

<220>
<221> MOD_RES
<222> (3)..(3)
<223> Lys, Arg, Met, Lys, Thr, Leu, Tyr or Ala

<220>
<221> MOD_RES
<222> (4)..(4)
<223> His, Gln, Arg, Lys, Thr, Leu, Val, Pro or Tyr

<220>
<221> MOD_RES
<222> (5)..(5)
<223> Gln, Thr, His, Arg, Pro, Ser, ala, Gln, Asn, Tyr, Lys,
      Asp or Leu

<220>
<221> MOD_RES
<222> (6)..(6)
<223> Phe, Leu or Glu

<220>
<221> MOD_RES
<222> (7)..(7)
<223> Tyr, Phe, His, Arg, Thr, Lys, Gln, Val or Glu

<220>

<221> MOD_RES
<222> (8)..(8)
<223> Val, Ile, Leu or Asp

<220>
<221> MOD_RES
<222> (9)..(9)
<223> Ser, Asp, Glu, Asn or Phe

<220>
<221> MOD_RES
<222> (10)..(10)
<223> Phe or Glu

<220>
<221> MOD_RES
<222> (11)..(11)
<223> Arg, Gln, Lys, Ser, Glu, Ala or Asn

<220>
<221> MOD_RES
<222> (12)..(12)
<223> Asp, Glu, Leu, Ala or Gln

<220>
<221> MOD_RES
<222> (13)..(13)
<223> Leu, Val, Met, Ile or Phe

<220>
<221> MOD_RES
<222> (14)..(14)
<223> Gly, His or Lys

<220>
<221> MOD_RES
<222> (15)..(15)
<223> Trp or Met

<220>
<221> MOD_RES
<222> (16)..(16)
<223> Gln, Leu, His, Glu, Asn, Asp, Ser or Gly

<220>
<221> MOD_RES
<222> (17)..(17)
<223> Asp, Asn, Ser, Lys, Arg, Glu or His

<220>
<221> MOD_RES
<222> (18)..(18)
<223> Trp or Ser

<220>
<221> MOD_RES
<222> (19)..(19)
<223> Ile or Val

<220>
<221> MOD_RES
<222> (20)..(20)
<223> Ile or Val

<220>

<221> MOD_RES
<222> (21)..(21)
<223> Ala, Ser, Tyr or Trp

<220>
<221> MOD_RES
<222> (23)..(23)
<223> Glu, Lys, Gln, Met, Pro, Leu, Arg, His or Lys

<220>
<221> MOD_RES
<222> (24)..(24)
<223> Gly, Glu, Asp, Lys, Ser, Gln, Arg or Phe

<220>
<221> MOD_RES
<222> (25)..(25)
<223> Tyr or Phe

<220>
<221> MOD_RES
<222> (26)..(26)
<223> Ala, Ser, Gly, Met, Gln, His, Glu, Asp, Leu, Asn, Lys
      or Thr

<220>
<221> MOD_RES
<222> (27)..(27)
<223> Ala or Pro

<220>
<221> MOD_RES
<222> (28)..(28)
<223> Tyr, Phe, Asn, Ala or Arg

<220>
<221> MOD_RES
<222> (29)..(29)
<223> Tyr, His, Glu, Phe or Arg

<220>
<221> MOD_RES
<222> (31)..(31)
<223> Glu, Asp, Ala, Ser, Tyr, His, Lys, Arg, Gln or Gly

<220>
<221> MOD_RES
<222> (33)..(33)
<223> Glu, Asp, Leu, Val, Lys, Gly, Thr, Ala or Gln

<220>
<221> MOD_RES
<222> (35)..(35)
<223> Ala, Ser, Ile, Asn, Pro, Glu, Asp, Phe, Gln or Leu

<220>
<221> MOD_RES
<222> (36)..(36)
<223> Phe, Tyr, Leu, Asn, Gly or Arg

<220>
<221> MOD_RES
<222> (37)..(37)
<223> Pro, Ser, Ala or Val

<220>
<221> MOD_RES
<222> (38)..(38)
<223> Leu, Met, Glu, Phe or Val

<220>
<221> MOD_RES
<222> (39)..(39)
<223> Asn, Asp, Thr, Gly, Ala, Arg, Leu or Pro

<220>
<221> MOD_RES
<222> (40)..(40)
<223> Ser, Ala, Glu, Asp, Thr, Leu, Lys, Gln or His

<220>
<221> MOD_RES
<222> (41)..(41)
<223> Tyr, His, Cys, Ile, Arg, Asp, Asn, Lys, Ser, Glu or Gly

<220>
<221> MOD_RES
<222> (42)..(42)
<223> Met, Leu, Phe, Val, Gly or Tyr

<220>
<221> MOD_RES
<222> (43)..(43)
<223> Asn, Glu, Thr, Pro, Lys, His, Gly, Met, Val or Arg

<220>
<221> MOD_RES
<222> (44)..(44)
<223> Ala, Ser, Gly, Pro or Phe

<220>
<221> MOD_RES
<222> (45)..(45)
<223> Thr, Ser, Leu, Pro, His or Met

<220>
<221> MOD_RES
<222> (46)..(46)
<223> Asn, Lys, Val, Thr or Gln

<220>
<221> MOD_RES
<222> (47)..(47)
<223> His, Tyr or Lys

<220>
<221> MOD_RES
<222> (48)..(48)
<223> Ala, Thr, Leu or Tyr

<220>
<221> MOD_RES
<222> (49)..(49)
<223> Ile, Thr, Val, Phe Tyr, Met or Pro

<220>
<221> MOD_RES
<222> (50)..(50)
<223> Val, Leu, Met, Ile or His

<220>
<221> MOD_RES
<222> (51)..(51)
<223> Gln, Arg or Thr

<220>
<221> MOD_RES
<222> (52)..(52)
<223> Thr, Ser, Ala, Asn or His

<220>
<221> MOD_RES
<222> (53)..(53)
<223> Leu, Asn or Ile

<220>
<221> MOD_RES
<222> (54)..(54)
<223> Val, Met, Leu, Pro or Ile

<220>
<221> MOD_RES
<222> (55)..(55)
<223> His, Asn, Arg, Lys, Tyr or Gln

<220>
<221> MOD_RES
<222> (56)..(56)
<223> Phe, Leu, Ser, Asn, Met, Ala, Arg, Glu, Gly or Gln

<220>
<221> MOD_RES
<222> (57)..(57)
<223> Ile, Met, Leu, Val, Lys, Gln, Ala or Tyr

<220>
<221> MOD_RES
<222> (58)..(58)
<223> Asn, Phe, Lys, Glu, Asp, Ala, Gln, Gly, Leu or Val

<220>
<221> MOD_RES
<222> (59)..(59)
<223> Pro, Asn, Ser, Val or Asp

<220>
<221> MOD_RES
<222> (60)..(60)
<223> Glu, Asp, Asn, Lys, Arg, Ser, Gly, Thr, Gln, Pro or His

<220>
<221> MOD_RES
<222> (61)..(61)
<223> Thr, His, Tyr, Ala, Ile, Lys, Asp, Ser, Gly or Arg

<220>
<221> MOD_RES
<222> (62)..(62)
<223> Val, Ile, Thr, Ala, Leu or Ser

<220>
<221> MOD_RES
<222> (63)..(63)
<223> Pro, Gly, Ser, Asp or Ala

<220>
<221> MOD_RES
<222> (64)..(64)
<223> Lys, Leu, Pro, Ala, Ser, Glu, Arg or Gly

<220>
<221> MOD_RES
<222> (65)..(65)
<223> Pro, Ala, Val, Thr or Ser

<220>
<221> MOD_RES
<222> (66)..(66)
<223> Cys, Val or Ser

<220>
<221> MOD_RES
<222> (68)..(68)
<223> Ala, Val or Thr

<220>
<221> MOD_RES
<222> (70)..(70)
<223> Thr, Ala, Glu, Val, Gly, Asp or Tyr

<220>
<221> MOD_RES
<222> (71)..(71)
<223> Gln, Lys, Glu, Arg or Val

<220>
<221> MOD_RES
<222> (72)..(72)
<223> Leu, Met, Thr or Tyr

<220>
<221> MOD_RES
<222> (73)..(73)
<223> Asn, Ser, Gly, Thr, Asp, Glu, Lys or Val

<220>
<221> MOD_RES
<222> (74)..(74)
<223> Ala, Pro, Gly or Ser

<220>
<221> MOD_RES
<222> (75)..(75)
<223> Ile, Thr, Leu or Val

<220>
<221> MOD_RES
<222> (76)..(76)
<223> Ser, Pro, Ala, Thr, Asn or Gly

<220>
<221> MOD_RES
<222> (77)..(77)
<223> Val, Ile, Leu or Met

<220>
<221> MOD_RES
<222> (79)..(79)
<223> Tyr, Phe, Arg, Thr, Tyr or Met

<220>
<221> MOD_RES
<222> (80)..(80)
<223> Phe, Tyr, His, Leu, Ile, Lys, Gln or Val

<220>
<221> MOD_RES
<222> (81)..(81)
<223> Asp, Leu, Asn or Glu

<220>
<221> MOD_RES
<222> (82)..(82)
<223> Asp, Ser, Arg, Asn, Glu, Ala, Lys, Gly or Pro

<220>
<221> MOD_RES
<222> (83)..(83)
<223> Ser, Asn, Glu, Asp, Val, Lys, Gly, Gln, Met, Glu, Asn
      or Lys

<220>
<221> MOD_RES
<222> (84)..(84)
<223> Ser, Asn, Glu, Asp, Val, Lys, Gly, Gln or Arg

<220>
<221> MOD_RES
<222> (85)..(85)
<223> Asn, Lys, Thr, Pro, Val, Ile, Arg, Ser or Gln

<220>
<221> MOD_RES
<222> (86)..(86)
<223> Val, Ile, Thr or Ala

<220>
<221> MOD_RES
<222> (87)..(87)
<223> Ile, Asn, Val, Leu, Tyr, Asp or Ala

<220>
<221> MOD_RES
<222> (88)..(88)
<223> Leu, Tyr, Lys or Ile

<220>
<221> MOD_RES
<222> (89)..(89)
<223> Lys, Arg, Asn, Tyr, Phe, Thr, Glu or Gly

<220>
<221> MOD_RES
<222> (90)..(90)
<223> Lys, Arg, His, Gln, Asn, Glu or Val

<220>
<221> MOD_RES
<222> (91)..(91)
<223> Tyr, His, Glu or Ile

<220>
<221> MOD_RES
<222> (92)..(92)
<223> Arg, Glu, Gln, Pro or Lys

<220>
<221> MOD_RES
<222> (93)..(93)
<223> Asn, Asp, Ala, Glu, Gly or Lys

<220>
<221> MOD_RES
<222> (94)..(94)
<223> Met or Ala

<220>
<221> MOD_RES
<222> (95)..(95)
<223> Val, Ile, Ala, Thr, Ser or Lys

<220>
<221> MOD_RES
<222> (96)..(96)
<223> Val or Ala

<220>
<221> MOD_RES
<222> (97)..(97)
<223> Arg, Lys, Gln, Asp, Glu, Val, Ala, Ser or Thr

<220>
<221> MOD_RES
<222> (98)..(98)
<223> Ala, Ser, Glu, Gly, Arg or Thr

<220>
<221> MOD_RES
<222> (100)..(100)
<223> Gly, Ala or Thr

<220>
<221> MOD_RES
<222> (102)..(102)
<223> His, Arg, Gly, Leu or Ser

<400> 42
Cys Xaa Xaa Xaa Xaa Xaa Xaa Xaa Xaa Xaa Xaa Xaa Xaa Xaa Xaa Xaa
1               5                   10                  15

Xaa Xaa Xaa Xaa Xaa Pro Xaa Xaa Xaa Xaa Xaa Xaa Xaa Cys Xaa Gly
            20                  25                  30

Xaa Cys Xaa Xaa Xaa Xaa Xaa Xaa Xaa Xaa Xaa Xaa Xaa Xaa Xaa Xaa
        35                  40                  45

Xaa Xaa Xaa Xaa Xaa Xaa Xaa Xaa Xaa Xaa Xaa Xaa Xaa Xaa Xaa Xaa
    50                  55                  60

Xaa Xaa Cys Xaa Pro Xaa Xaa Xaa Xaa Xaa Xaa Xaa Xaa Leu Xaa Xaa
65                  70                  75                  80

Xaa Xaa Xaa Xaa Xaa Xaa Xaa Xaa Xaa Xaa Xaa Xaa Xaa Xaa Xaa Xaa
                85                  90                  95

Xaa Xaa Cys Xaa Cys Xaa
            100


<210> 43

<211> 96
<212> PRT
<213> Artificial Sequence

<220>
<223> Description of Artificial Sequence: Synthetic
      peptide

<400> 43
Leu Tyr Val Asp Phe Ser Asp Val Gly Trp Asp Asp Trp Ile Val Ala
1               5                   10                  15

Pro Pro Gly Tyr Gln Ala Phe Tyr Cys His Gly Glu Cys Pro Phe Pro
            20                  25                  30

Leu Ala Asp His Phe Asn Ser Thr Asn His Ala Val Val Gln Thr Leu
        35                  40                  45

Val Asn Ser Val Asn Ser Lys Ile Pro Lys Ala Cys Cys Val Pro Thr
    50                  55                  60

Glu Leu Ser Ala Ile Ser Met Leu Tyr Leu Asp Glu Asn Glu Lys Val
65                  70                  75                  80

Val Leu Lys Tyr Asn Gln Glu Met Val Val Glu Gly Cys Gly Cys Arg
                85                  90                  95

<210> 44
<211> 96
<212> PRT
<213> Artificial Sequence

<220>
<223> Description of Artificial Sequence: Synthetic
      peptide

<400> 44
Leu Tyr Val Asp Phe Ser Asp Val Gly Trp Asn Asp Trp Ile Val Ala
1               5                   10                  15

Pro Pro Gly Tyr His Ala Phe Tyr Cys His Gly Glu Cys Pro Phe Pro
            20                  25                  30

Leu Ala Asp His Leu Asn Ser Thr Asn His Ala Val Val Gln Thr Leu
        35                  40                  45

Val Asn Ser Val Asn Ser Lys Ile Pro Lys Ala Cys Cys Val Pro Thr
    50                  55                  60

Glu Leu Ser Ala Ile Ser Met Leu Tyr Leu Asp Glu Asn Glu Lys Val
65                  70                  75                  80

Val Leu Lys Tyr Asn Gln Glu Met Val Val Glu Gly Cys Gly Cys Arg
                85                  90                  95

<210> 45
<211> 102

<212> PRT
<213> Artificial Sequence

<220>
<223> Description of Artificial Sequence: Synthetic
      construct

<220>
<221> MOD_RES
<222> (2)..(2)
<223> Lys or Arg

<220>
<221> MOD_RES
<222> (3)..(3)
<223> Lys or Arg

<220>
<221> MOD_RES
<222> (11)..(11)
<223> Arg or Gln

<220>
<221> MOD_RES
<222> (16)..(16)
<223> Gln or Leu

<220>
<221> MOD_RES
<222> (19)..(19)
<223> Ile or Val

<220>
<221> MOD_RES
<222> (23)..(23)
<223> Glu or Gln

<220>
<221> MOD_RES
<222> (26)..(26)
<223> Ala or Ser

<220>
<221> MOD_RES
<222> (35)..(35)
<223> Ala or Ser

<220>
<221> MOD_RES
<222> (39)..(39)
<223> Asn or Asp

<220>
<221> MOD_RES
<222> (41)..(41)
<223> Tyr or Cys

<220>
<221> MOD_RES
<222> (50)..(50)
<223> Val or Leu

<220>
<221> MOD_RES
<222> (52)..(52)

<223> Ser or Thr

<220>
<221> MOD_RES
<222> (56)..(56)
<223> Phe or Leu

<220>
<221> MOD_RES
<222> (57)..(57)
<223> Ile or Met

<220>
<221> MOD_RES
<222> (58)..(58)
<223> Asn or Lys

<220>
<221> MOD_RES
<222> (60)..(60)
<223> Glu, Asp or Asn

<220>
<221> MOD_RES
<222> (61)..(61)
<223> Thr, Ala or Val

<220>
<221> MOD_RES
<222> (65)..(65)
<223> Pro or Ala

<220>
<221> MOD_RES
<222> (71)..(71)
<223> Gln or Lys

<220>
<221> MOD_RES
<222> (73)..(73)
<223> Asn or Ser

<220>
<221> MOD_RES
<222> (75)..(75)
<223> Ile or Thr

<220>
<221> MOD_RES
<222> (80)..(80)
<223> Phe or Tyr

<220>
<221> MOD_RES
<222> (82)..(82)
<223> Asp or Ser

<220>
<221> MOD_RES
<222> (84)..(84)
<223> Ser or Asn

<220>
<221> MOD_RES
<222> (89)..(89)

<223> Lys or Arg

<220>
<221> MOD_RES
<222> (91)..(91)
<223> Tyr or His

<220>
<221> MOD_RES
<222> (97)..(97)
<223> Arg or Lys

<400> 45
Cys Xaa Xaa His Glu Leu Tyr Val Ser Phe Xaa Asp Leu Gly Trp Xaa
1               5                   10                  15

Asp Trp Xaa Ile Ala Pro Xaa Gly Tyr Xaa Ala Tyr Tyr Cys Glu Gly
            20                  25                  30

Glu Cys Xaa Phe Pro Leu Xaa Ser Xaa Met Asn Ala Thr Asn His Ala
        35                  40                  45

Ile Xaa Gln Xaa Leu Val His Xaa Xaa Xaa Pro Xaa Xaa Val Pro Lys
    50                  55                  60

Xaa Cys Cys Ala Pro Thr Xaa Leu Xaa Ala Xaa Ser Val Leu Tyr Xaa
65                  70                  75                  80

Asp Xaa Ser Xaa Asn Val Ile Leu Xaa Lys Xaa Arg Asn Met Val Val
                85                  90                  95

Xaa Ala Cys Gly Cys His
            100


<210> 46
<211> 323
<212> PRT
<213> Homo sapiens

<400> 46
Met His Val Arg Ser Leu Arg Ala Ala Ala Pro His Ser Phe Val Ala
1               5                   10                  15

Leu Trp Ala Pro Leu Phe Leu Leu Arg Ser Ala Leu Ala Asp Phe Ser
            20                  25                  30

Leu Asp Asn Glu Val His Ser Ser Phe Ile His Arg Arg Leu Arg Ser
        35                  40                  45

Gln Glu Arg Arg Glu Met Gln Arg Glu Ile Leu Ser Ile Leu Gly Leu
    50                  55                  60

Pro His Arg Pro Arg Pro His Leu Gln Gly Lys His Asn Ser Ala Pro
65                  70                  75                  80

Met Phe Met Leu Asp Leu Tyr Asn Ala Met Ala Val Glu Glu Gly Gly
                85                  90                  95

Gly Pro Gly Gly Gln Gly Phe Ser Tyr Pro Tyr Lys Ala Val Phe Ser
            100                 105                 110

Thr Gln Gly Pro Pro Leu Ala Ser Leu Gln Asp Ser His Phe Leu Thr
        115                 120                 125

Asp Ala Asp Met Val Met Ser Phe Val Asn Leu Val Glu His Asp Lys
    130                 135                 140

Glu Phe Phe His Pro Arg Tyr His His Arg Glu Phe Arg Phe Asp Leu
145                 150                 155                 160

Ser Lys Ile Pro Glu Gly Glu Ala Val Thr Ala Ala Glu Phe Arg Ile
                165                 170                 175

Tyr Lys Asp Tyr Ile Arg Glu Arg Phe Asp Asn Glu Thr Phe Arg Ile
            180                 185                 190

Ser Val Tyr Gln Val Leu Gln Glu His Leu Gly Arg Glu Ser Asp Leu
        195                 200                 205

Phe Leu Leu Asp Ser Arg Thr Leu Trp Ala Ser Glu Glu Gly Trp Leu
    210                 215                 220

Val Phe Asp Ile Thr Ala Thr Ser Asn His Trp Val Val Asn Pro Arg
225                 230                 235                 240

His Asn Leu Gly Leu Gln Leu Ser Val Glu Thr Leu Asp Gly Gln Ser
                245                 250                 255

Ile Asn Pro Lys Leu Ala Gly Leu Ile Gly Arg His Gly Pro Gln Asn
            260                 265                 270

Lys Gln Pro Phe Met Val Ala Phe Phe Lys Ala Thr Glu Val His Phe
        275                 280                 285

Arg Ser Ile Arg Ser Thr Gly Ser Lys Gln Arg Ser Gln Asn Arg Ser
    290                 295                 300

Lys Thr Pro Lys Asn Gln Glu Ala Leu Arg Met Ala Asn Val Ala Glu
305                 310                 315                 320

Asn Ser Ser

<210> 47
<211> 63
<212> PRT
<213> Homo sapiens

<400> 47
Arg Ala Pro Arg Ser Gln Gln Pro Phe Val Val Thr Phe Phe Arg Ala
1               5                   10                  15

Ser Pro Ser Pro Ile Arg Thr Pro Arg Ala Val Arg Pro Leu Arg Arg
            20                  25                  30

Arg Gln Pro Lys Lys Ser Asn Glu Leu Pro Gln Ala Asn Arg Leu Pro
        35                  40                  45

Gly Ile Phe Asp Asp Val His Gly Ser His Gly Arg Gln Val Cys
    50                  55                  60

<210> 48
<211> 42
<212> PRT
<213> Homo sapiens

<400> 48
Arg Ser Ile Arg Ser Thr Gly Ser Lys Gln Arg Ser Gln Asn Arg Ser
1               5                   10                  15

Lys Thr Pro Lys Asn Gln Glu Ala Leu Arg Met Ala Asn Val Ala Glu
            20                  25                  30

Asn Ser Ser Ser Asp Gln Arg Gln Ala Cys
        35                  40


<210> 49
<211> 42
<212> PRT
<213> Mus musculus

<400> 49
Arg Thr Thr Arg Ser Ala Ser Ser Arg Arg Arg Gln Gln Ser Arg Asn
1               5                   10                  15

Arg Ser Thr Gln Ser Gln Asp Val Ser Arg Gly Ser Gly Ser Ser Asp
            20                  25                  30

Tyr Asn Gly Ser Glu Leu Lys Thr Ala Cys
        35                  40


<210> 50
<211> 41
<212> PRT
<213> Homo sapiens

<400> 50
Arg Ser Val Arg Ala Ala Asn Lys Arg Lys Asn Gln Asn Arg Asn Lys
1               5                   10                  15

Ser Ser Ser His Gln Asp Ser Ser Arg Met Ser Ser Val Gly Asp Tyr
            20                  25                  30

Asn Thr Ser Glu Gln Lys Gln Ala Cys
        35                  40


<210> 51
<211> 33
<212> PRT
<213> Drosophila sp.

<400> 51
Arg Ser Lys Arg Ser Ala Ser His Pro Arg Lys Arg Lys Lys Ser Val
1               5                   10                  15

Ser Pro Asn Asn Val Pro Leu Leu Glu Pro Met Glu Ser Thr Arg Ser
            20                  25                  30

Cys


<210> 52
<211> 35
<212> PRT
<213> Drosophila sp.

<400> 52

Arg Ser Ile Arg Asp Val Ser Gly Gly Glu Gly Gly Gly Lys Gly Gly
1               5                   10                  15

Arg Asn Lys Arg His Ala Arg Arg Pro Thr Arg Arg Lys Asn His Asp
            20                  25                  30

Asp Thr Cys
        35

<210> 53
<211> 55
<212> PRT
<213> Homo sapiens

<400> 53
Arg His Val Arg Ile Ser Arg Ser Leu His Gln Asp Glu His Ser Trp
1               5                   10                  15

Ser Gln Ile Arg Pro Leu Leu Val Thr Phe Gly His Asp Gly Lys Gly
            20                  25                  30

His Pro Leu His Lys Arg Glu Lys Arg Gln Ala Lys His Lys Gln Arg
        35                  40                  45

Lys Arg Leu Lys Ser Ser Cys
    50                  55

<210> 54
<211> 56
<212> PRT
<213> Homo sapiens

<400> 54
Arg Ile Ser Arg Ser Leu Pro Gln Gly Ser Gly Asn Trp Ala Gln Leu
1               5                   10                  15

Arg Pro Leu Leu Val Thr Phe Gly His Asp Gly Arg Gly His Ala Leu
            20                  25                  30

Thr Arg Arg Arg Arg Ala Lys Arg Ser Pro Lys His His Ser Gln Arg
        35                  40                  45

Ala Arg Lys Lys Asn Lys Asn Cys
    50                  55

<210> 55
<211> 22
<212> PRT
<213> Xenopus sp.

<400> 55
Arg Cys Lys Arg Pro Arg Arg Lys Arg Ser Tyr Ser Lys Leu Pro Phe
1               5                   10                  15

Thr Ala Ser Asn Ile Cys
            20

<210> 56
<211> 13
<212> PRT
<213> Homo sapiens

<400> 56
Arg Lys Lys Arg Ser Thr Gly Val Leu Leu Pro Leu Gln
1               5                   10

<210> 57
<211> 40
<212> PRT
<213> Homo sapiens

<400> 57
Lys Ser Lys Asn Lys Lys Lys Gln Arg Lys Gly Pro His Arg Lys Ser
1               5                   10                  15

Gln Thr Leu Gln Phe Asp Glu Gln Thr Leu Lys Lys Ala Arg Arg Lys
            20                  25                  30

Gln Trp Ile Glu Pro Arg Asn Cys
        35                  40

<210> 58
<211> 4
<212> PRT
<213> Artificial Sequence

<220>
<223> Description of Artificial Sequence: Synthetic
      peptide

<400> 58
Gly Gly Pro Pro
1